# EUROPEAN PATENT SPECIFICATION

(11) **EP 2 914 634 B1**
(45) Date of publication and mention of the grant of the patent: **06.12.2017**
(21) Application number: 13851307.2
(22) Date of filing: 31.10.2013
(51) Int. Cl.: C07K 16/46, A61K 39/395, G06F 19/16, C07K 16/28

(54) **CRYSTAL STRUCTURES OF HETERODIMERIC FC DOMAINS**
KRISTALLSTRUKTUREN AUS HETERODIMEREN FC-DOMÄNEN
STRUCTURES CRISTALLINES DE DOMAINES FC HÉTÉRODIMÈRES

(30) Priority: 02.11.2012 US 201213668098; 17.04.2013 US 201361813084 P
(43) Date of publication of application: 09.09.2015
(73) Proprietor: Zymeworks Inc., Vancouver, British Colombia V6H 3V9 (CA)
(72) Inventor: SPRETER VON KREUDENSTEIN, Thomas, Vancouver British Columbia V6E 1S8 (CA); DIXIT, Surjit Bhimarao, Richmond British Columbia V7E 3N5 (CA); LARIO, Paula Irene, Vancouver British Columbia V5P 4X2 (CA); ESCOBAR-CABRERA, Eric, Burnaby British Columbia V5A 4B3 (CA); BOULANGER, Martin J., Victoria British Columbia V8P 5S1 (CA); SUITS, Michael D.L., Waterloo Ontario N2L 2J6 (CA)
(74) Representative: Grund, Martin
(86) International application number: PCT/CA2013/050832
(87) International publication number: WO 2014/067011

(56) References cited:
- WO-A1-2010/068722
- WO-A1-2012/058768
- WO-A1-2013/063702
- WO-A1-2013/166604
- WO-A1-2014/004586
- WO-A2-2014/012085
- WO-A2-2014/018572

## Description

### TECHNICAL FIELD

The present disclosure relates to Fc heterodimer proteins in crystalline form, a cystallizable composition comprising such Fc heterodimer proteins, and methods for identifying mutations which promote heterodimeric Fc chain pair formation.

### BACKGROUND

There is a drive in the pharmaceutical industry towards the development of bispecific therapeutics that can concurrently bind two or more distinct targets or epitopes in order to achieve novel mechanisms of action and efficacy. *See*, Beck et al., 2010, Nature Reviews, Immunology 10: 345-352; Carter, 2011, Experimental Cell Research 317: 1261-1269; Kontermann, 2012, mABs 4: 182-197; and Segal et al., 2001, Journal of Immunology Methods 248:1-6. In recent years, a number of bispecific formats based on either antibody or other protein domains have been designed with the goal of creating a modular molecular scaffold. *See*, Kontermann, 2012, mABs 4:182-197; and Klein et al., 2012, mABs 27:4(6). From this, it is clear that modular multi-domain, multi-functional monoclonal antibodies, with their intrinsic therapeutically relevant features combined with the experiences gained in the biopharmaceutical development of these molecules as therapeutics, makes this class of molecules an attractive molecular class for pharmaceutical development provided that such molecules do not substantially deviate from their native structural and functional characteristics.

Initial IgG-like bispecific antibody development centered on use of a hybrid hybridoma of two cells that produces two different antibodies of interest. *See*, Milstein and Cuello, 1983, Nature 305: 537-540. Co-expression of the four different antibody chains (two heavy and two light) in such a fused cell leads to the non-selective formation of up to ten different combinations of heavy and light chain pairs, from which the one correct bispecific molecule is recovered through laborious purification. Improving on this, some workers have used either natural or engineered differences in Protein A binding affinities of the two antibody heavy chains for selective isolation of the heterodimer from the homodimers. *See* Lindhofer et al., 1995, Journal of Immunology 155: 219-225; Igawa and Tsunoda, 2007, United States Patent Publication No. 2009/0263392 A1; Davis and Smith, 2010, "Readily Isolated Bispecific Antibodies with Native Immunoglobulin Format", United States Patent Publication No. 2010/00331527; and Klein et al., 2012, MAbs. 27:4(6). The bispecific antibody of interest that is obtained in any of these non-selective chain pairing expression strategies appears to be limited to a maximum of 12.5% of the total antibody yield in cases where both light-heavy and heavy-heavy chain pairing is essential or 50% if selective light-heavy chain pairing requirement is abrogated such as by using a common light chain. In either case this approach will significantly impact the cost of goods.

In order to overcome this impact and diminish the formation of unwanted Fc chain pairs, structure guided attempts to engineer mutations resulting in selective pairing of preferred heavy chains when co-expressed in a recombinant manner is desirable. Prominent among these rational design efforts is the knob-into-hole strategy, developed by Presta, Carter and coworkers, which employs steric point mutations in the CH3-CH3 interface to preferentially drive Fc heterodimerisation and prevent formation of homodimers. *See,* Ridgway and Presta, 1996, Protein Engineering 9: 617-621; Merchant et al. 1998, Nature Biotechnology 17: 677-681; and Atwell et al., 1997, Journal of Molecular Biology 270: 26-35. Such designs have yielded high heterodimer selectivity, but have caused about 11 °C lowering in thermal stability of the CH3 domain relative to the wild type. In contrast to this steric complementarity approach in the knob-into-hole designs, Gunasekaran and coworkers have recently employed electrostatic complementarity design strategy to achieve the selective heterodimerization goal. *See*, Gunasekaran et al., 2010, The Journal of Biological Chemistry 285: 19637-19646. Davis and coworkers have designed strand exchange engineered domain (SEED) CH3 which is comprised of alternating segments of human IgA and IgG CH3 sequences leading to preferentially associating heterodimers. *See* Davis et al., 2010, PEDS 23: 195-202. The engineered CH3 domains of both these approaches have melting temperatures of the CH3 domains of ∼68 °C.
WO 2012/058768 relates generally to heterodimeric constructs, but does not disclose their crystalline forms.

Alternately, an annealing based approach for producing bispecific antibodies by mixing two different antibodies has been pursued in other technologies. *See* Jackman et al., 2010, J. Biol. Chem 285: 20850-20859; and Strop et al., 2012, J. Mol. Biol. 420, 204-219. These rational engineering approaches favor heterodimer formation by destabilizing the natural homodimer interface and result in antibodies comprising less stable CH3 domains than the parent molecule. A protein with reduced stability of its native folded state is potentially prone to a number of aggregation related challenges in its handling and development. *See,* Wang, 2005, International Journal of Pharmaceutics 289: 1-30; and Demarest et al., 2008, Current Opinion in Drug Discovery and Development 11: 675-687. Further, the mutations in the IgG Fc region and the reduced stability of the CH3 domain could have an impact on immunogenicity and pharmacokinetic properties, which are important drug like properties that have to be validated for successful design of a modular bispecific scaffold. WO 2010/068722 A1 discloses one example of human IgG Fc variant crystals.

Given the above background, there is a need in the art for Fc heterodimer proteins in crystalline form, cystallizable compositions comprising such Fc heterodimer proteins, and methods for identifying mutations which promote heterodimeric Fc chain pair formation. Such articles and methods are needed in order to develop polypeptide constructs that comprise antigen-binding domains that are linked to an Fc heterodimer protein comprising CH3 domains which have been modified to select for heterodimers with favorable drug-like properties such as ease of manufacturing and analytical characterization; formulation and stability of the therapeutic at the requisite drug concentrations; and pharmacokinetic properties, immunogenicity and toxicity that are similar to Fc heterodimer proteins without a modified CH3 domain. An antibody platform that takes into consideration all of these aspects concurrently would significantly empower the drug developer in the design of best-in-class bi- and multi-specific therapeutic candidates.

### SUMMARY

The present invention is embodied in the claims. The disclosed embodiments address the needs presented in the prior art. Disclosed are the atomic coordinates of compositions comprising Fc heterodimer proteins in crystalline form derived from high resolution x-ray diffraction. Further disclosed are systems and methods for using all or a portion of these atomic coordinates to identify and design improved Fc heterodimer proteins. Further disclosed are compositions comprising a mixture of (i) a solubilized Fc heterodimer protein and (ii) a mother liquor solution. The mother liquor solution comprises between 2% and 10% (v/v) ethylene glycol, between 10% and 25% (w/v) polyethylene glycol having an average molecular weight of between 2000 Daltons and 10000 Daltons, and between 0.05 M and 0.40 M ammonium iodide. Further disclosed are systems and methods of identifying a mutation which promotes heterodimeric Fc chain pair formation in which structure based modeling is performed to identify a candidate mutation to an Fc chain using all or a portion of the disclosed three-dimensional atomic coordinates.

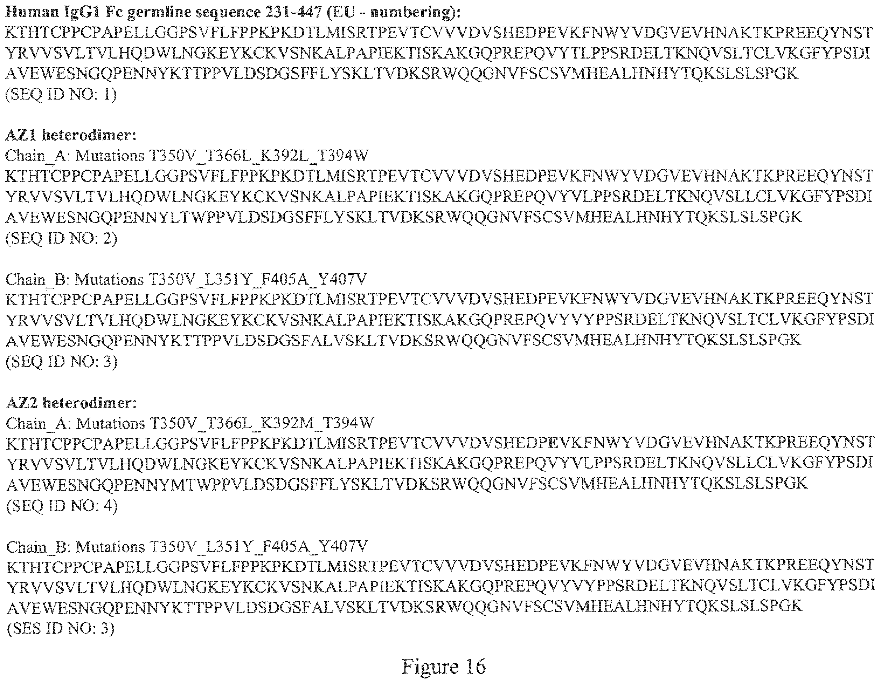

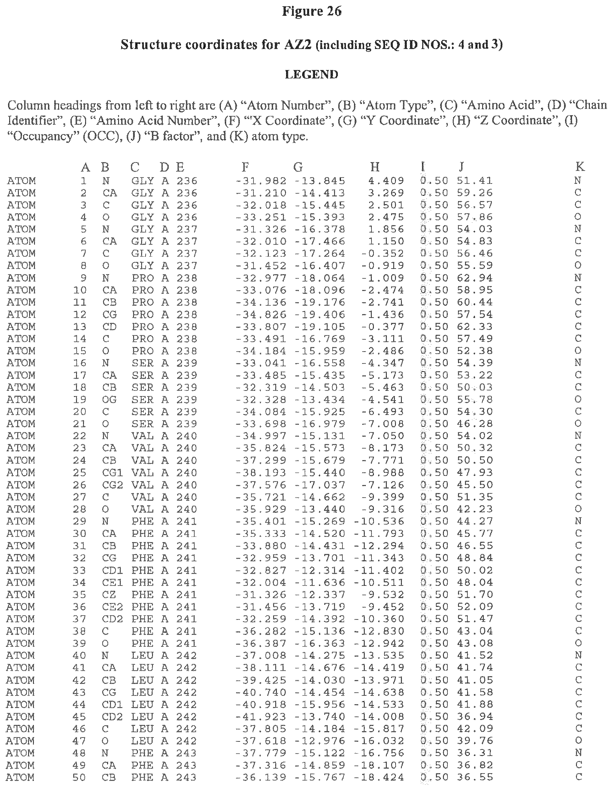

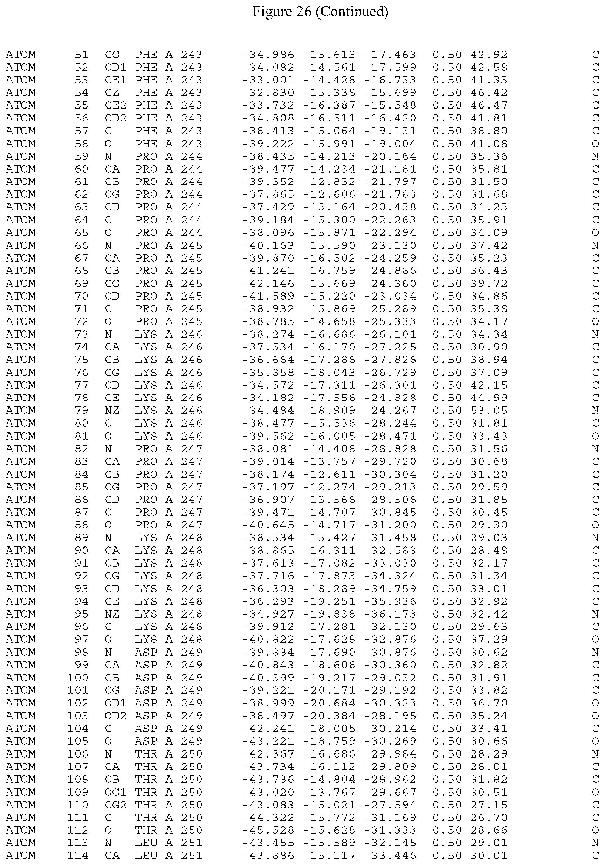

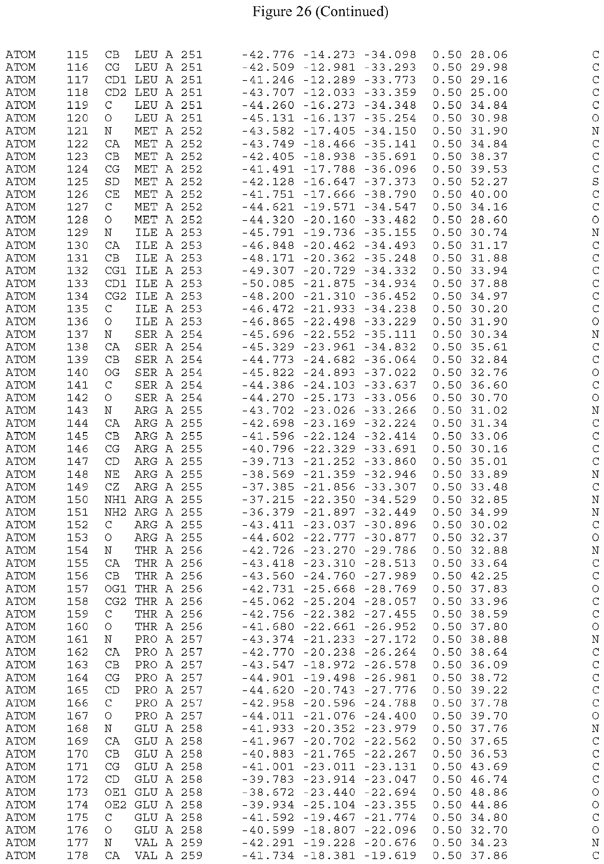

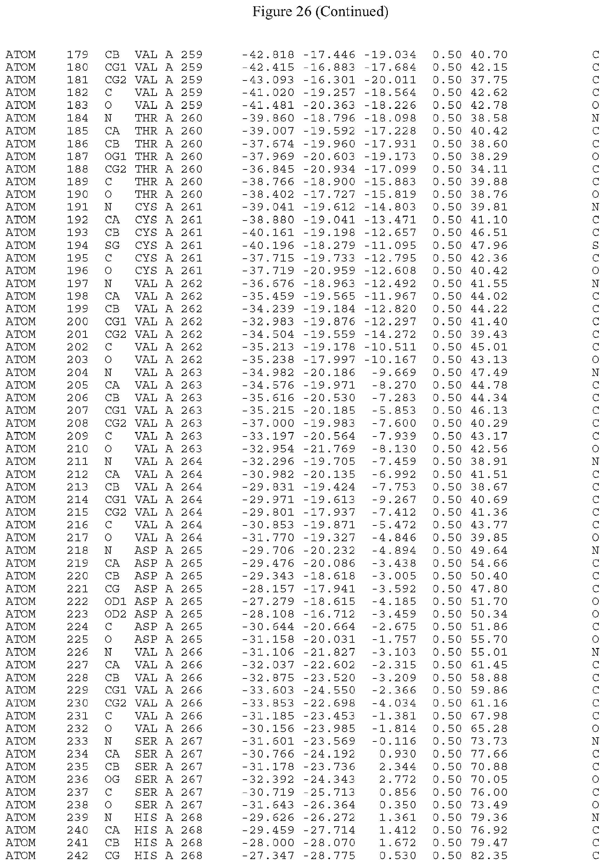

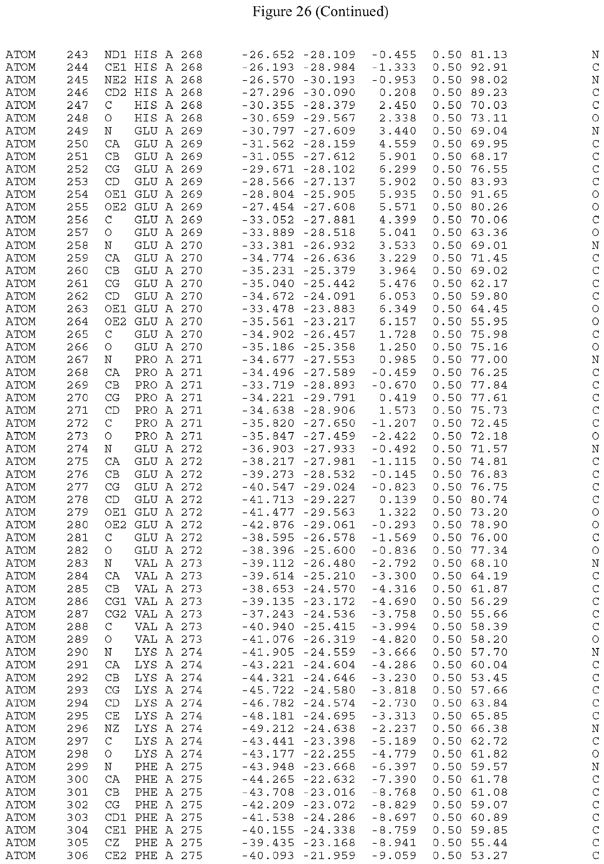

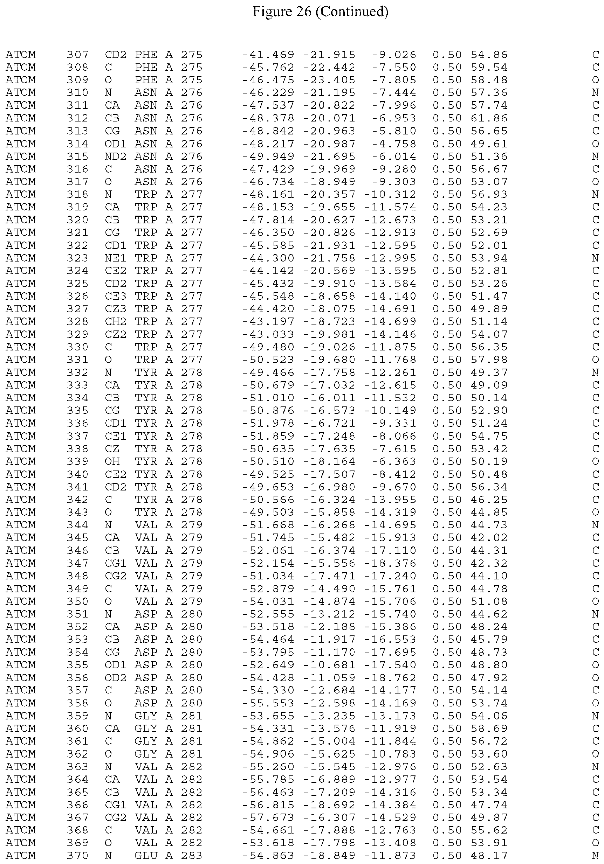

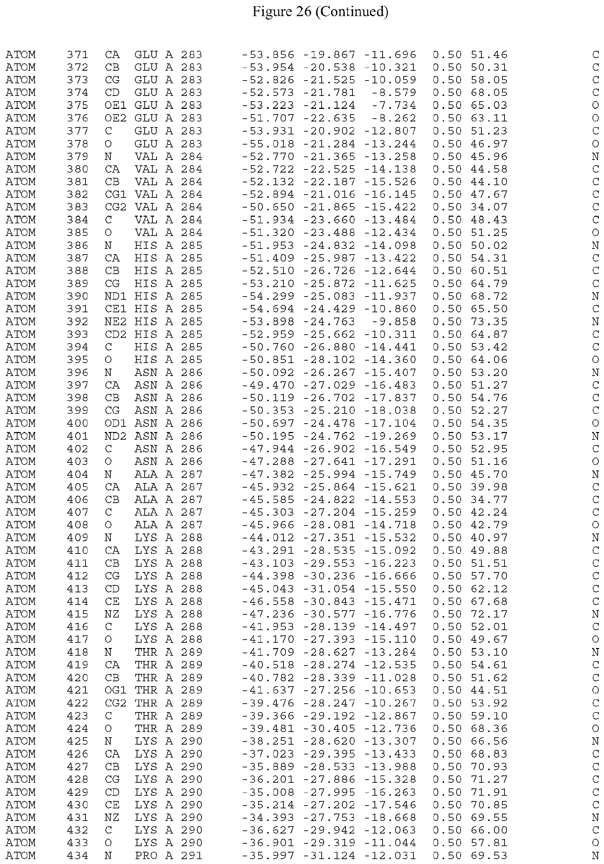

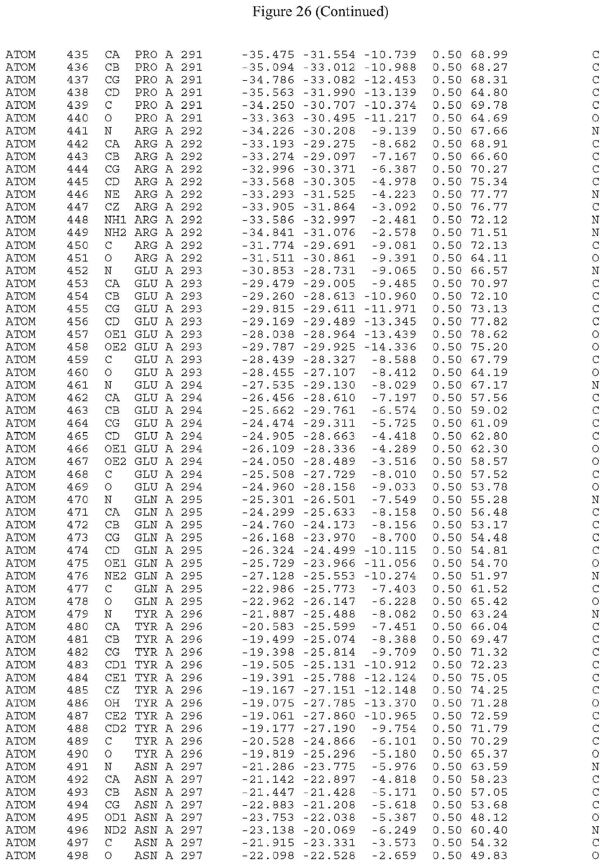

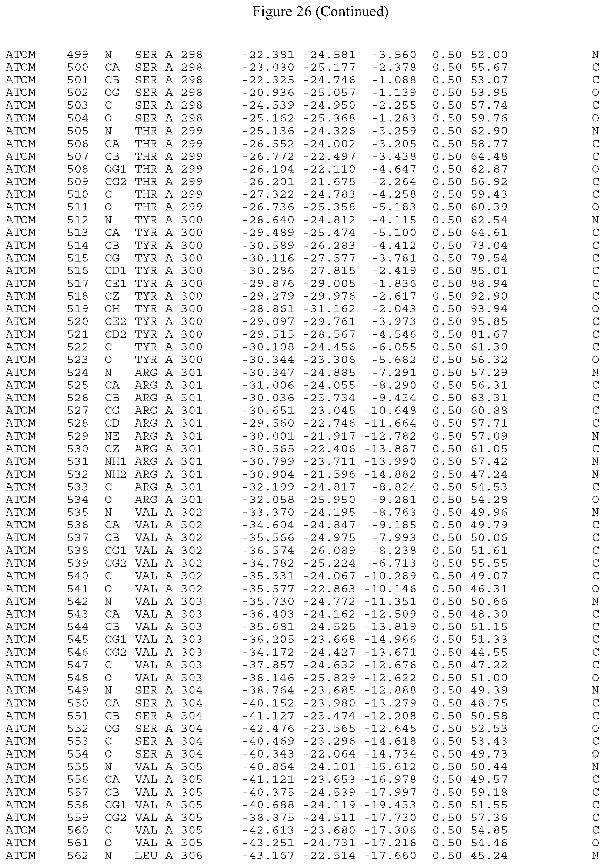

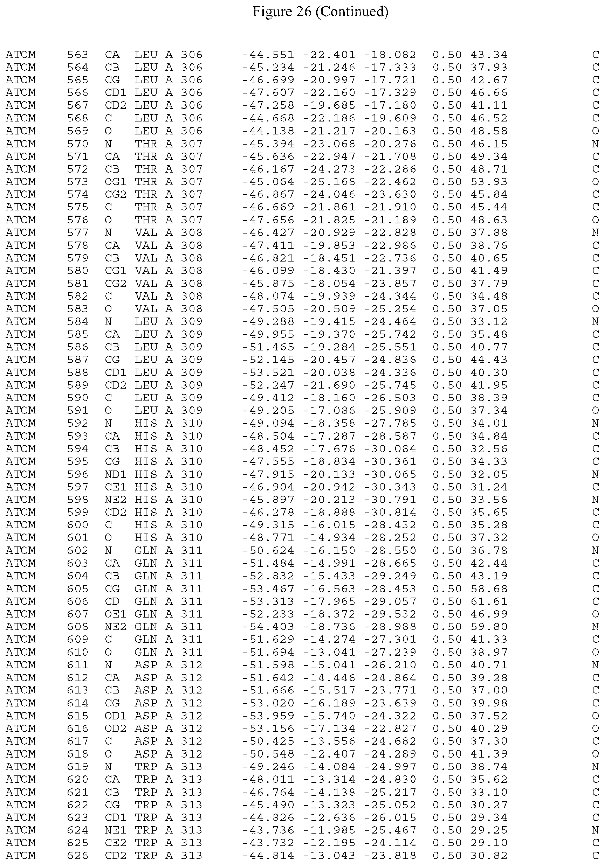

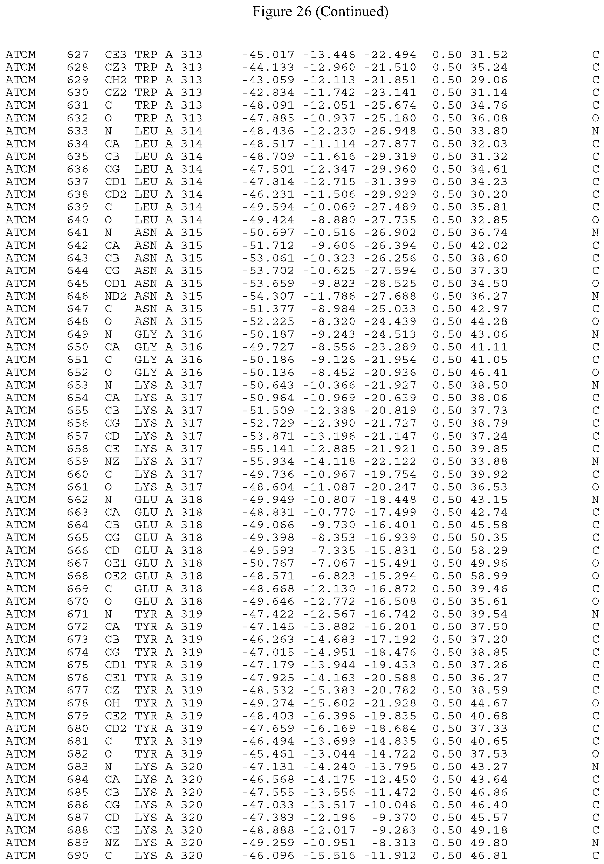

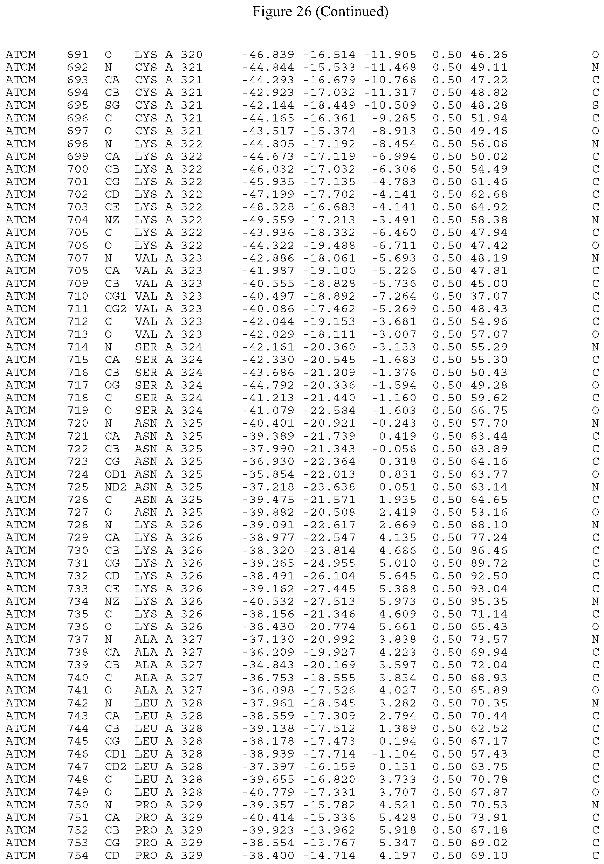

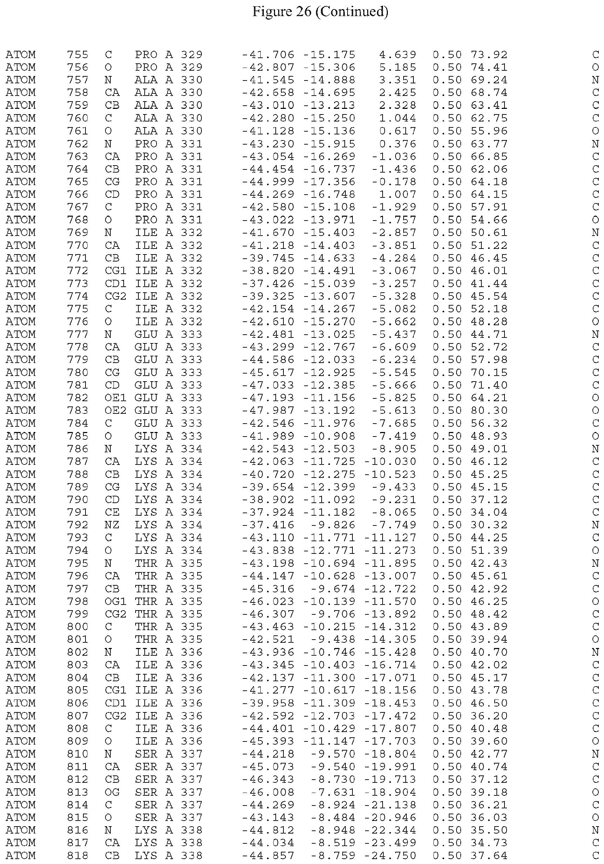

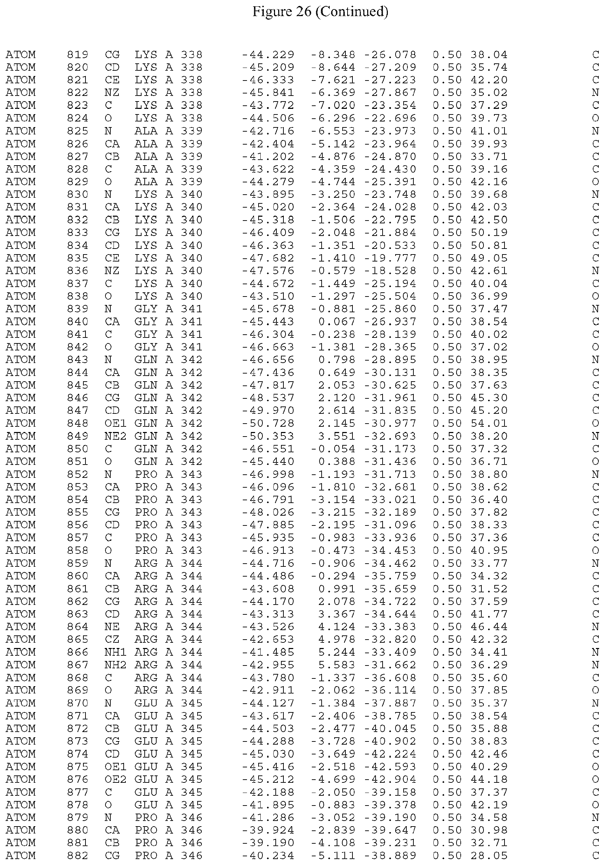

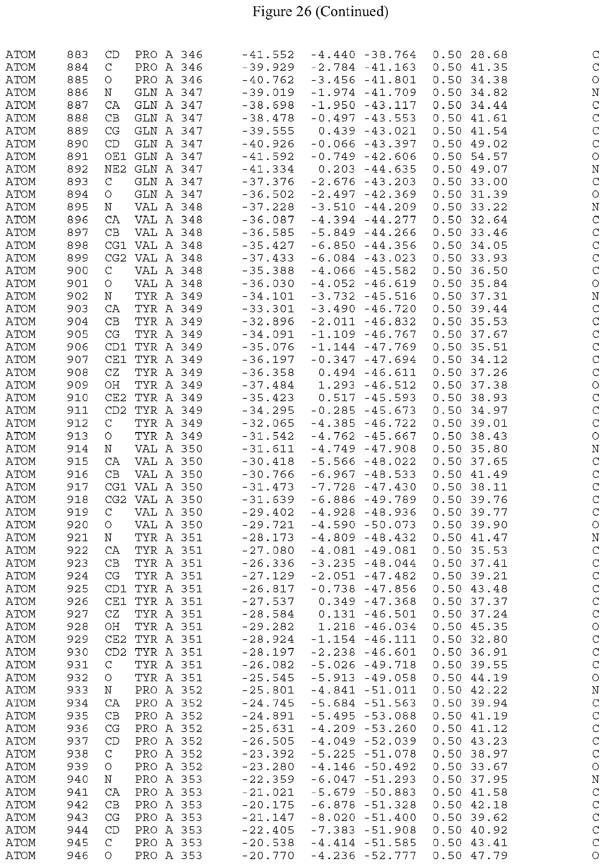

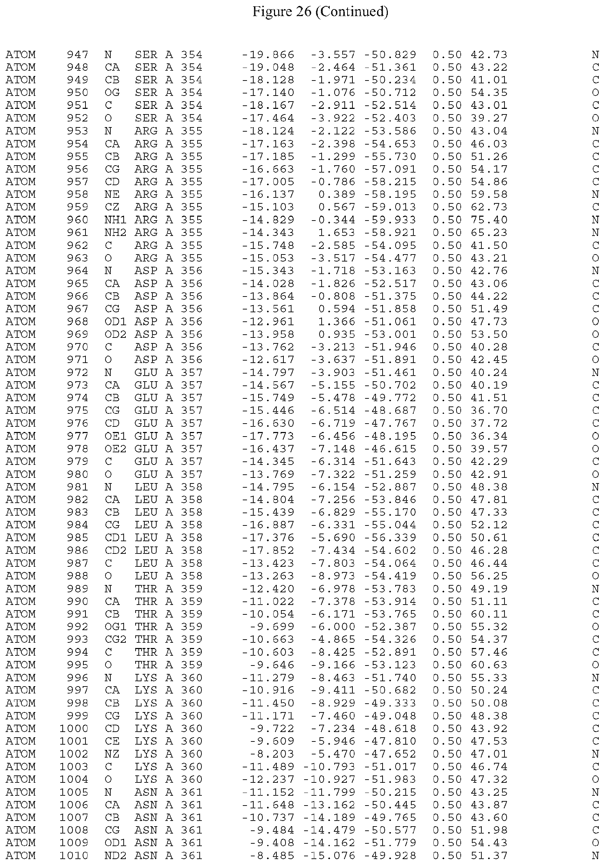

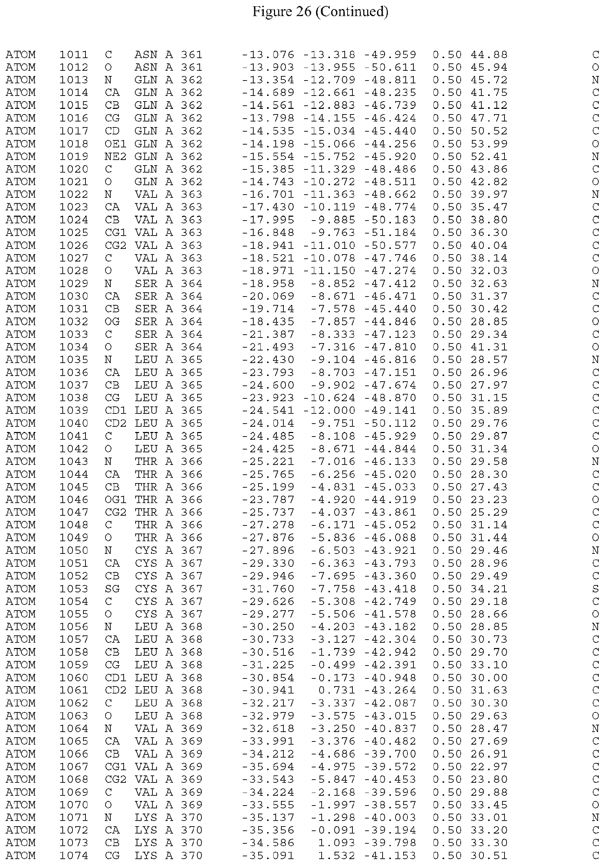

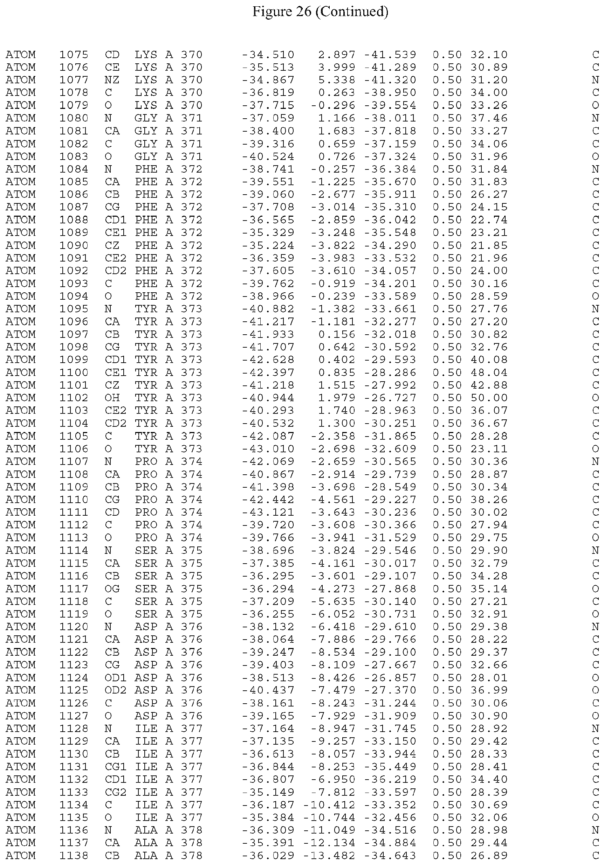

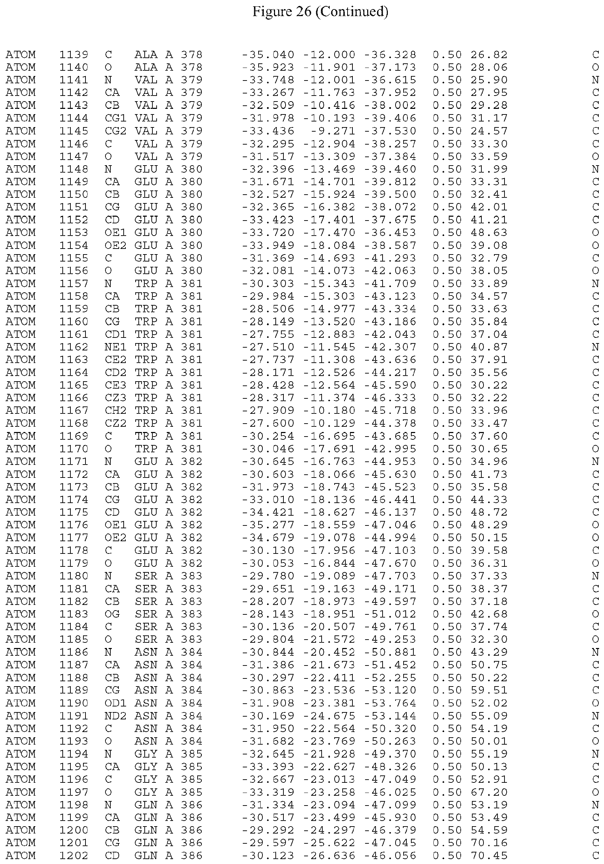

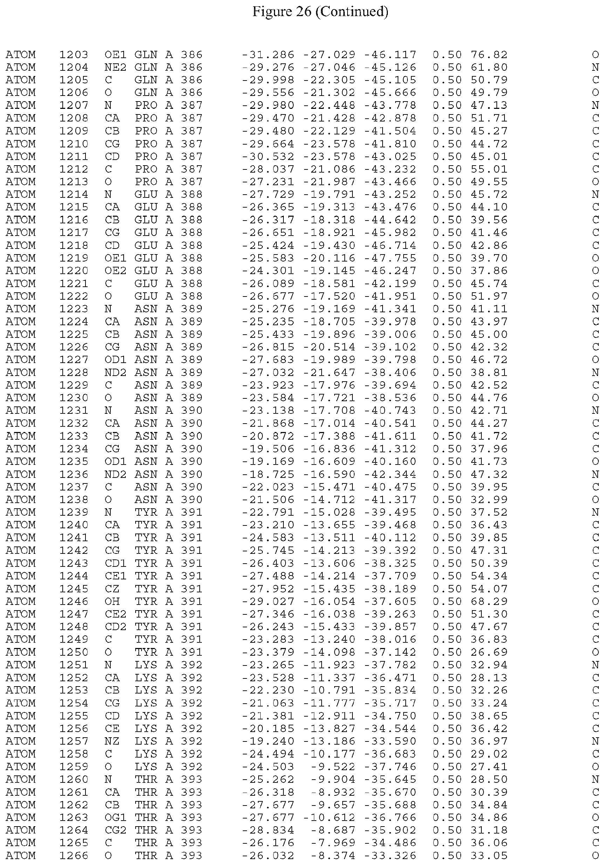

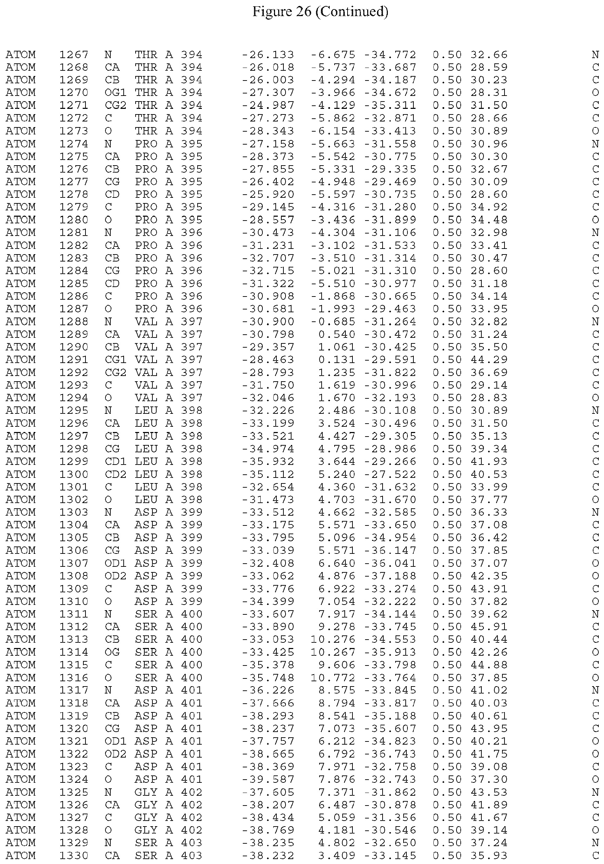

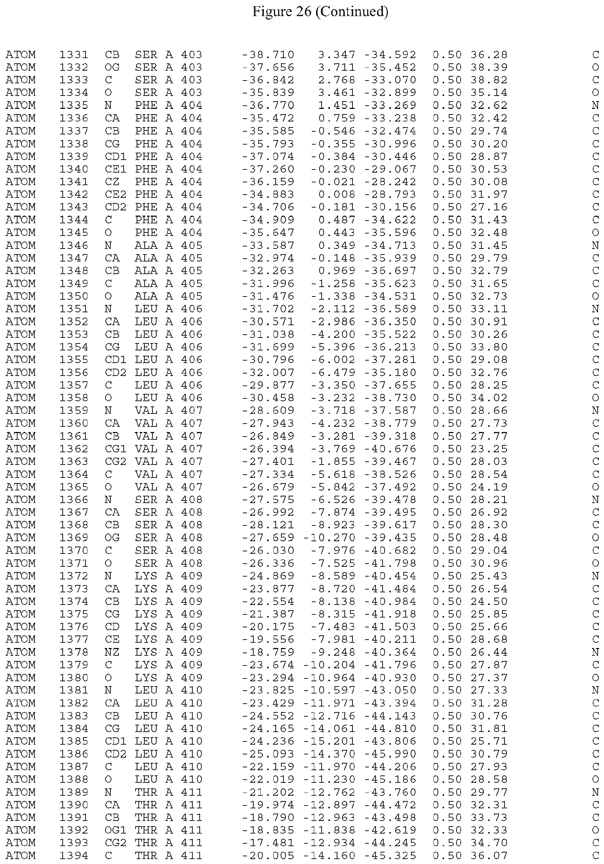

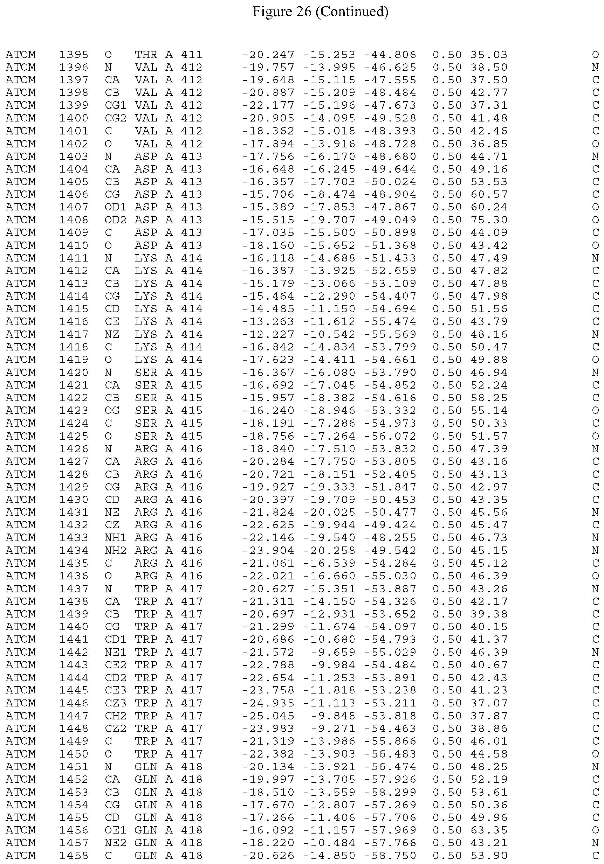

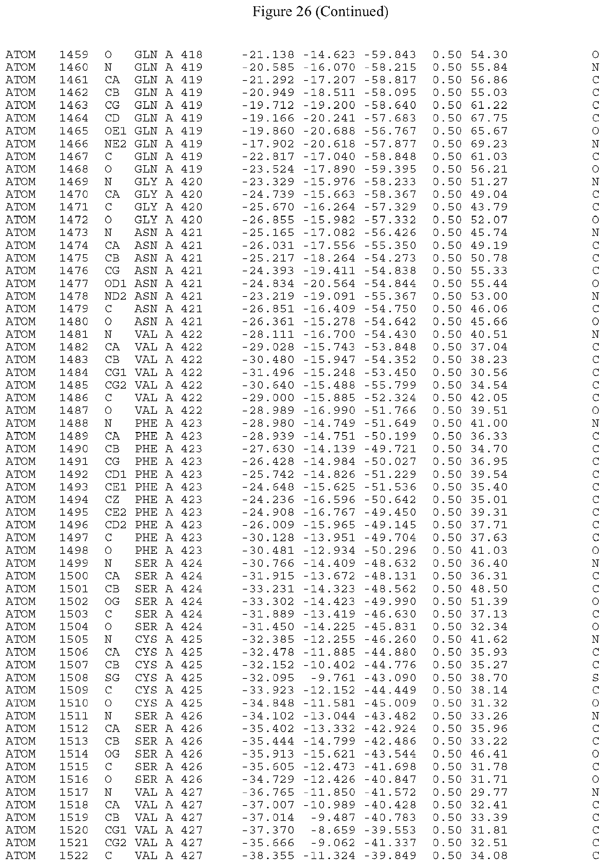

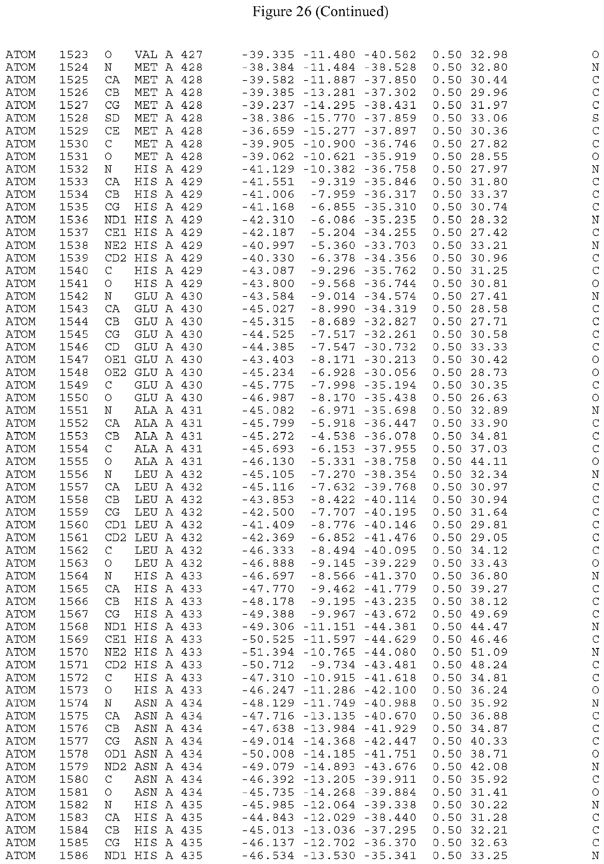

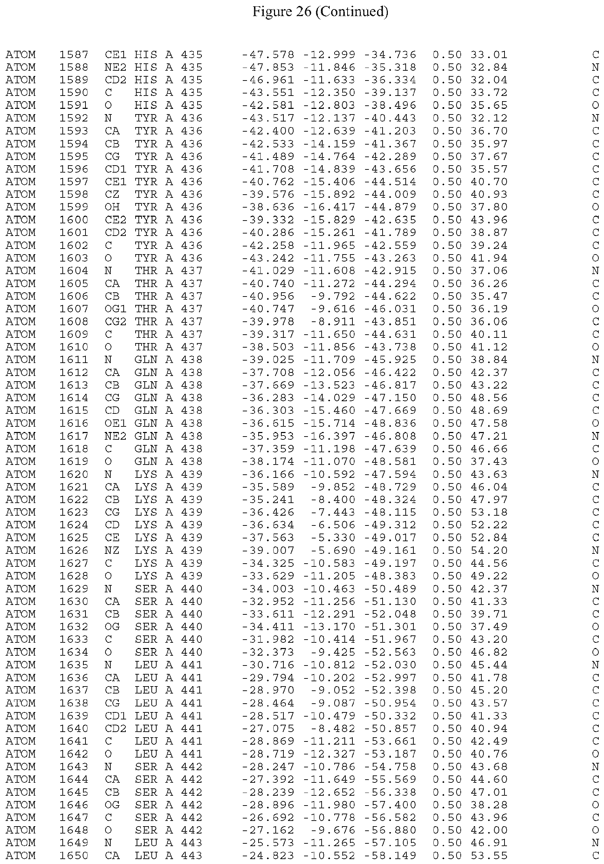

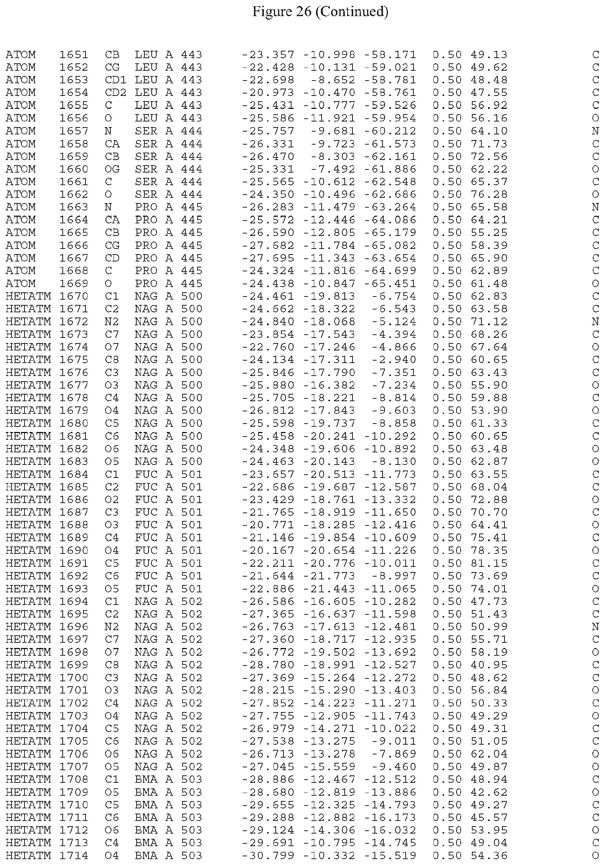

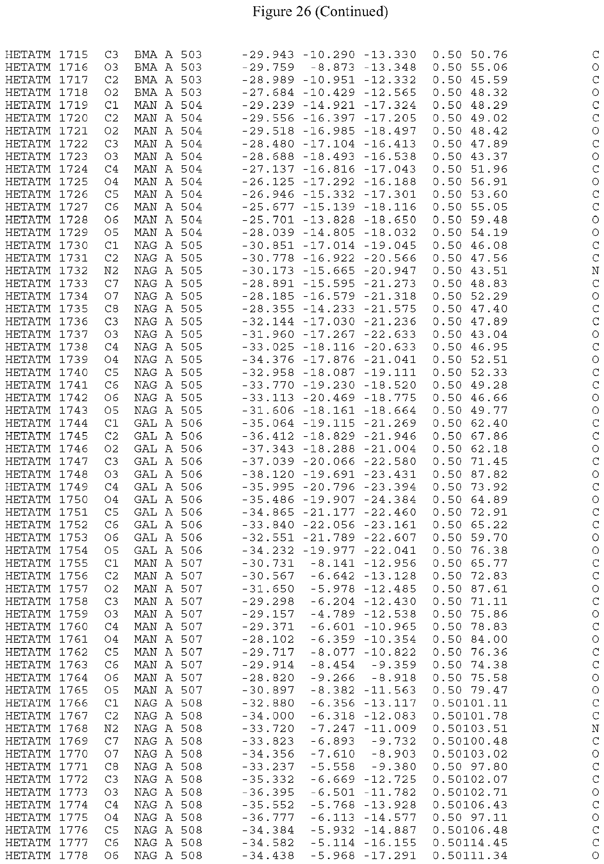

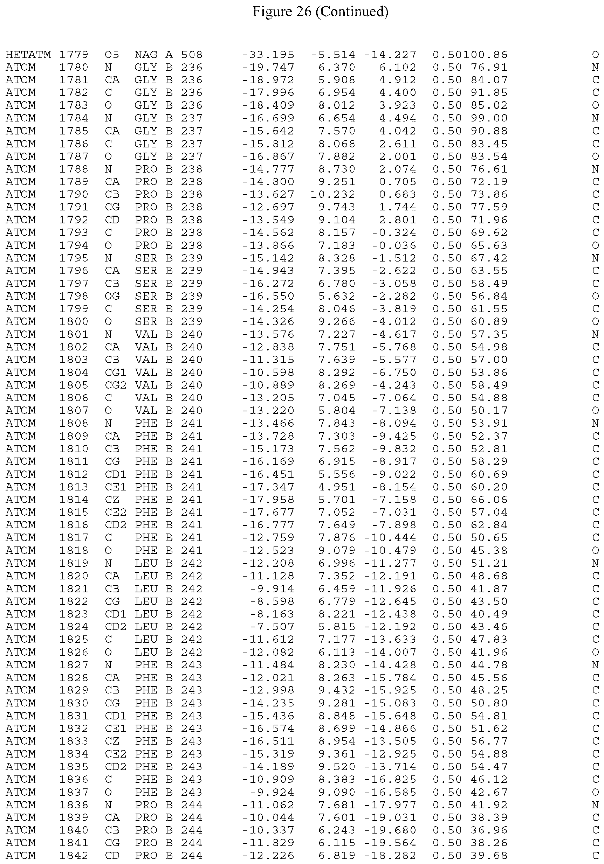

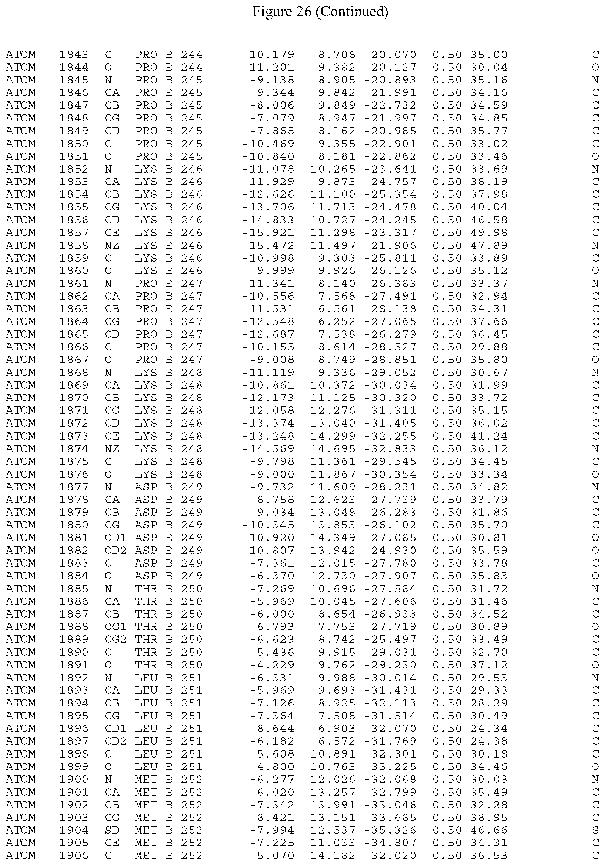

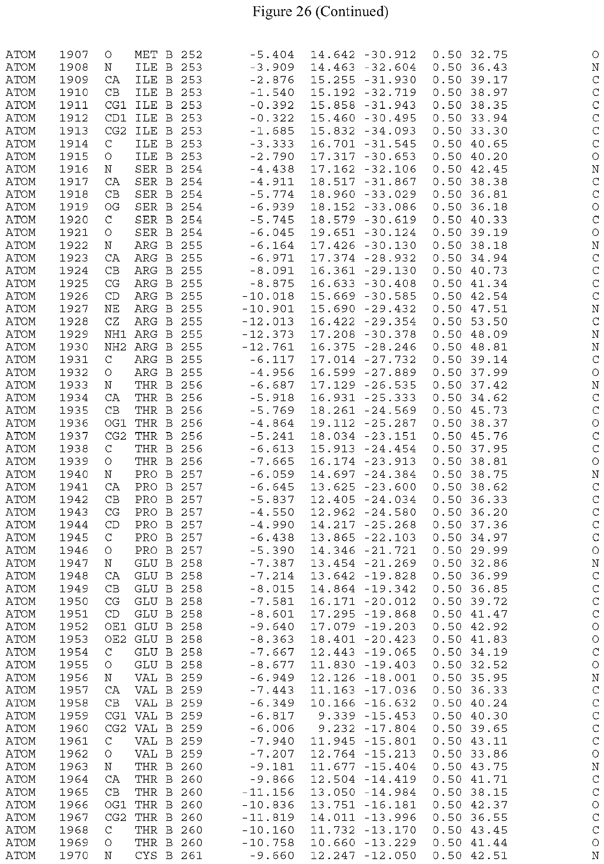

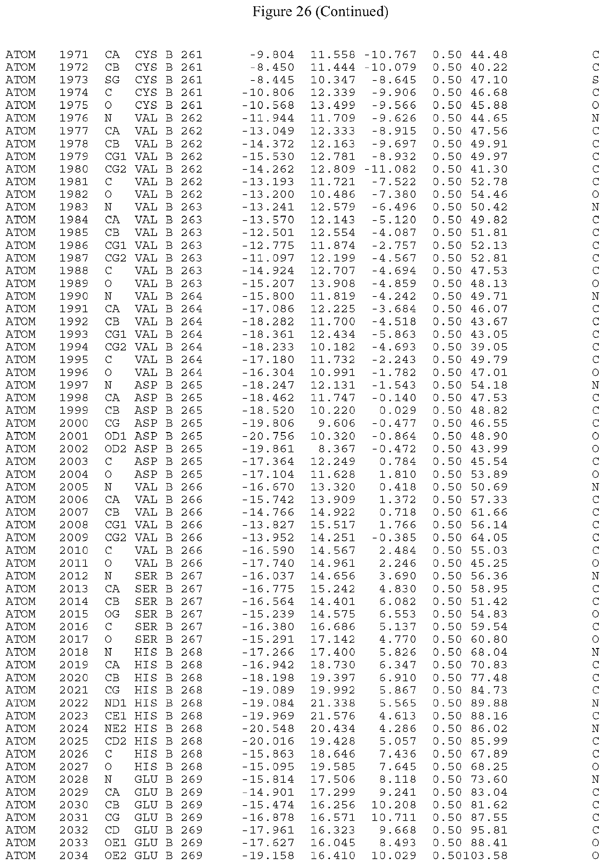

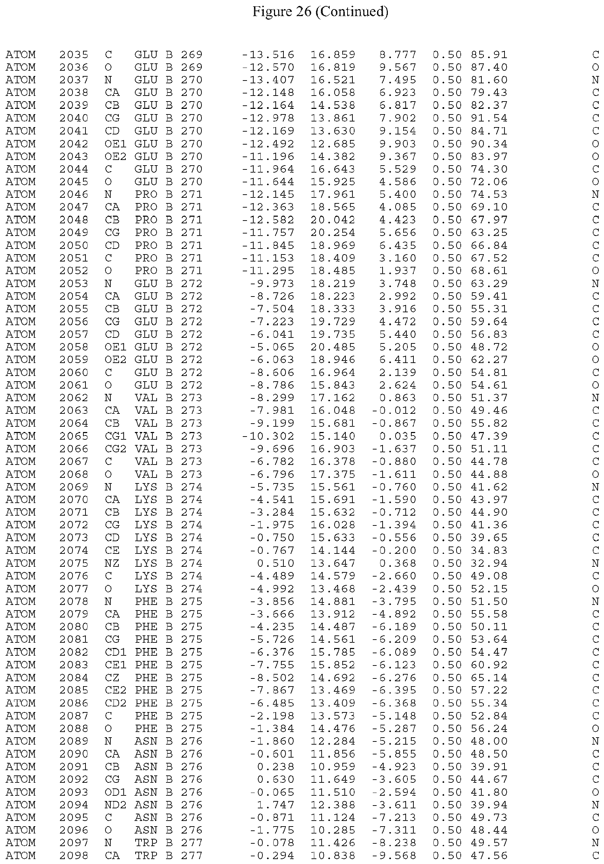

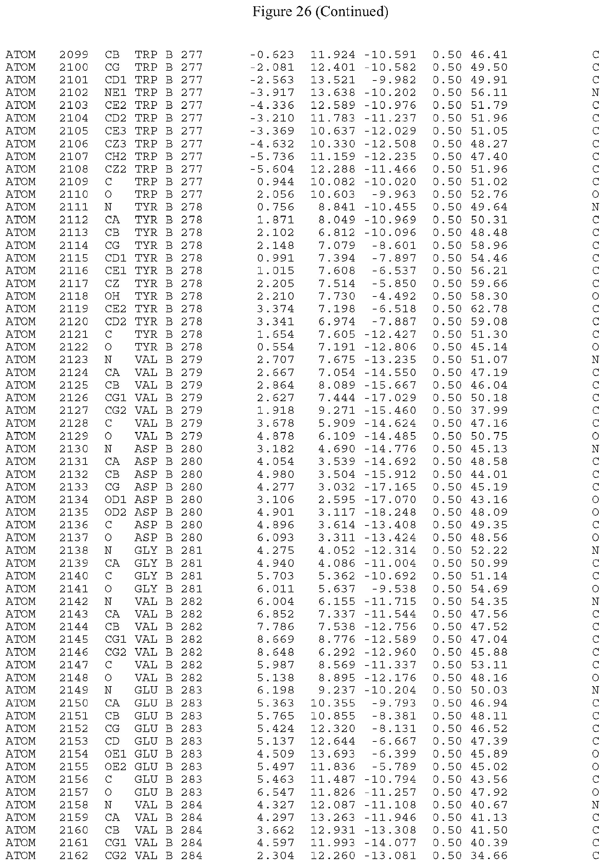

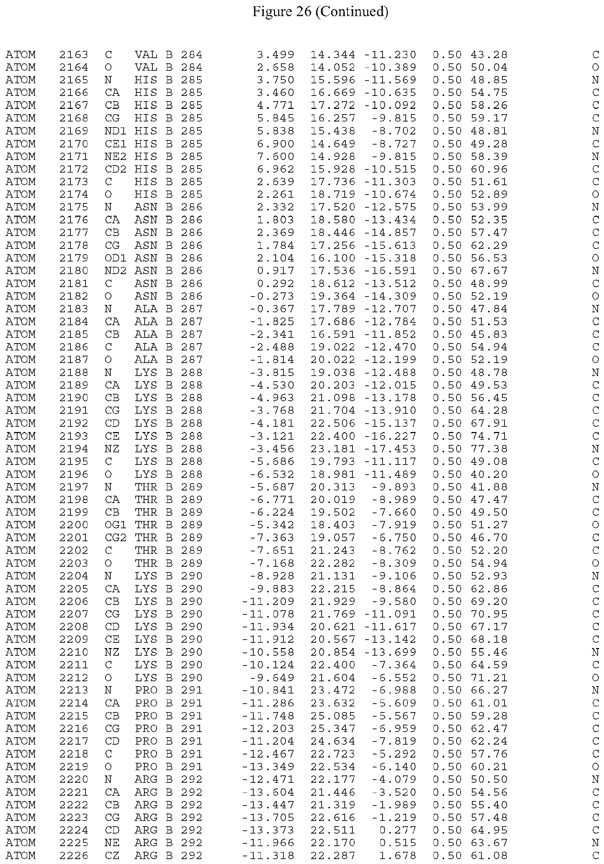

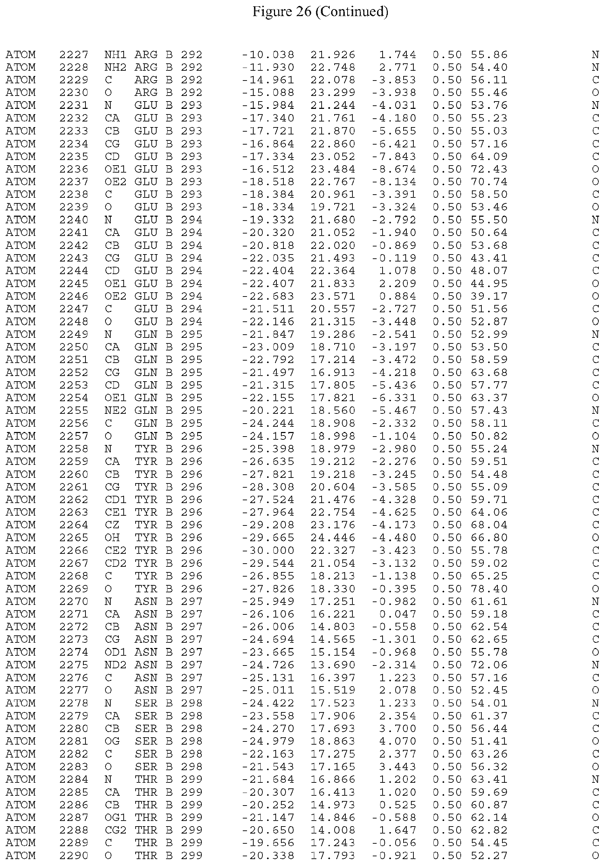

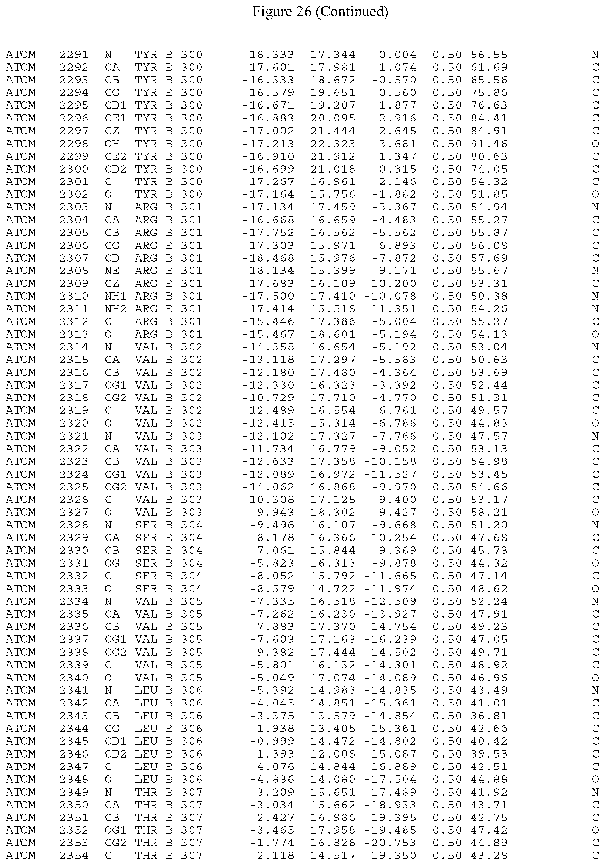

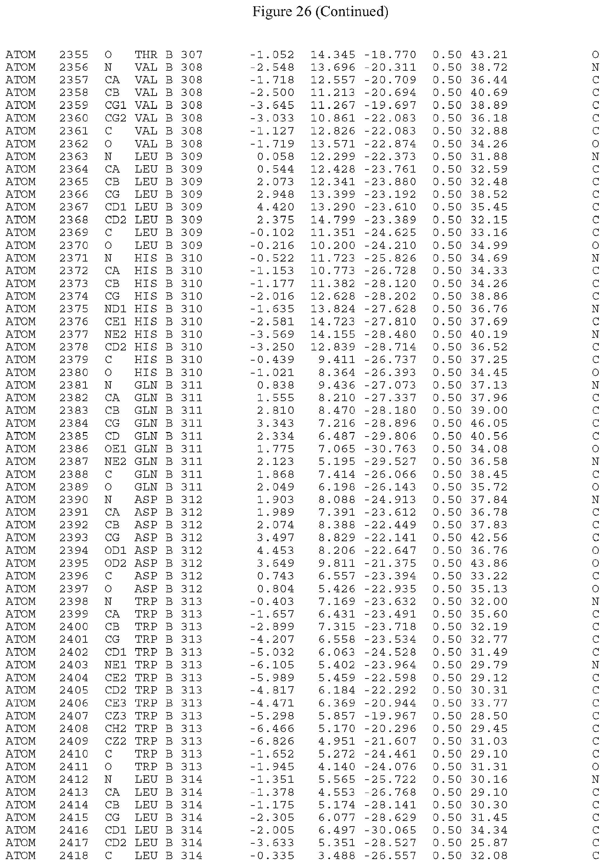

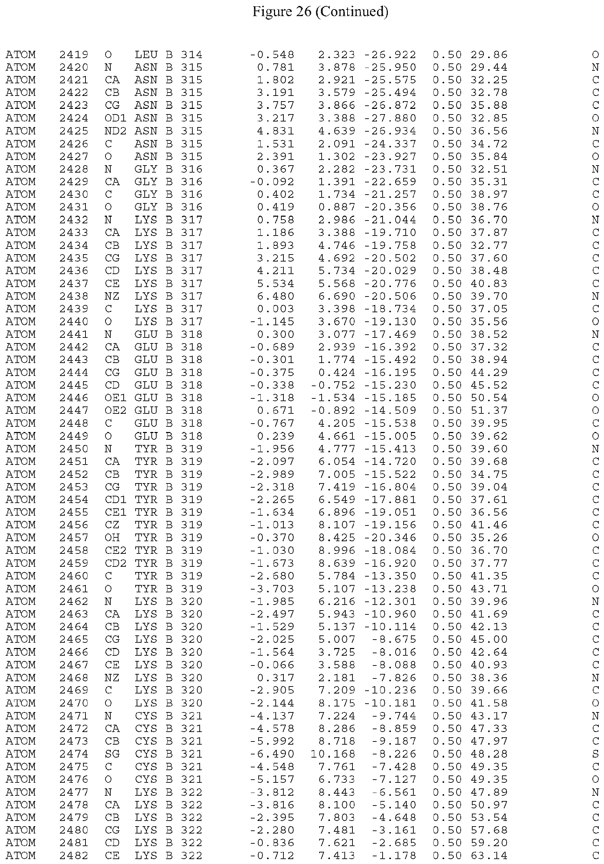

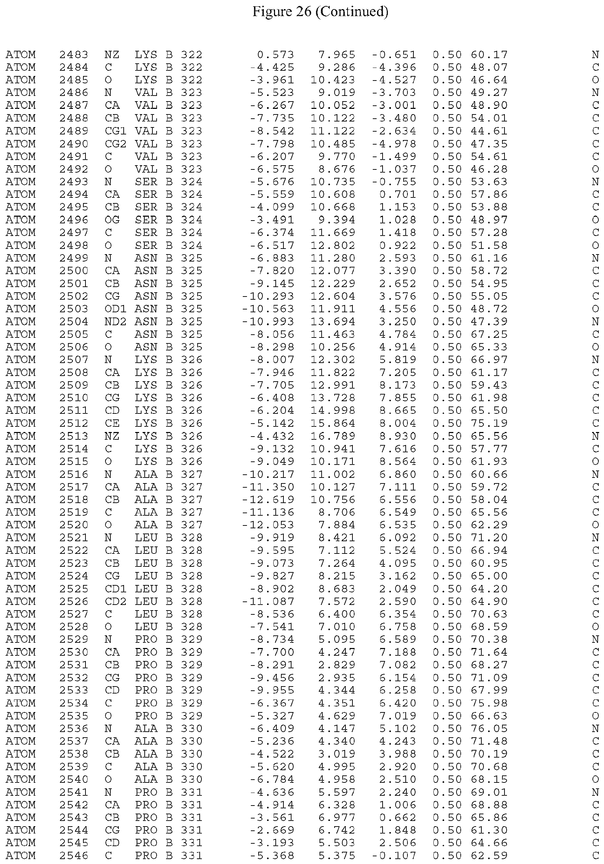

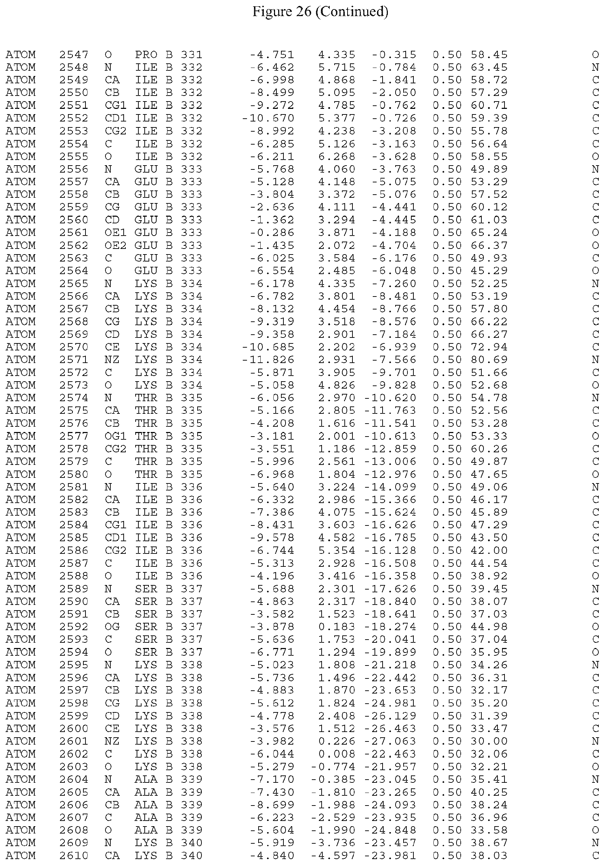

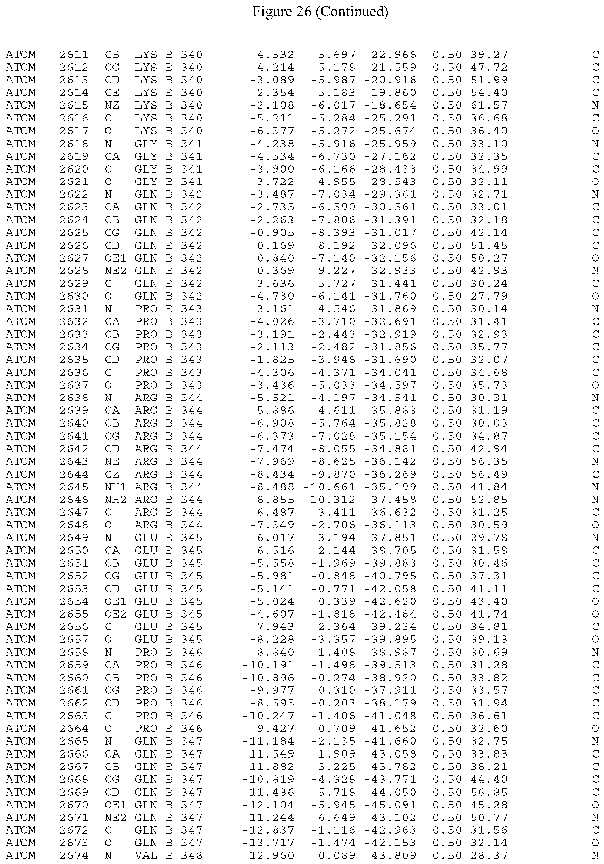

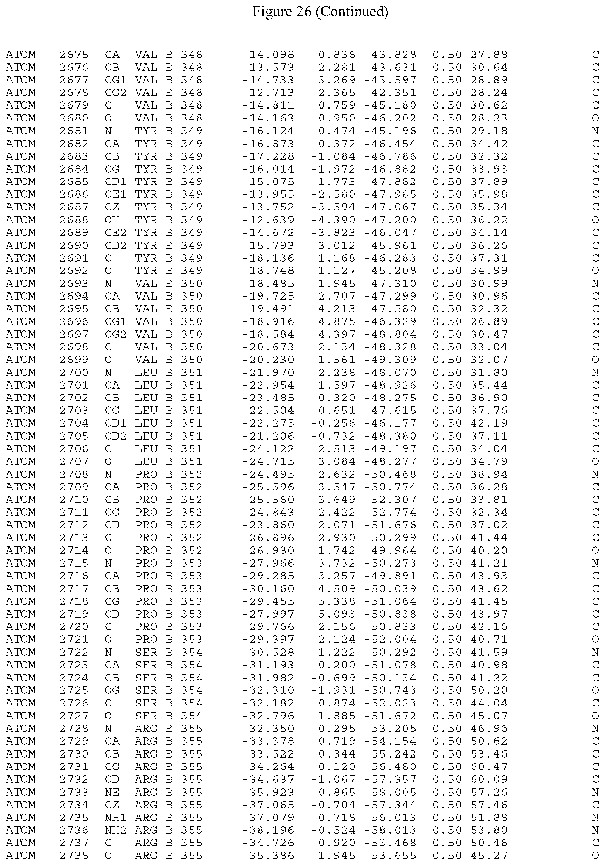

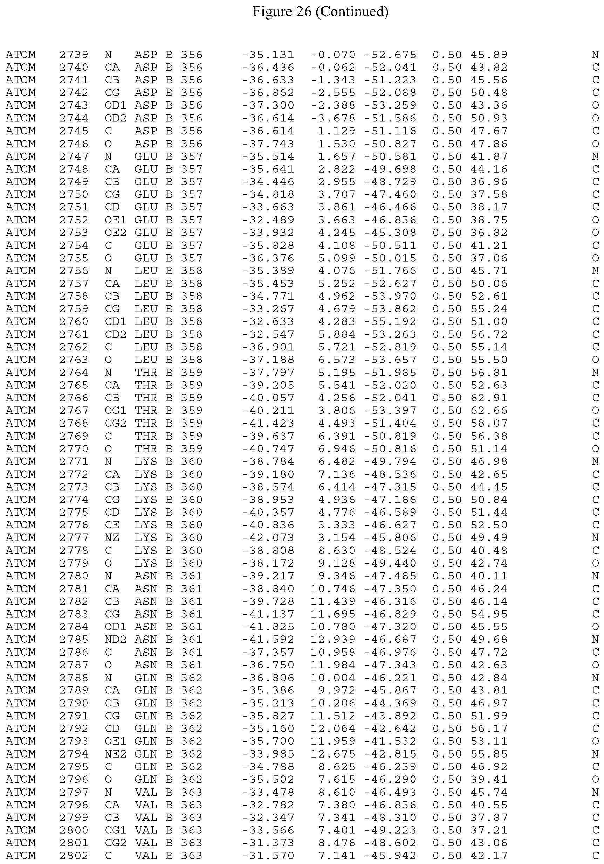

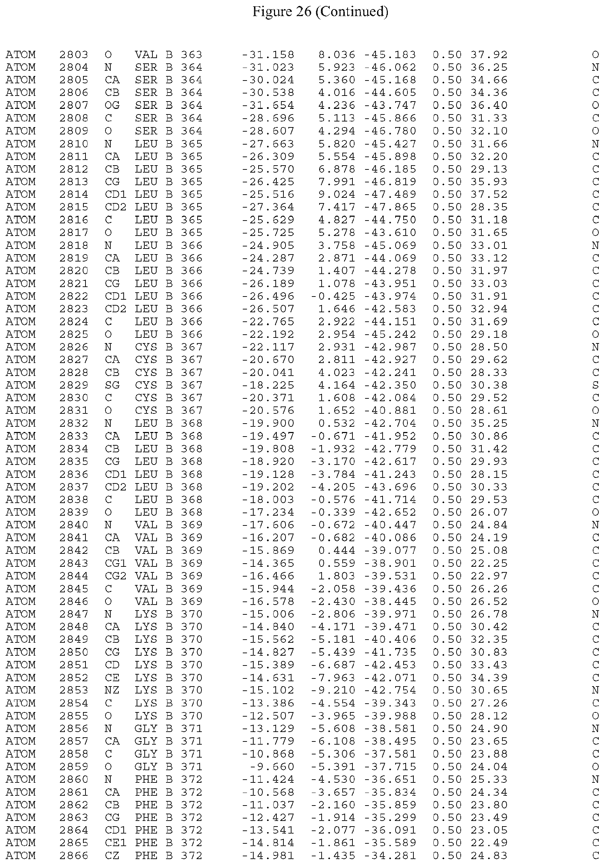

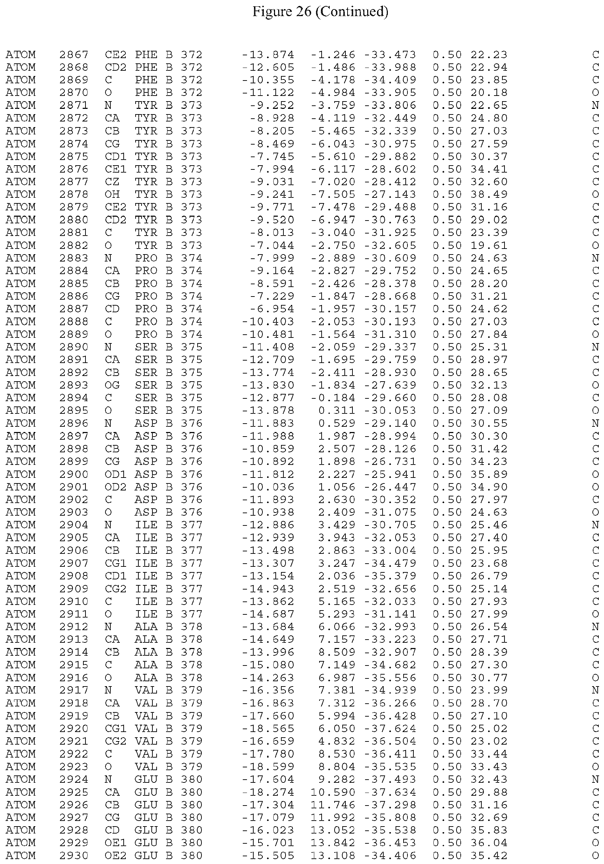

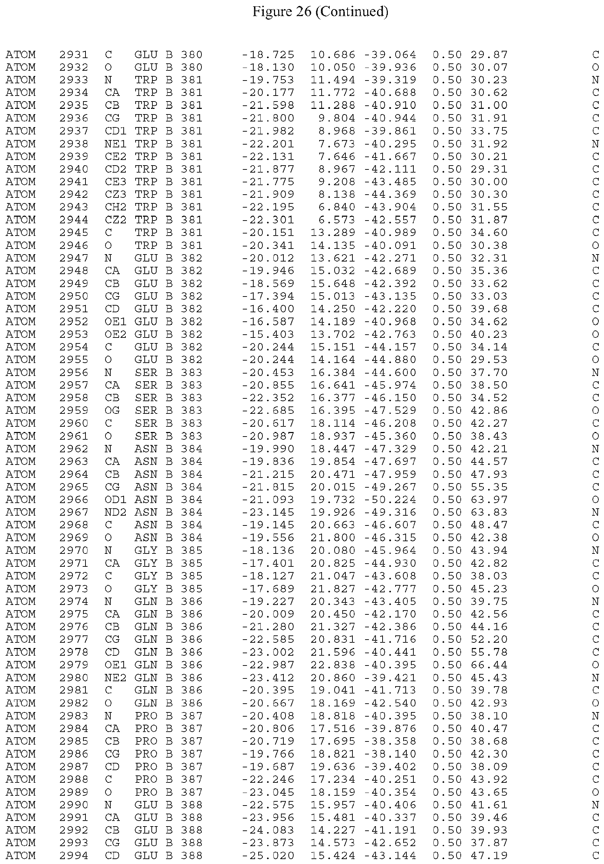

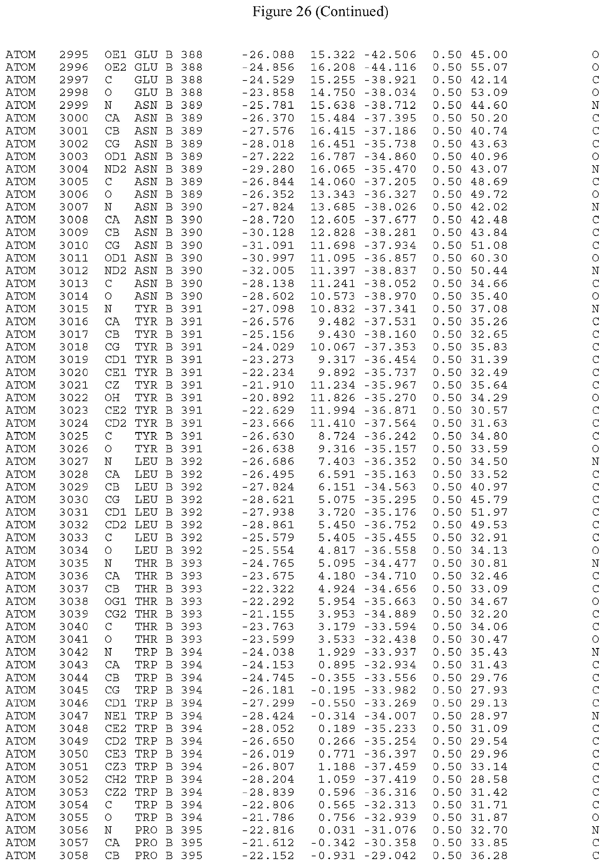

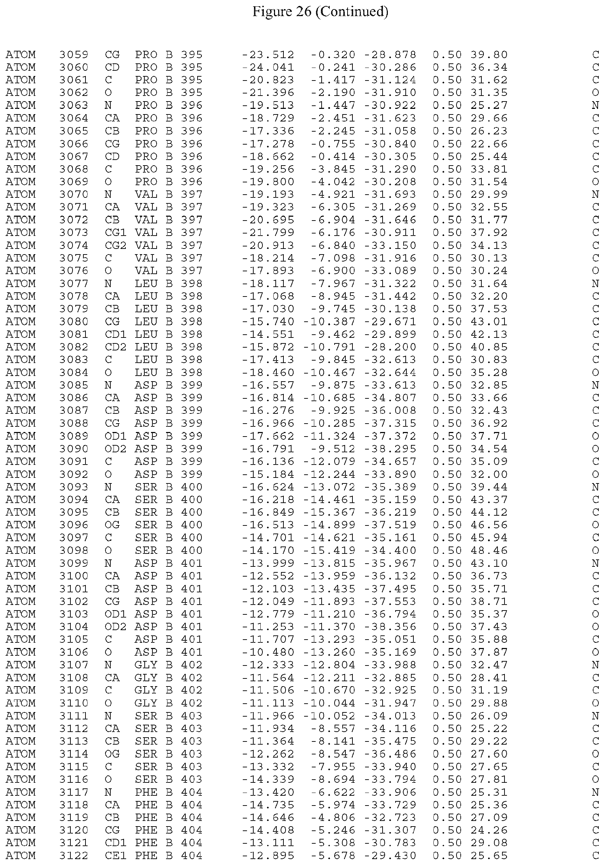

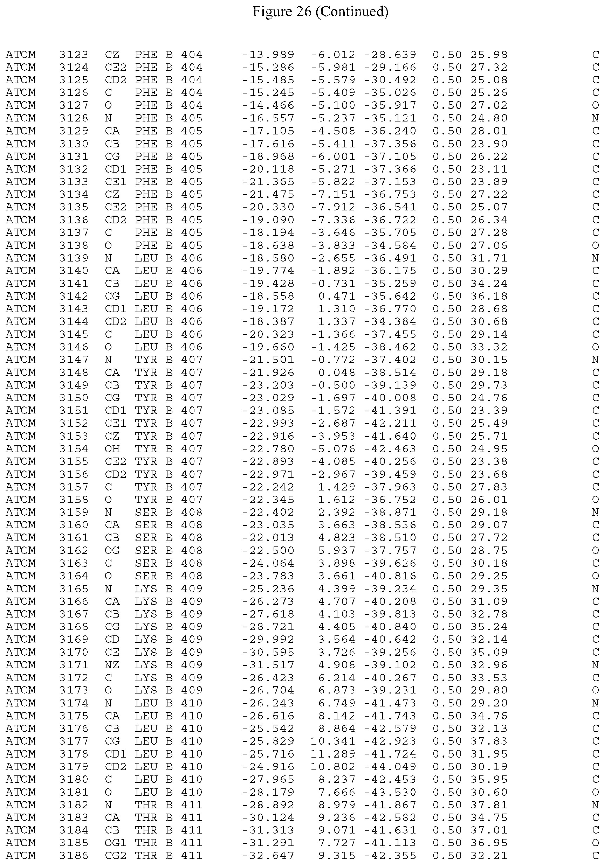

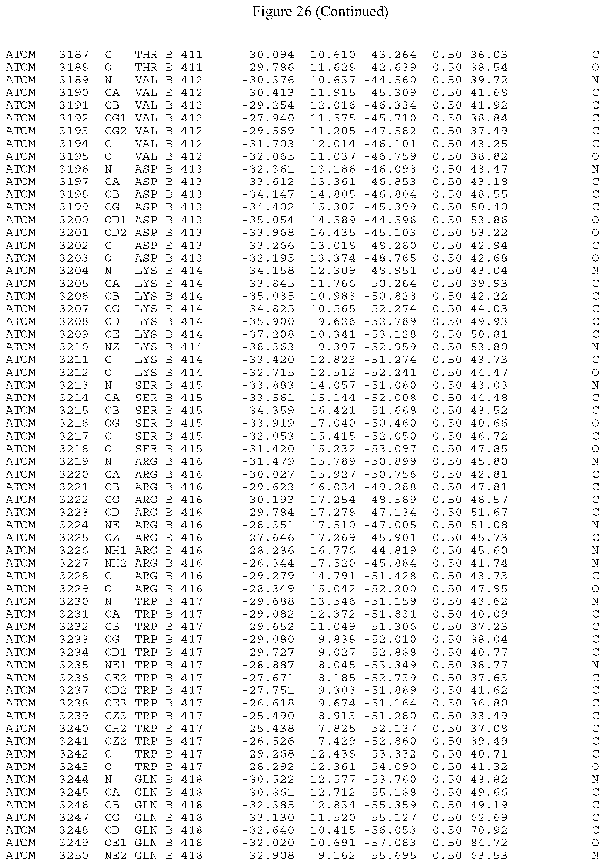

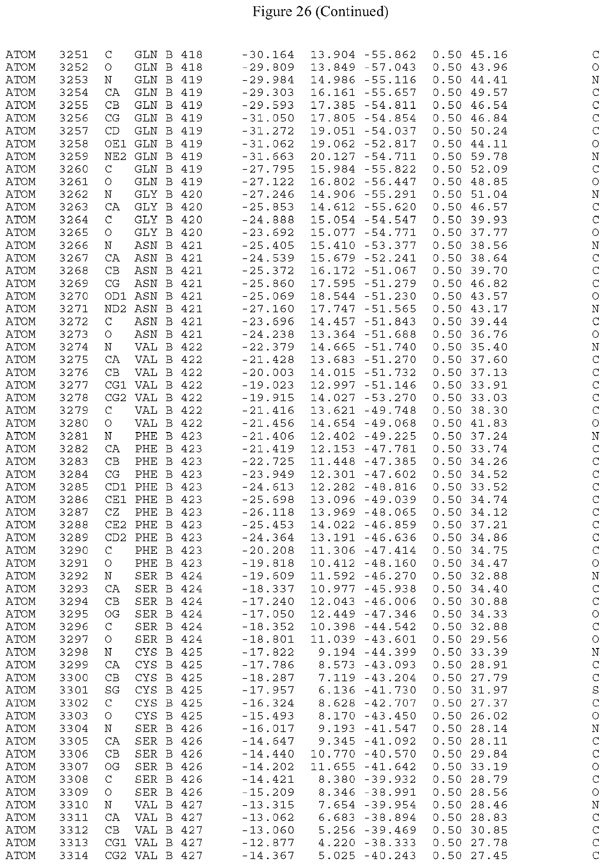

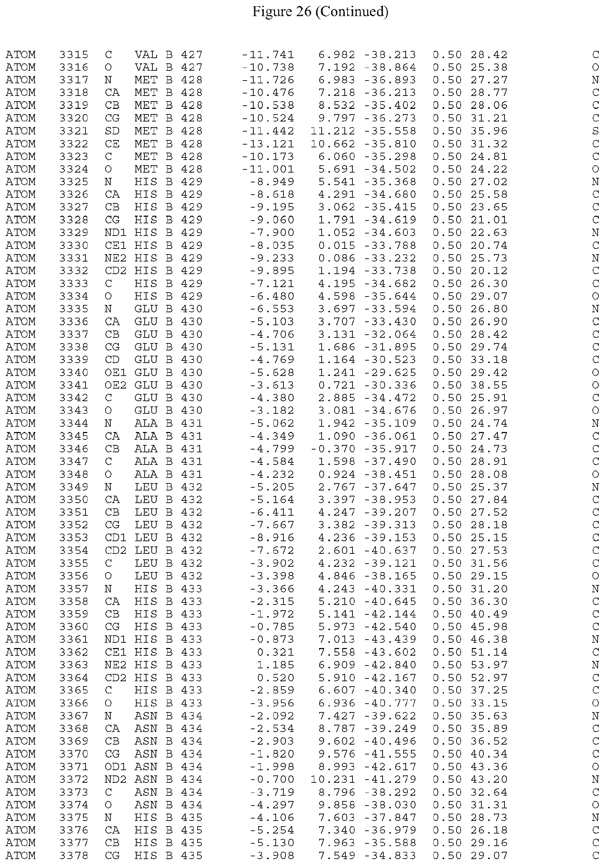

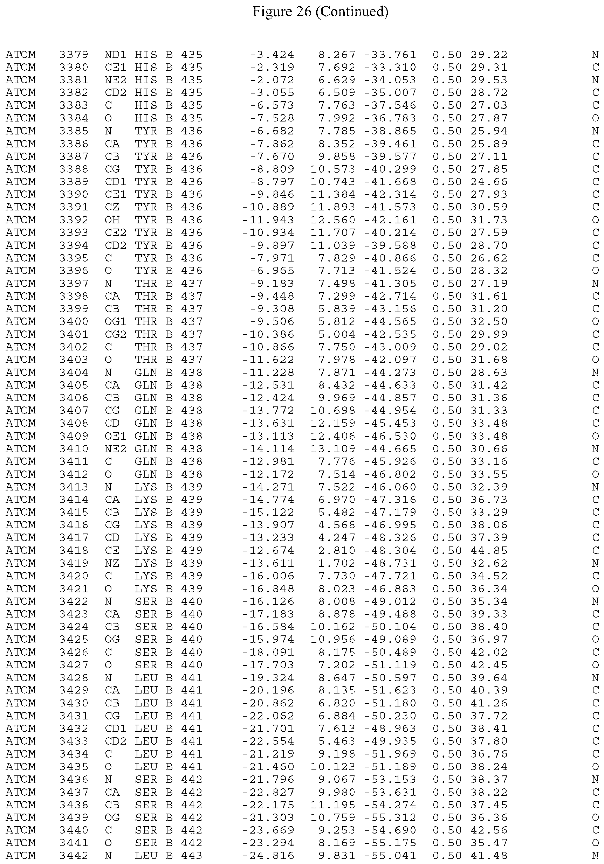

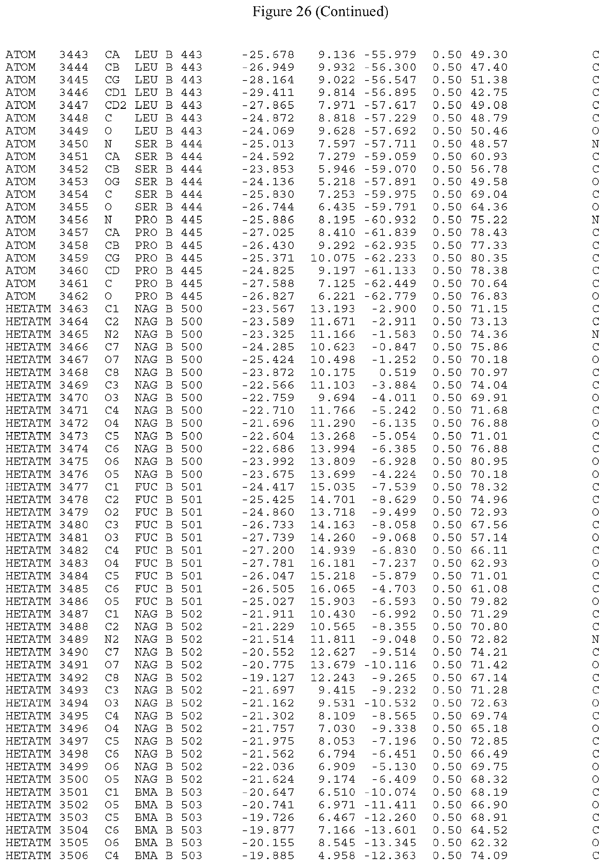

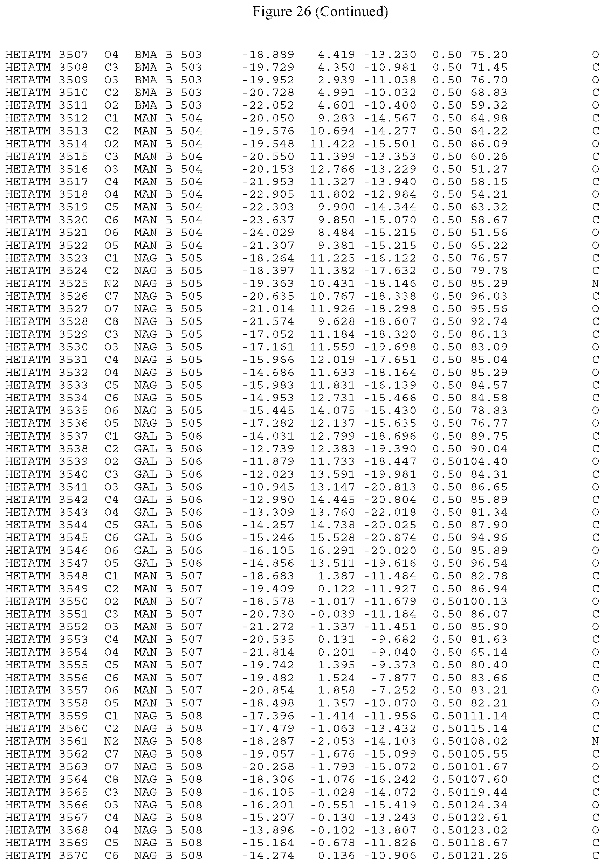

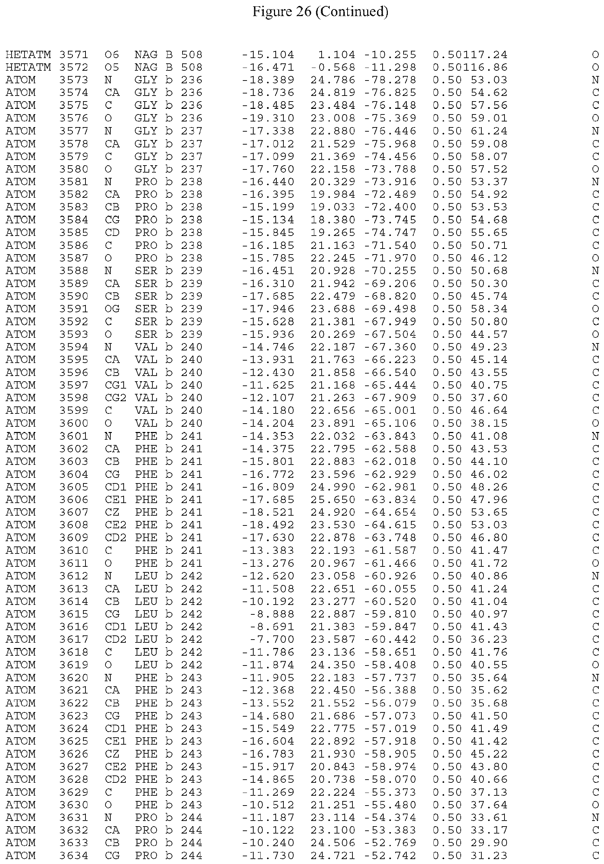

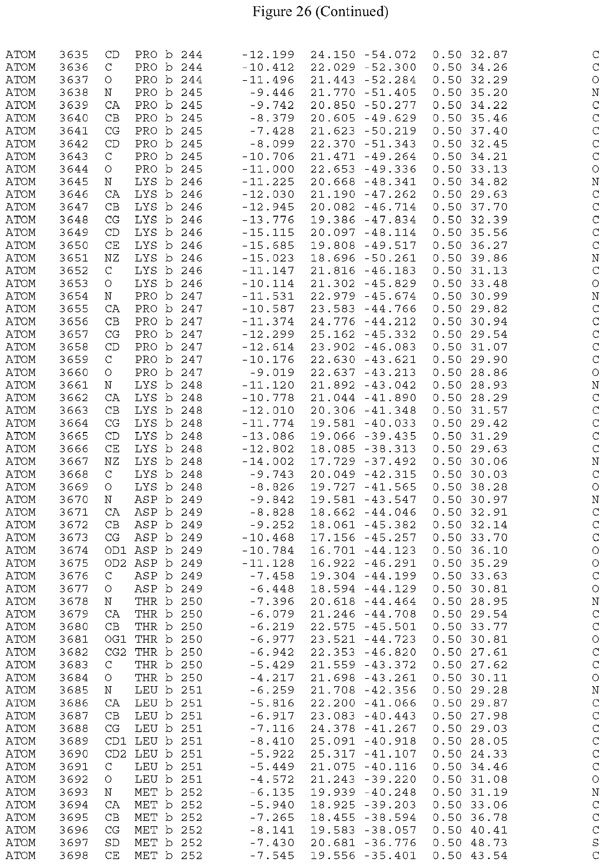

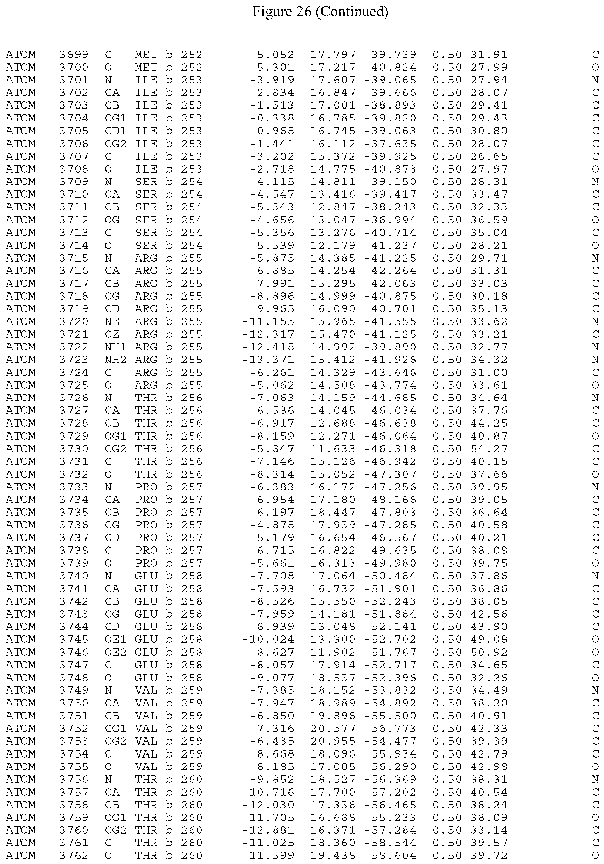

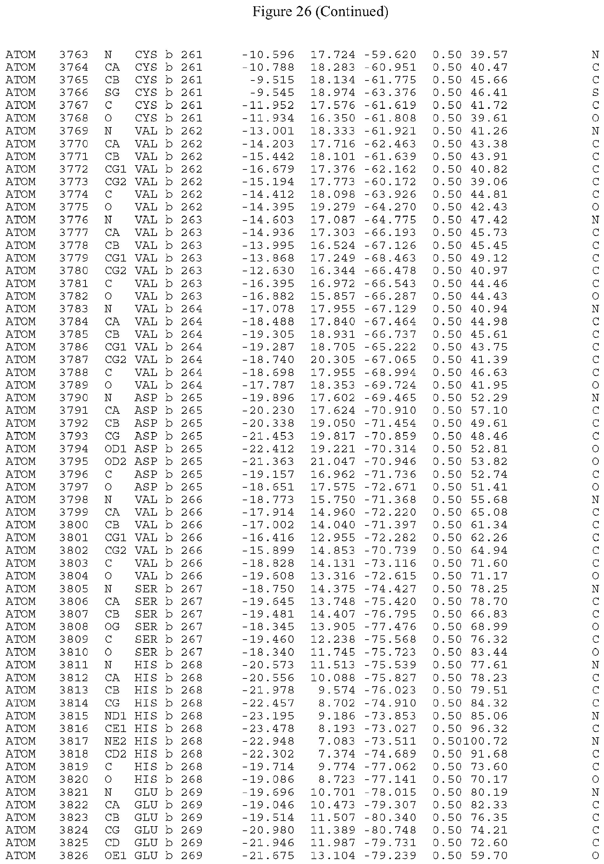

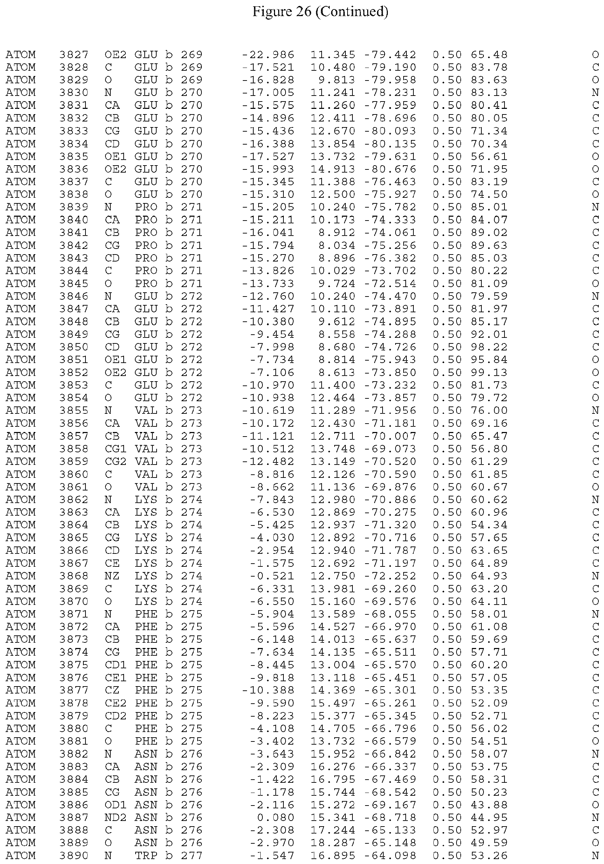

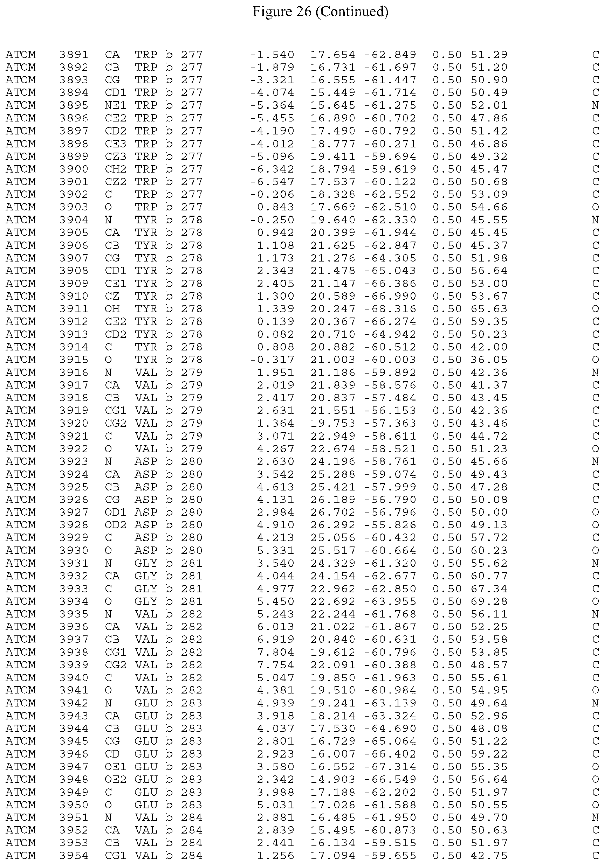

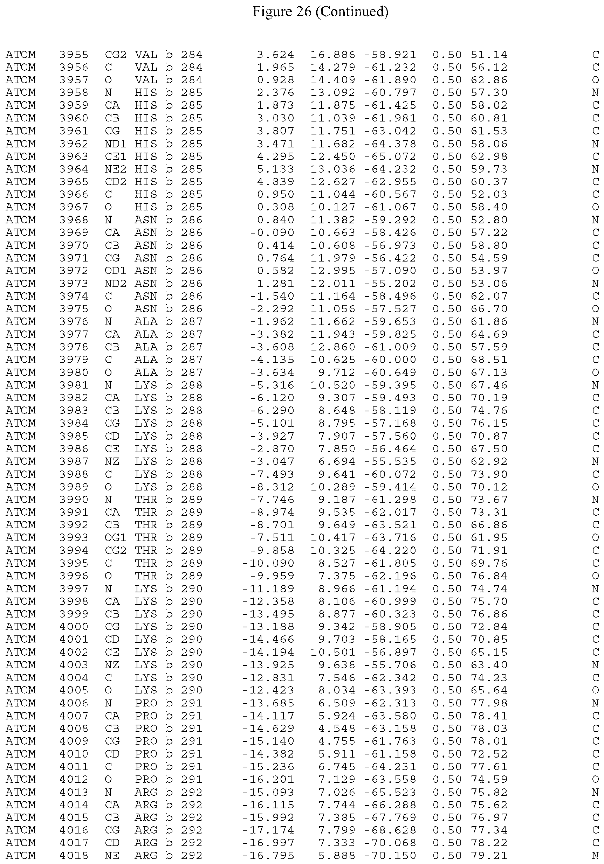

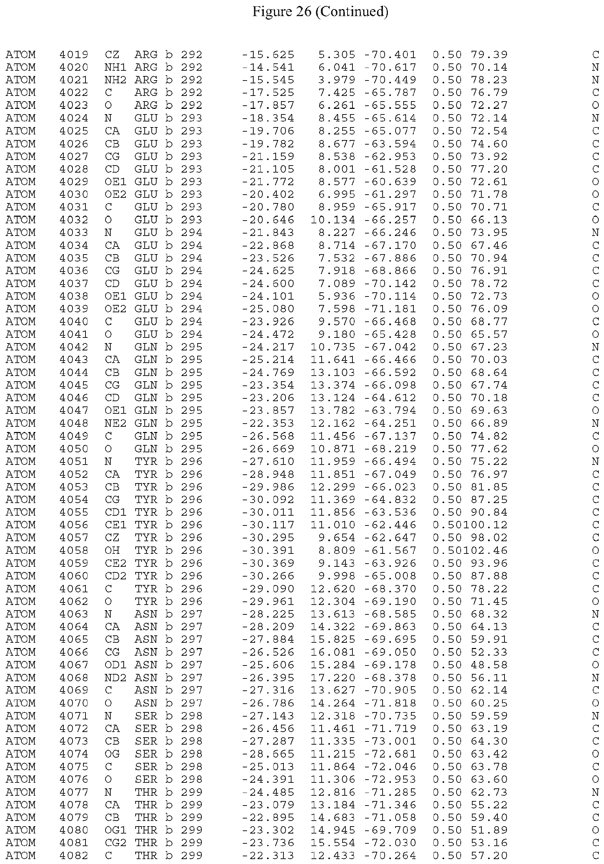

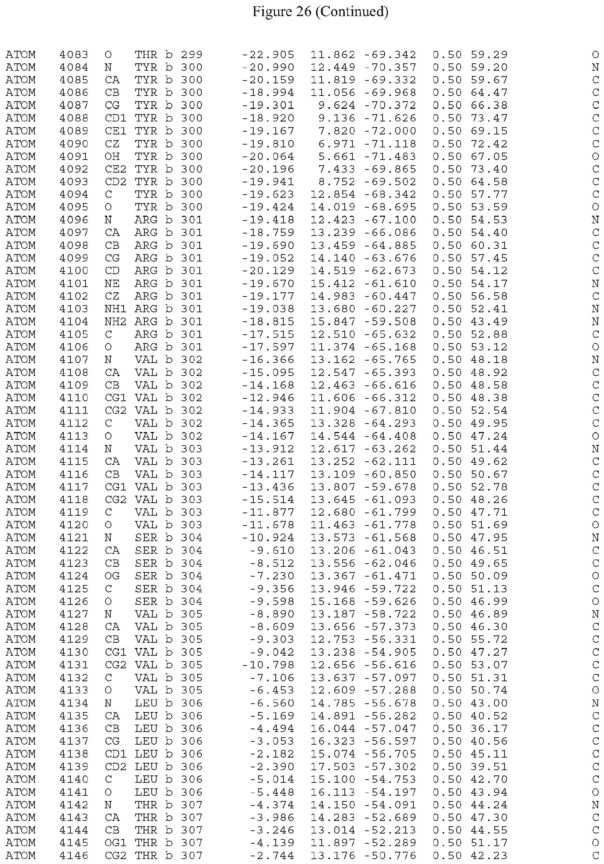

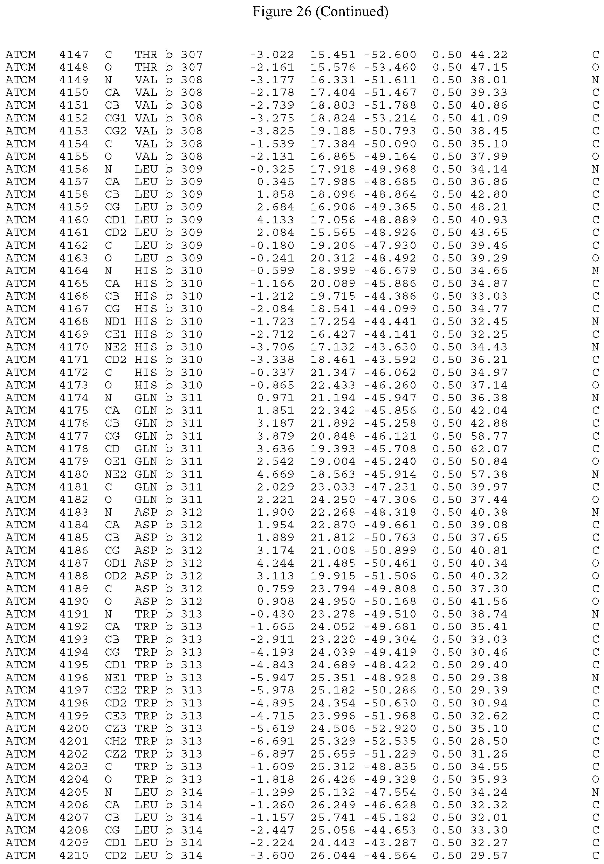

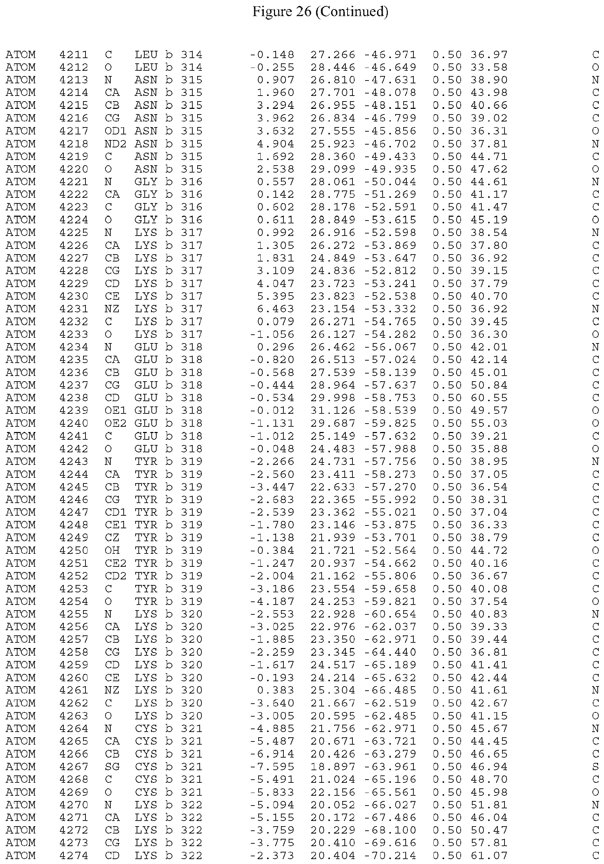

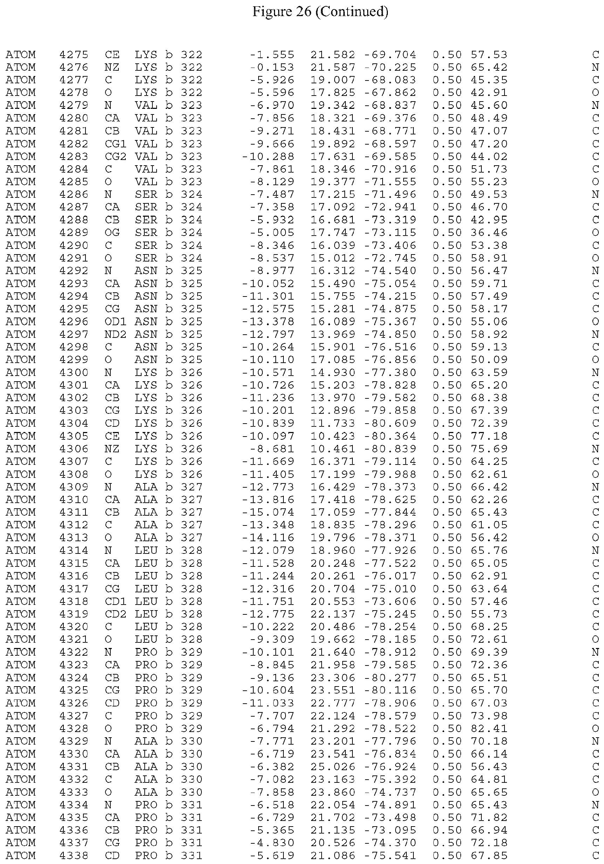

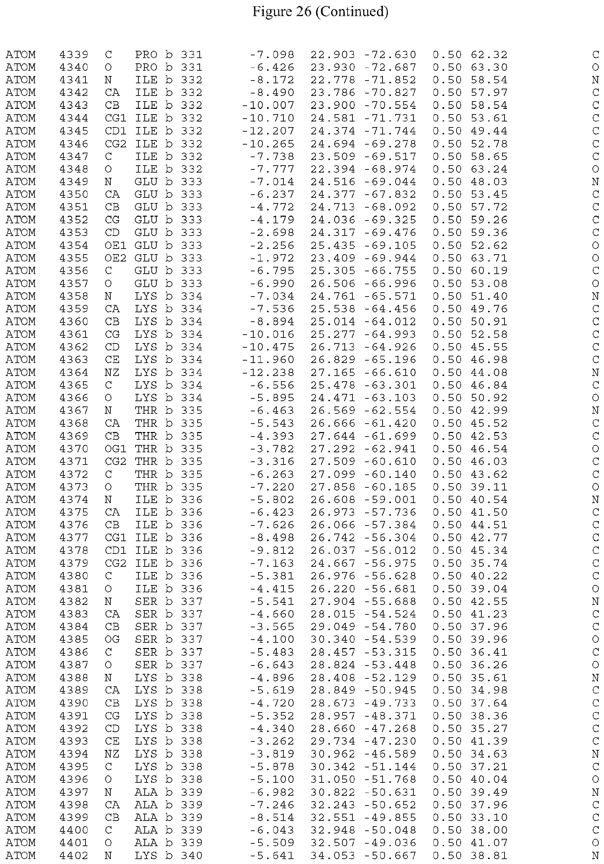

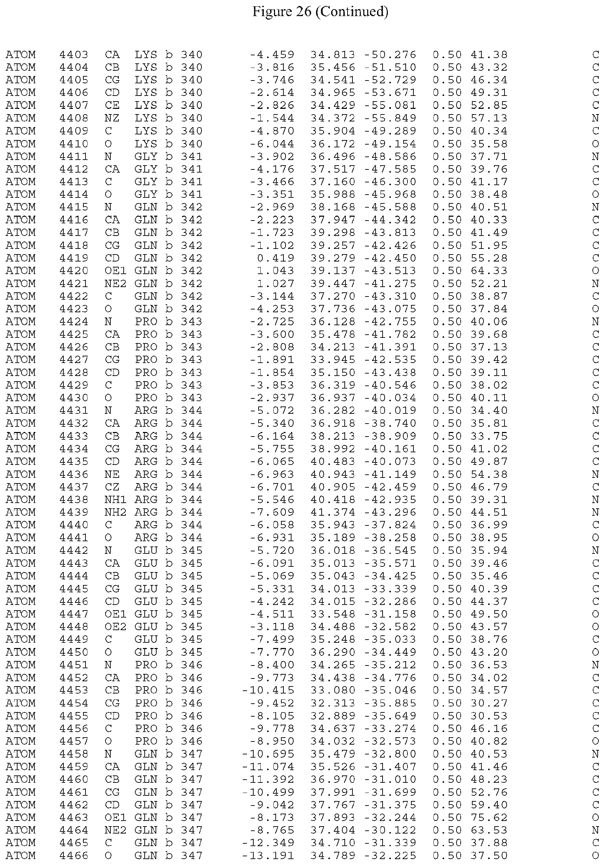

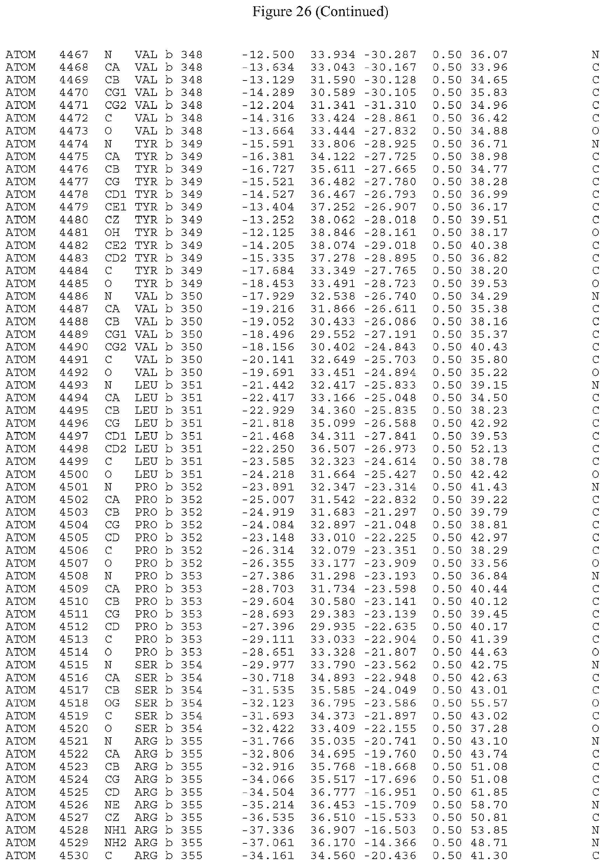

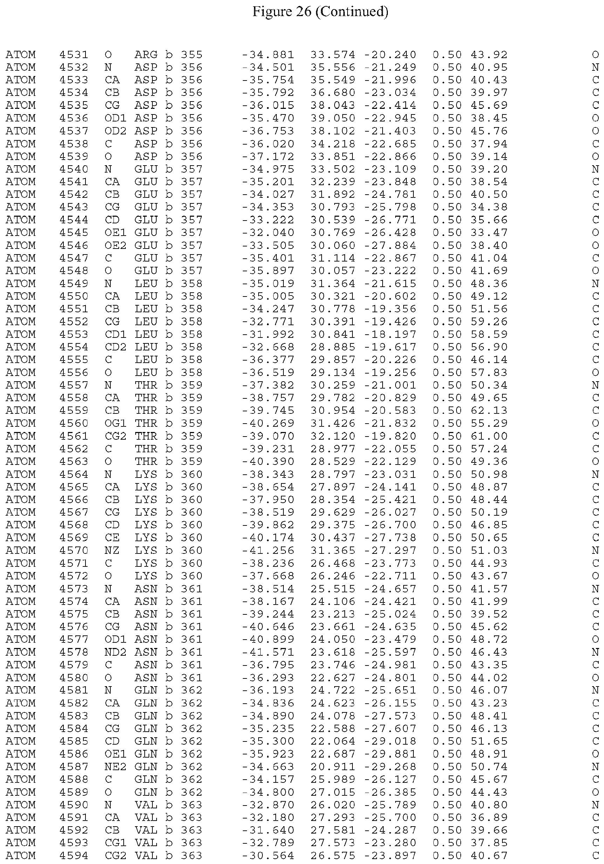

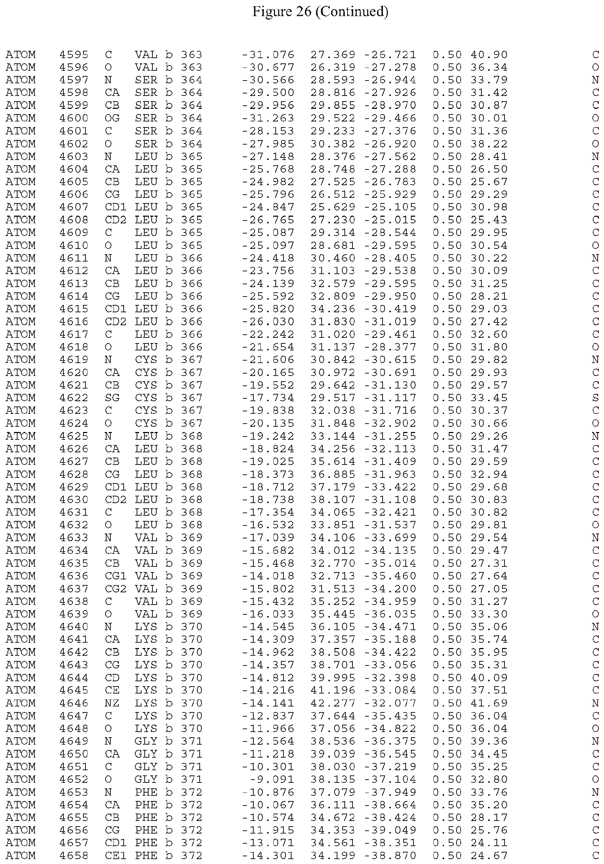

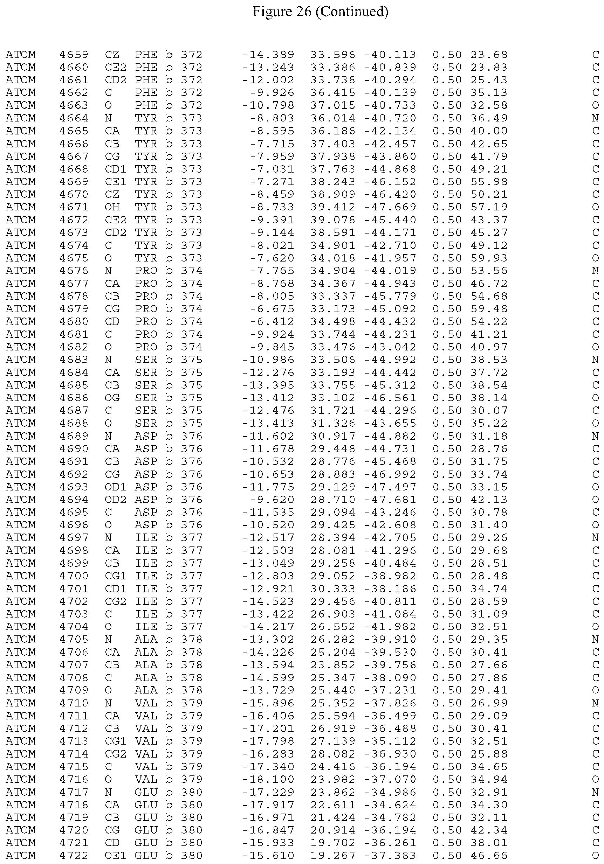

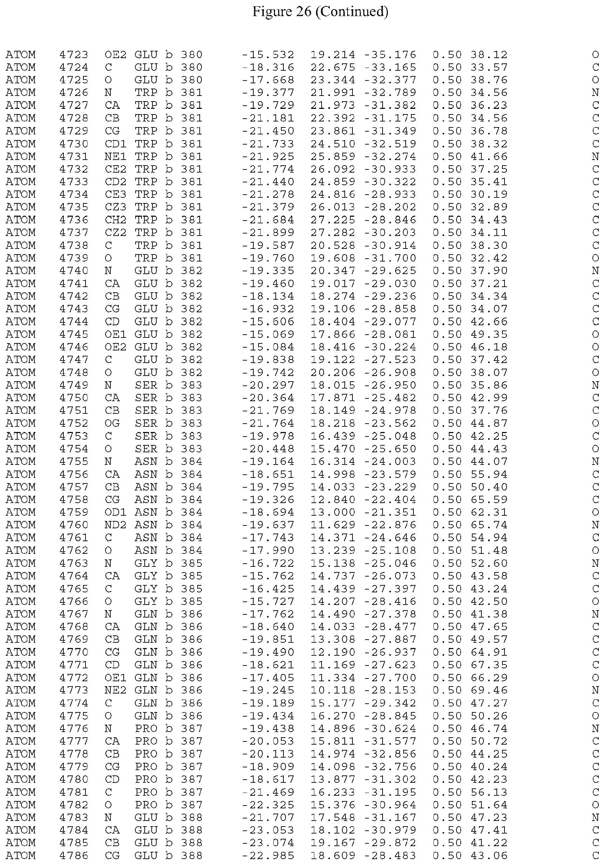

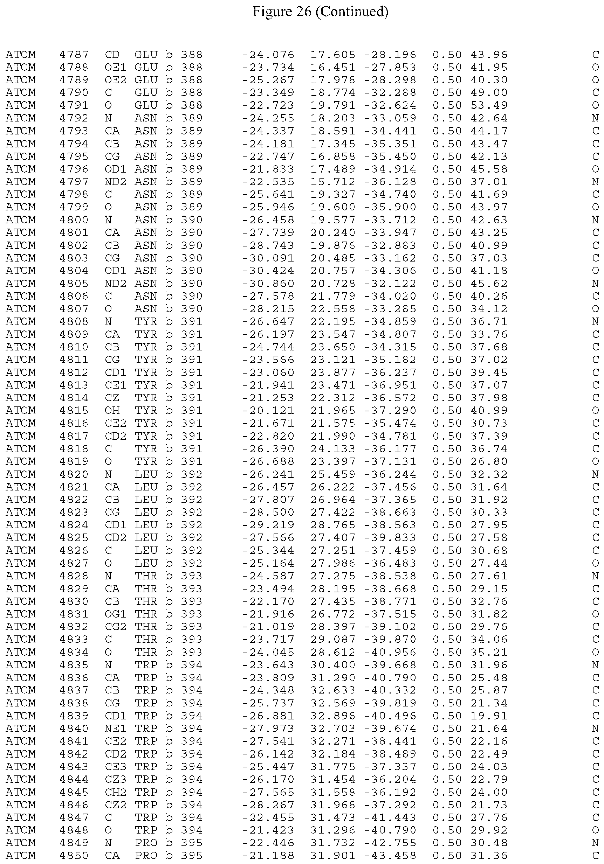

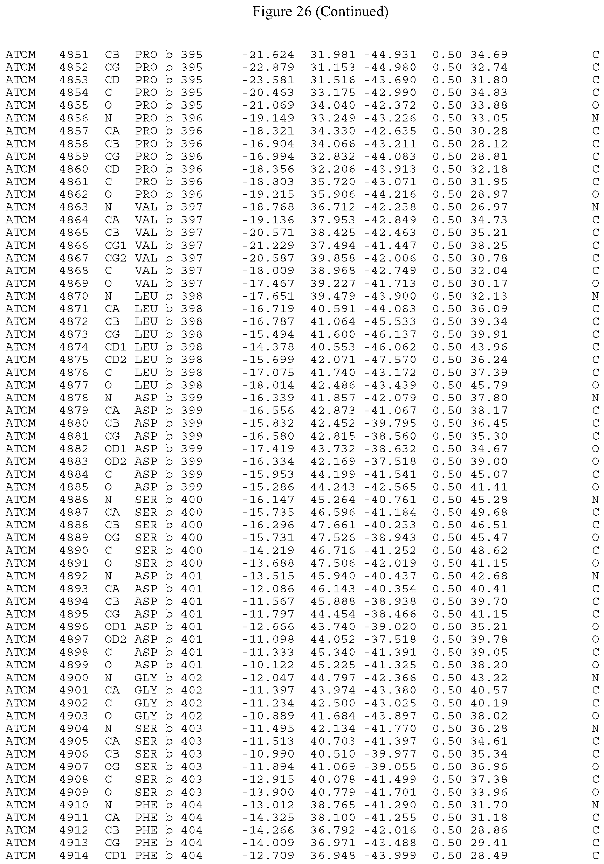

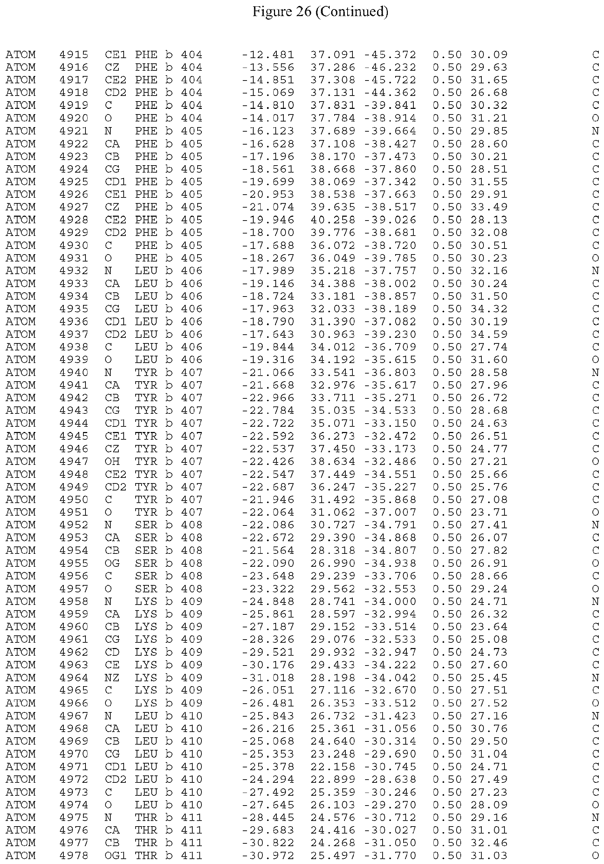

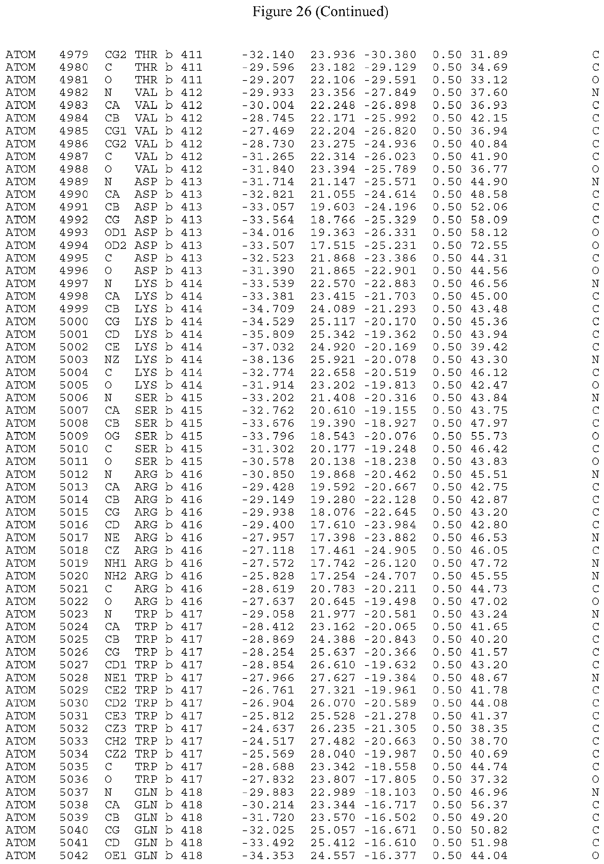

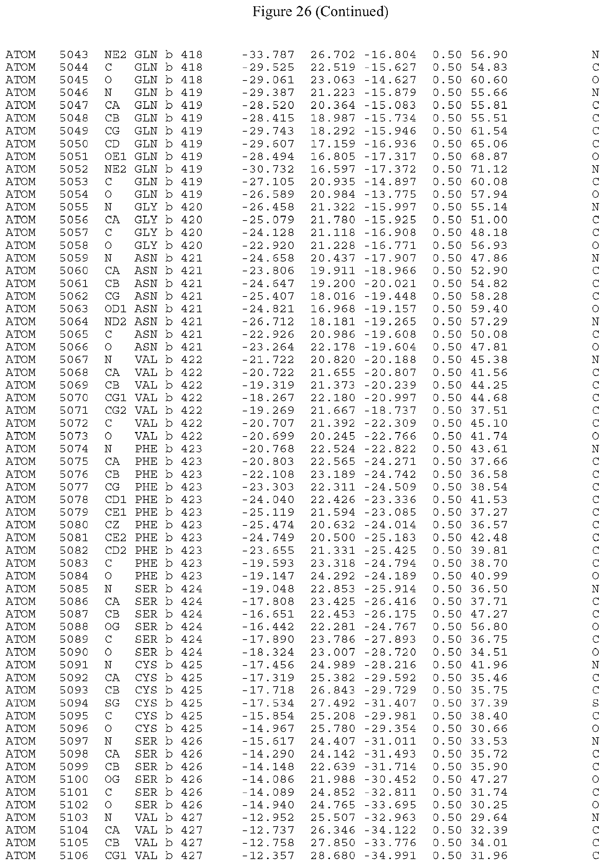

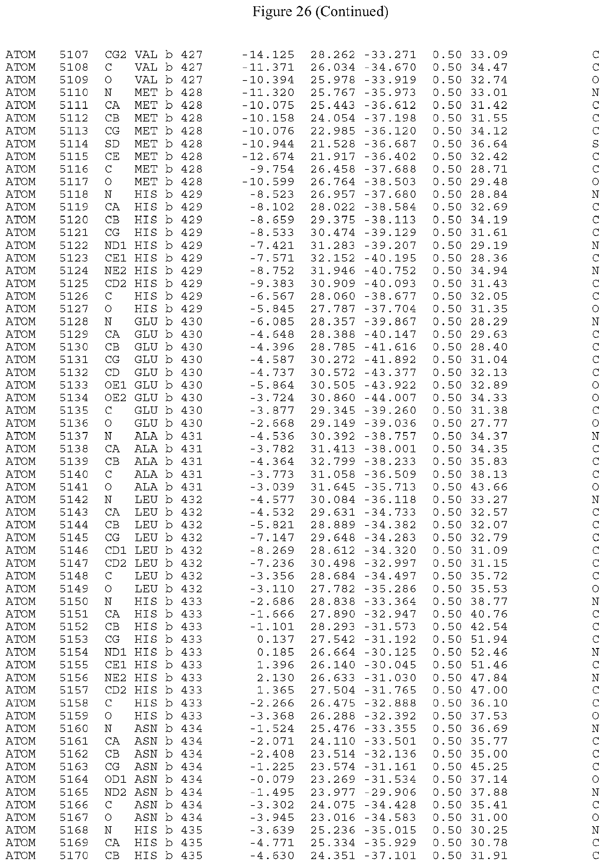

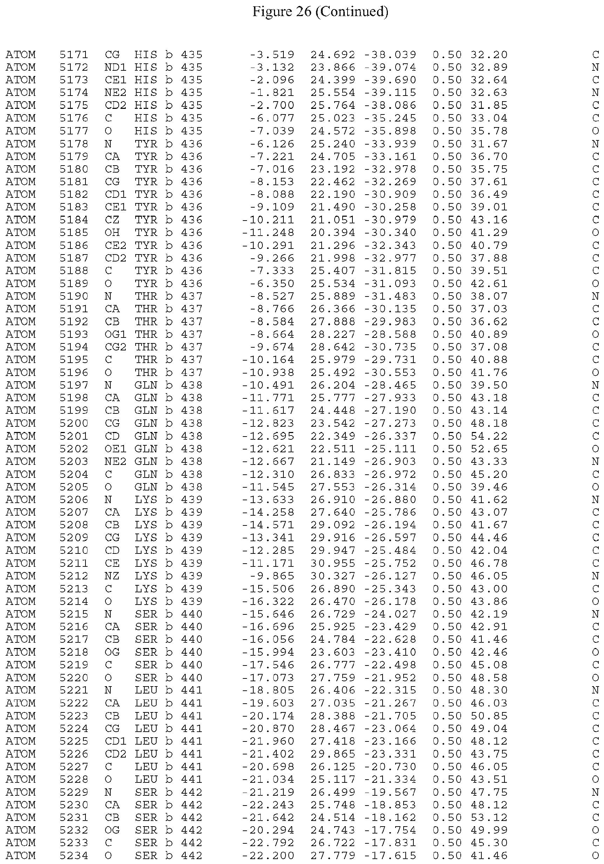

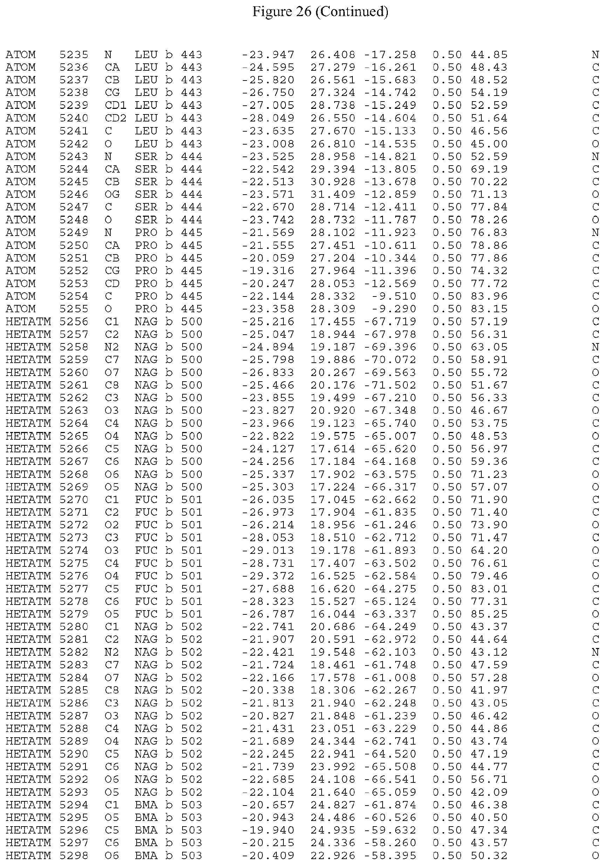

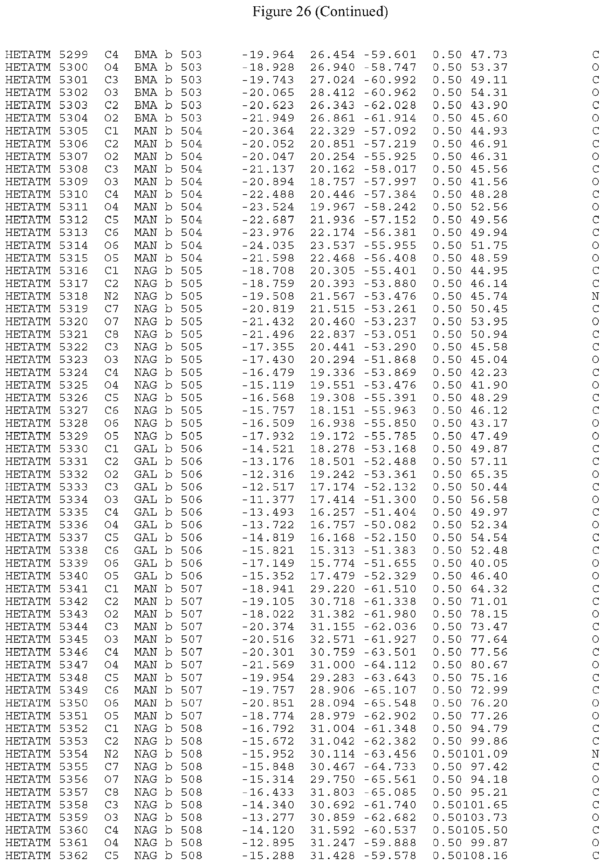

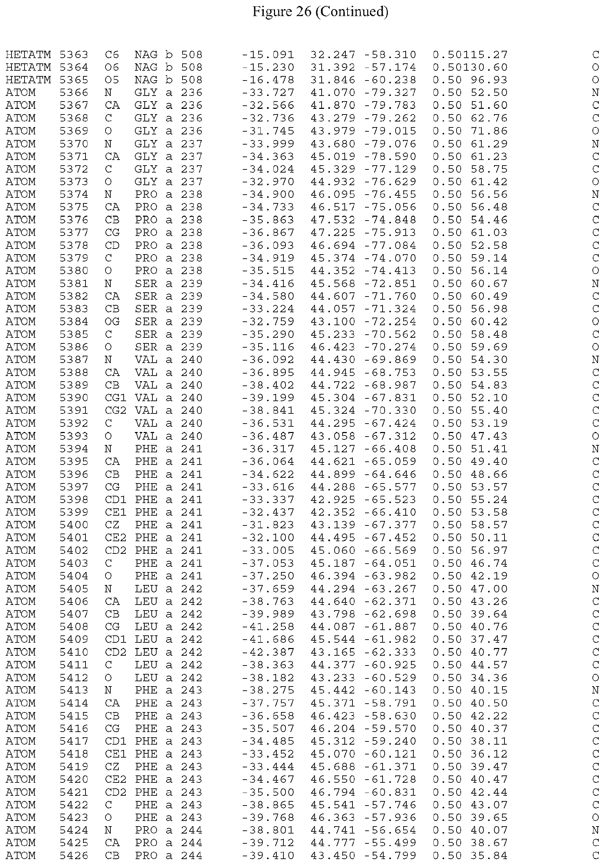

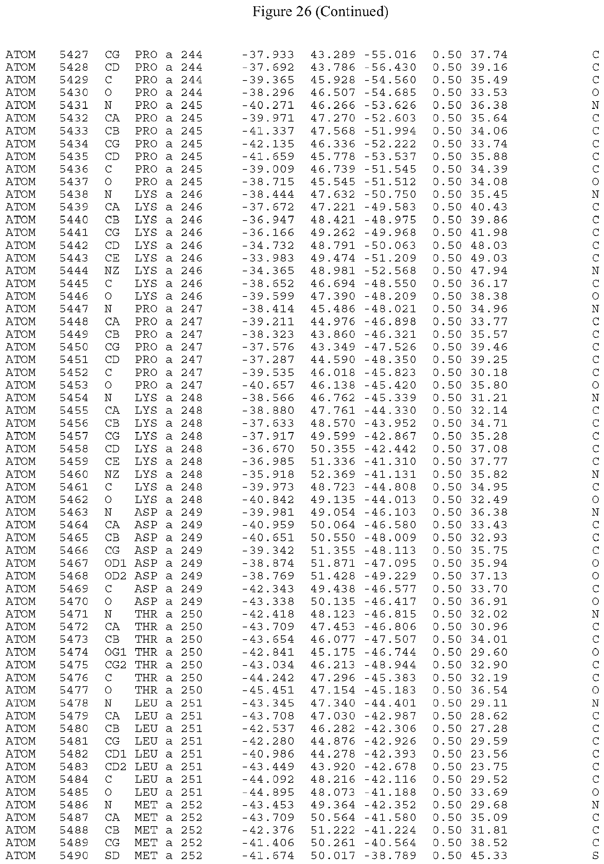

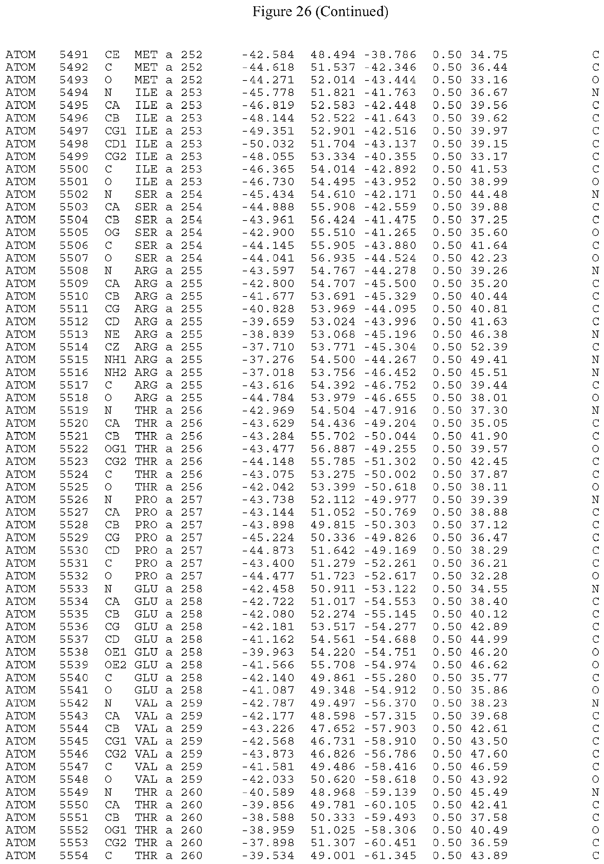

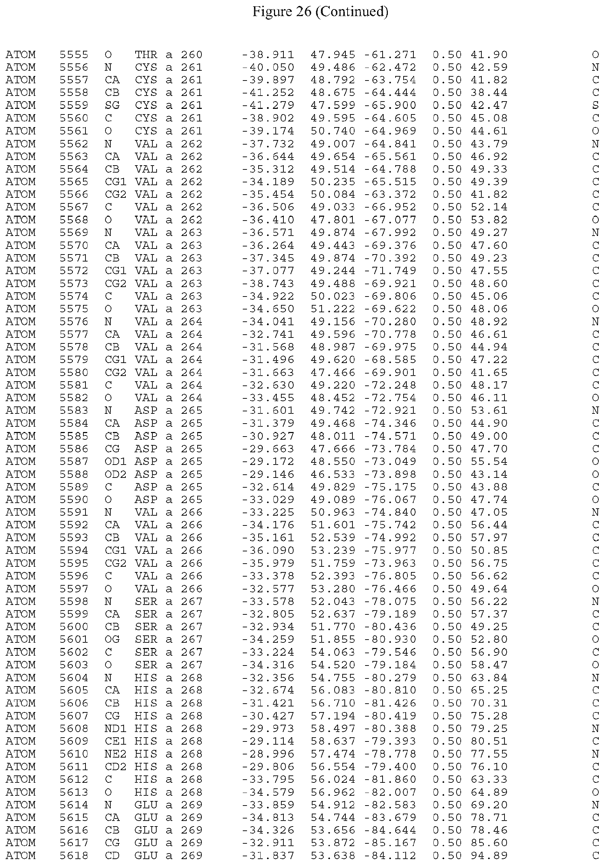

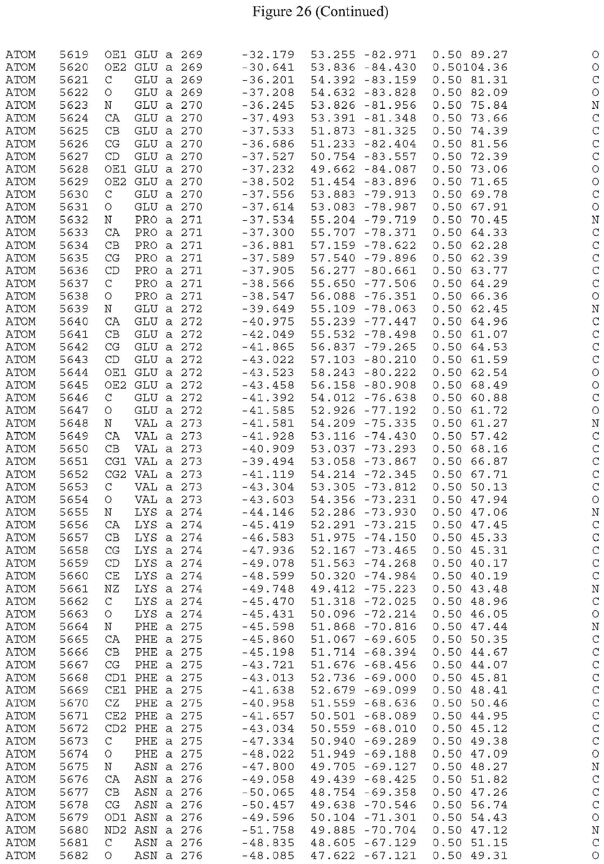

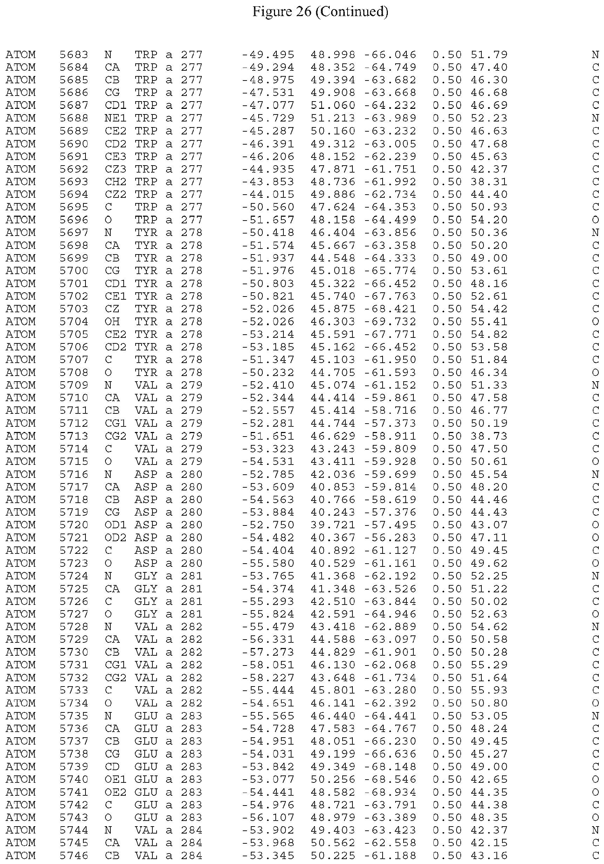

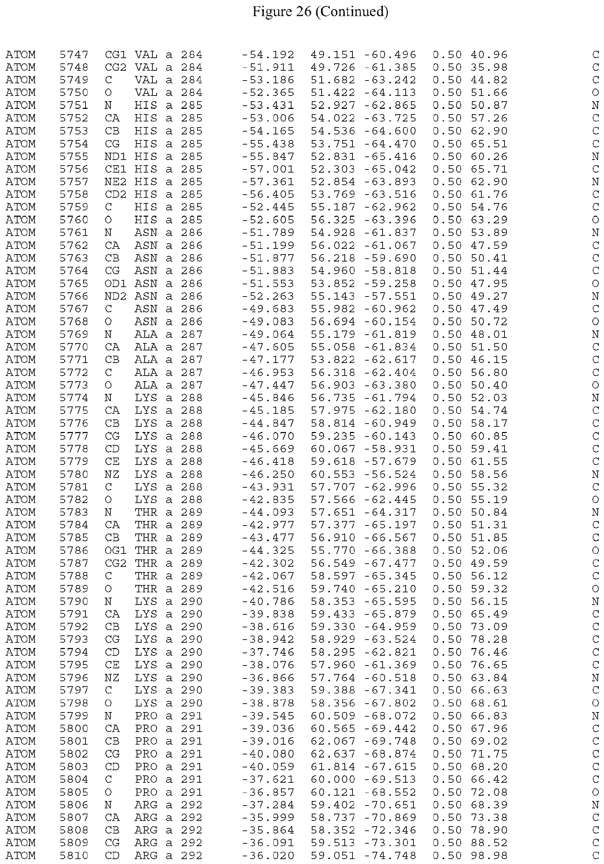

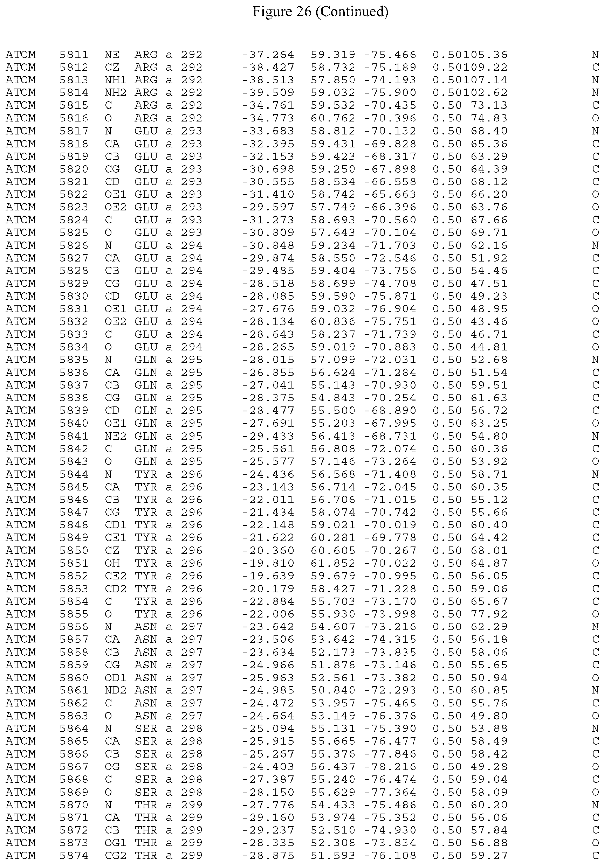

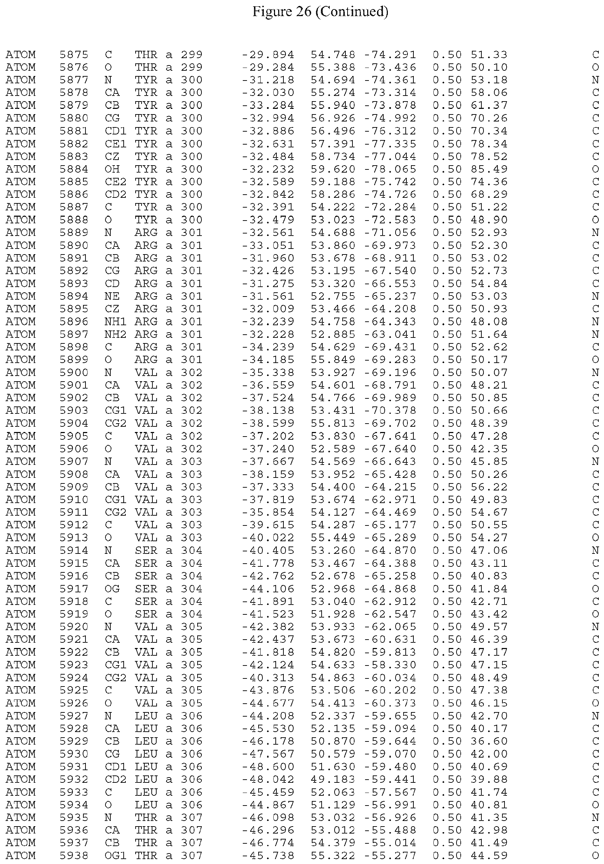

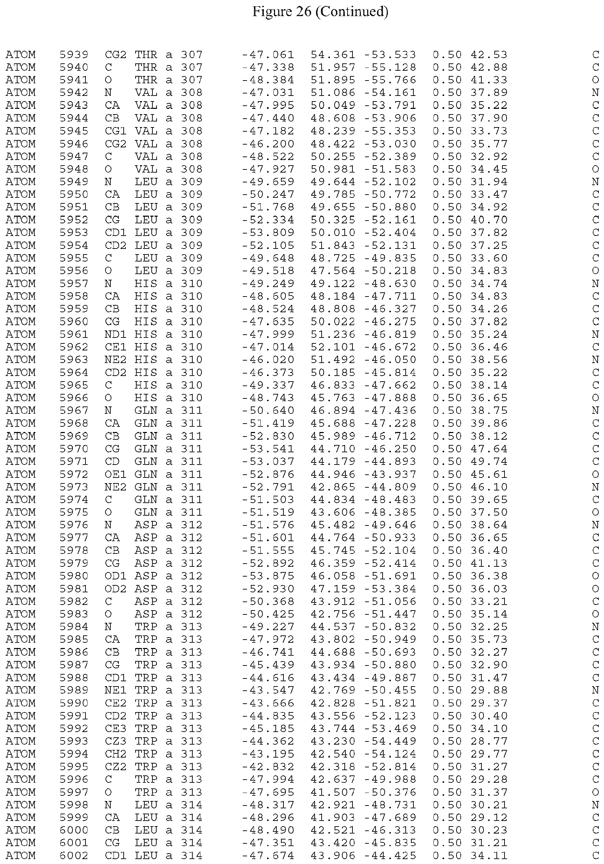

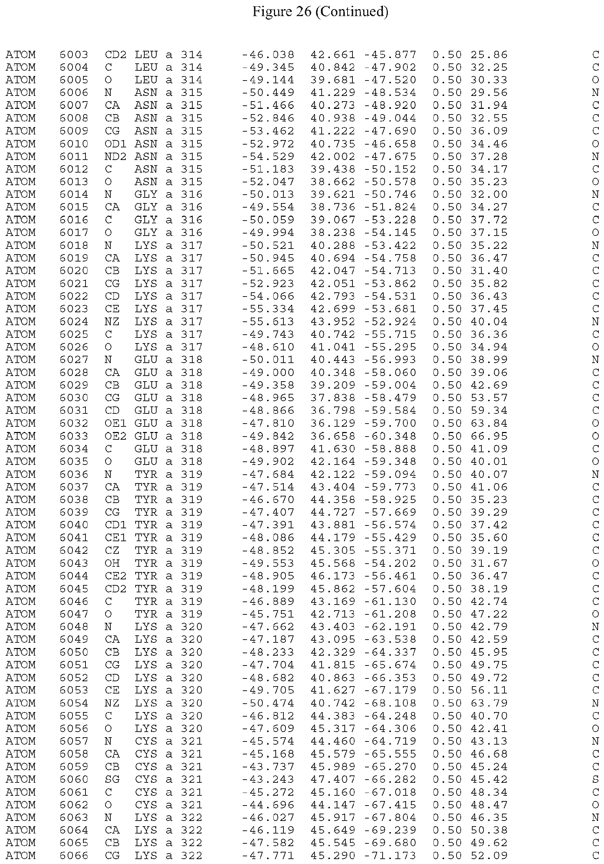

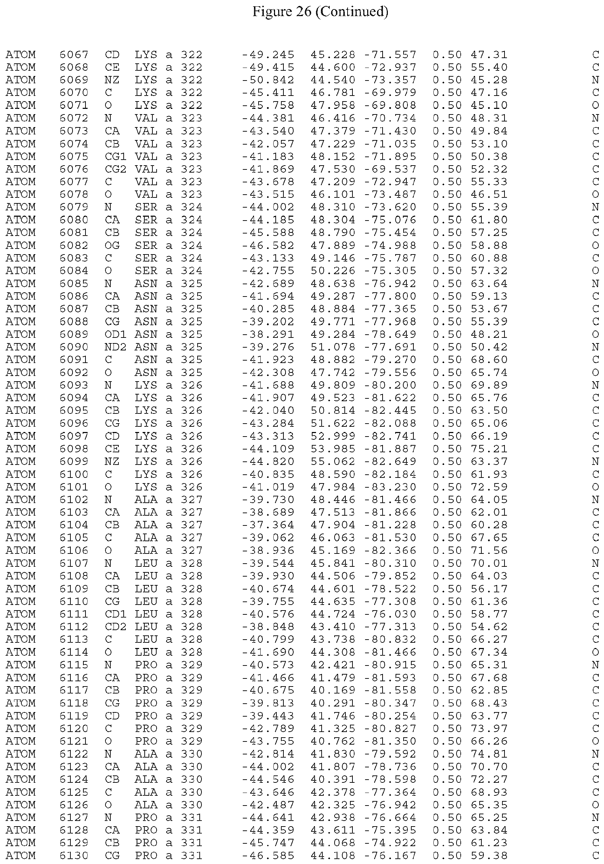

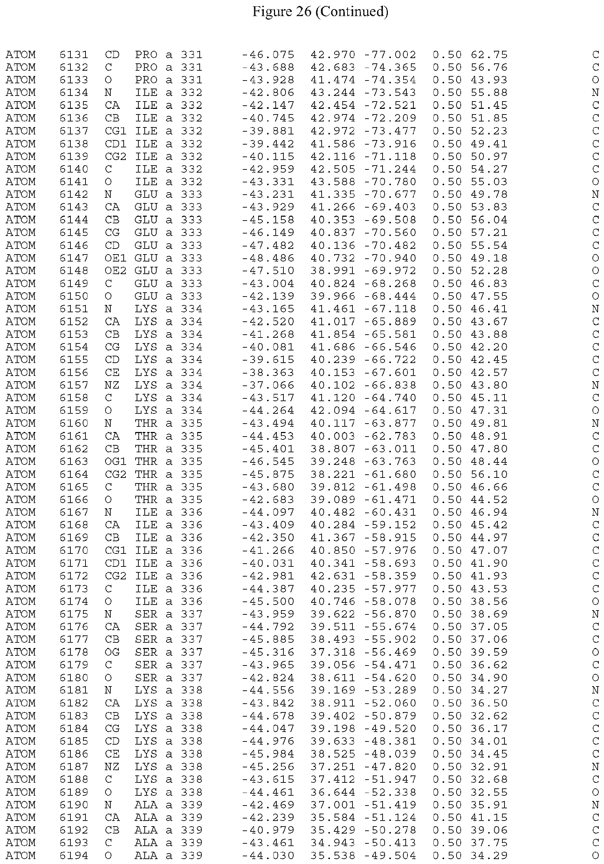

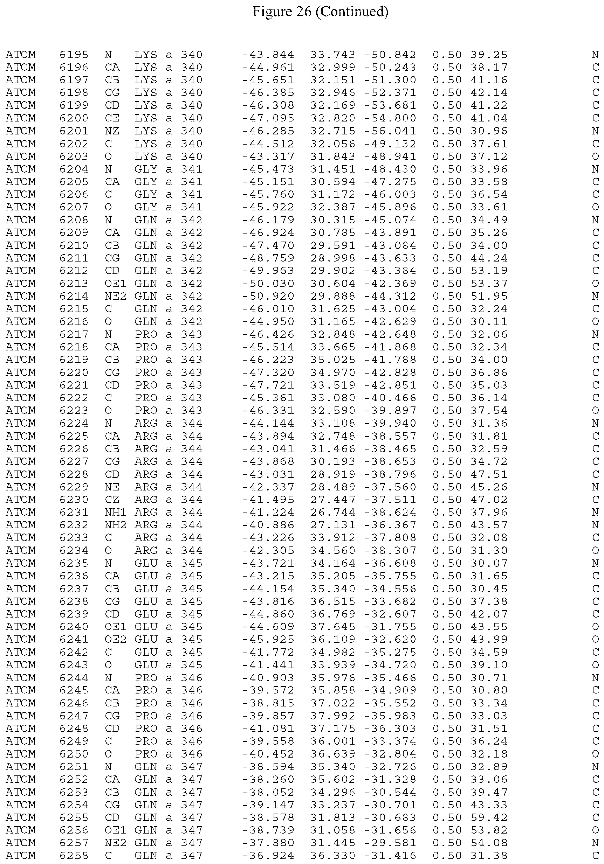

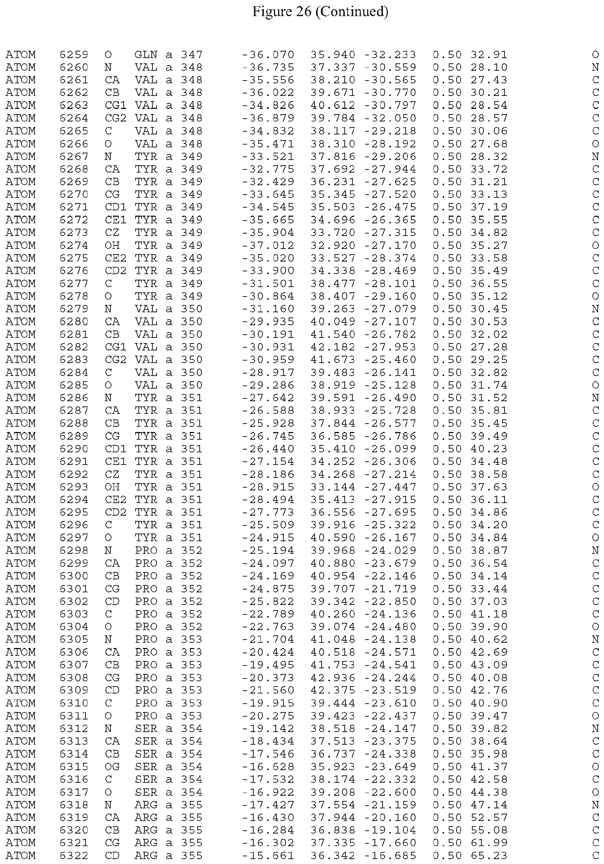

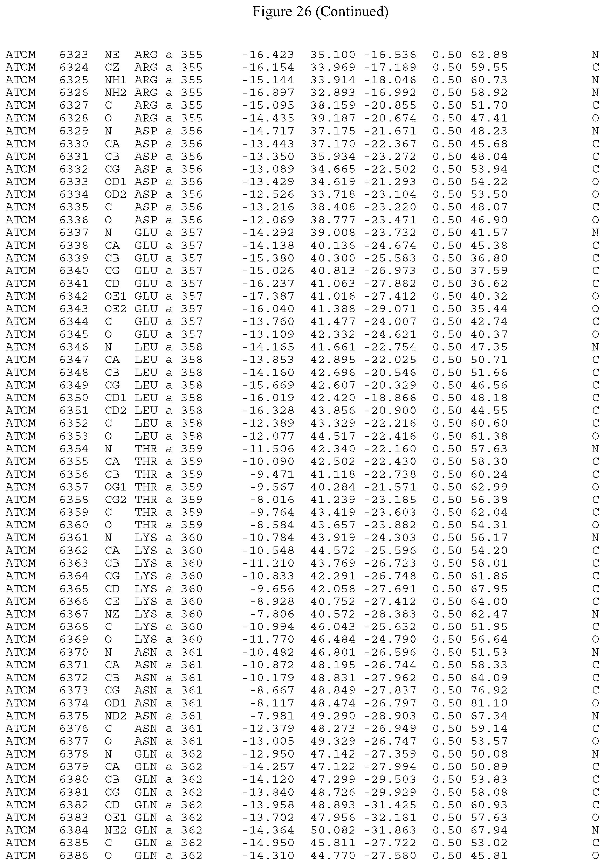

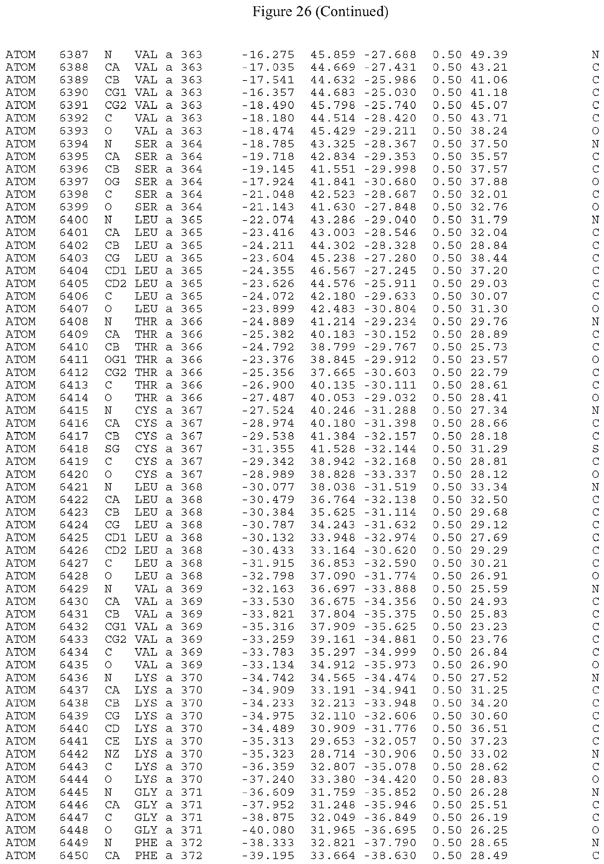

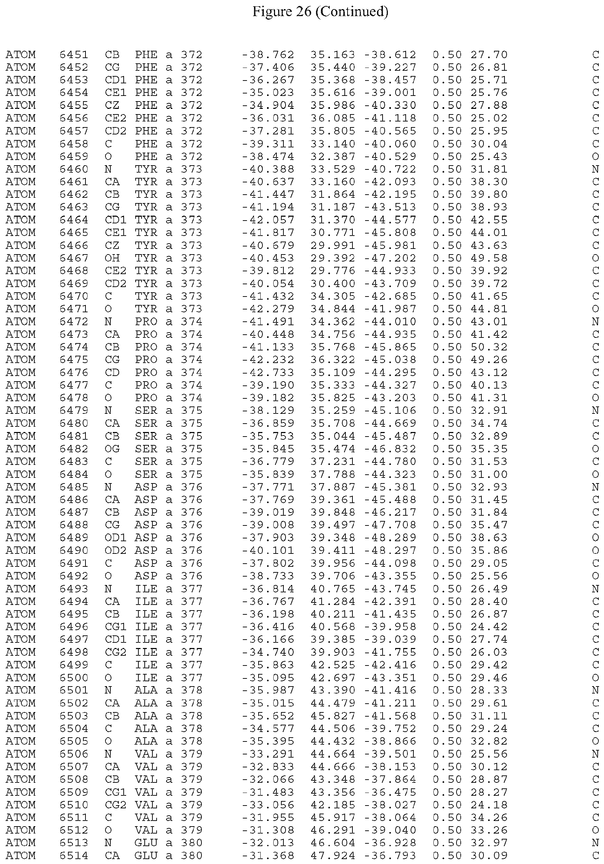

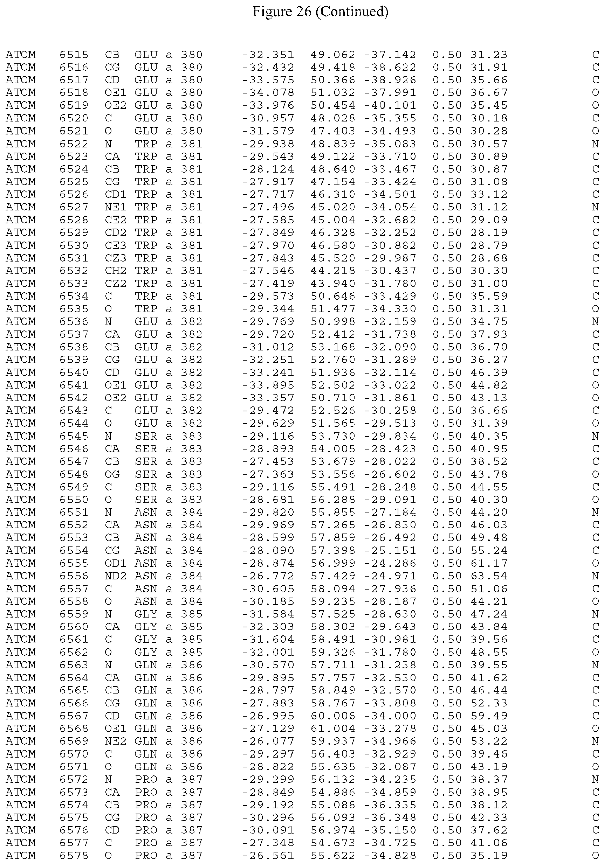

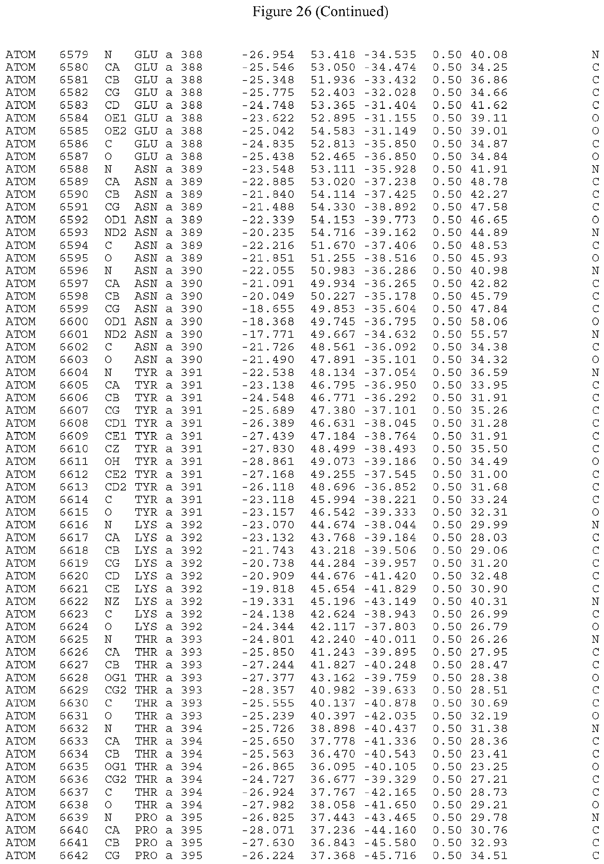

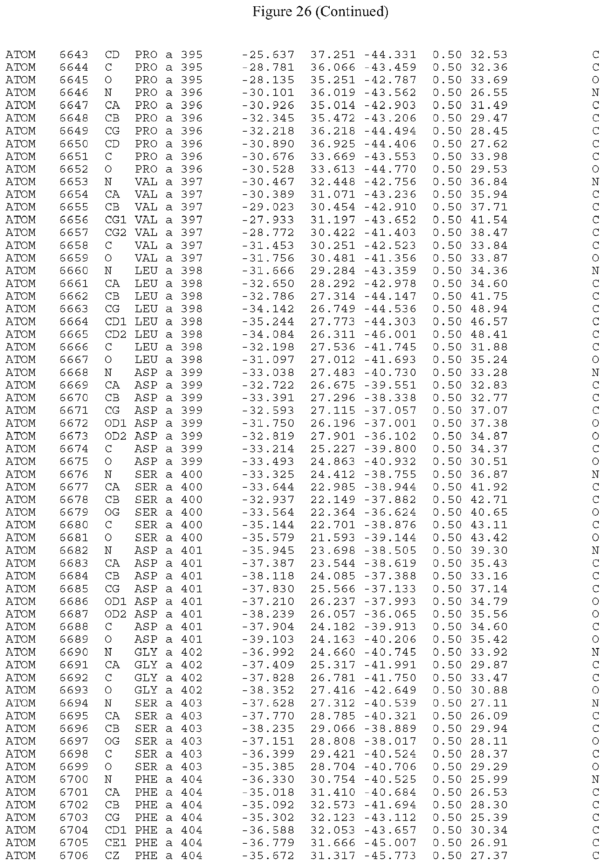

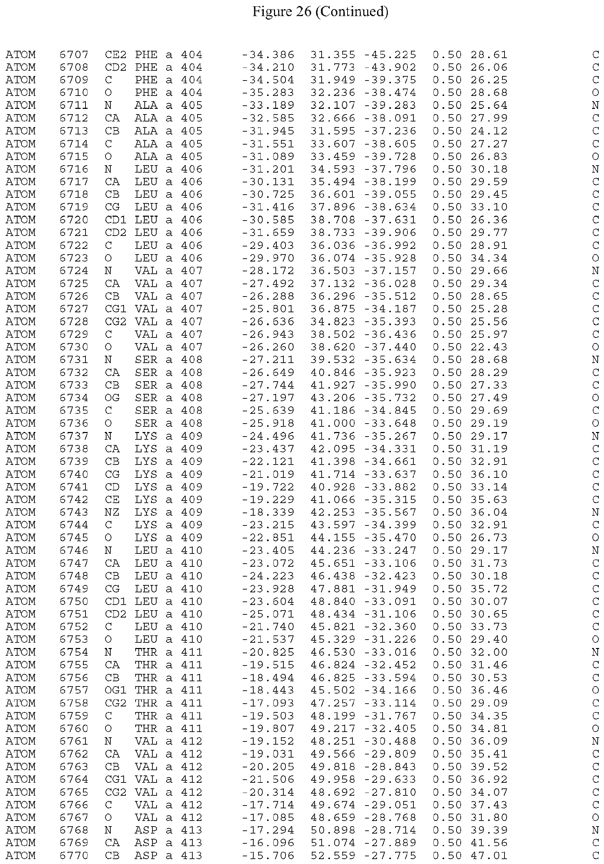

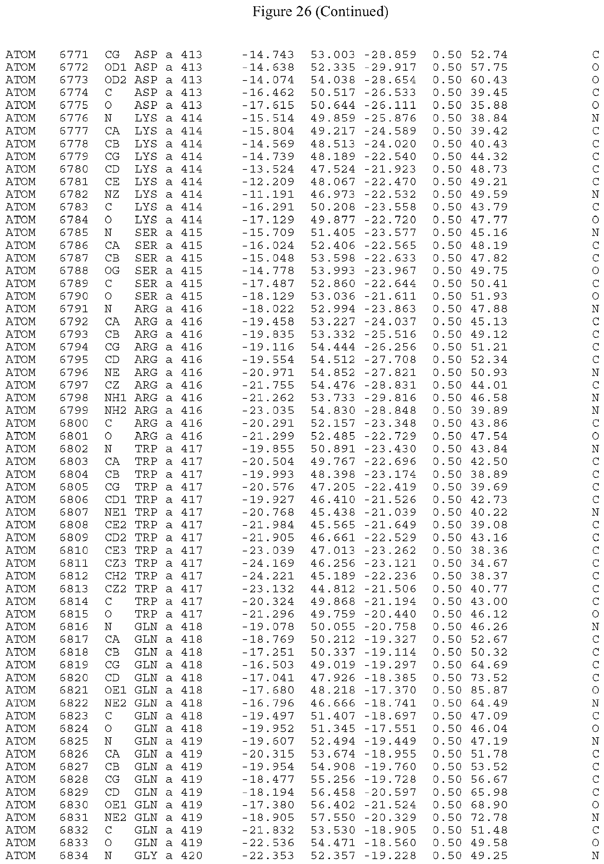

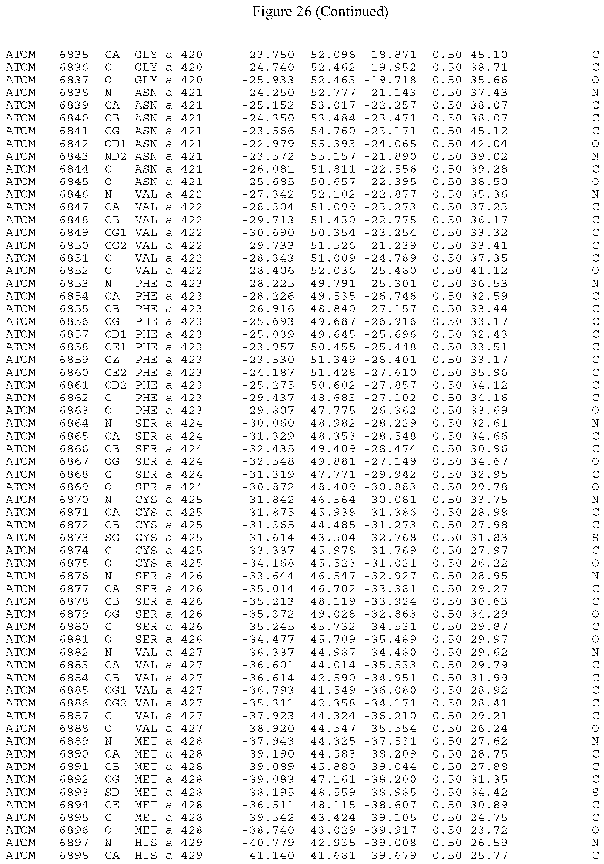

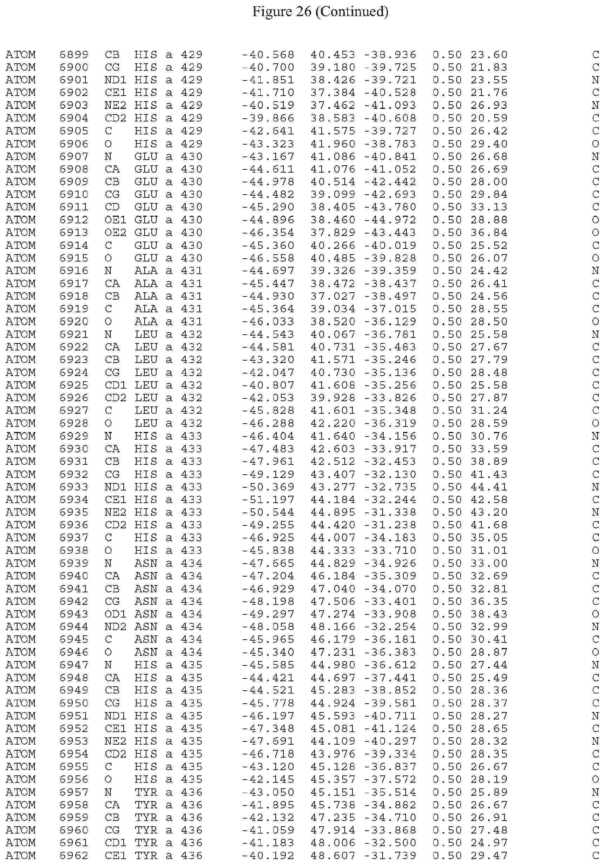

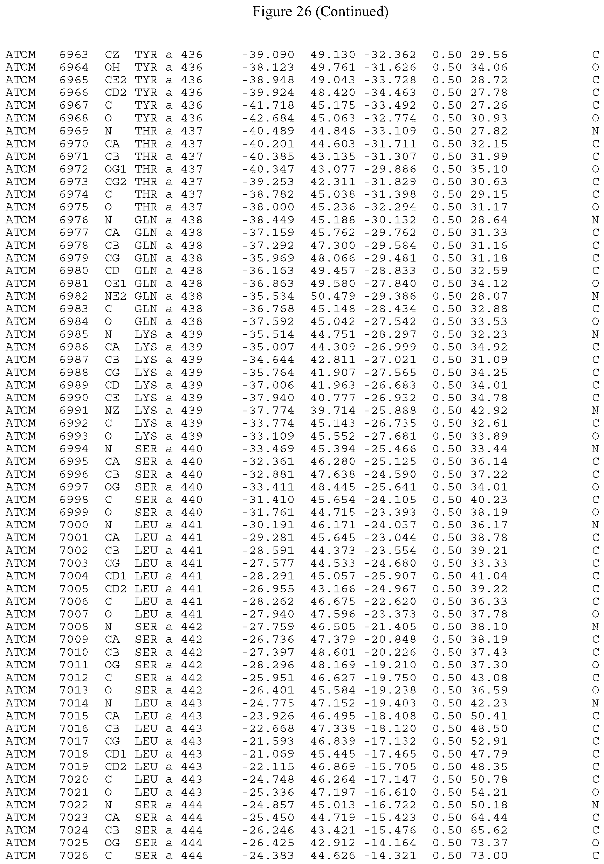

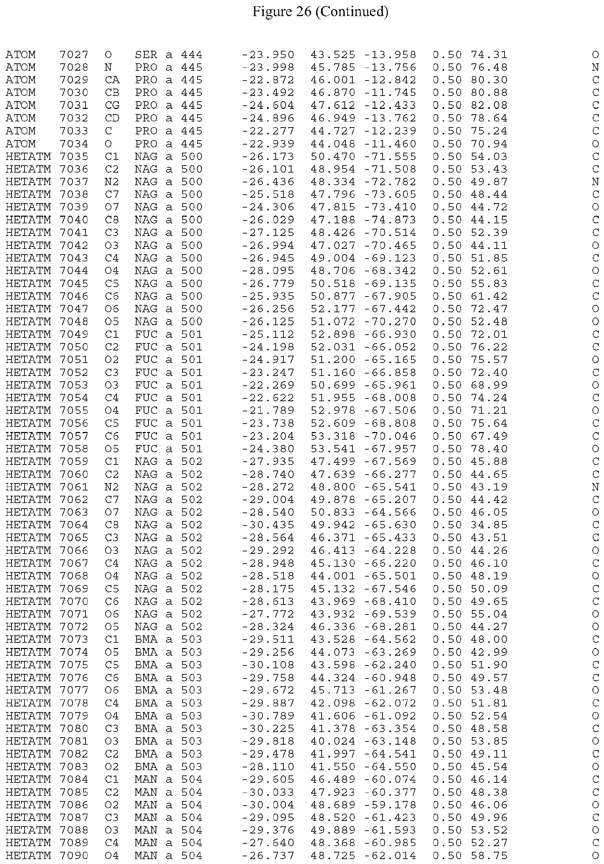

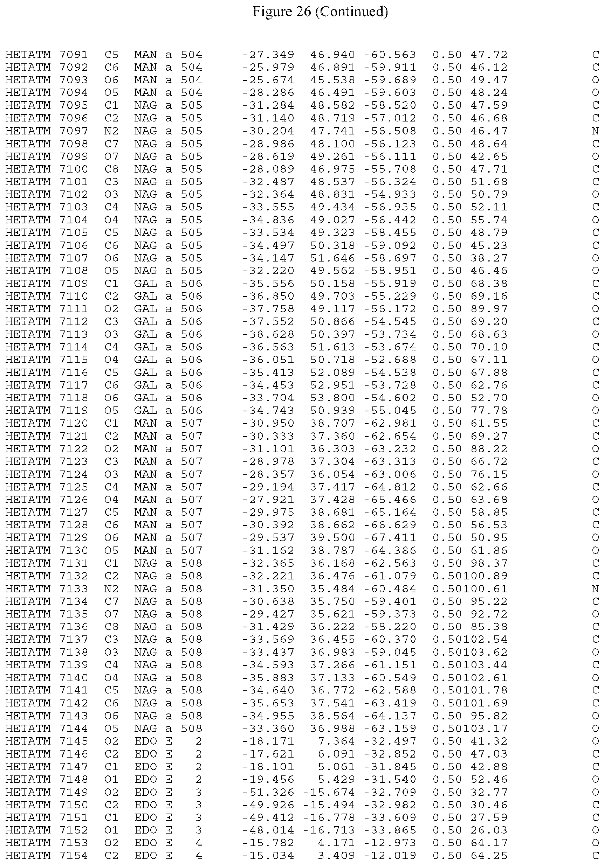

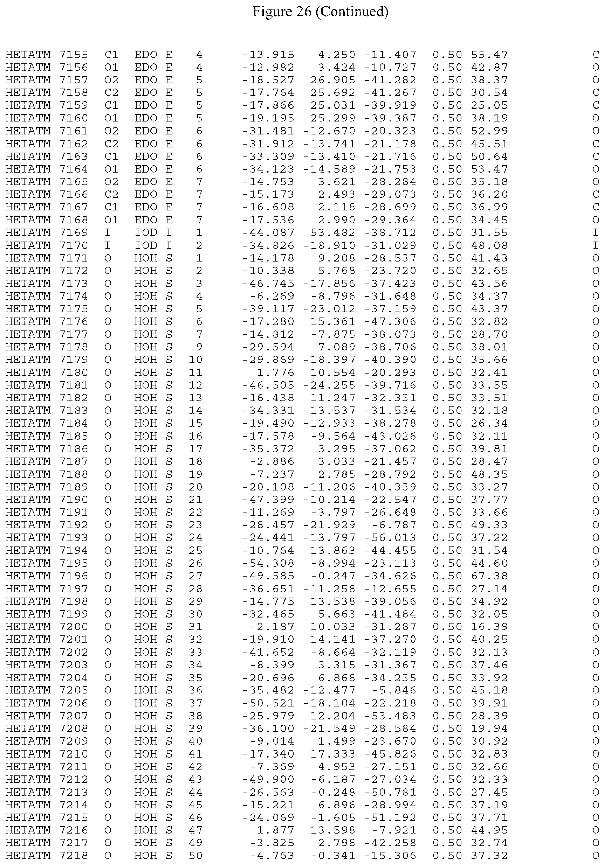

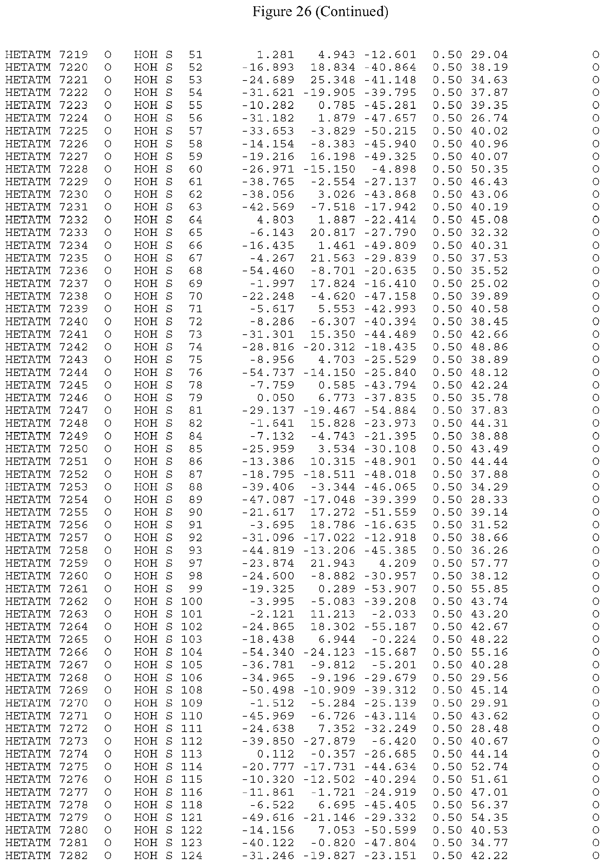

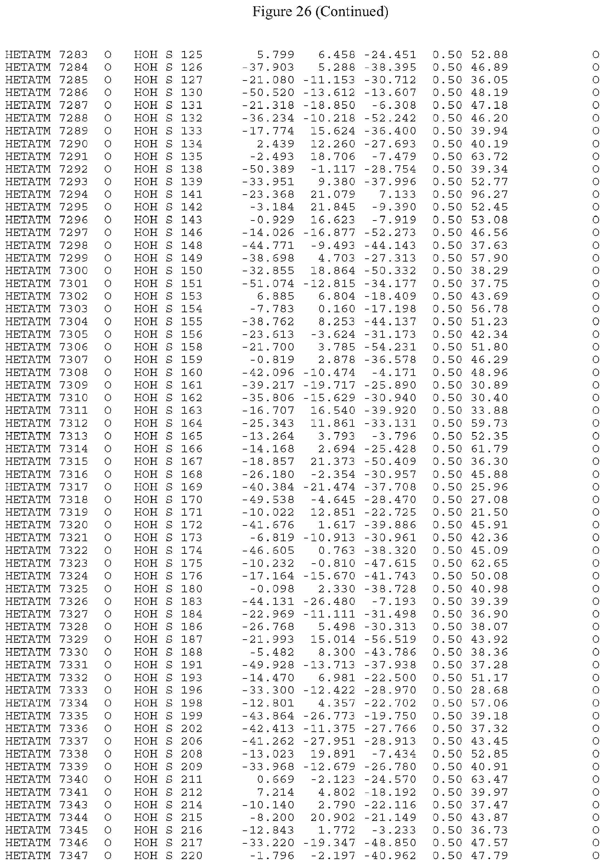

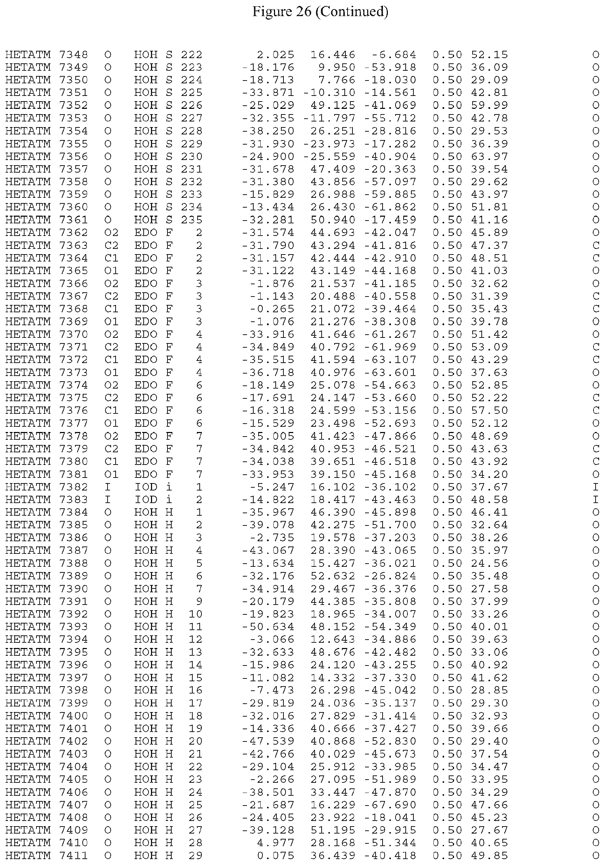

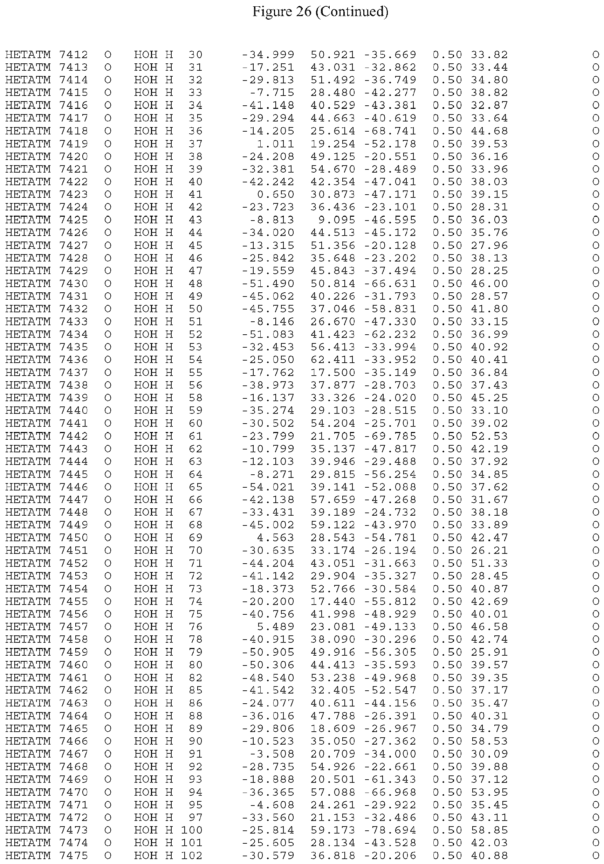

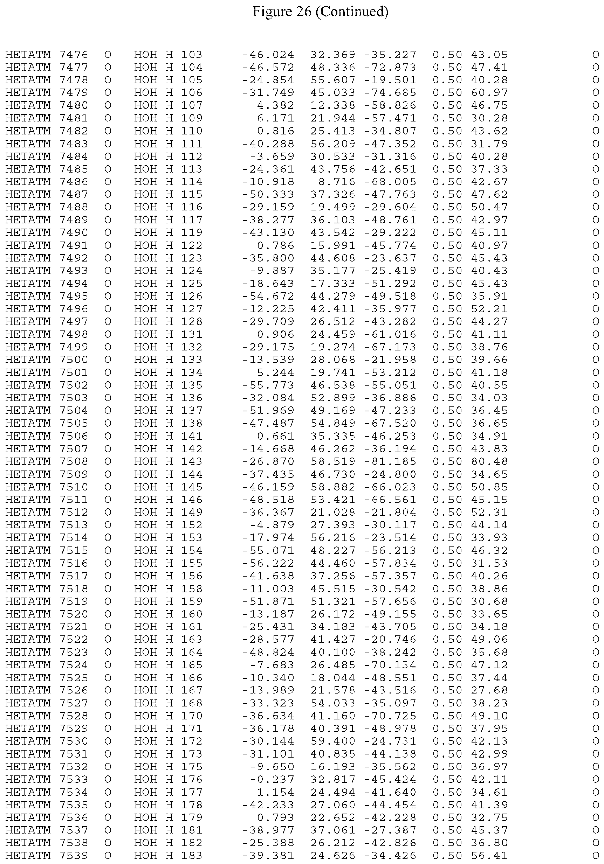

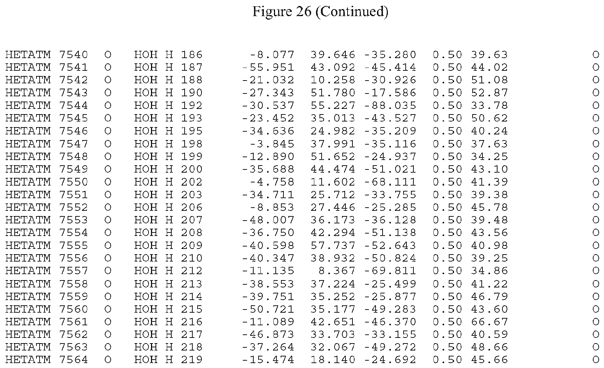

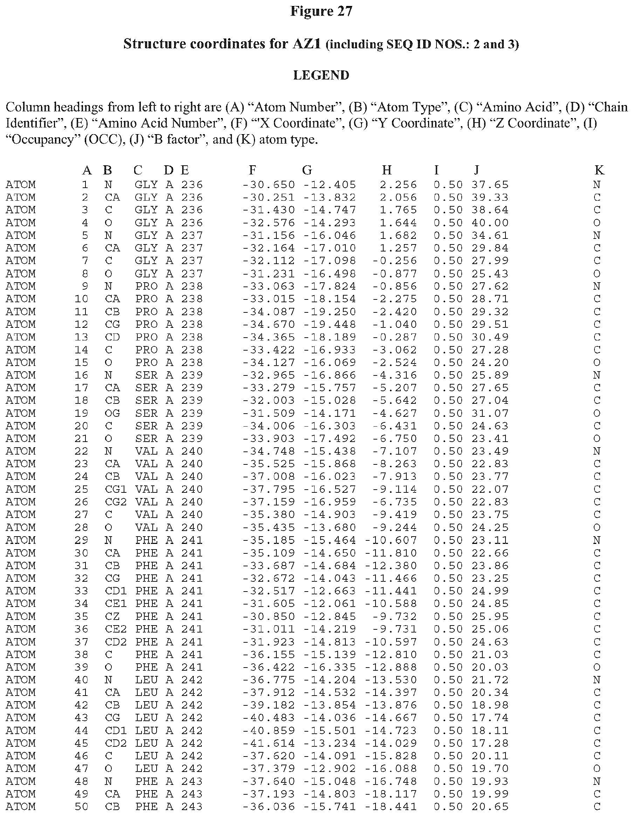

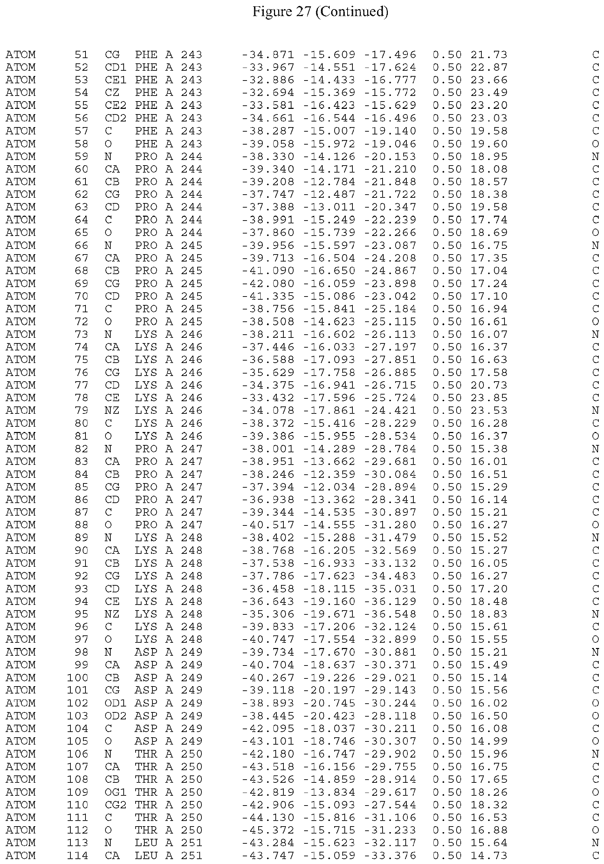

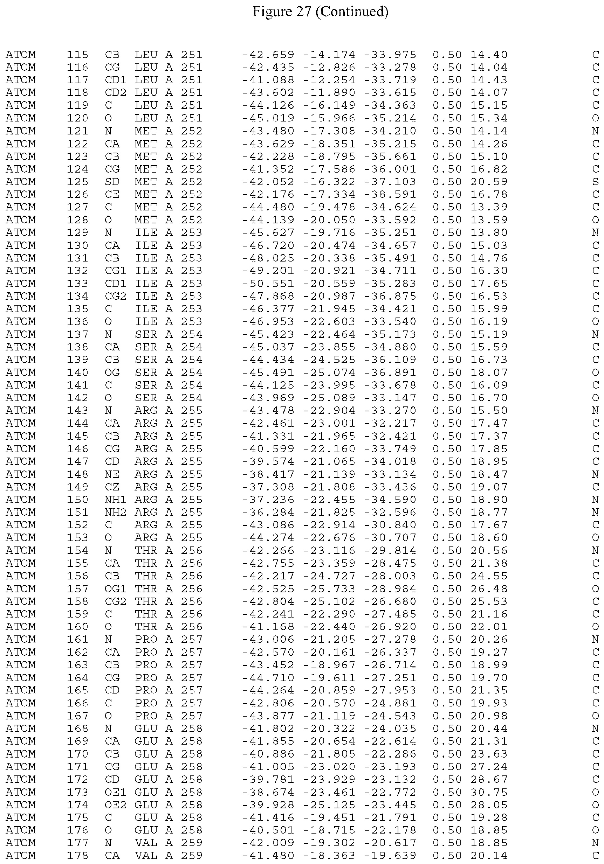

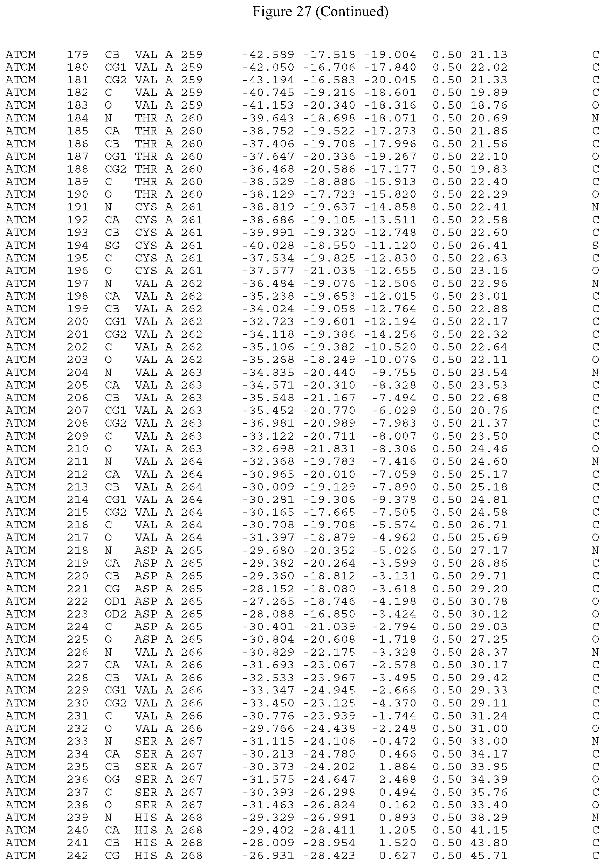

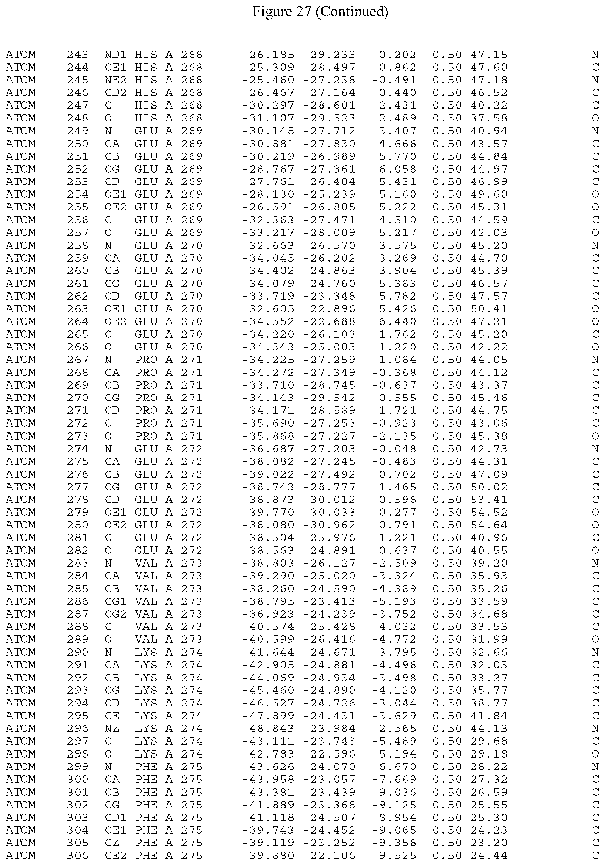

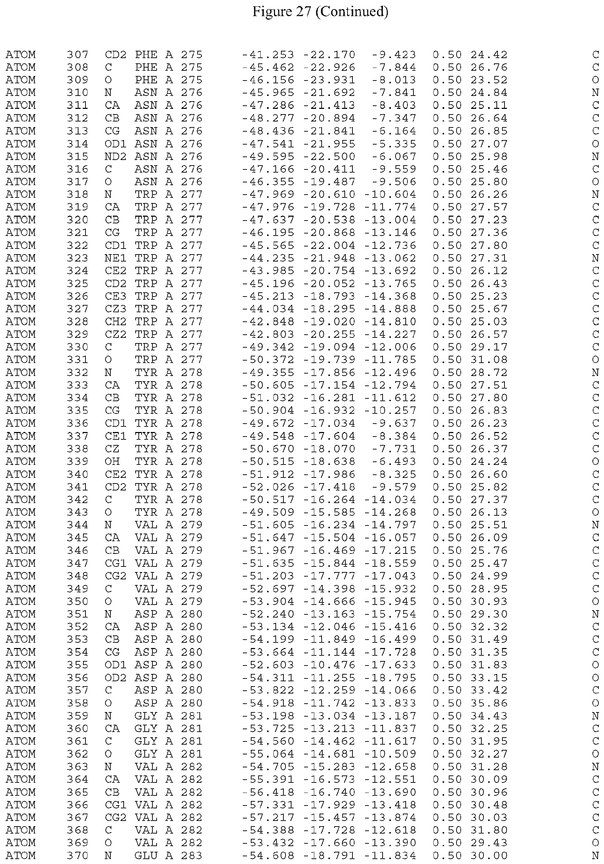

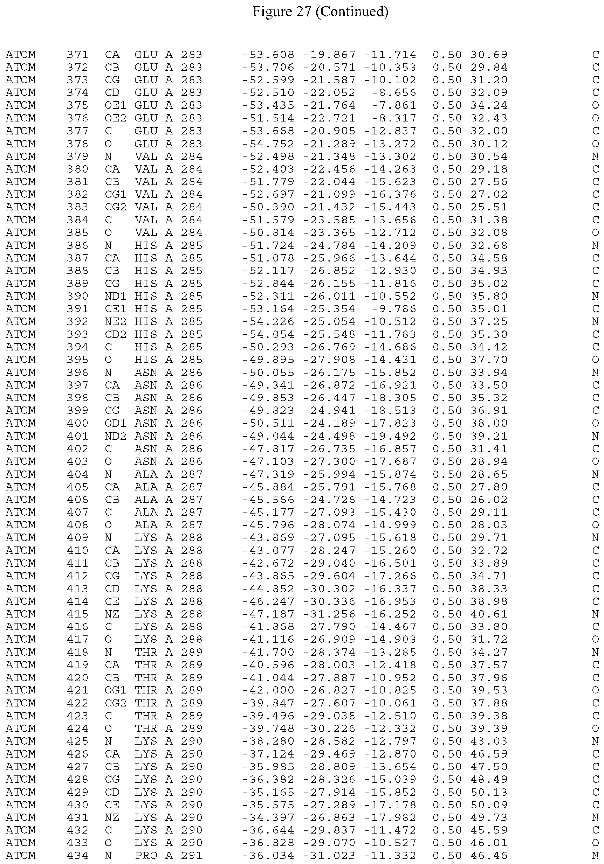

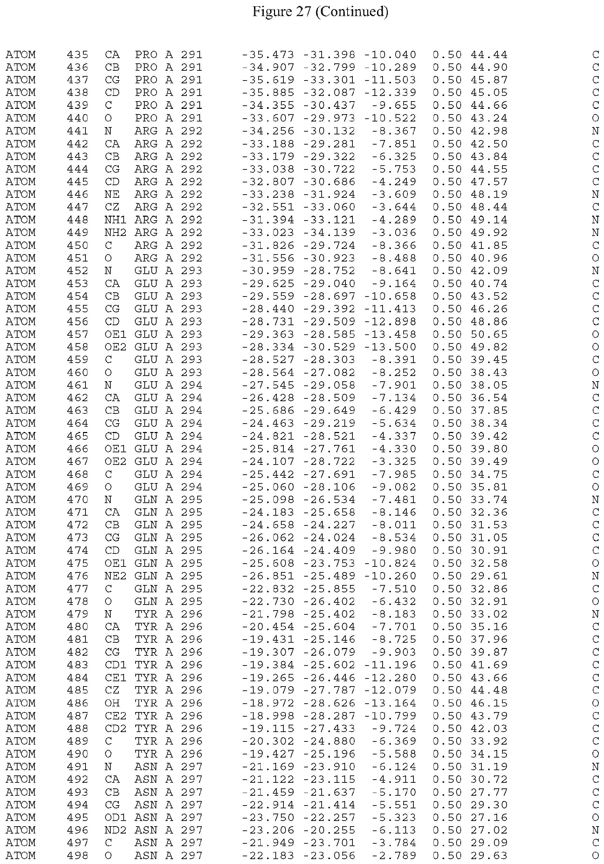

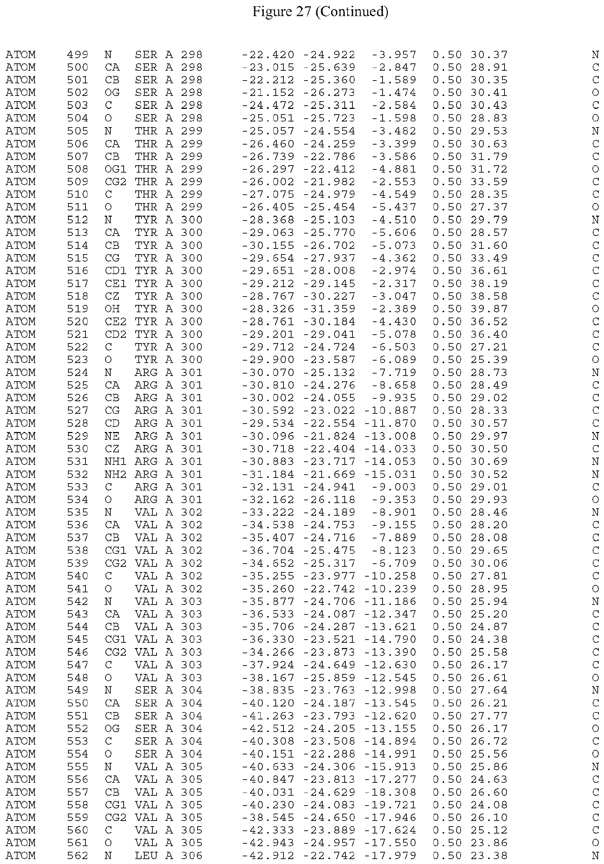

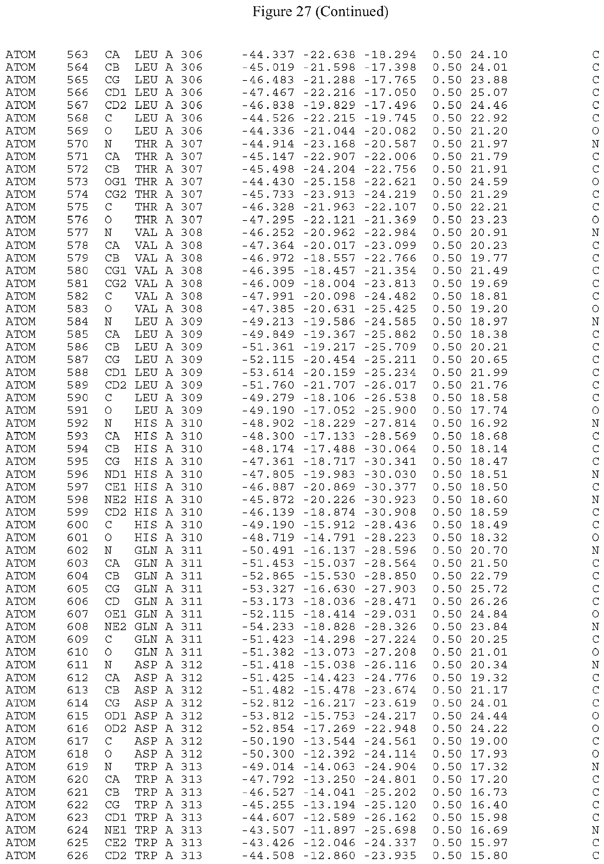

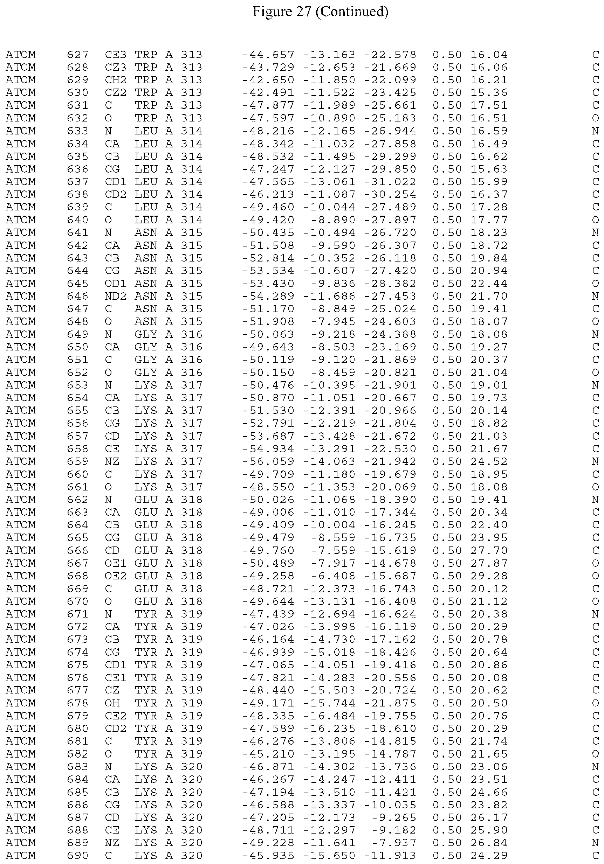

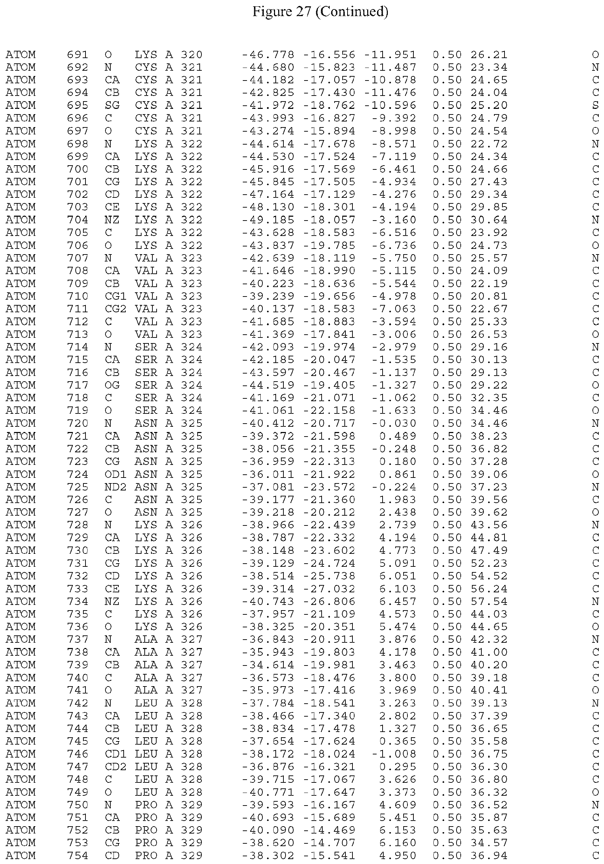

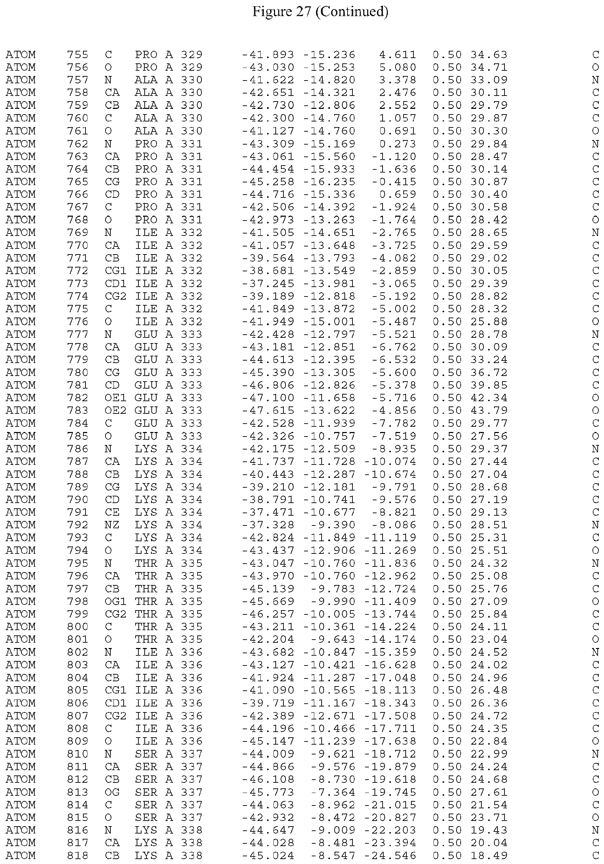

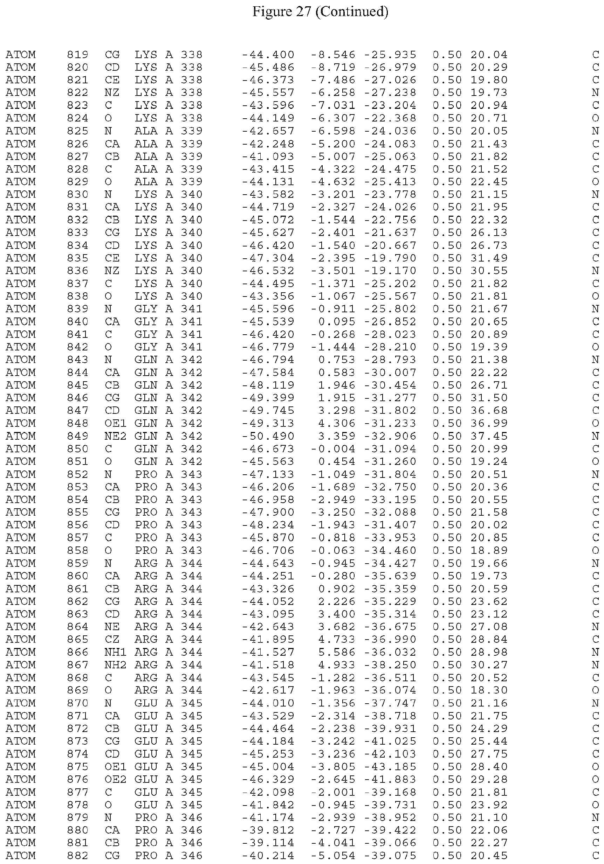

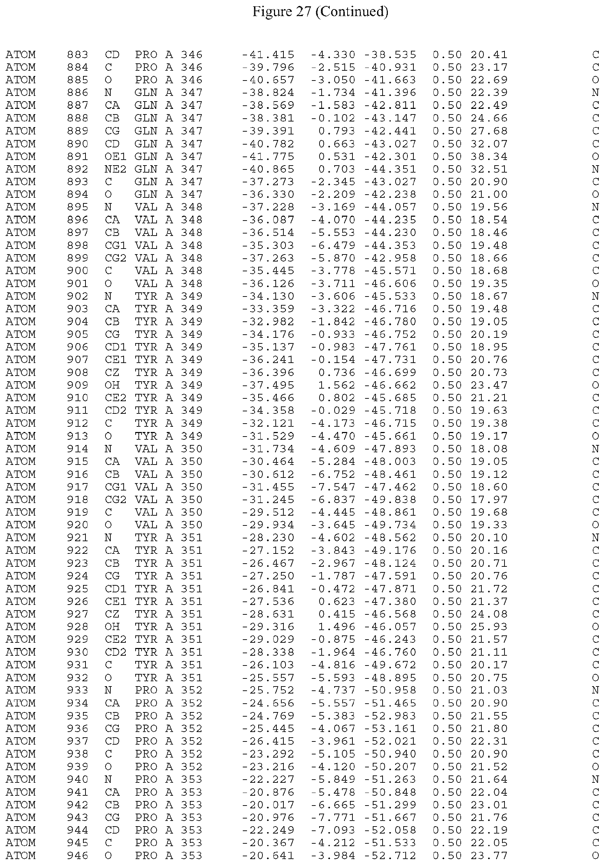

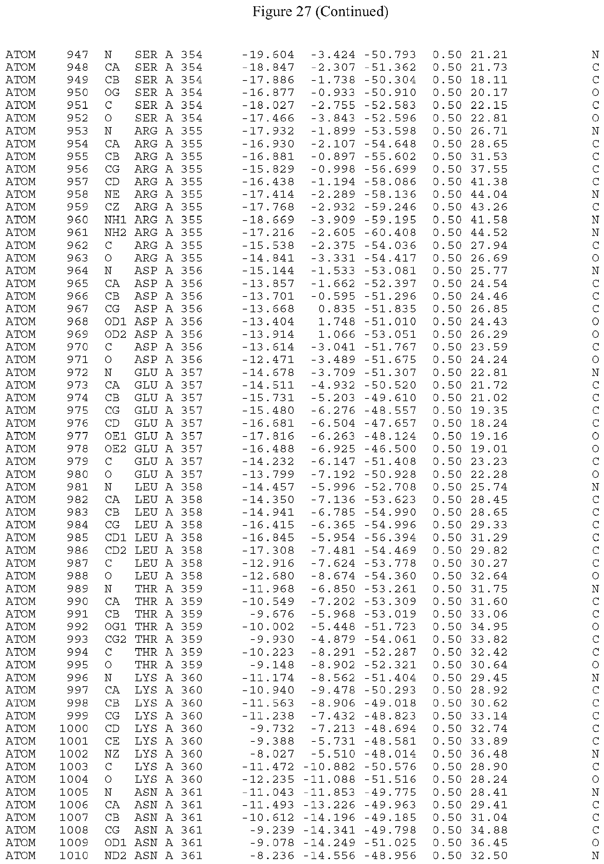

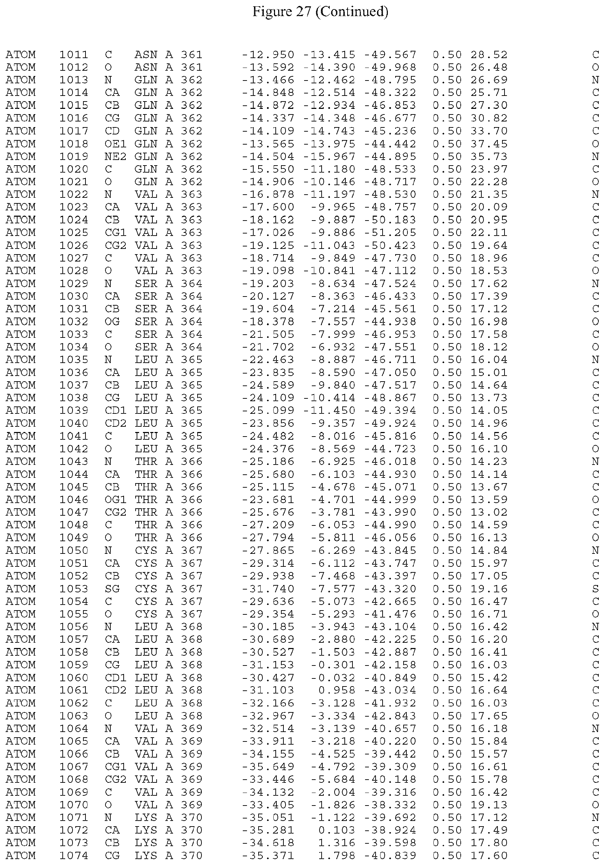

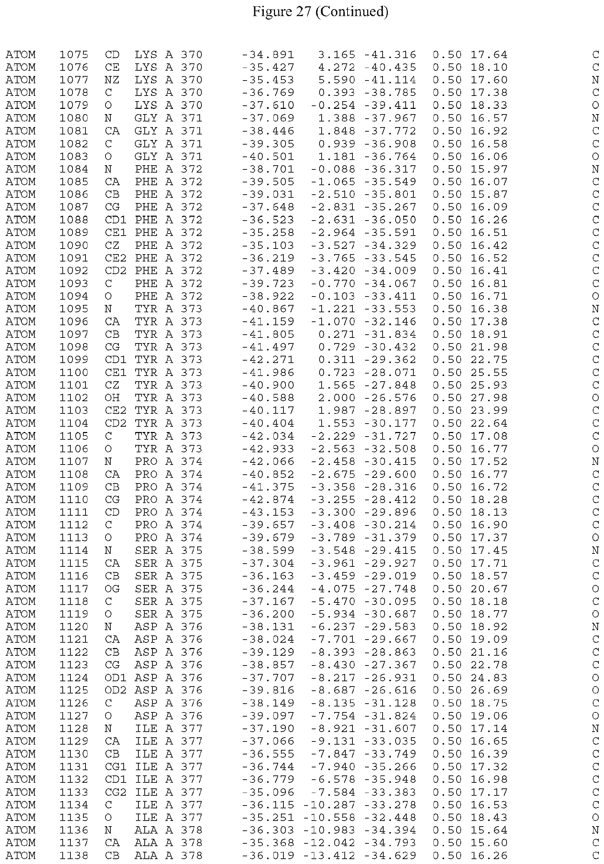

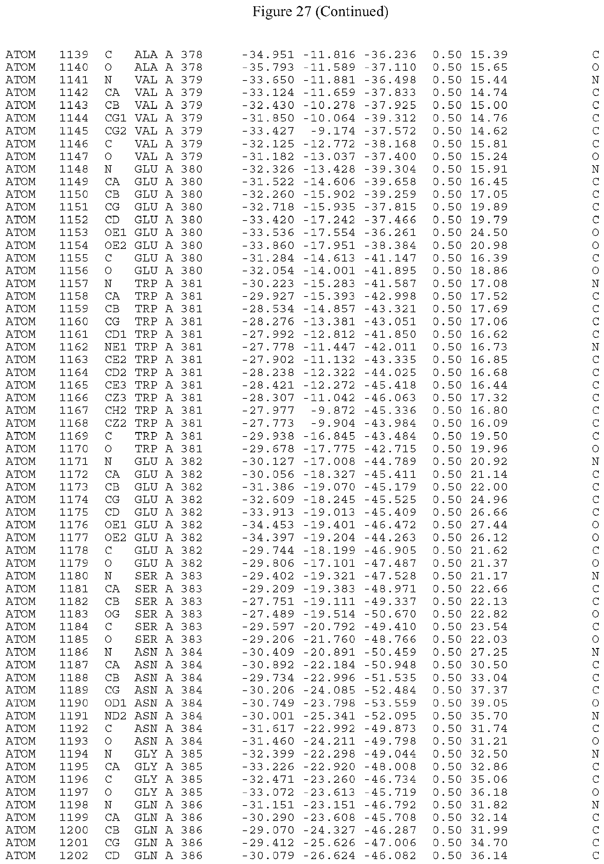

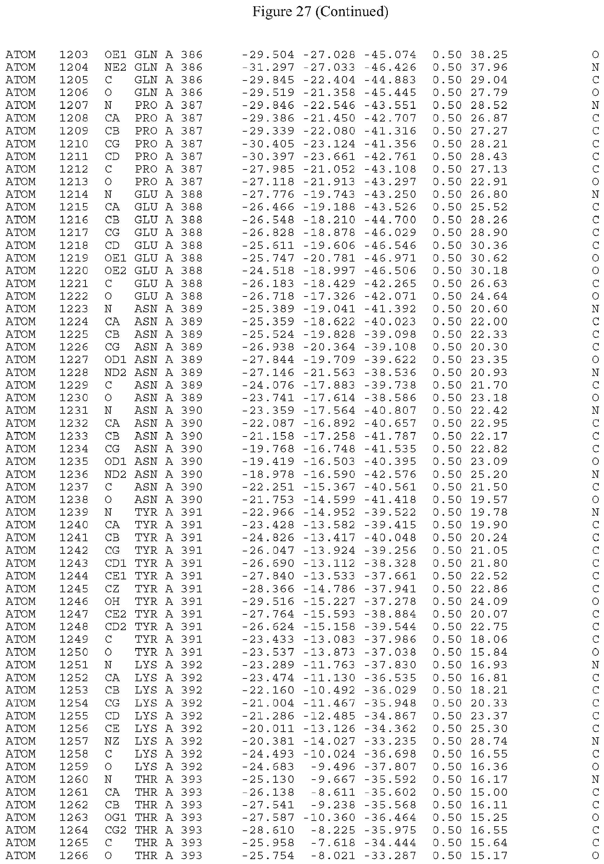

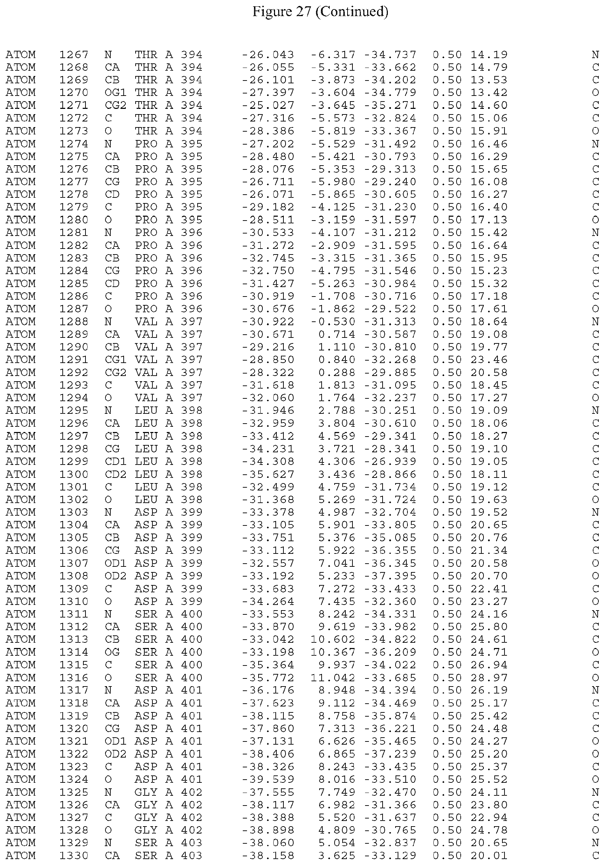

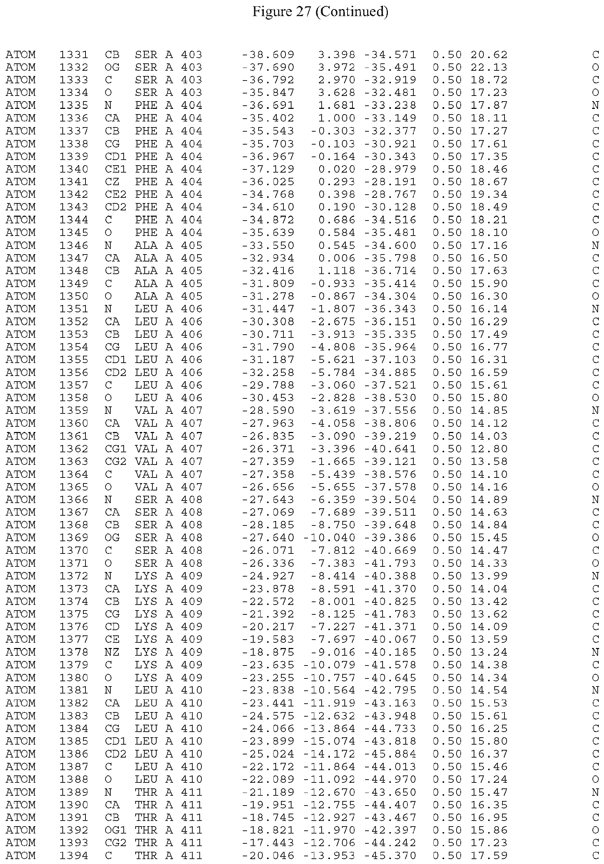

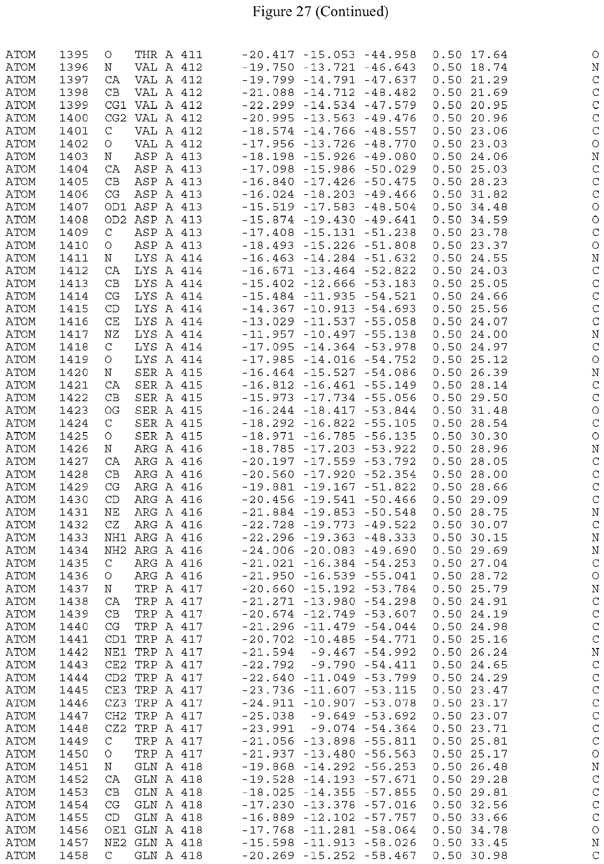

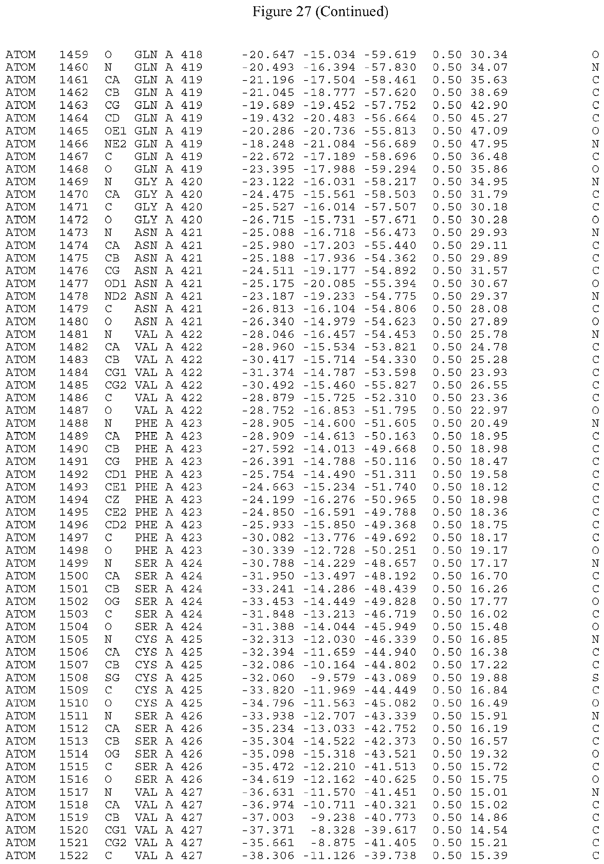

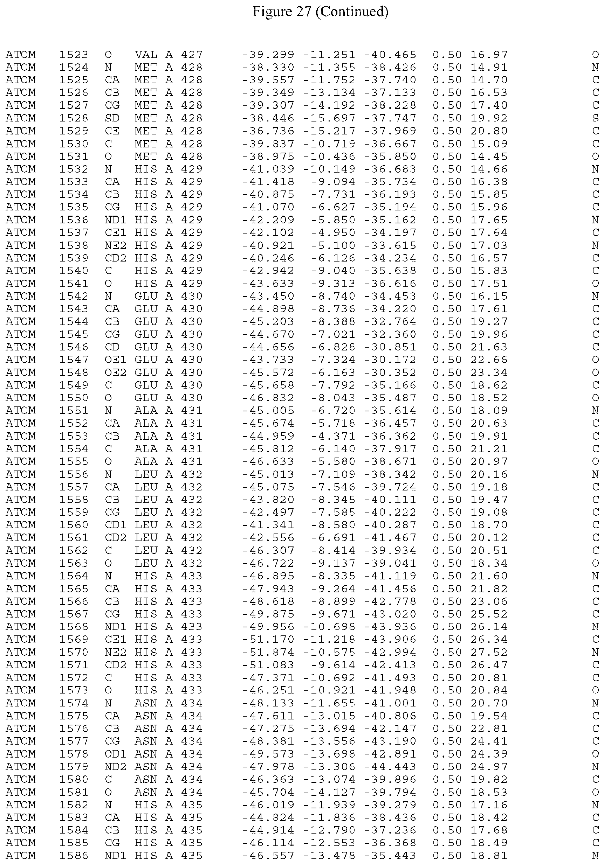

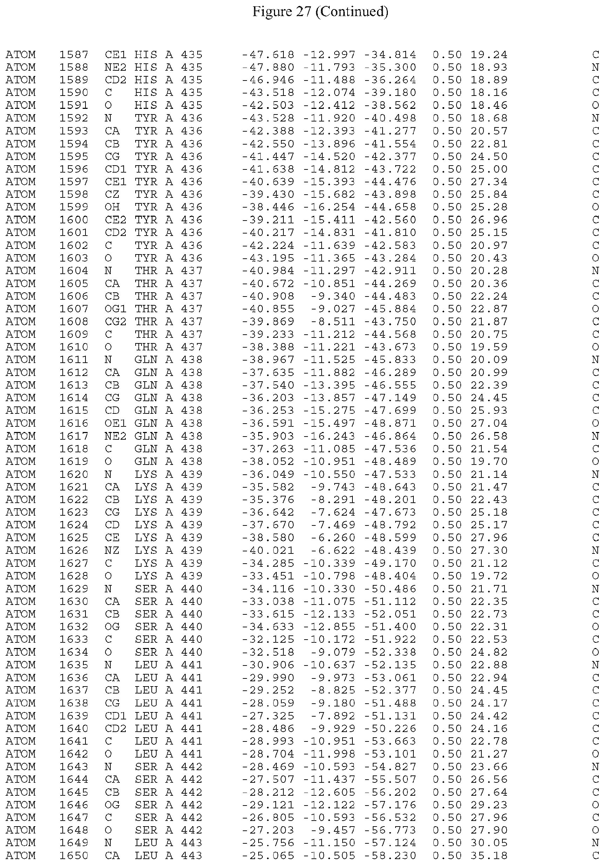

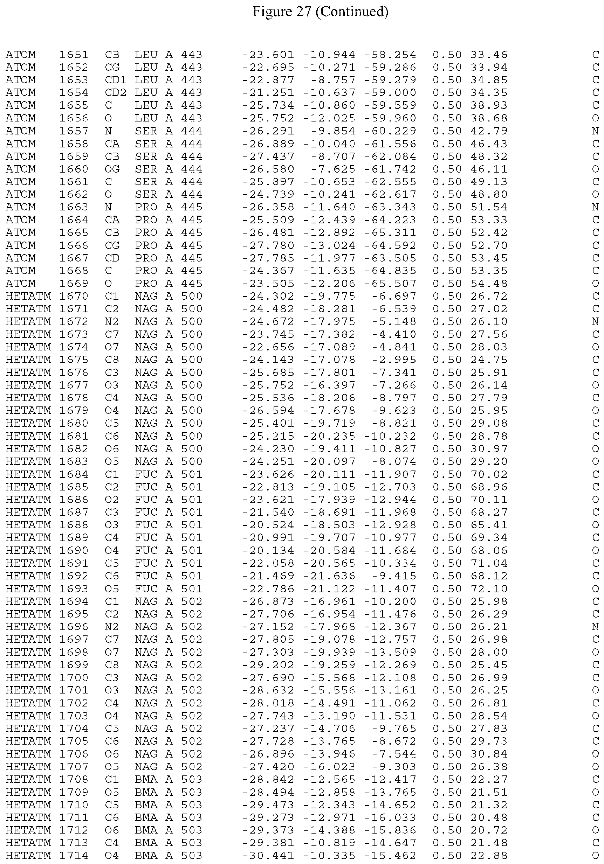

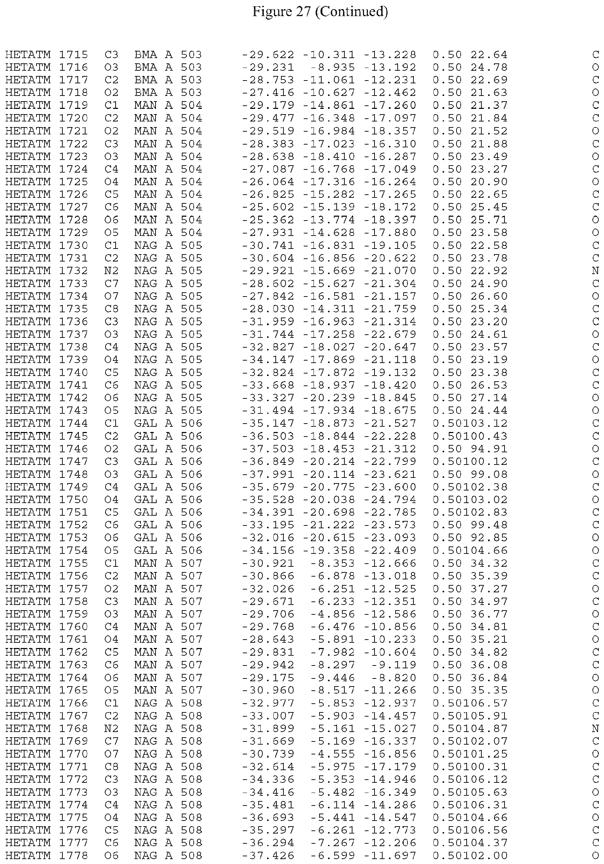

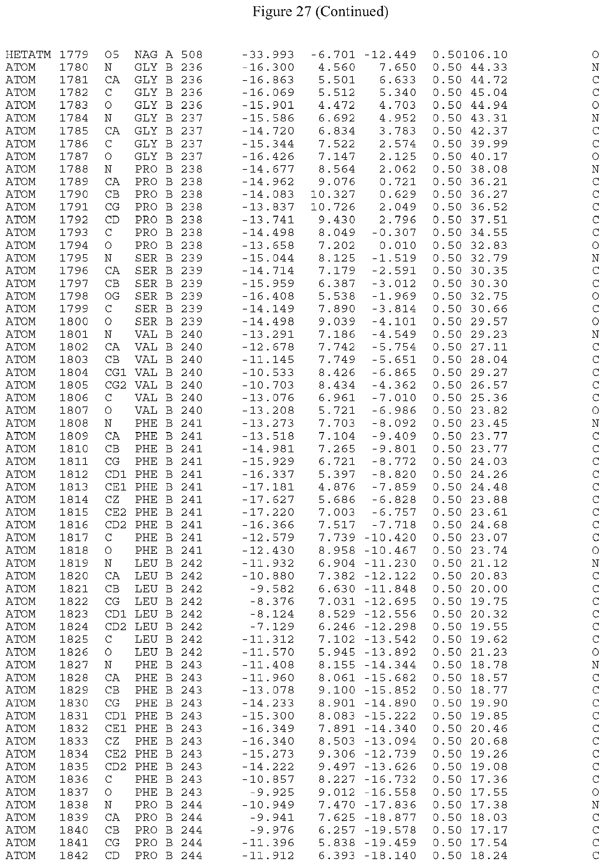

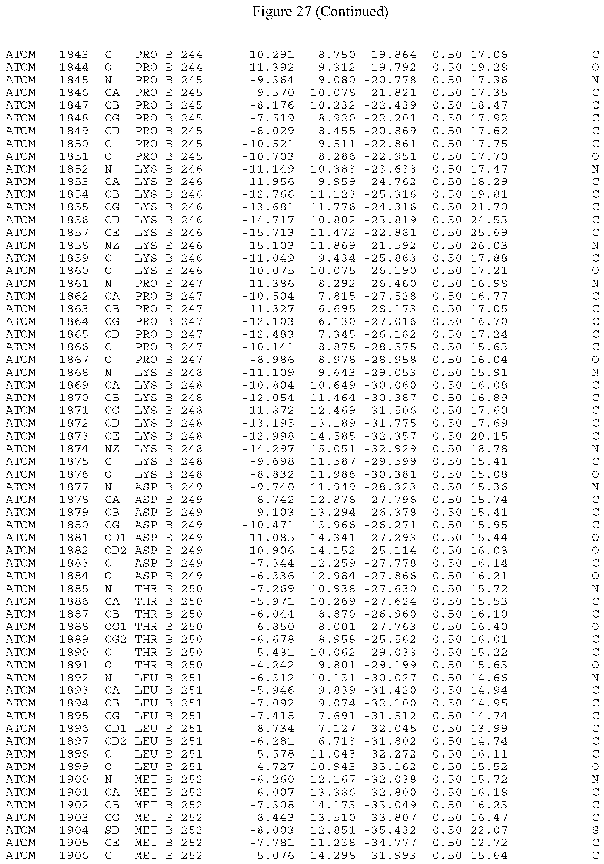

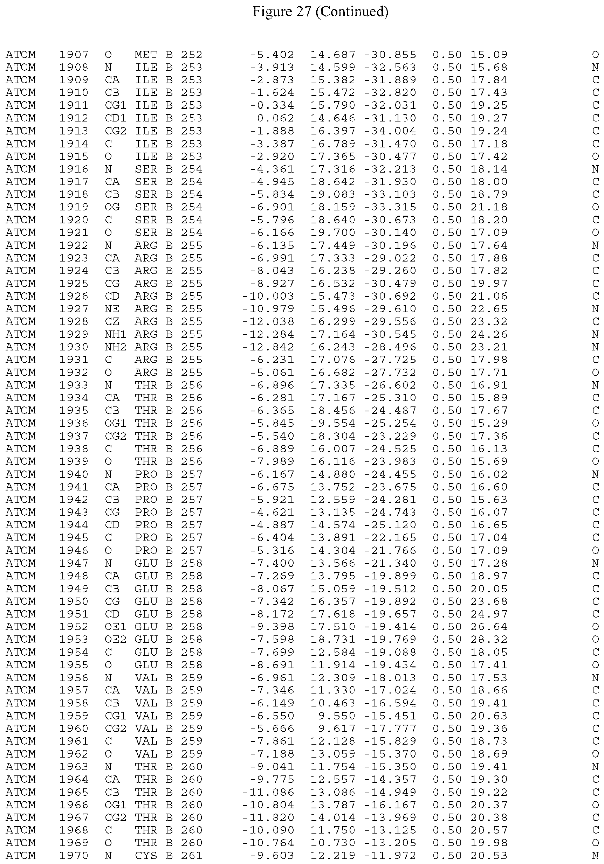

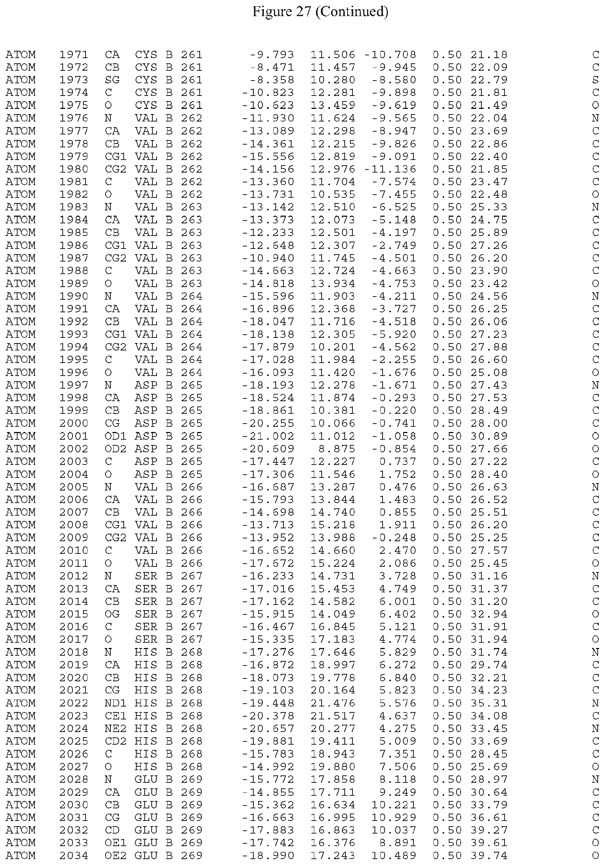

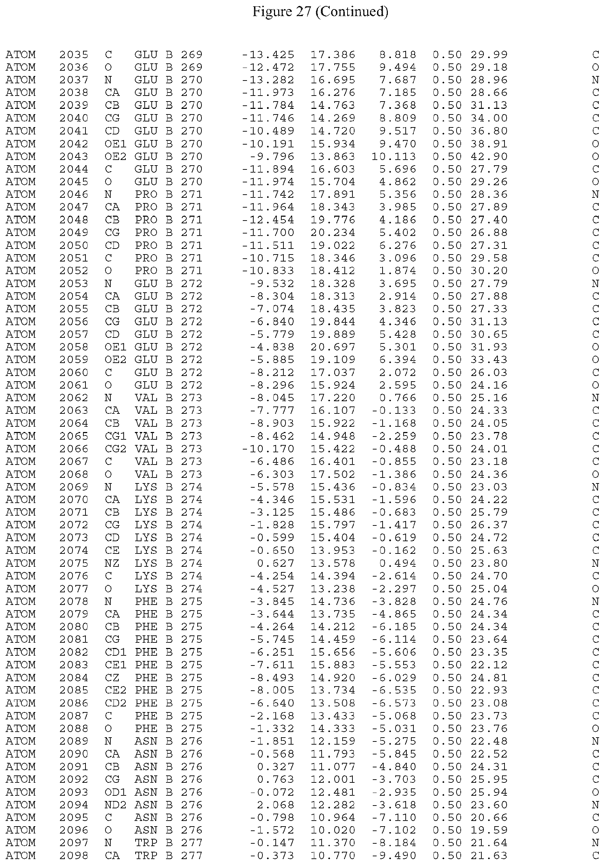

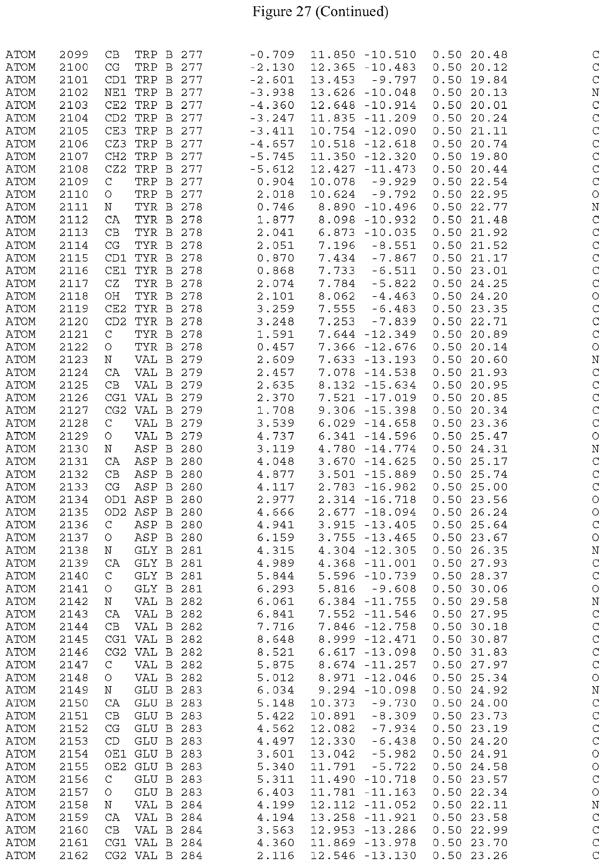

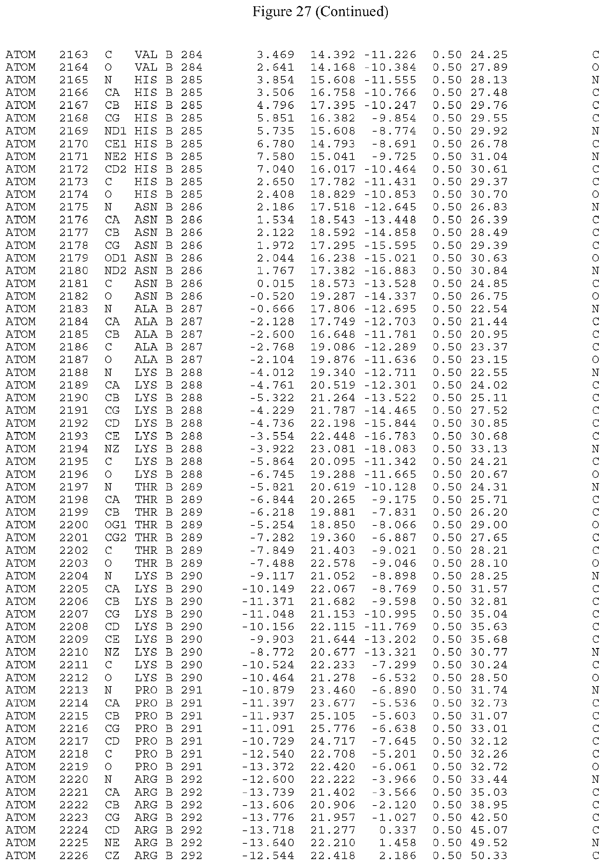

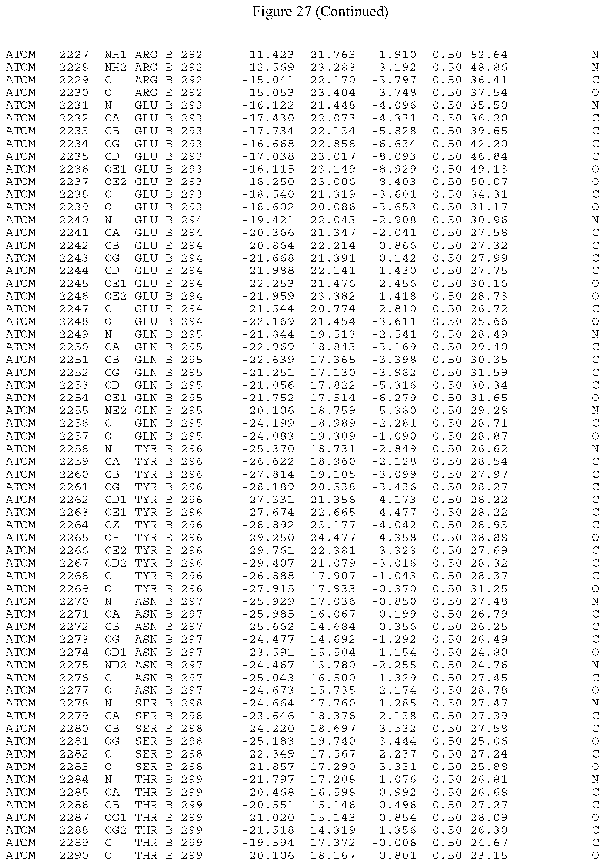

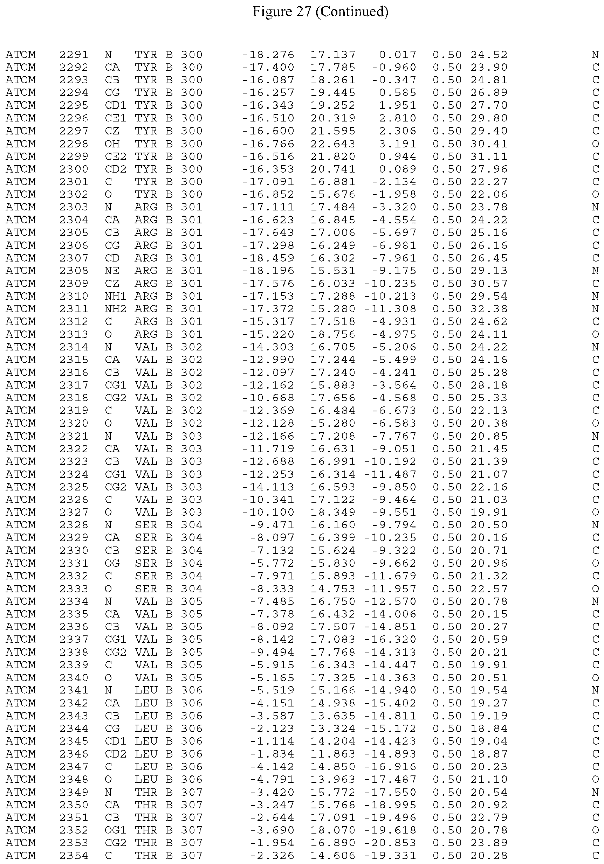

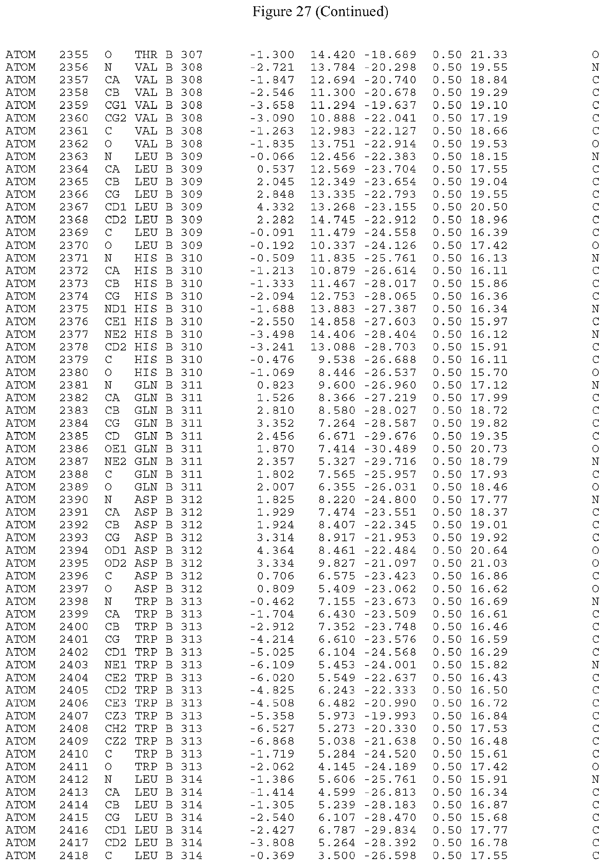

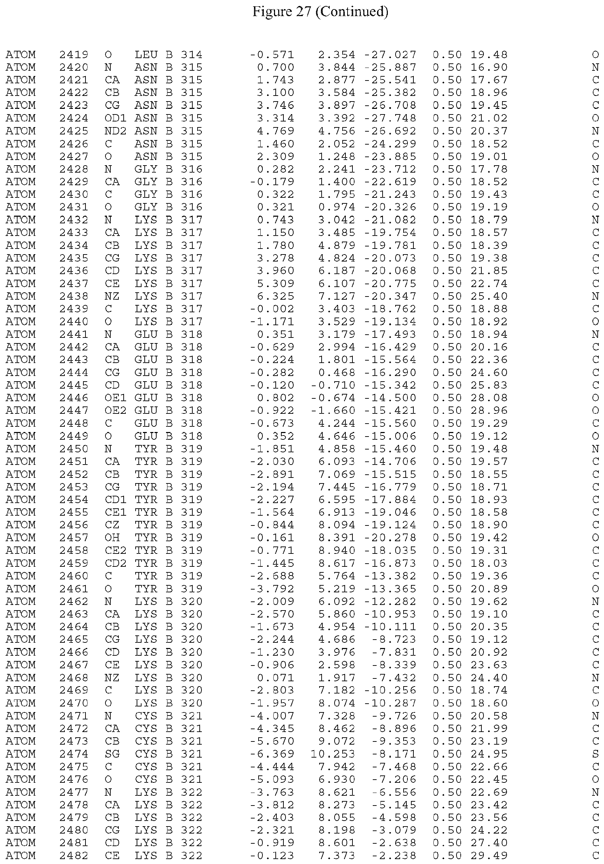

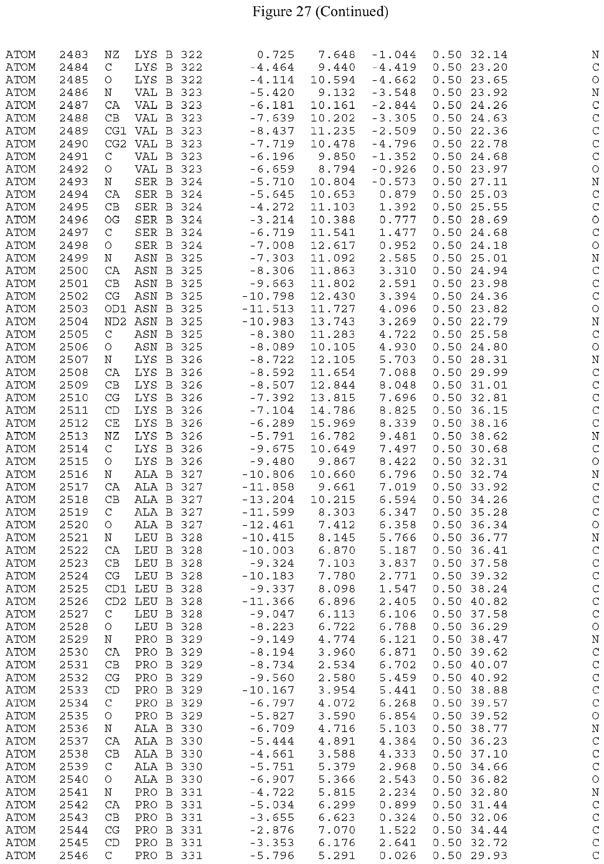

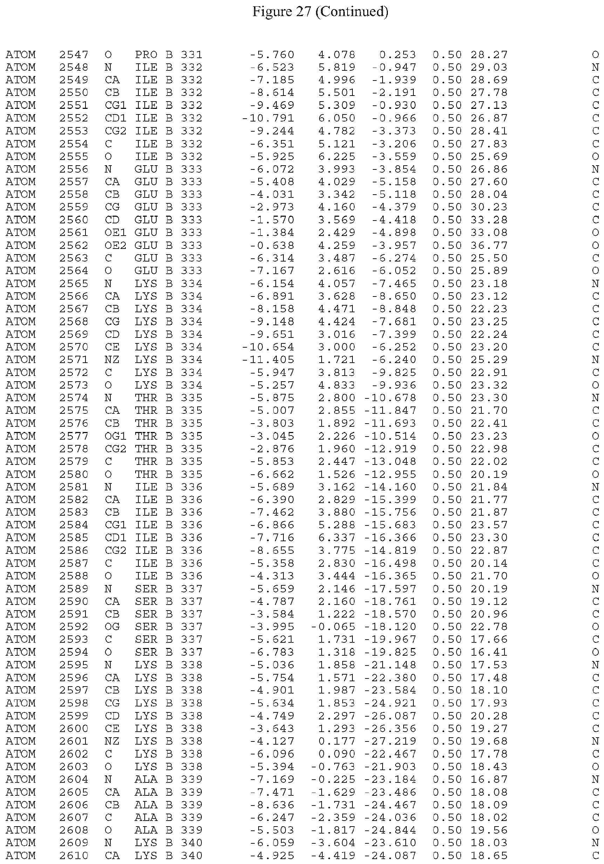

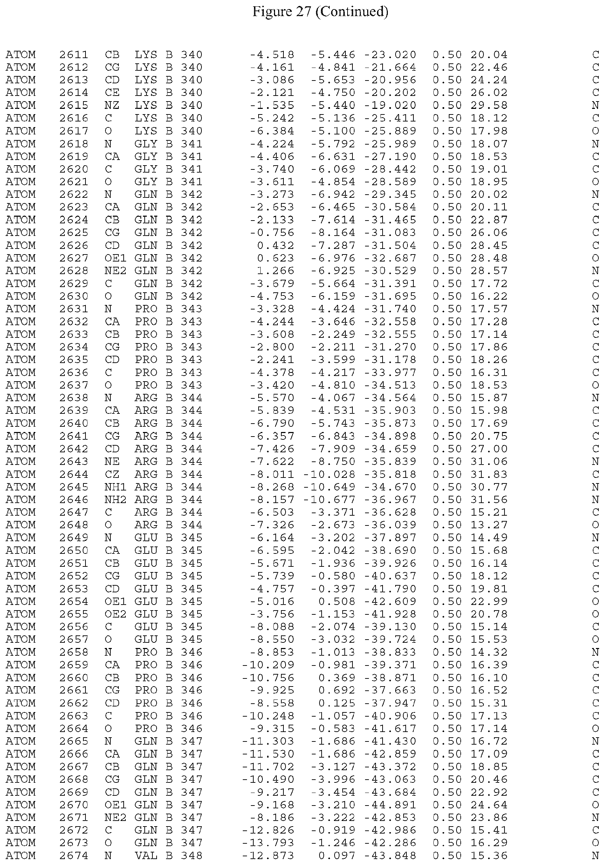

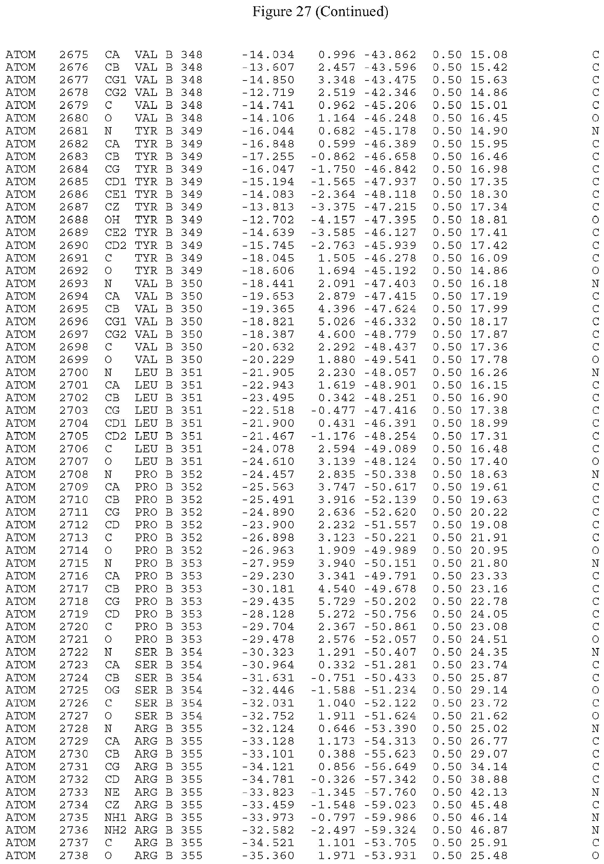

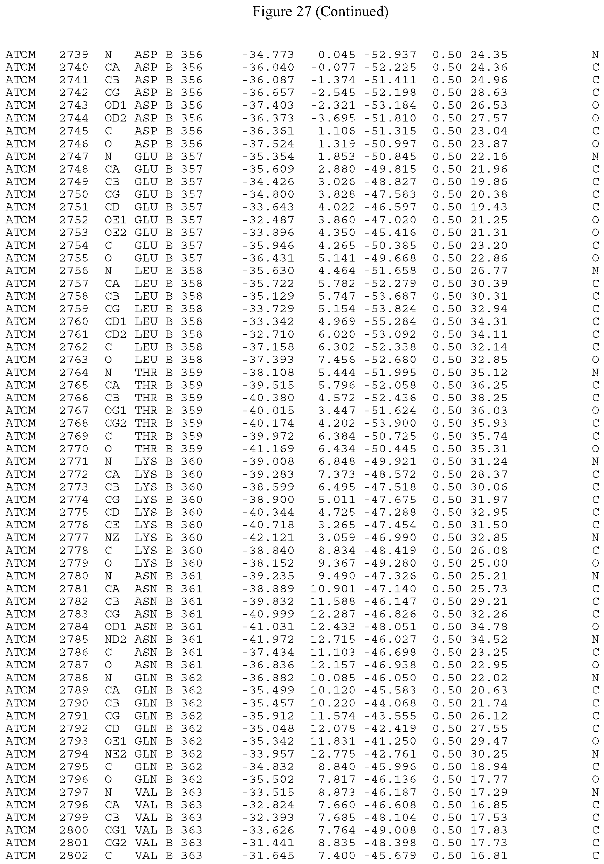

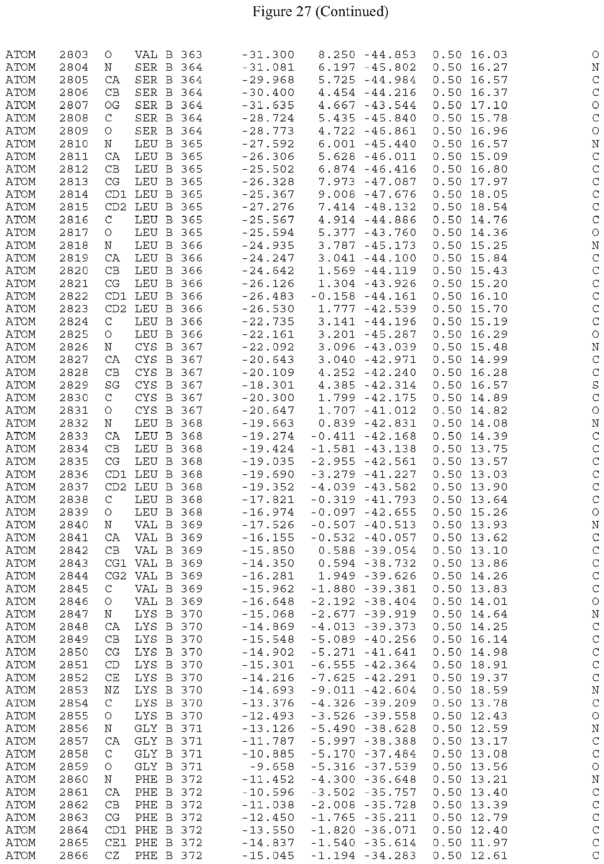

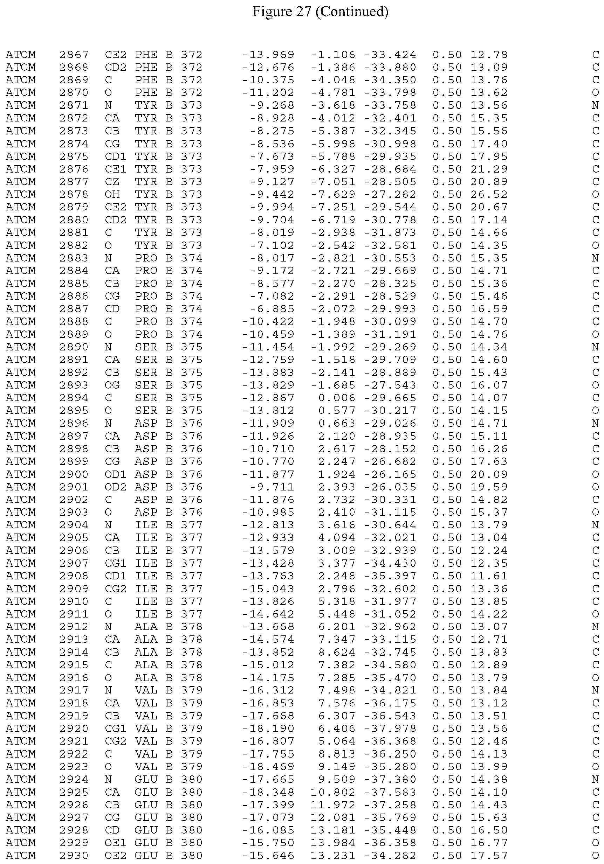

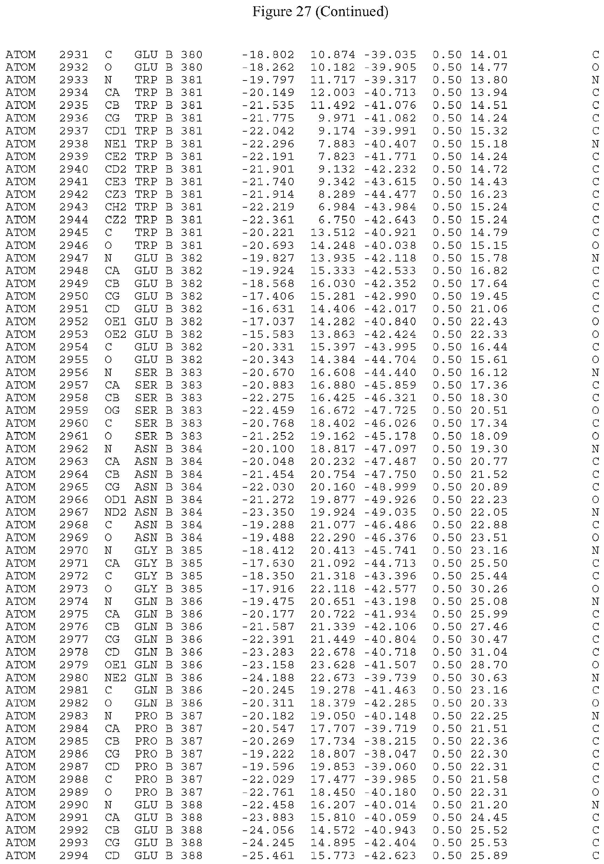

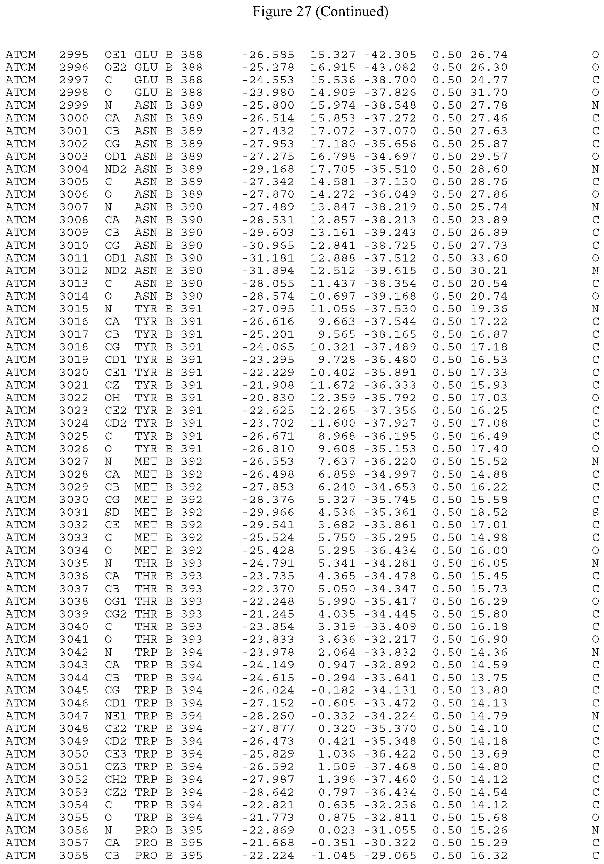

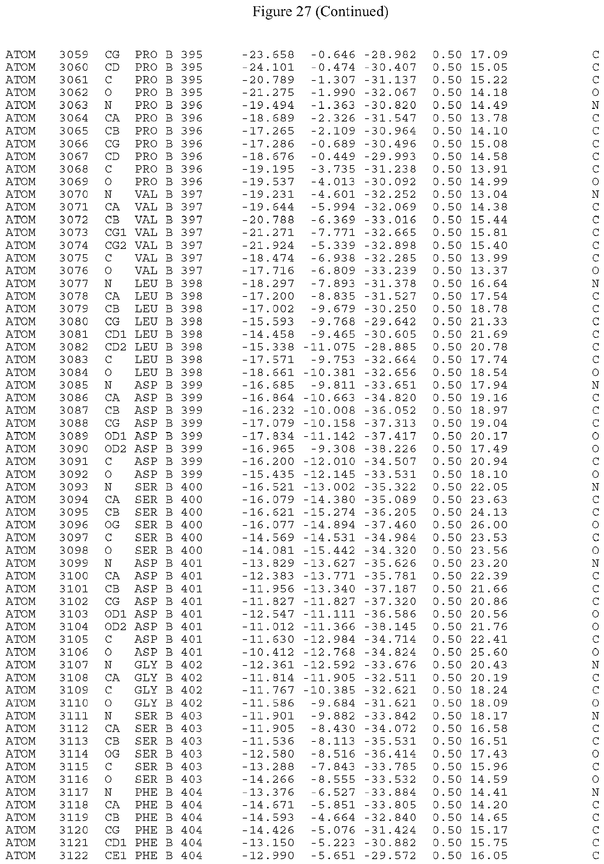

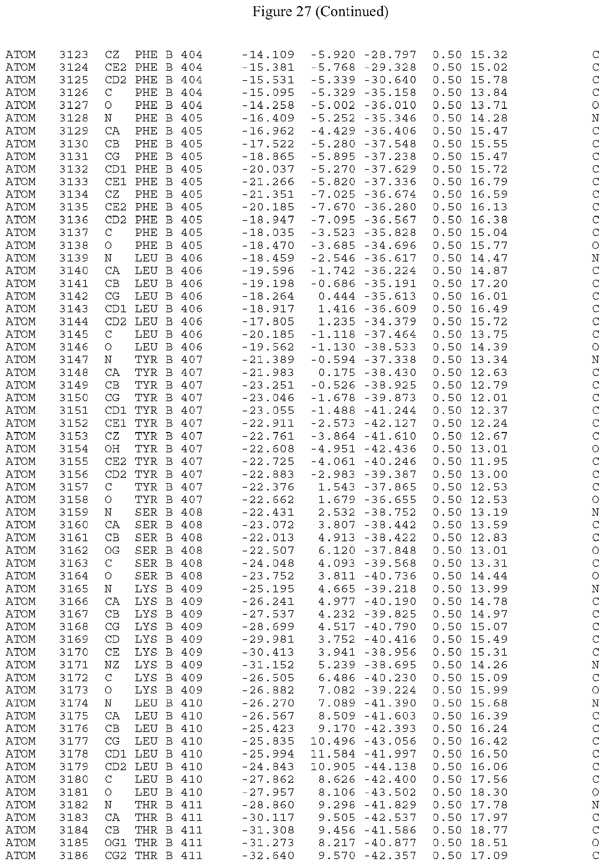

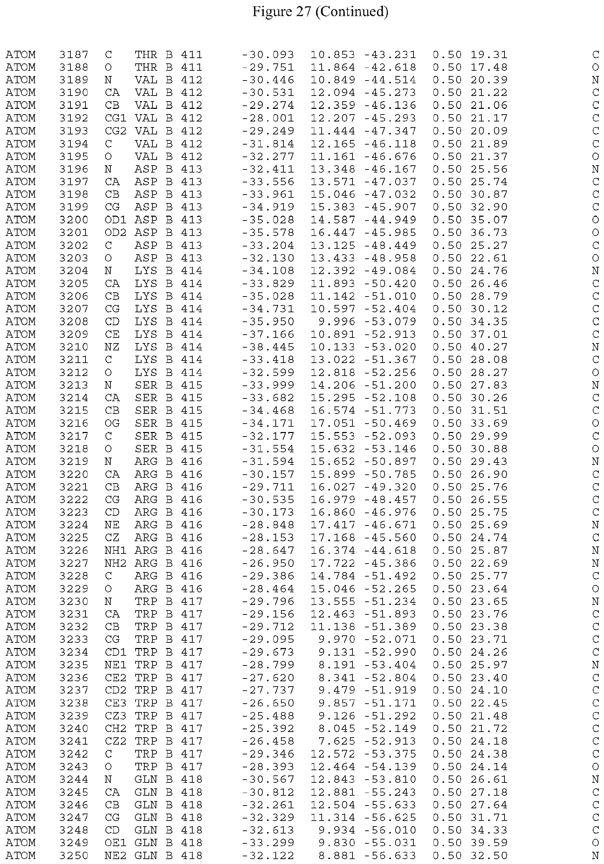

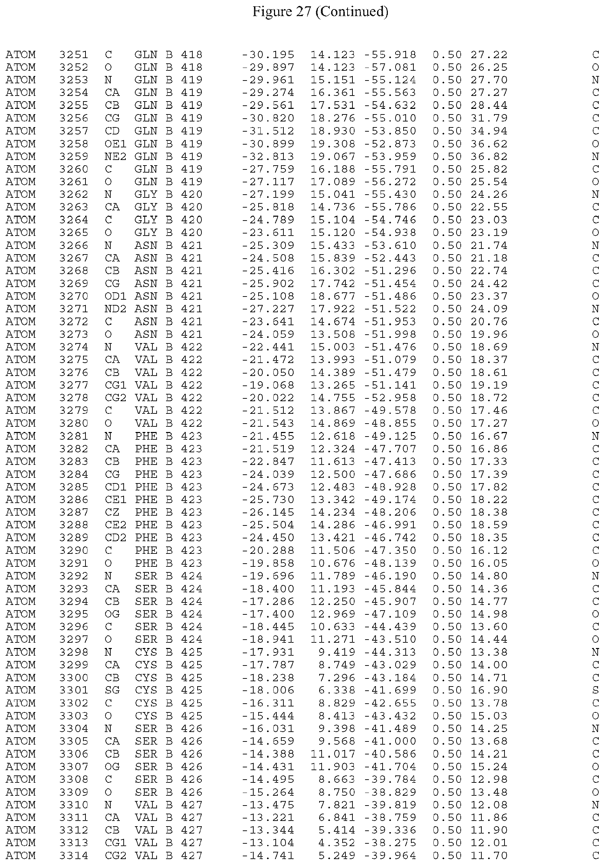

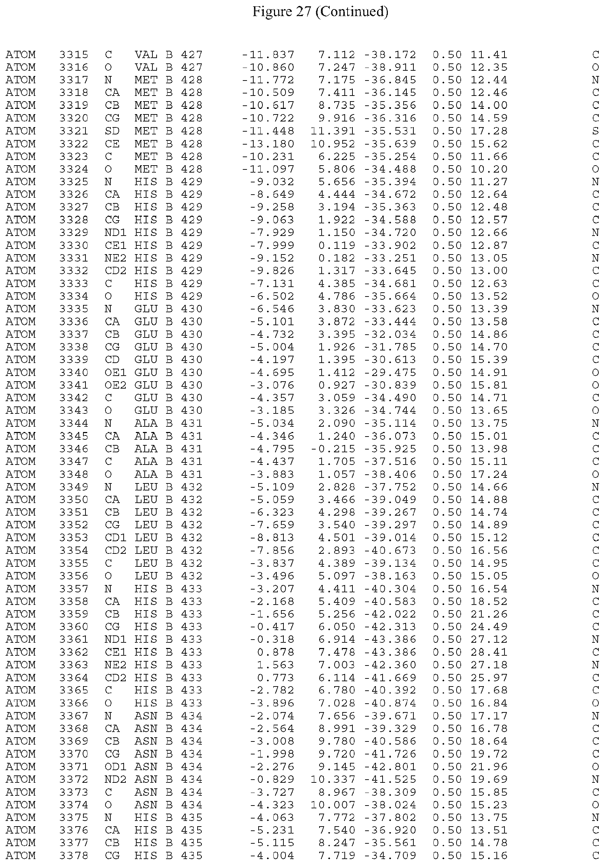

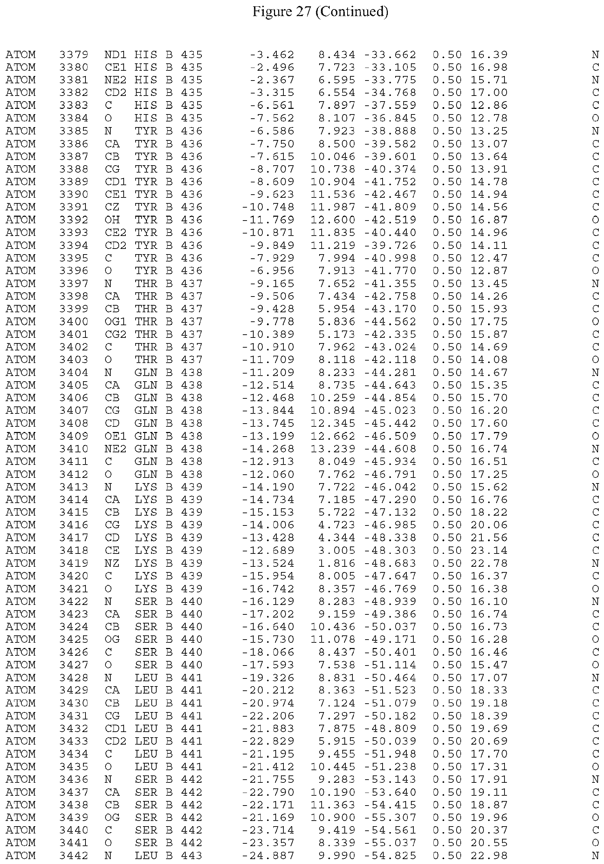

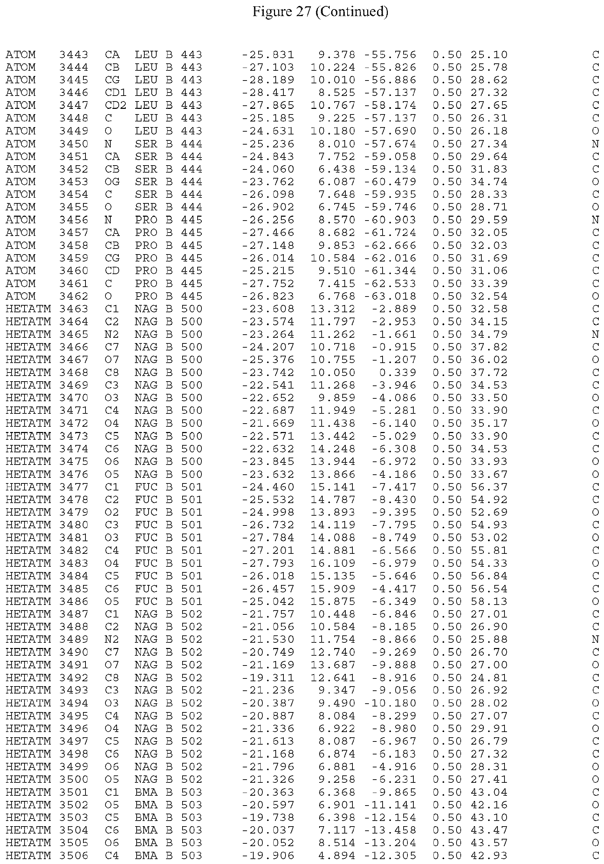

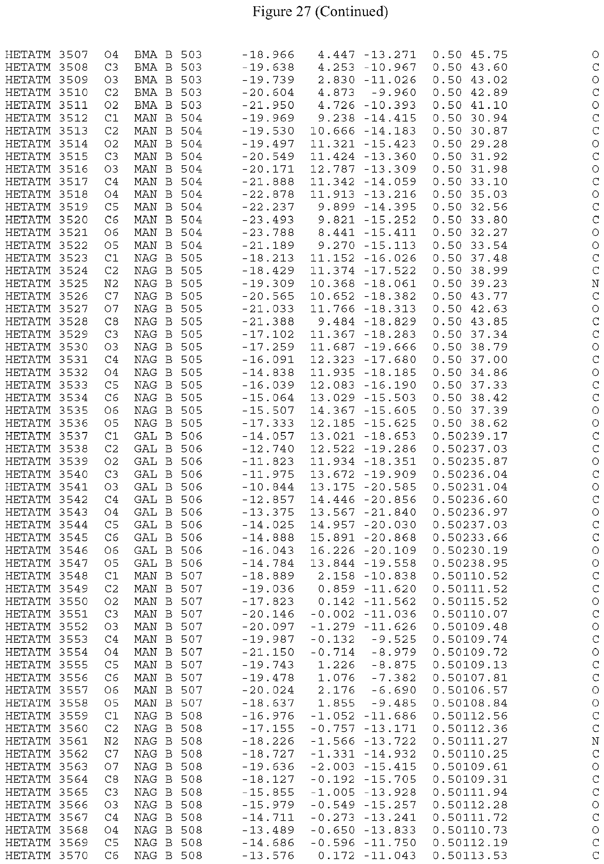

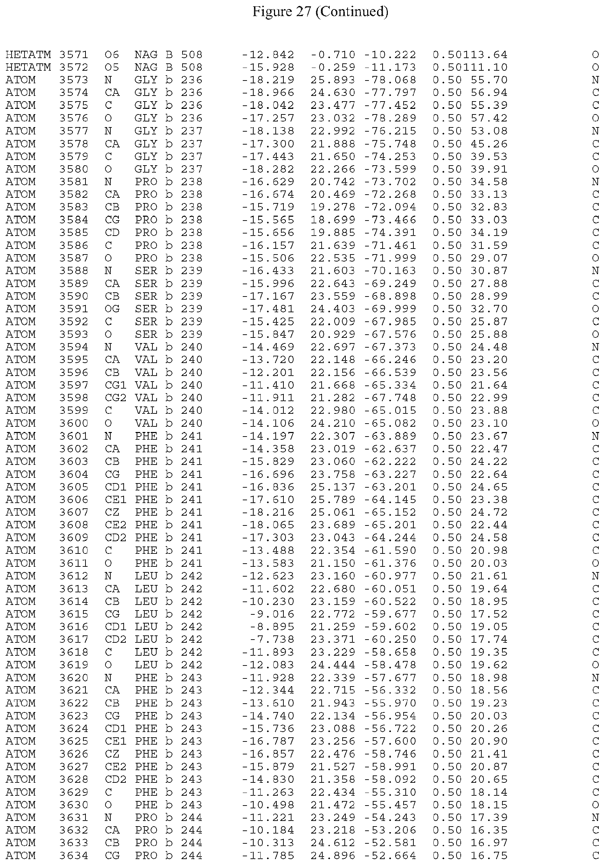

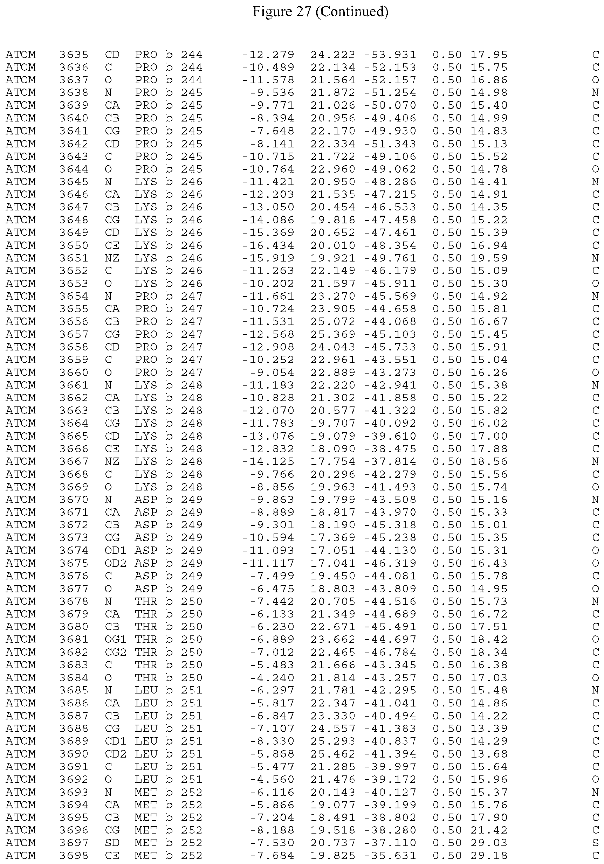

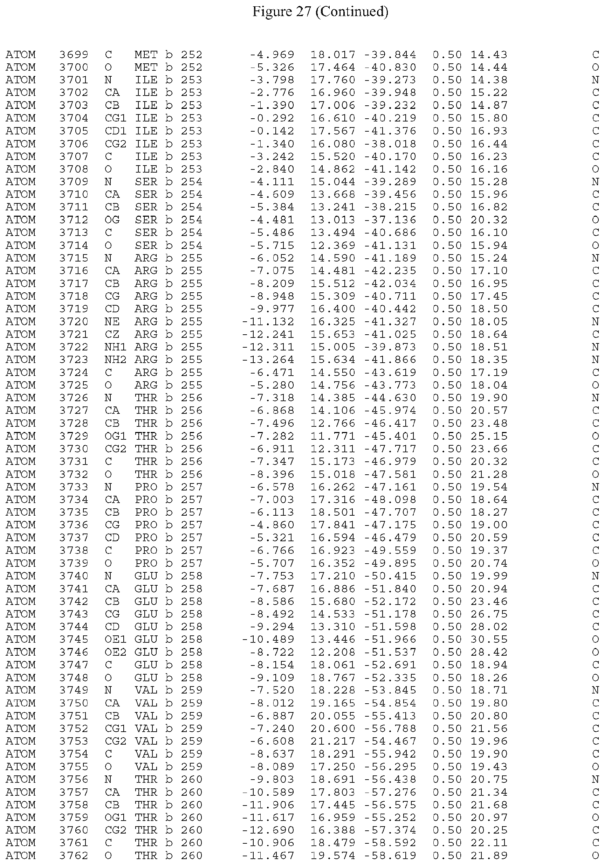

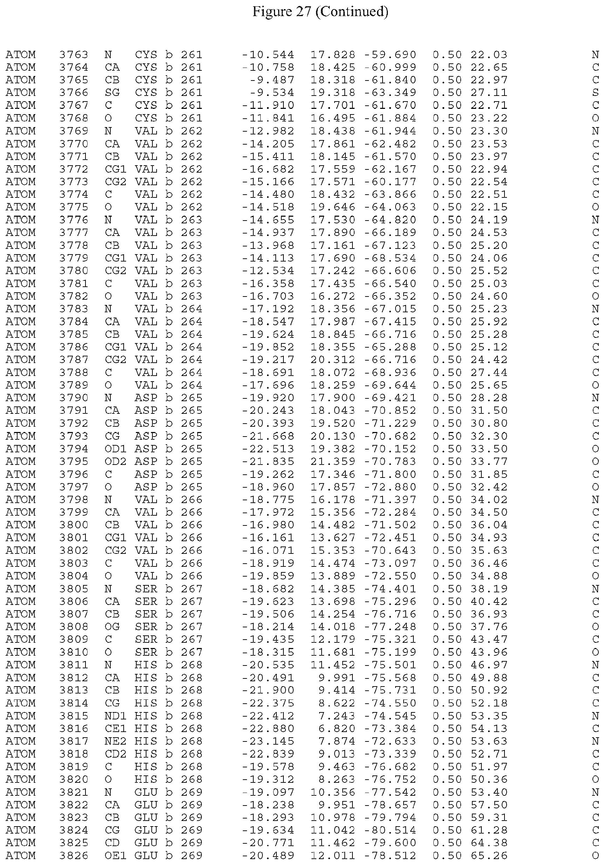

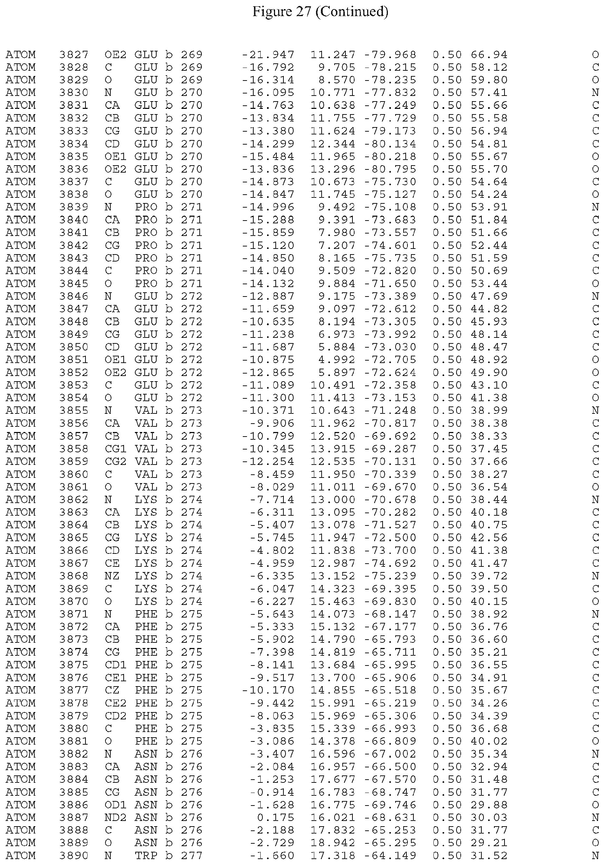

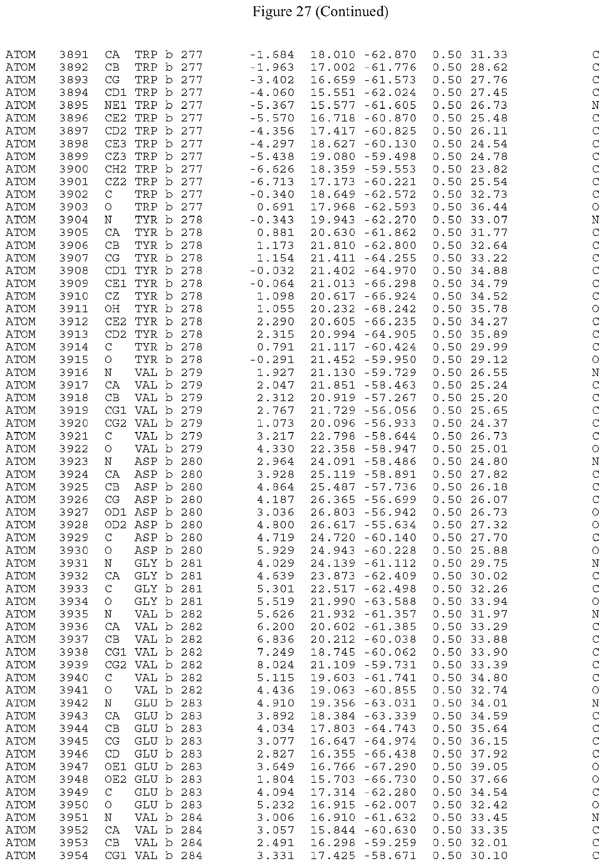

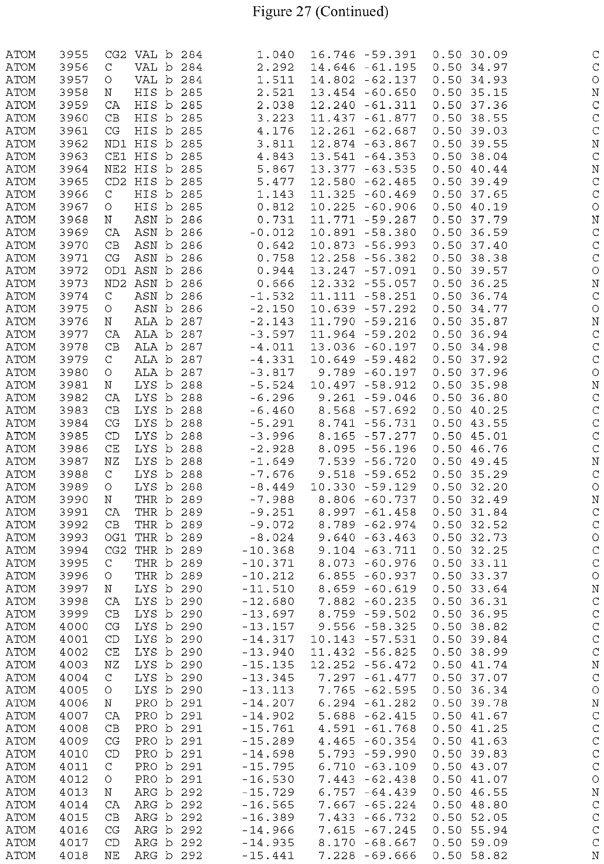

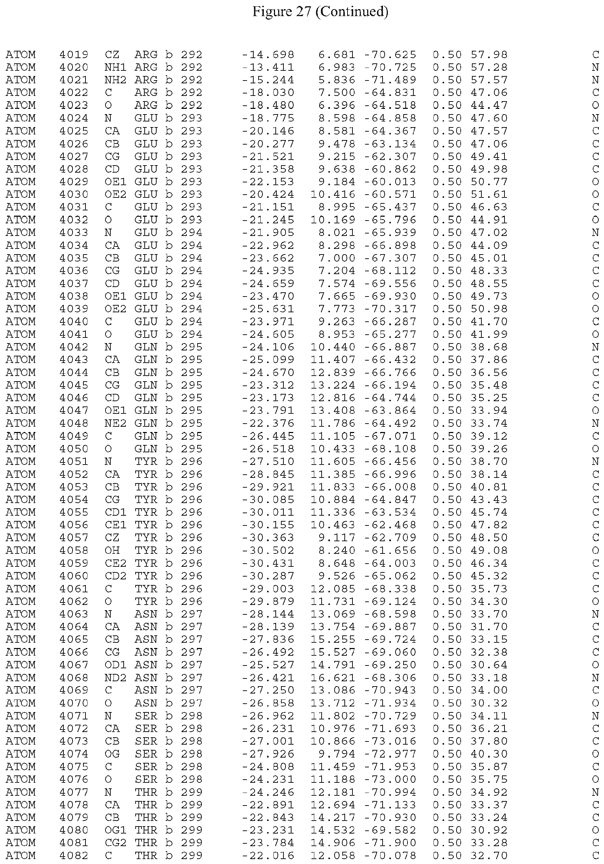

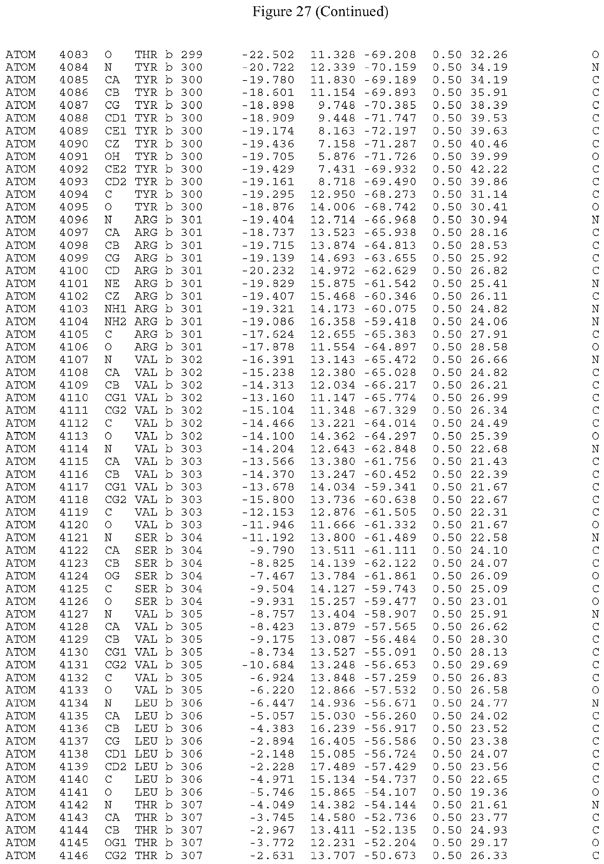

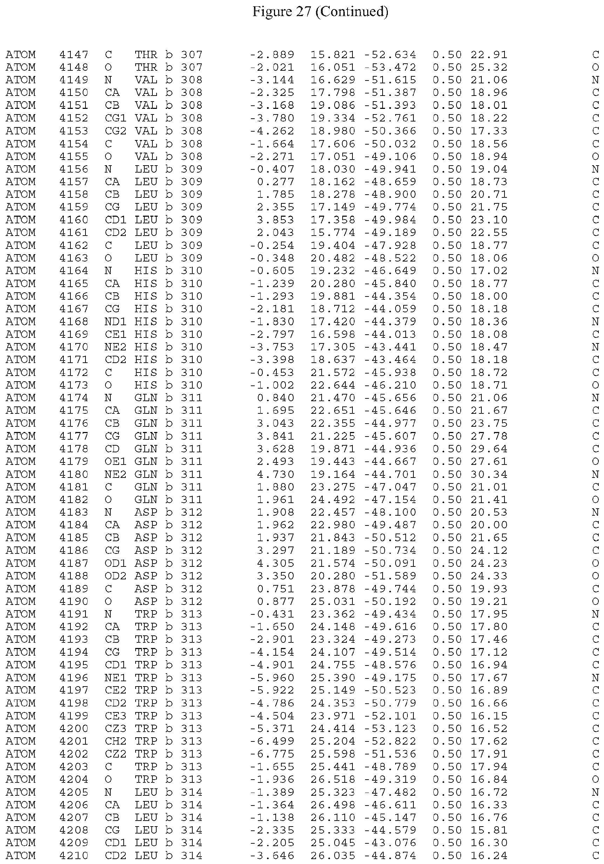

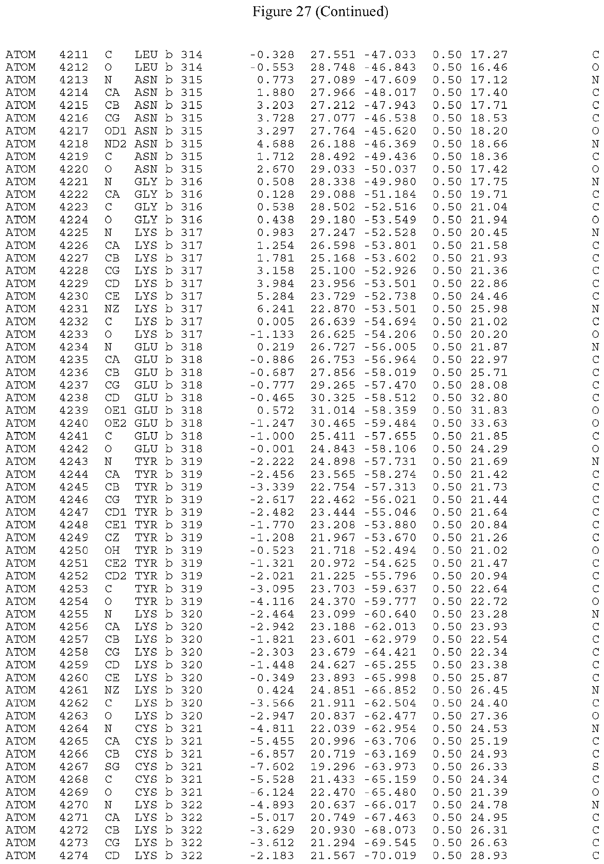

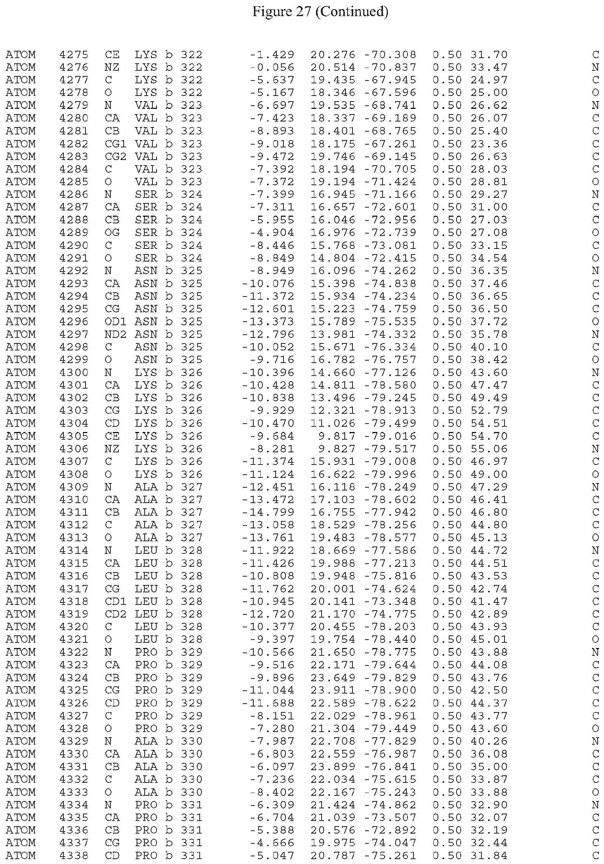

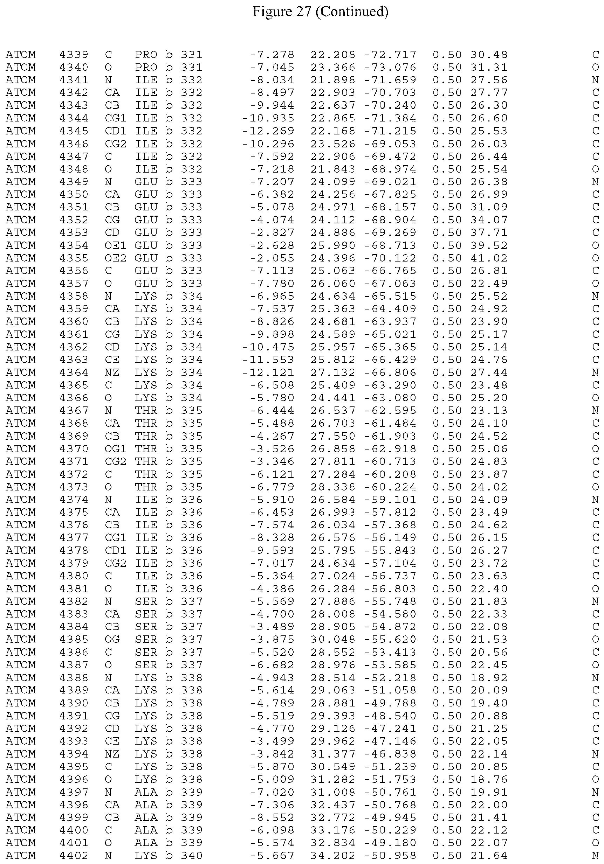

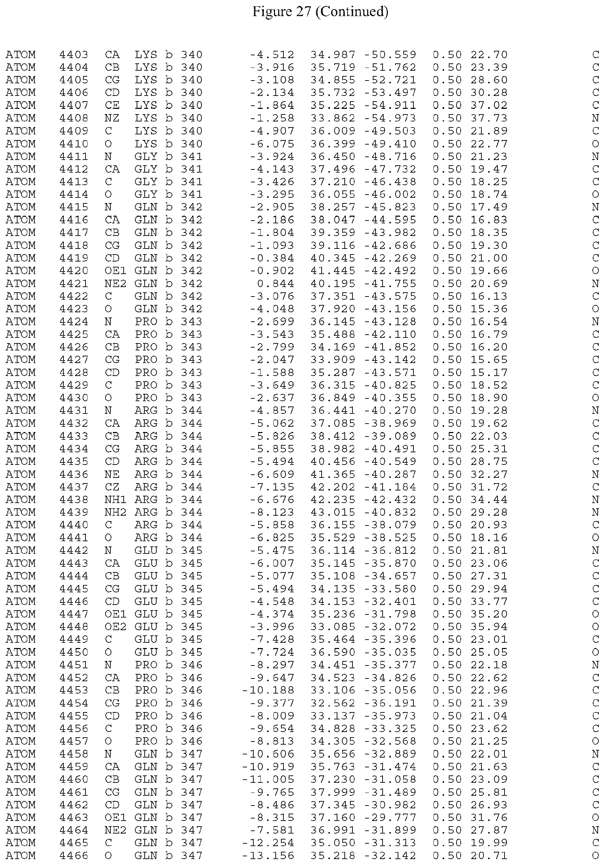

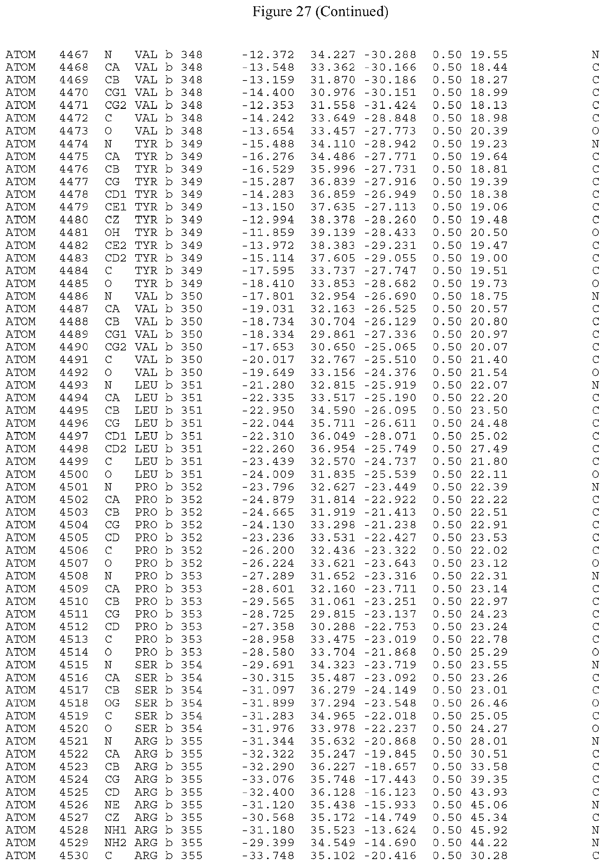

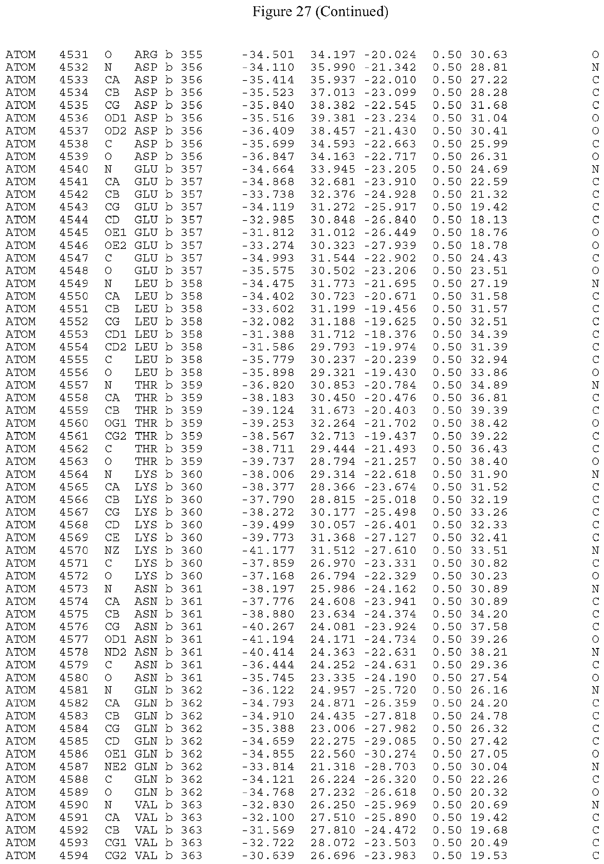

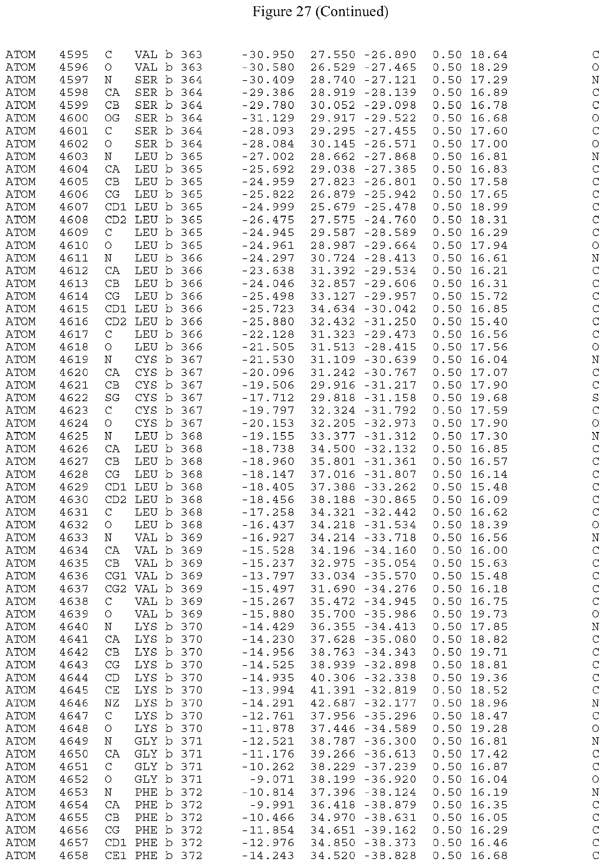

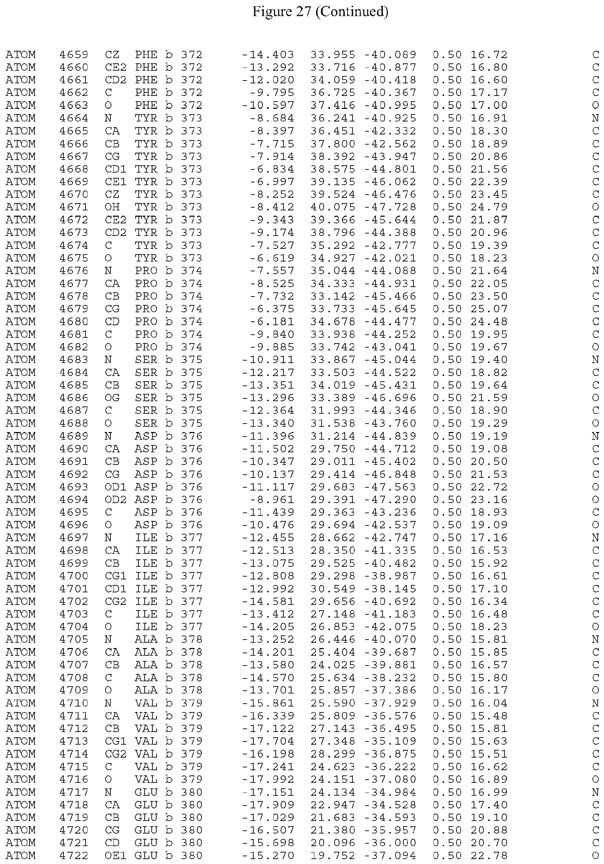

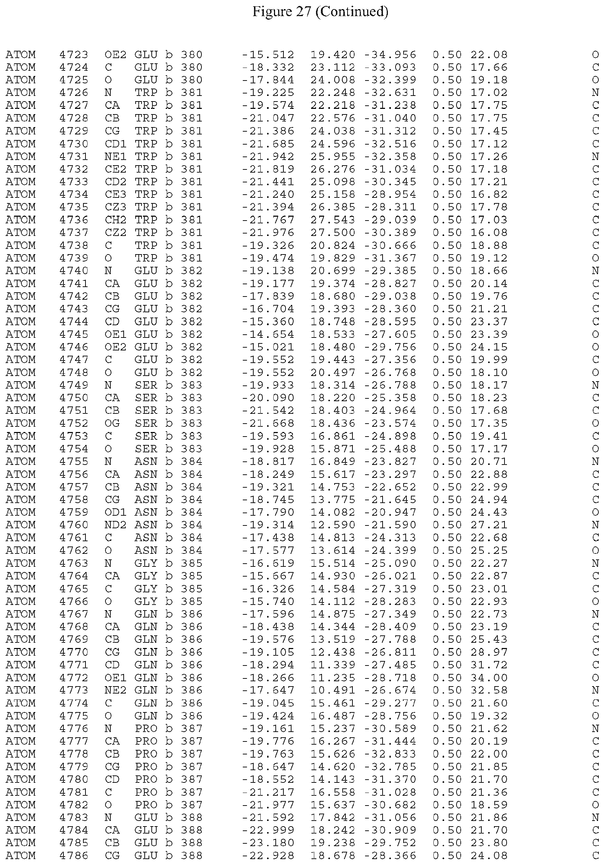

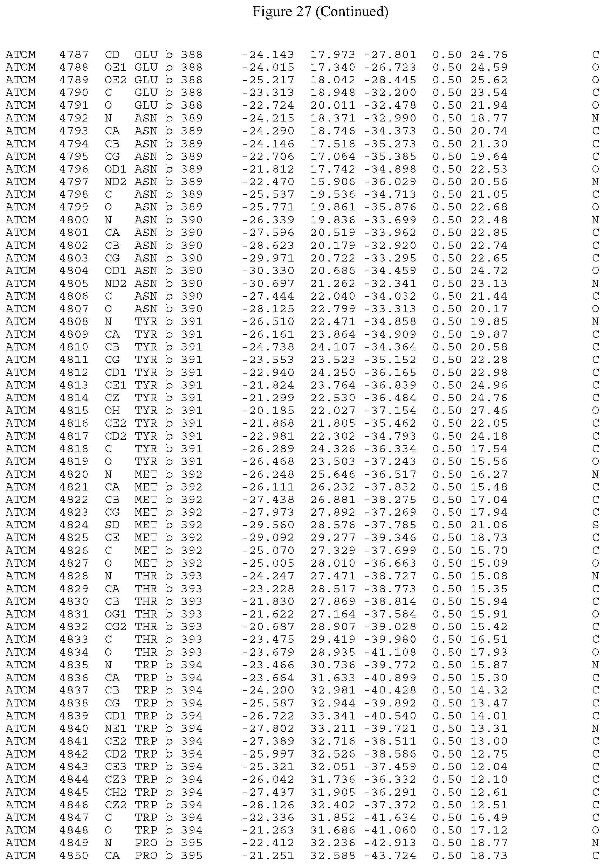

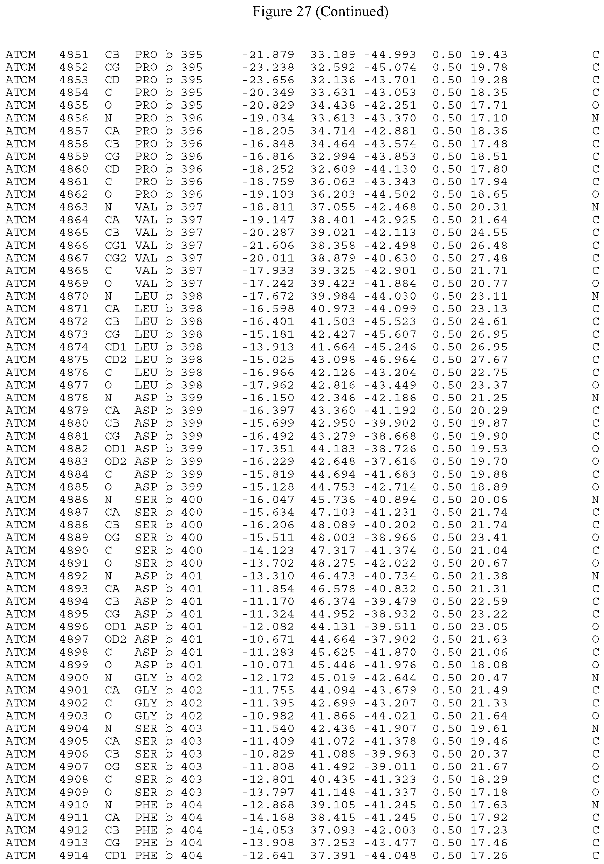

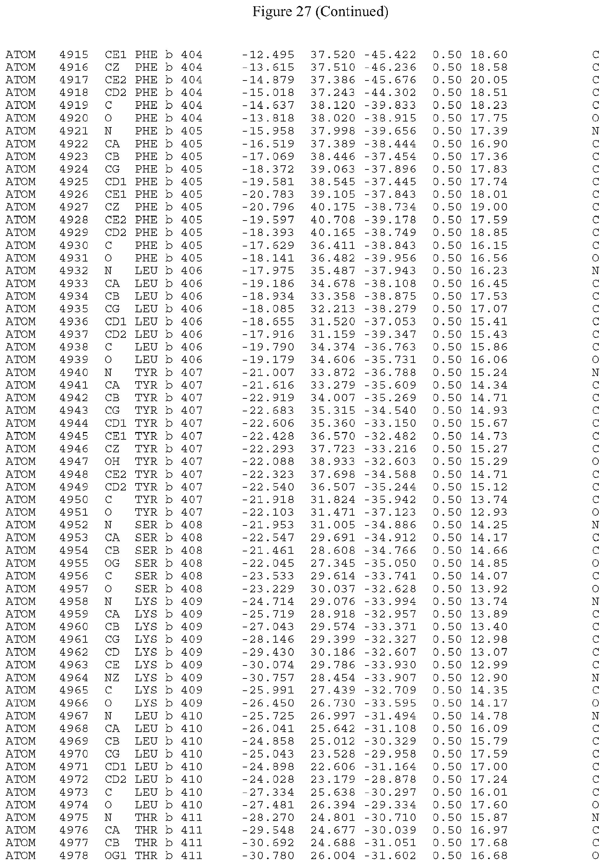

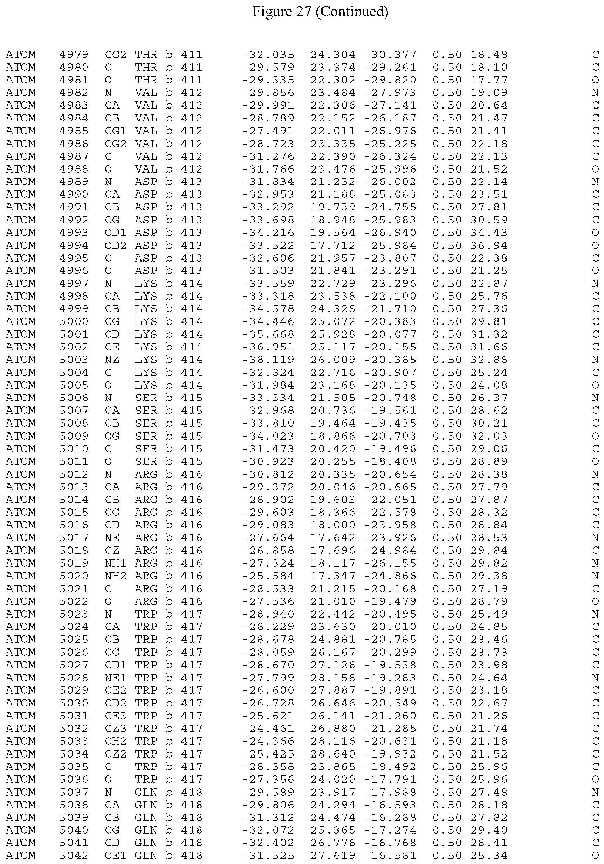

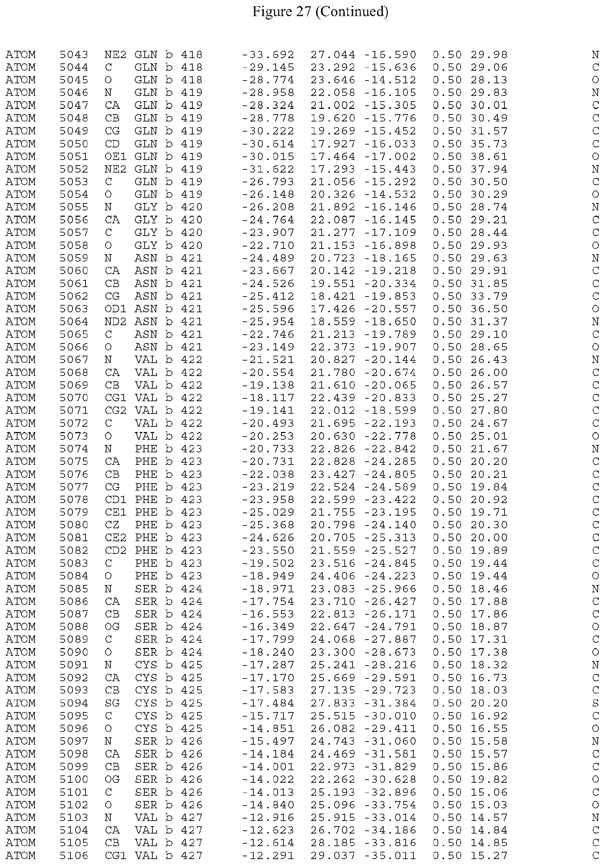

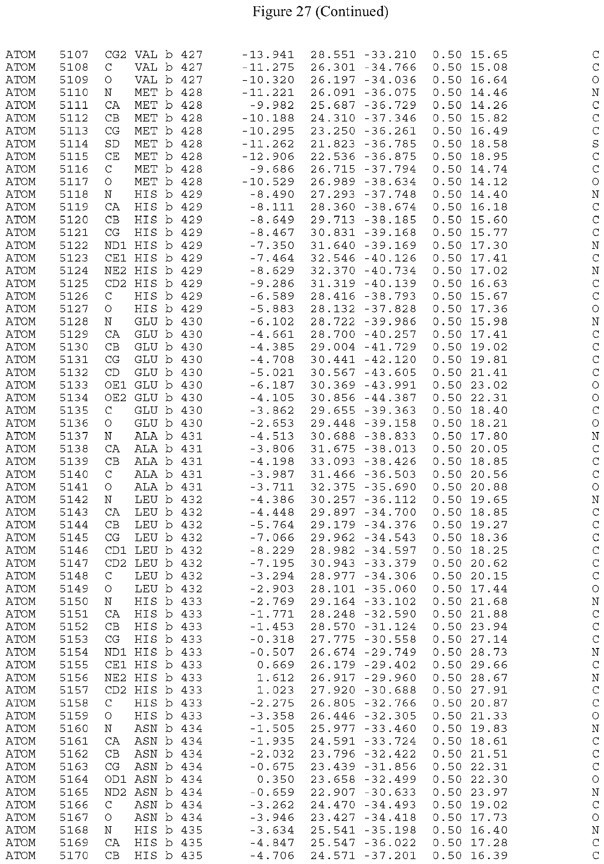

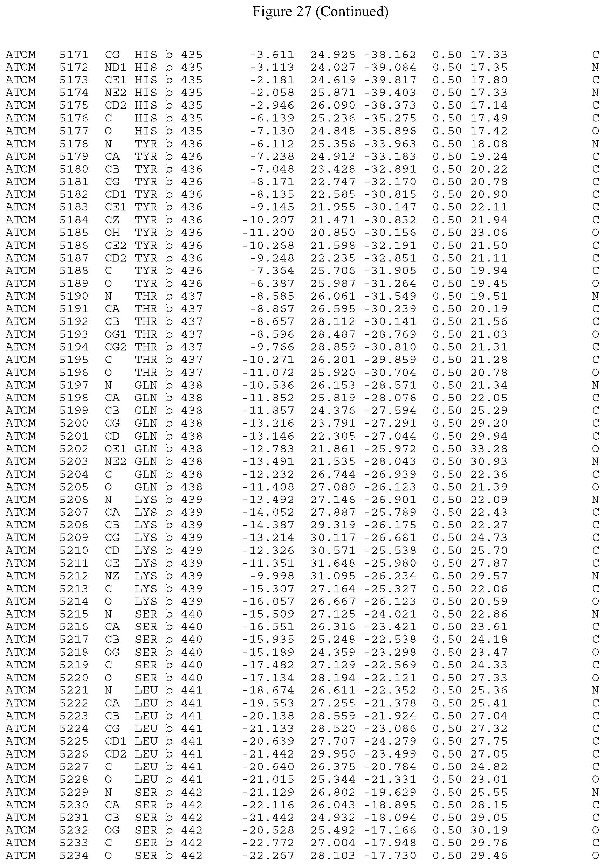

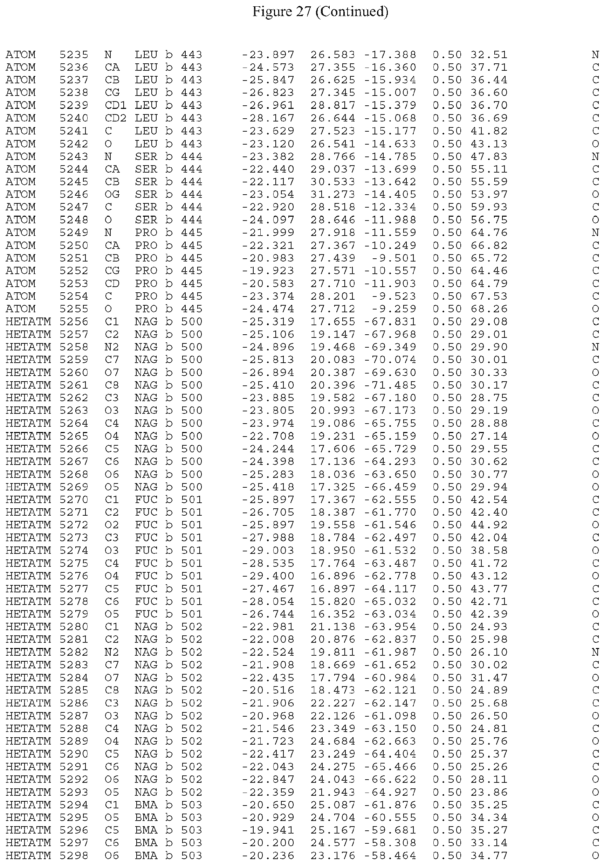

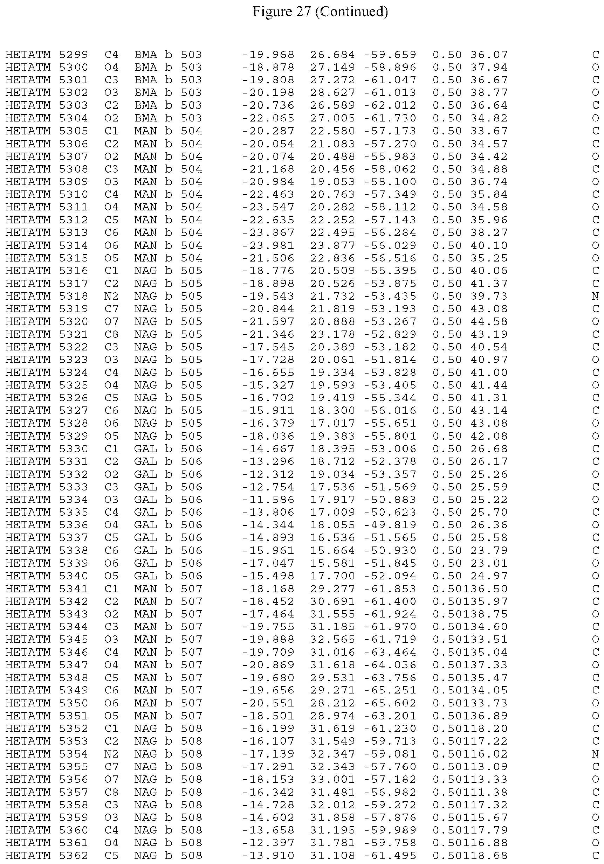

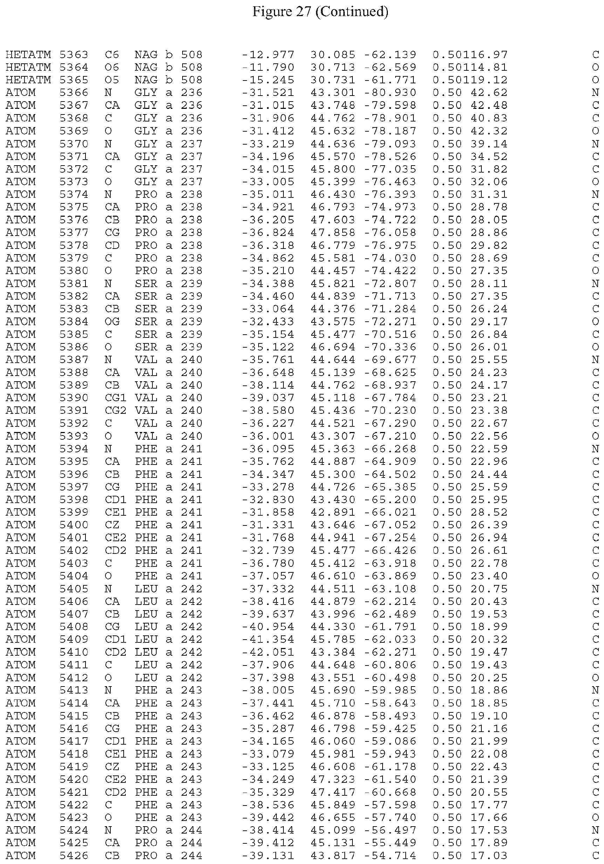

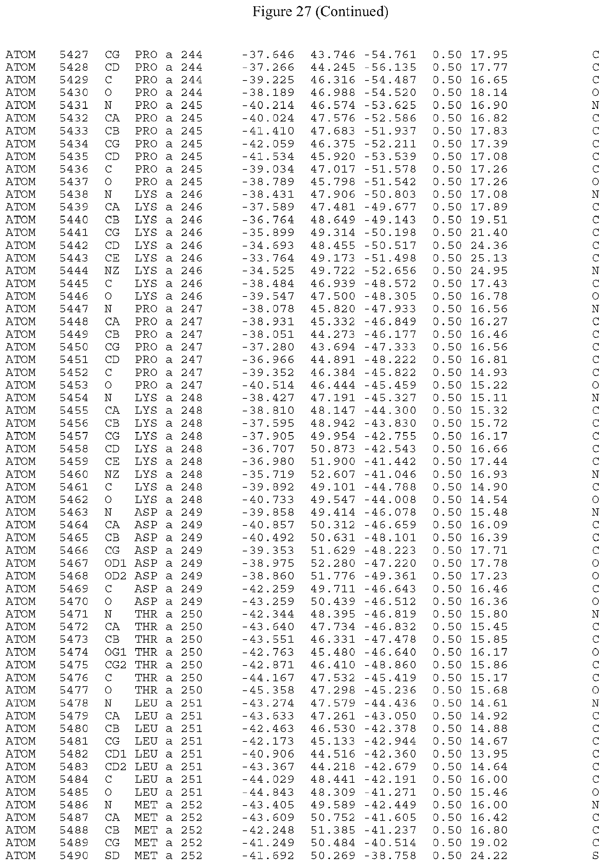

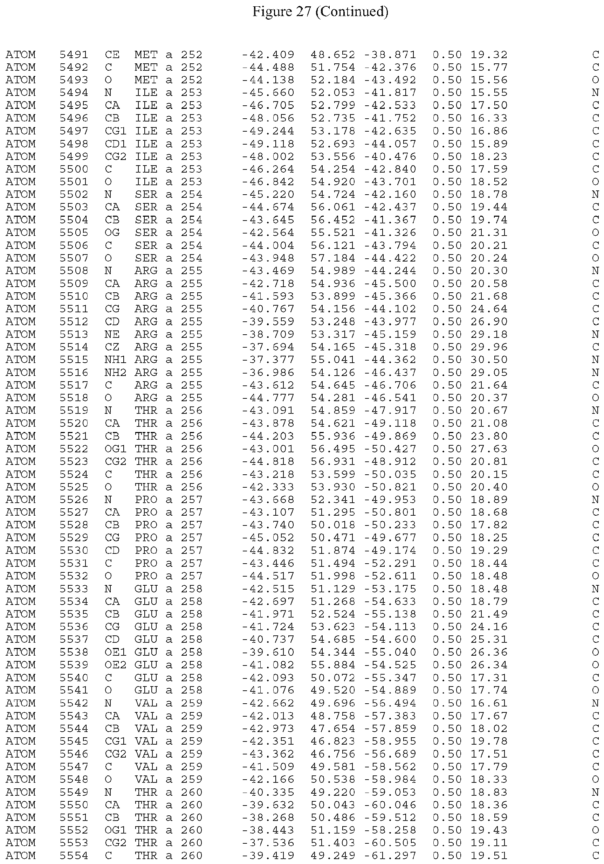

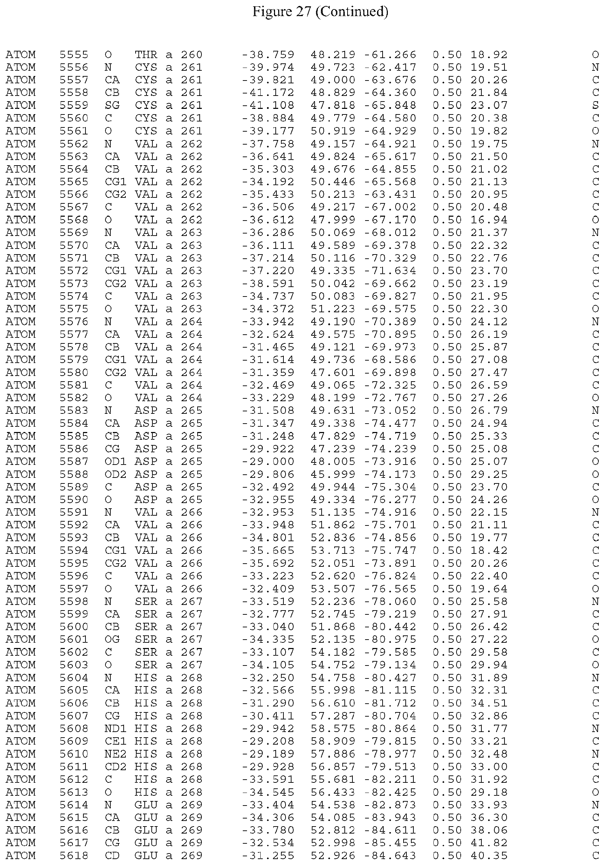

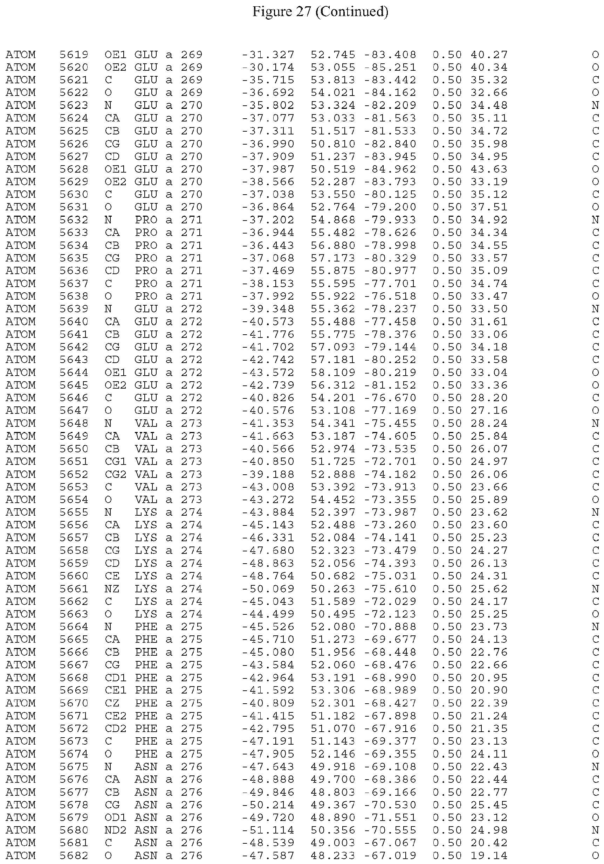

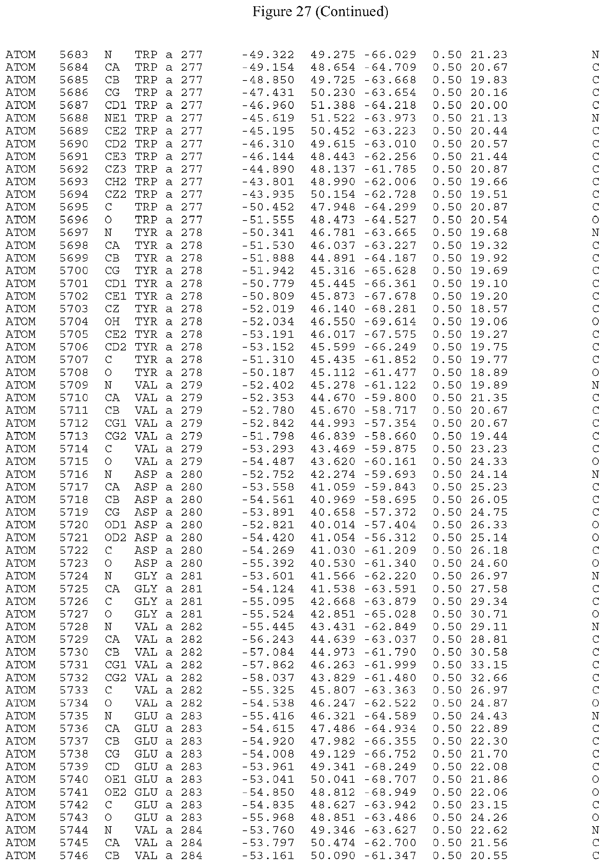

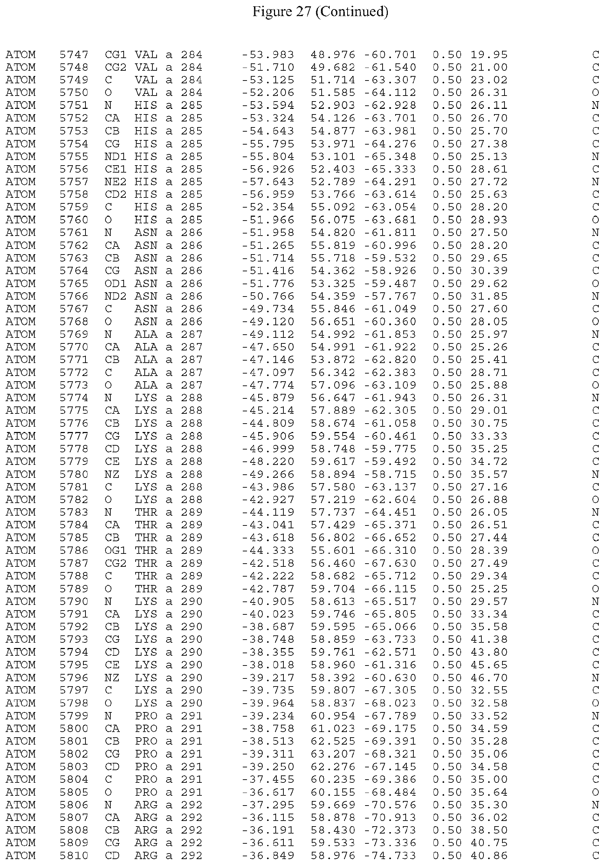

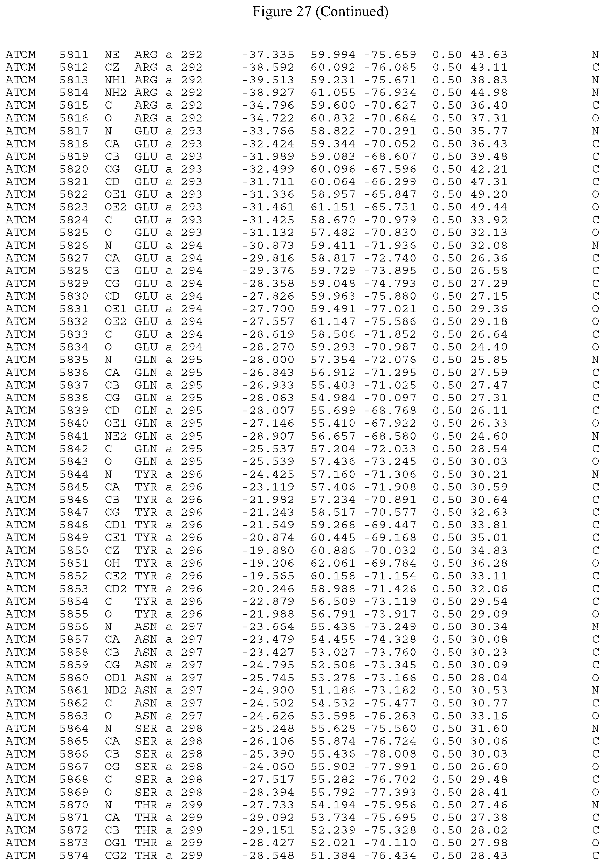

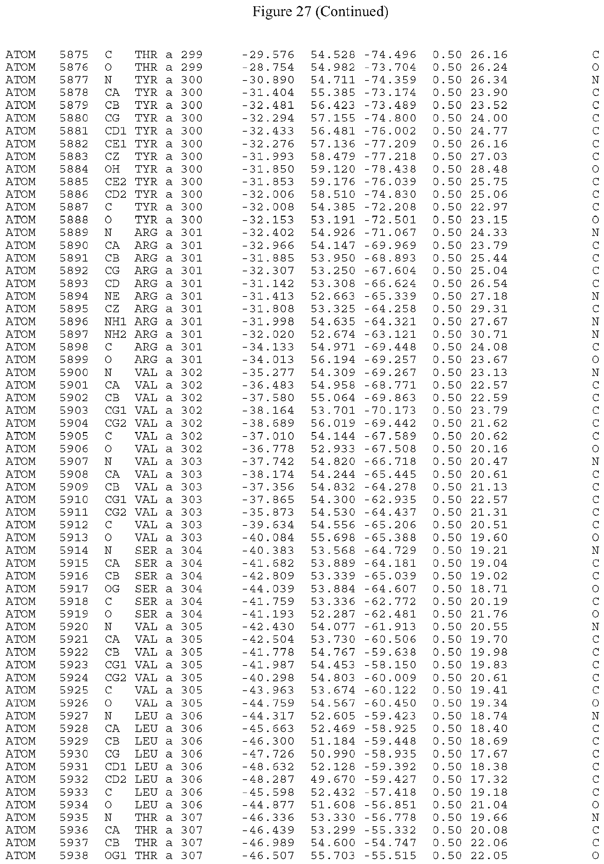

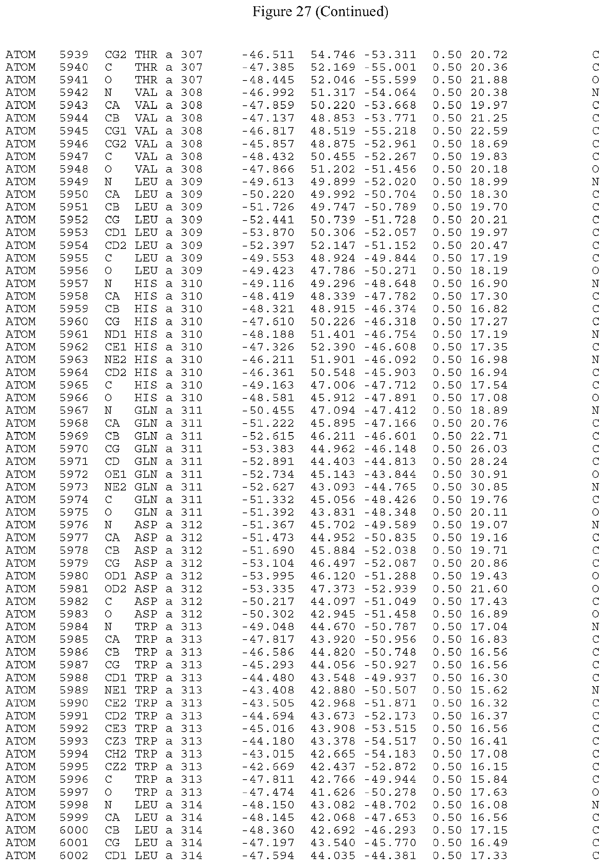

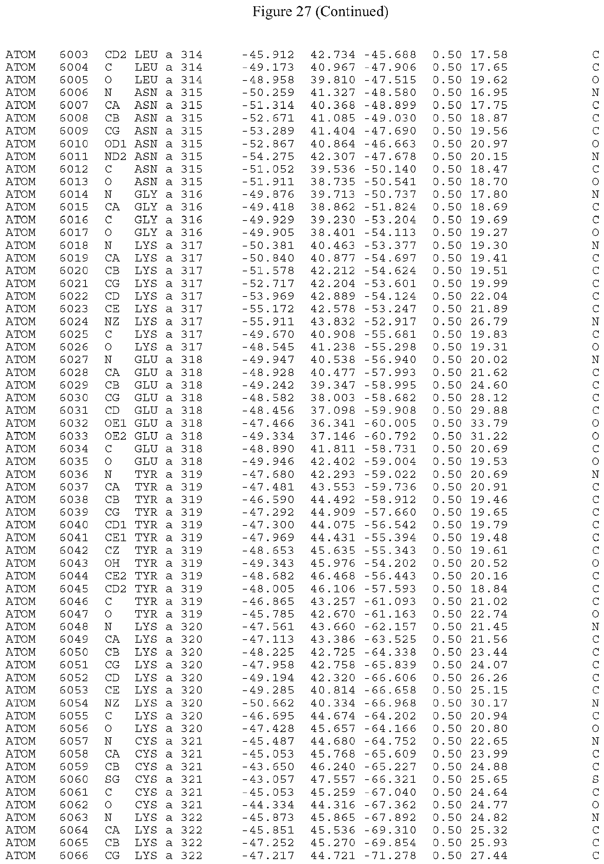

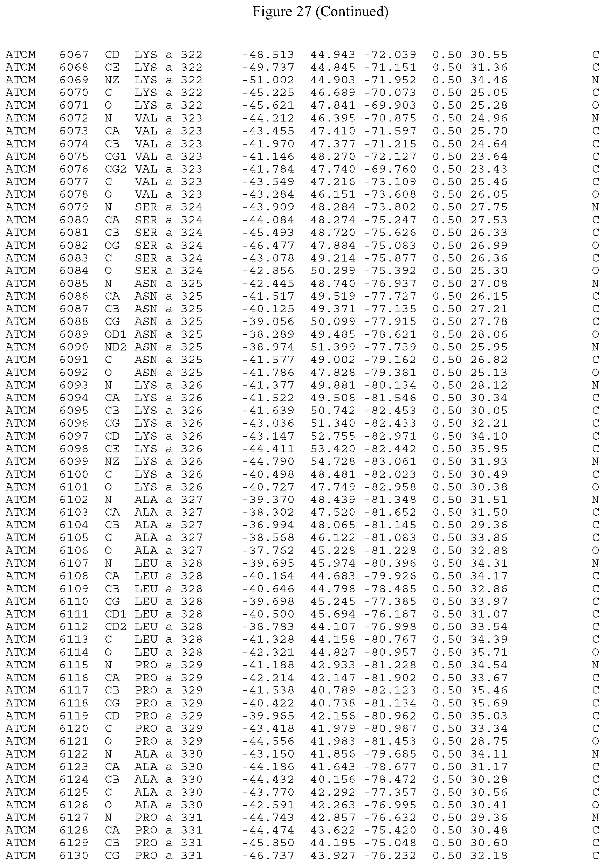

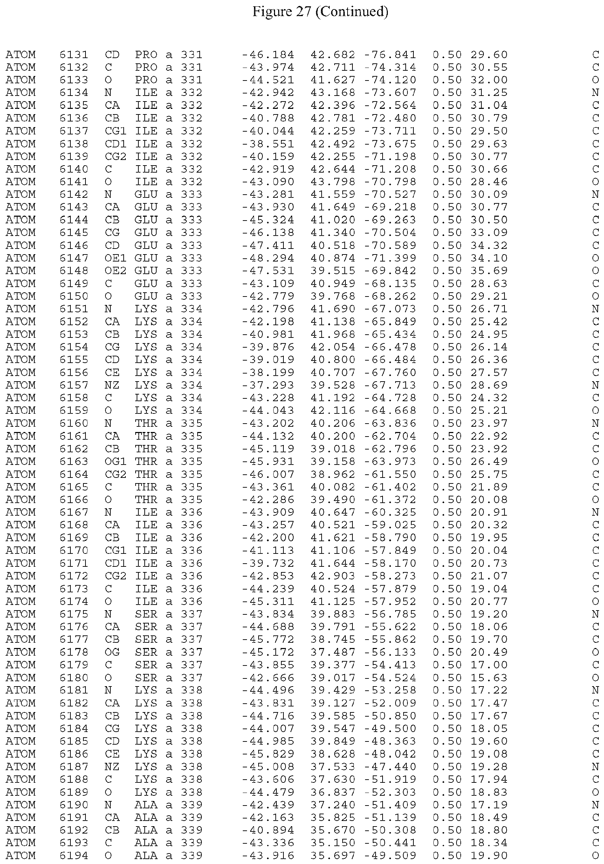

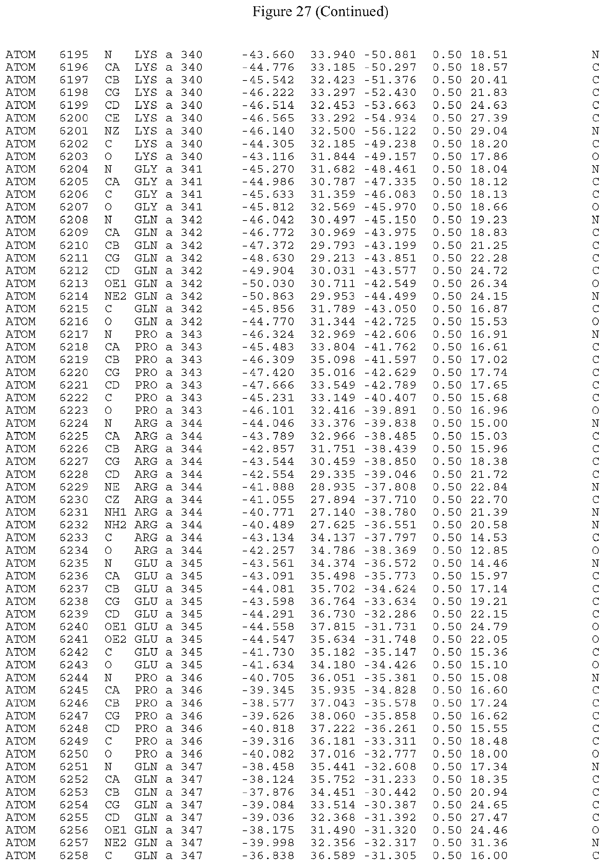

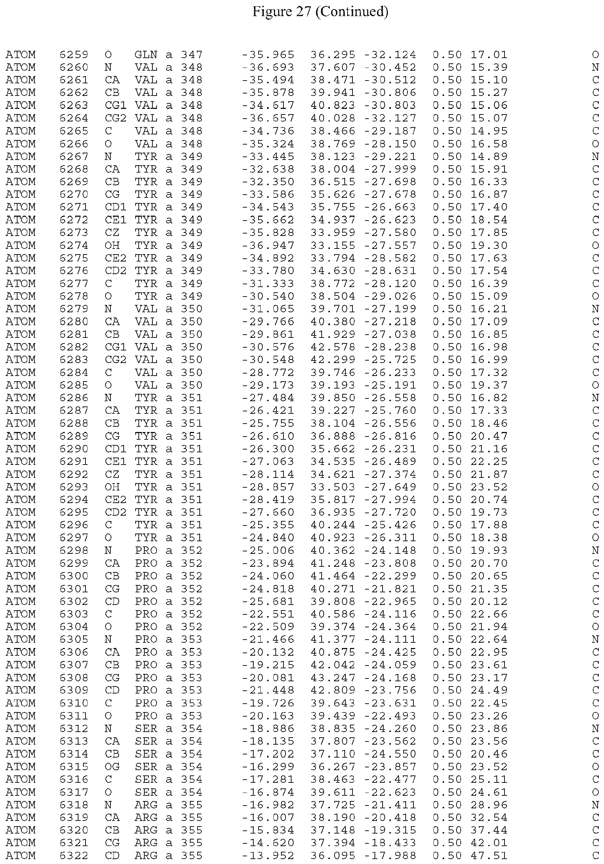

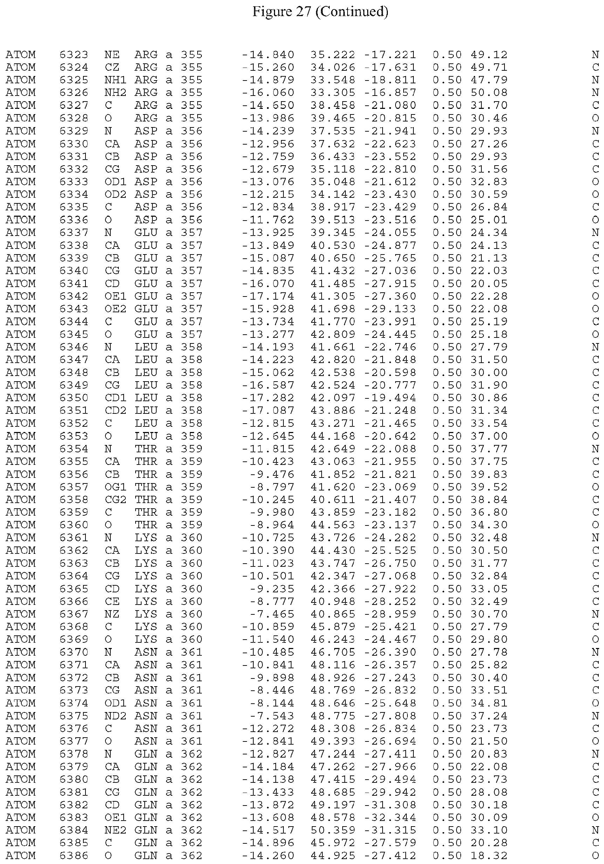

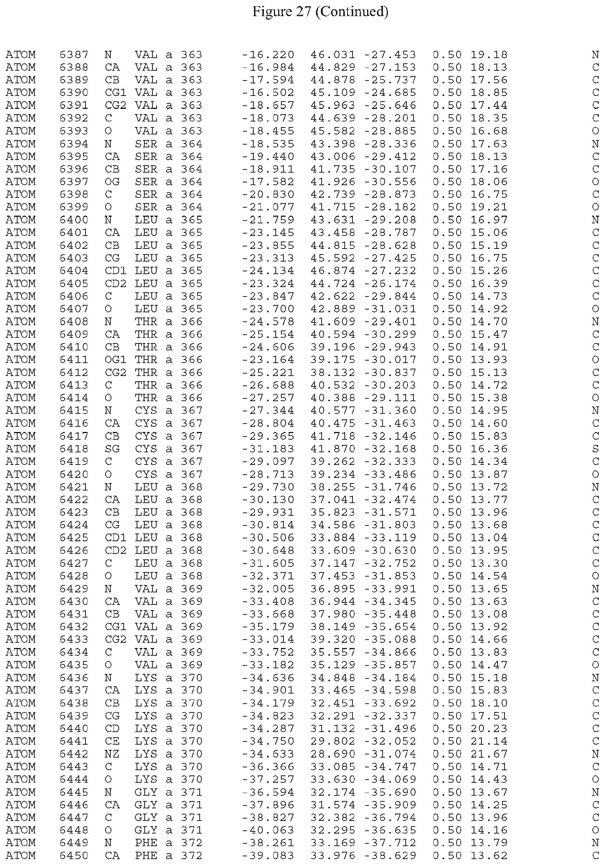

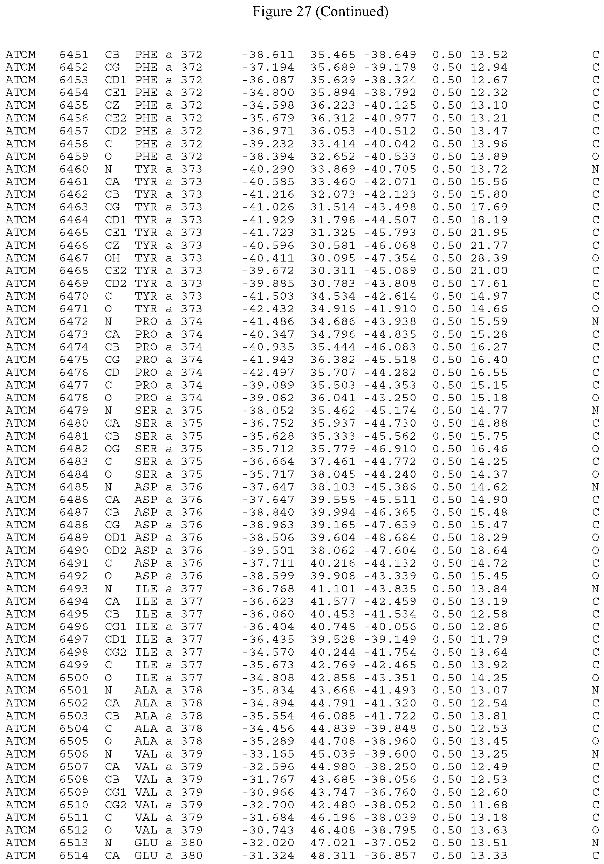

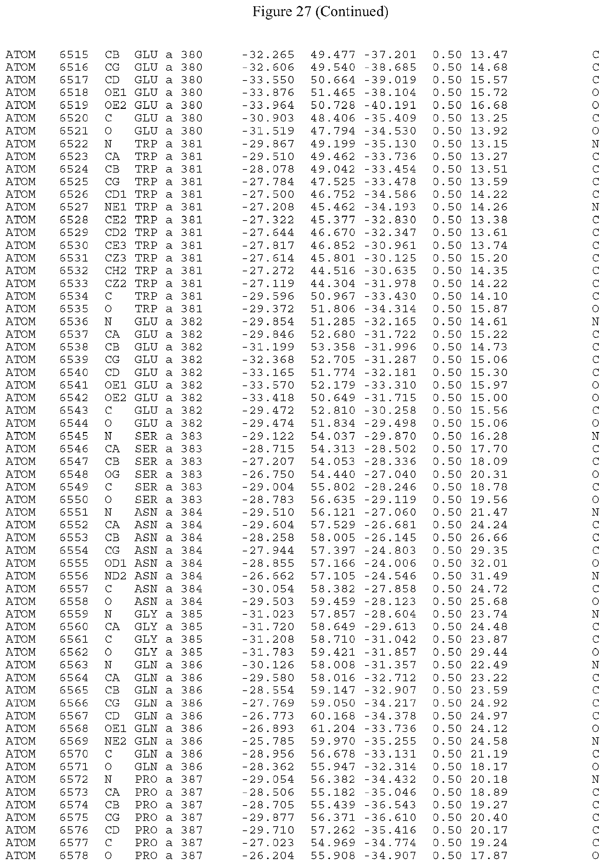

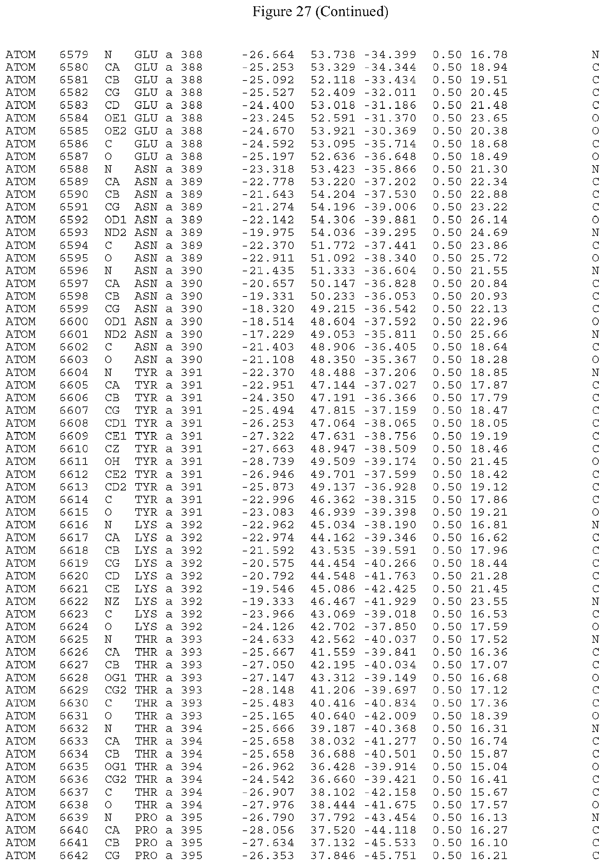

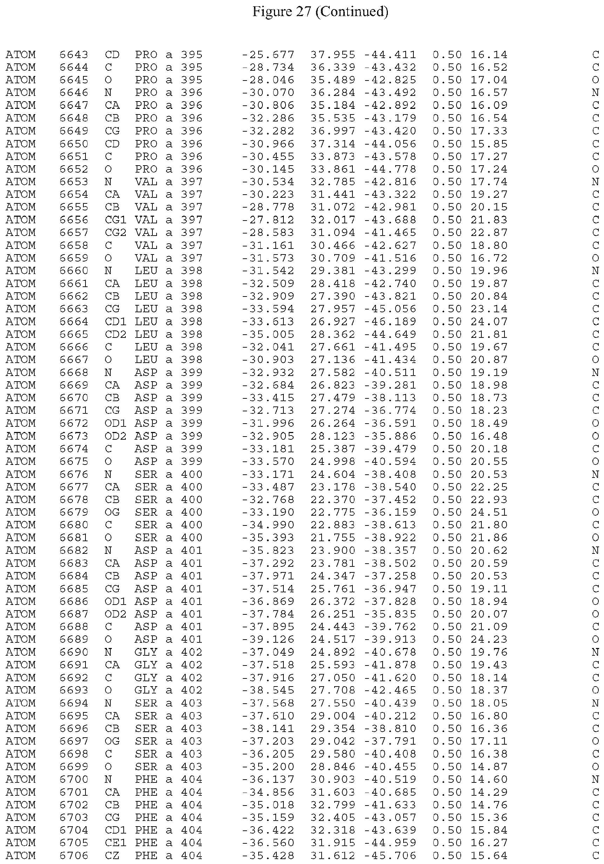

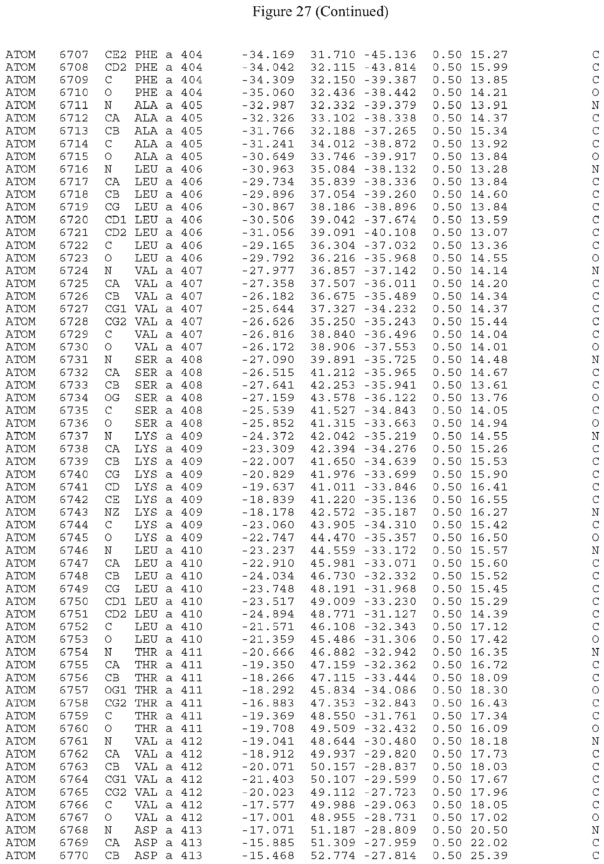

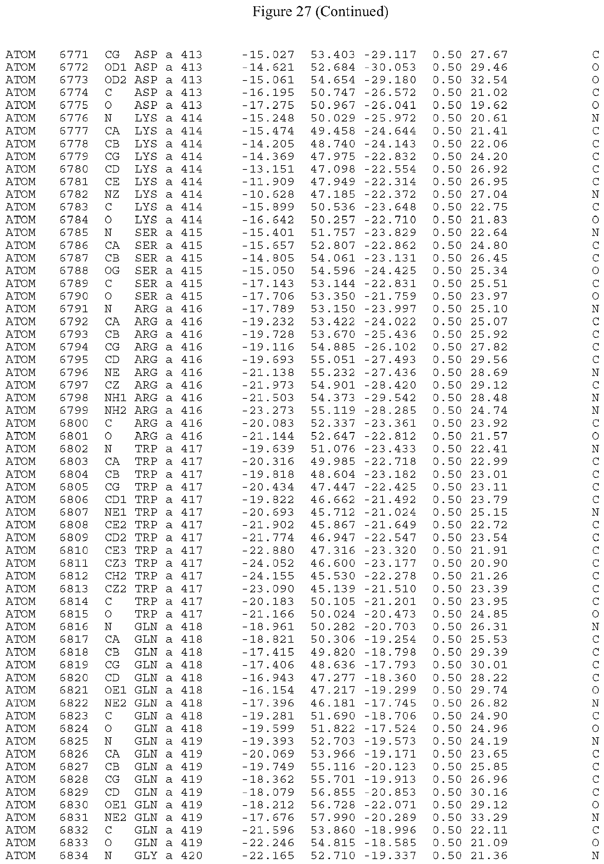

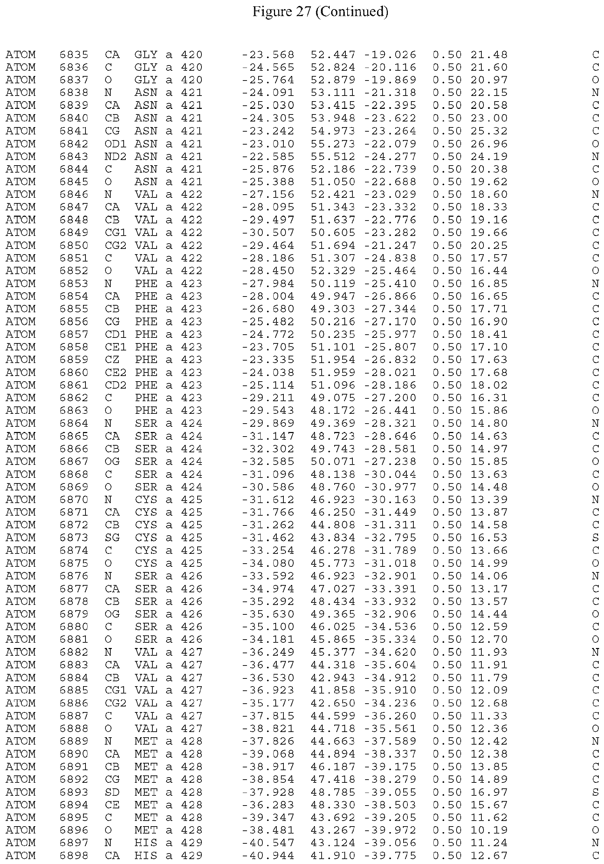

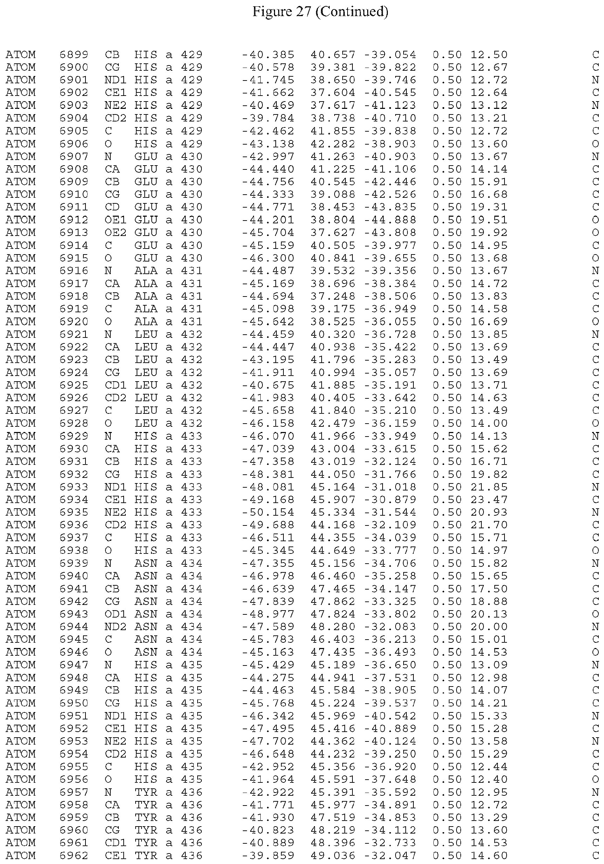

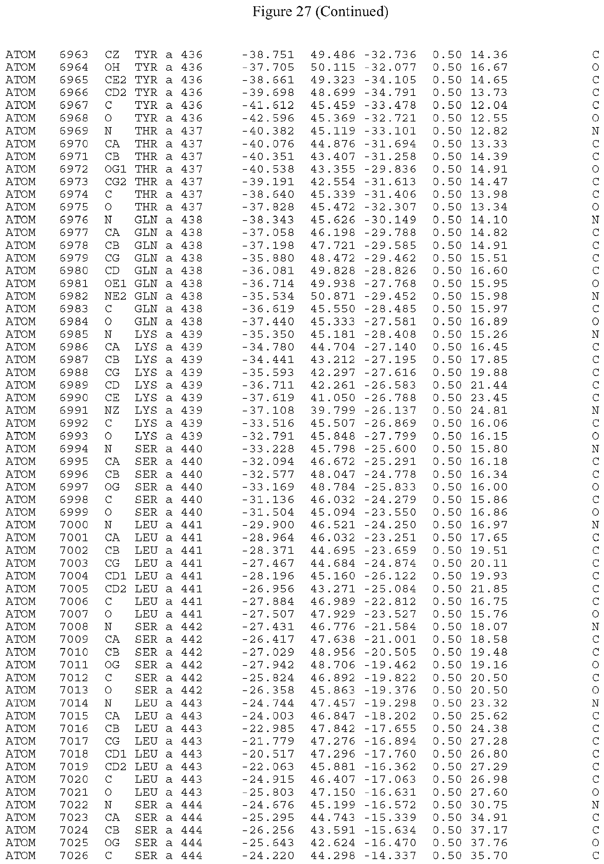

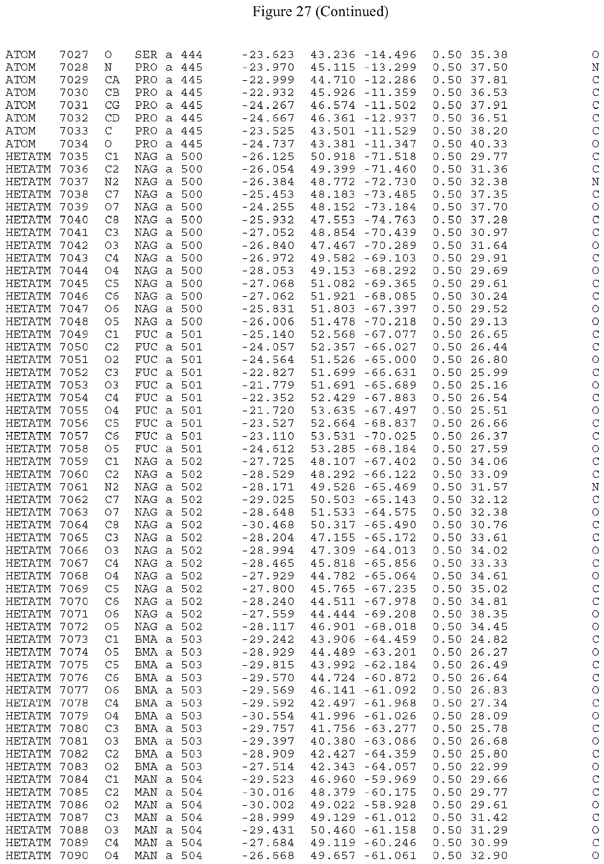

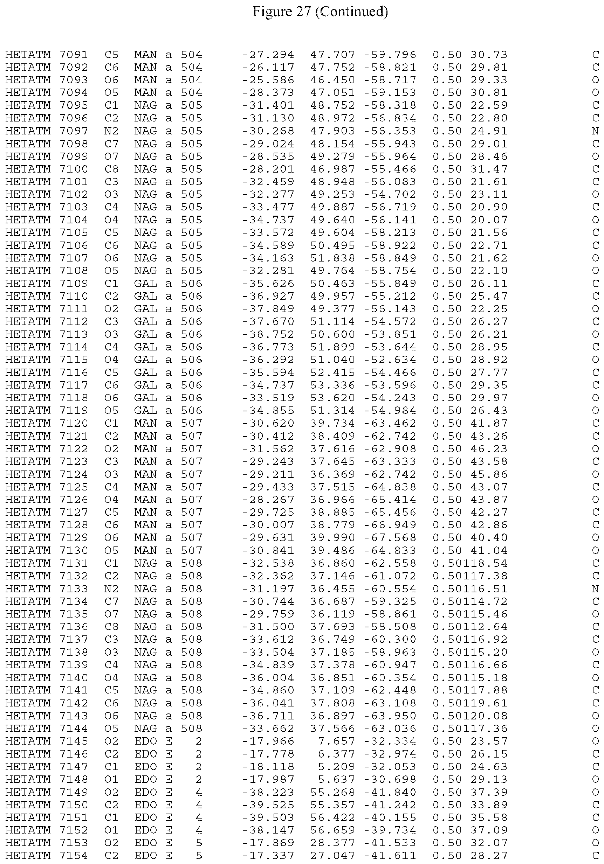

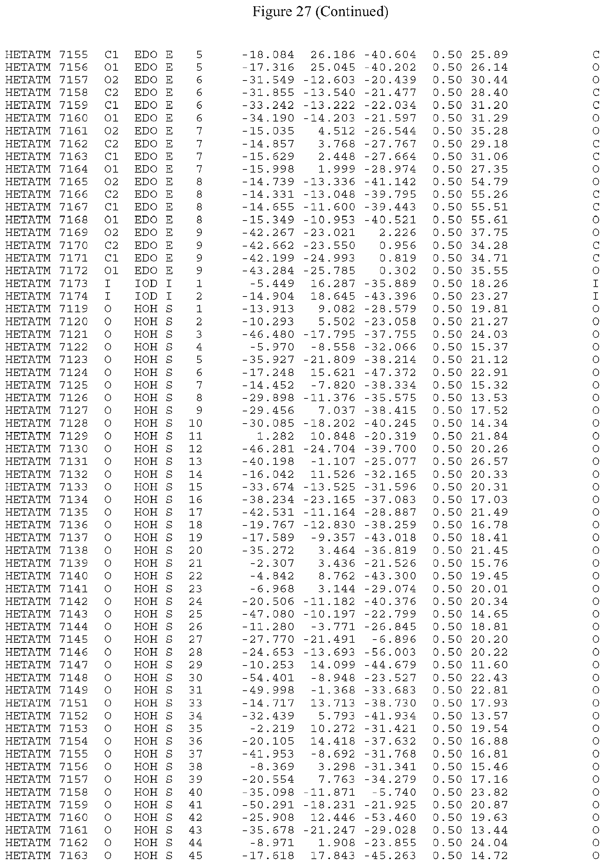

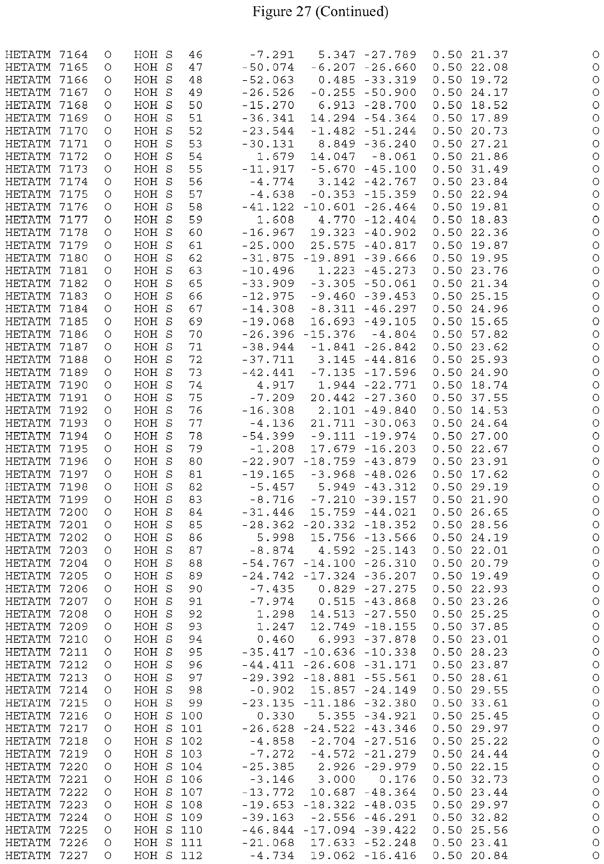

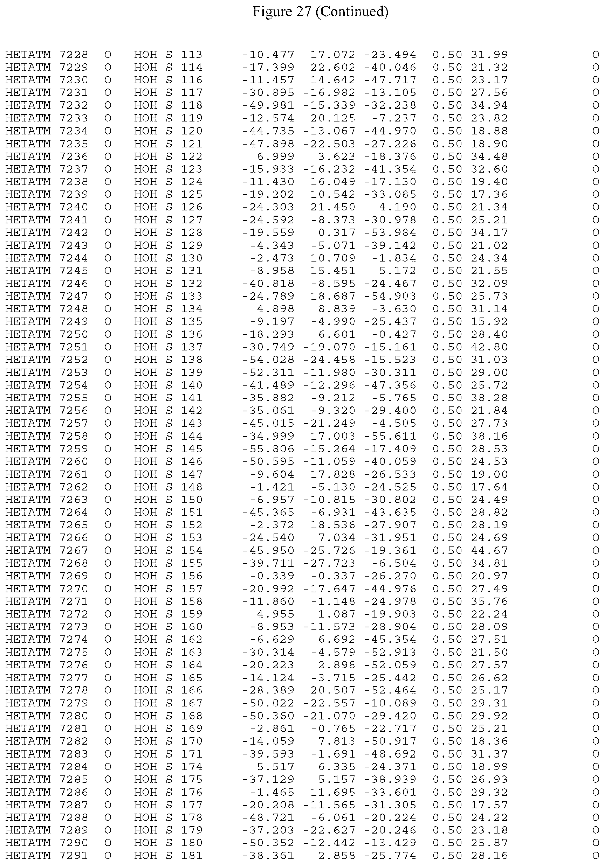

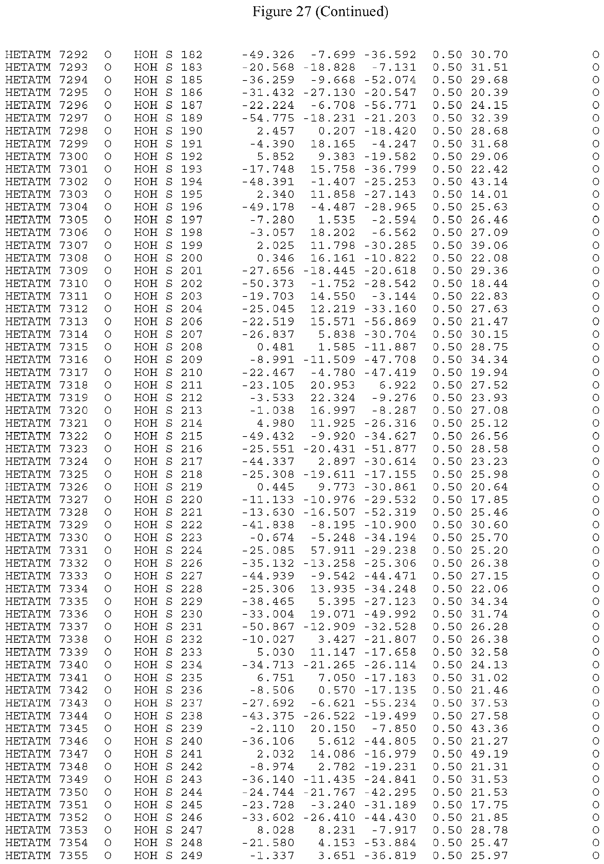

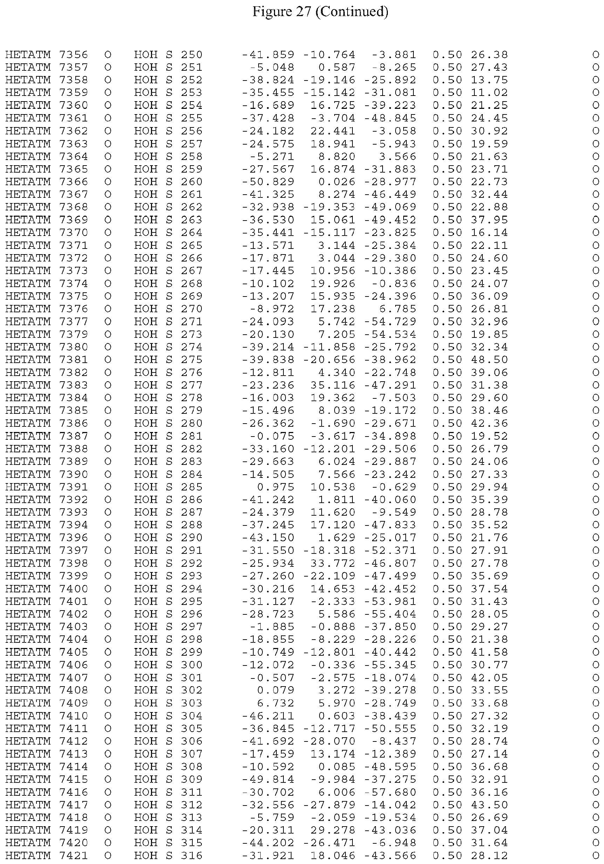

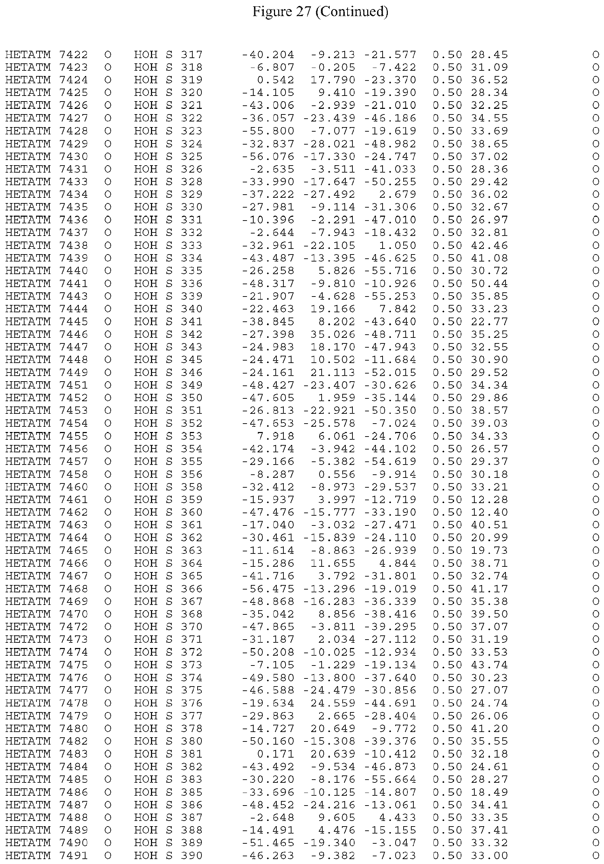

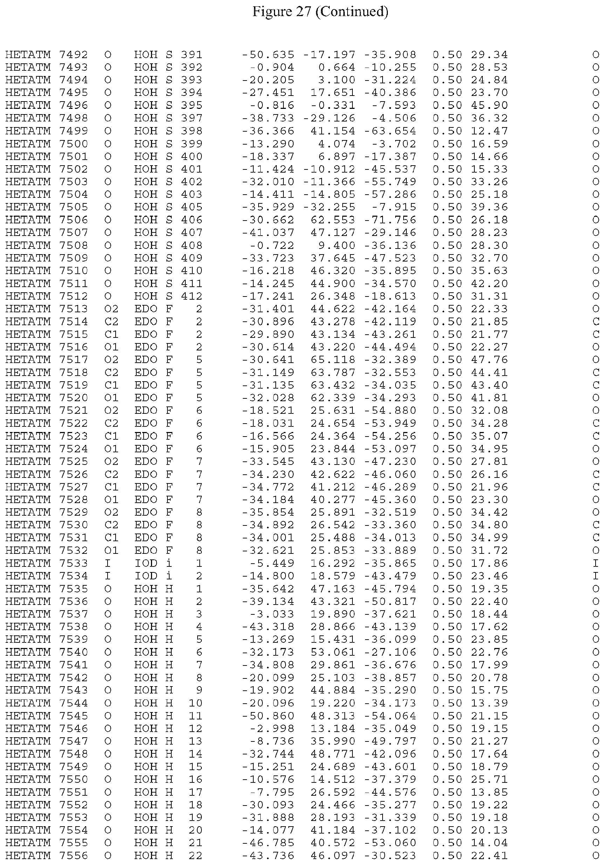

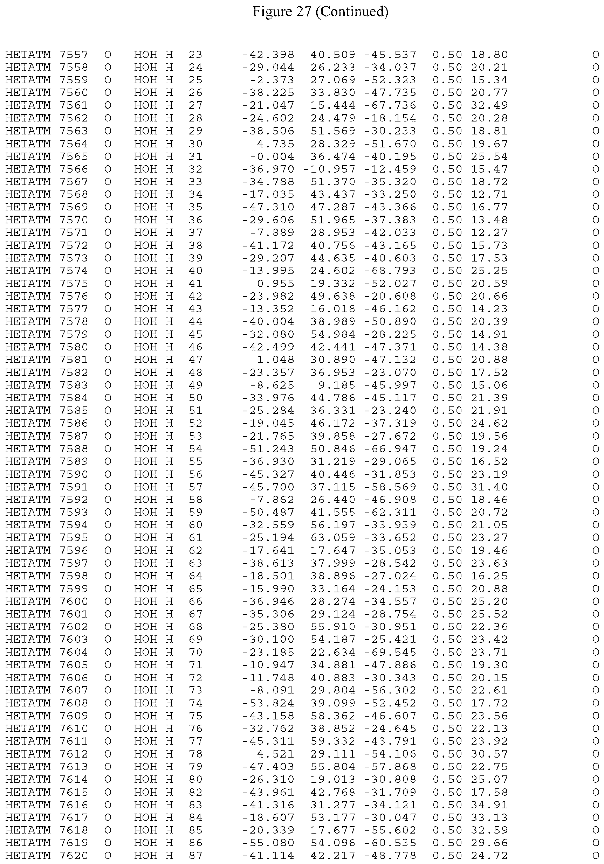

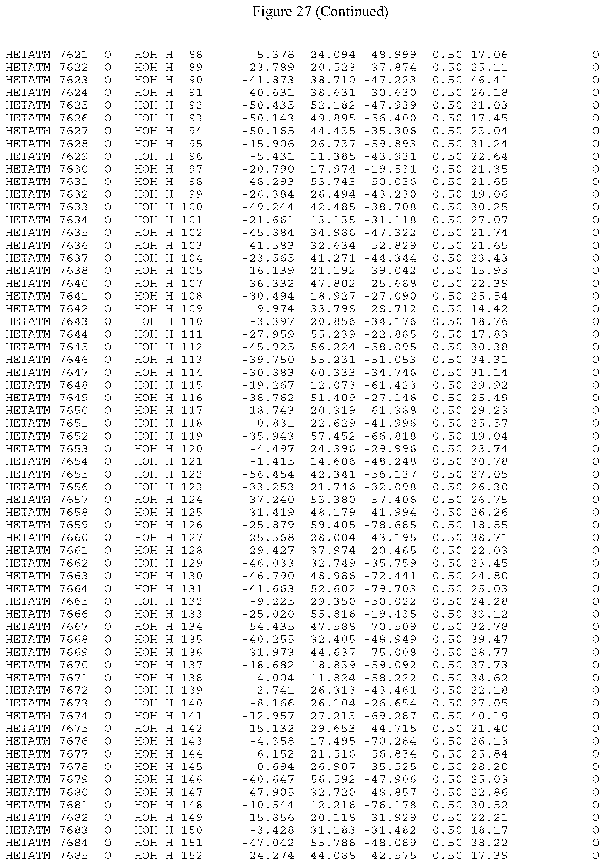

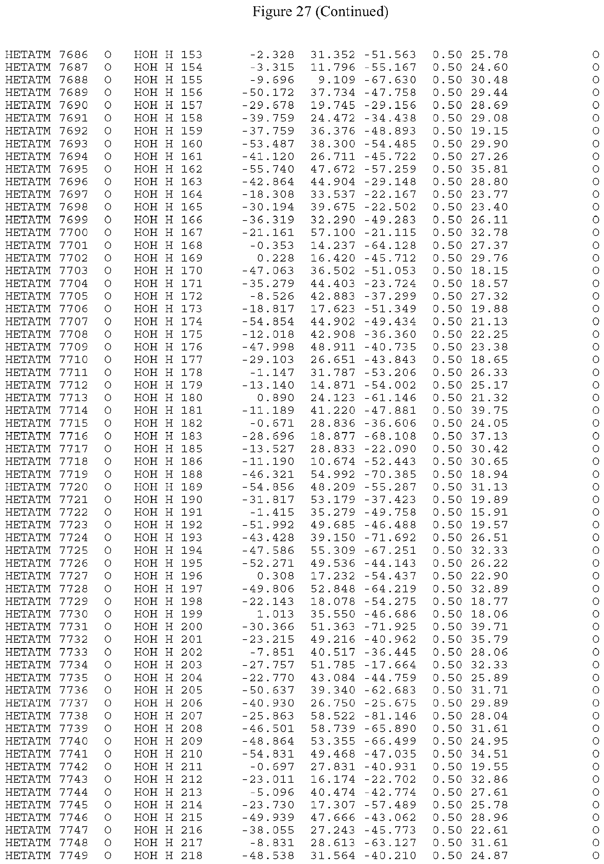

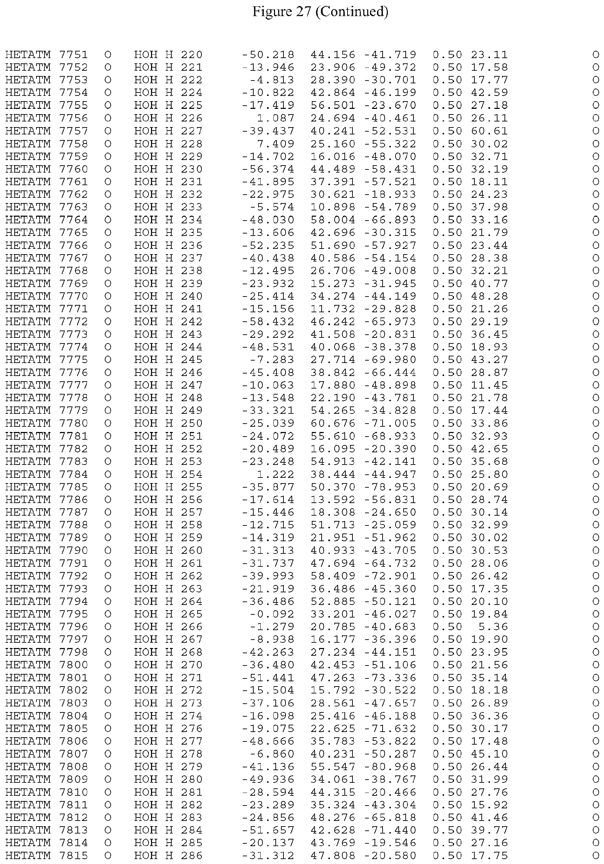

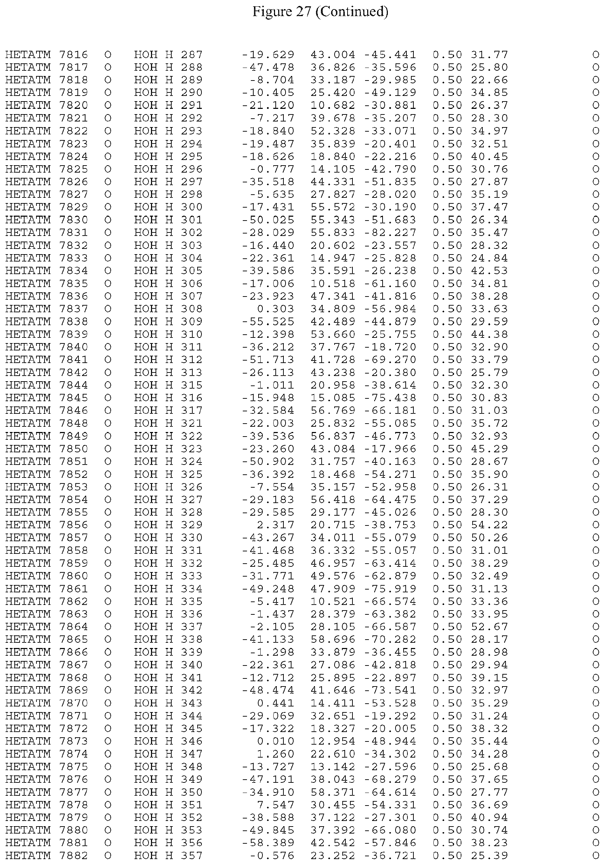

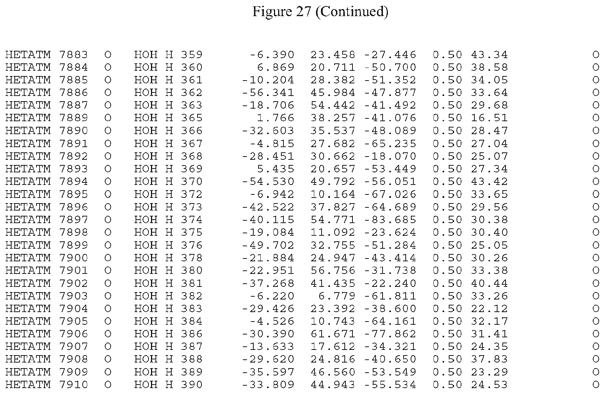

One aspect of the present disclosure provides a composition comprising an Fc heterodimer protein in crystalline form. In this aspect, the Fc heterodimer protein comprises the amino acid sequences set forth in (i) SEQ ID NOS: 2 and 3 or (ii) SEQ ID NOS: 4 and 3 of Figure 16. The crystal is in space group P2₁2₁2₁ with unit cell dimensions a = 49 ± 2 Å, b = 75 ± 2 Å, c = 149 ± 2 A, α = β = γ = 90°. In some embodiments, the Fc heterodimer protein comprises the amino acid sequences set forth in SEQ ID NOS: 2 and 3 and has a three dimensional structure characterized by the atomic coordinates of (i) chains A and B of Figure 27 or (ii) chains a and b of Figure 27. In some embodiments, the Fc heterodimer protein comprises the amino acid sequences set forth in SEQ ID NOS: 4 and 3 and has a three dimensional structure characterized by the atomic coordinates of (i) chains A and B of Figure 26 or (ii) chains a and b of Figure 26. In some embodiments, the Fc heterodimer protein comprises the amino acid sequences set forth in SEQ ID NOS: 2 and 3 forming a CH3 domain interface, and the Fc heterodimer protein provides complementary hydrophobic and electrostatic surfaces, created by residues 366, 392, 394 of SEQ ID NO: 2 and residues 351, 405, 407 of SEQ ID NO: 3, at the CH3 domain interface with distinct surface complementarity relative to wild type Fc interface surfaces. In some embodiments, the Fc heterodimer protein comprises the amino acid sequences set forth in SEQ ID NOS: 2 and 3 forming a CH3 domain interface, and the Fc heterodimer protein provides complementary hydrophobic and electrostatic surfaces, created by residues 366, 392, 394 of SEQ ID NO: 3 and residues 351, 405, 407 of SEQ ID NO: 2, at the CH3 domain interface with distinct surface complementarity relative to wild type Fc interface surfaces. In some embodiments, the Fc heterodimer protein comprises the amino acid sequences set forth in SEQ ID NOS: 4 and 3 forming a CH3 domain interface, and the Fc heterodimer protein provides complementary hydrophobic and electrostatic surfaces, created by residues 366, 392, 394 of SEQ ID NO: 4 and residues 351, 405, 407 of SEQ ID NO: 3, at the CH3 domain interface with distinct surface complementarity relative to corresponding wild type Fc interface surfaces. In some embodiments, the Fc heterodimer protein comprises the amino acid sequences set forth in SEQ ID NOS: 4 and 3 forming a CH3 domain interface, and the Fc heterodimer protein provides complementary hydrophobic and electrostatic surfaces, created by residues 366, 392, 394 of SEQ ID NO: 3 and residues 351, 405, 407 of SEQ ID NO: 4, at the CH3 domain interface with distinct surface complementarity relative to corresponding wild type Fc interface surfaces. In some embodiments, the Fc heterodimer protein comprises a D399-K409 salt bridge.

Another aspect provides a method of obtaining the above-identified composition by producing and purifying the Fc heterodimer protein and subjecting the purified Fc heterodimer protein to conditions which promote crystallization, thereby obtaining the Fc heterodimer protein in crystalline form. In some embodiments, the conditions which promote crystallization comprise mixing the purified Fc heterodimer protein with a mother liquor solution. In some embodiments the mother liquor solution comprises between 2% and 10% (v/v) ethylene glycol, between 10% and 25% (w/v) polyethylene glycol having an average molecular weight of between 2000 Daltons and 10000 Daltons, and between 0.05 M and 0.40 M ammonium iodide. In some embodiments, the mother liquor solution comprises 5% (v/v/) ethylene glycol, 18% (w/v) polyethylene glycol having an average molecular weight of 3350 Daltons, and 0.15 M ammonium iodide. In some embodiments, the purified Fc heterodimer protein is mixed with a first aliquot of the mother liquor solution and suspended over a second aliquot of the mother liquor in a hanging drop method. In some embodiments, the purified Fc heterodimer protein is mixed with a first aliquot of the mother liquor solution in a 2:1 ratio, a 1:1 ratio, a 3:1 ratio, or a 0.5:2 ratio. In some embodiments, a sitting drop method rather than a hanging drop method is used. In some embodiments, the purified Fc heterodimer protein is incubated at a temperature of between 15 °C and 25 °C after the mixing.

Another aspect provides a crystallizable composition comprising a mixture of (i) a solubilized Fc heterodimer protein comprising the amino acid sequence set forth in (a) SEQ ID NOS: 2 and 3 or (b) SEQ ID NOS: 3 and 4 of Figure 16 and (ii) a mother liquor solution. The mother liquor solution comprises between 2% and 10% (v/v) ethylene glycol, between 10% and 25% (w/v) polyethylene glycol having an average molecular weight of between 2000 Daltons and 10000 Daltons, and between 0.05 M and 0.40 M ammonium iodide. In some embodiments, the mother liquor solution comprises 5% (v/v/) ethylene glycol, 18% (w/v) polyethylene glycol having an average molecular weight of 3350 Daltons, and 0.15 M ammonium iodide.

Another aspect provides a method of identifying a mutation which promotes heterodimeric Fc chain pair formation. The method comprises performing structure based modeling, using a suitably programmed computer, to identify a candidate mutation to an Fc chain using a three-dimensional atomic crystal structure of an Fc heterodimer protein which is defined by the atomic coordinates of any combination of chains a, b, A, and B of Figures 26 or 27 determined from an X-ray diffraction quality crystal of the Fc heterodimer protein. The Fc heterodimer protein comprises the amino acid sequences as set forth in (i) SEQ ID NOS: 2 and 3 or (ii) SEQ ID NOS: 4 and 3, and the X-ray diffraction quality crystal is in an orthorhombic space group. In some embodiments, the orthorhombic space group is P2₁2₁2₁ and has unit cell dimensions a = 49 ± 2 Å, b = 75 ± 2 Å, c = 149 ± 2 Å, α = β = γ = 90°. In some embodiments, the structure based modeling comprises (a) identifying a plurality of residues on the three-dimensional structure that influence heterodimeric Fc chain pair formation, (b) modeling a plurality of three-dimensional Fc structures using the three-dimensional atomic crystal structure as a template, wherein each three-dimensional Fc structure in the plurality of three-dimensional Fc structures includes mutations to one or more of the residues in the plurality of residues, (c) comparing each three-dimensional Fc structure in the plurality of three-dimensional Fc structures to the three-dimensional atomic crystal structure, and (d) selecting one of the three-dimensional Fc structure in the plurality of three-dimensional Fc structures based on the comparing (c). In some embodiments, the comparing (c) compares a calculated thermodynamic property of the three-dimensional atomic crystal structure to a calculated thermodynamic property of a three-dimensional Fc structure in the plurality of three-dimensional Fc structures. In some embodiments, the thermodynamic property is entropy, average energy, average enthalpy, free energy or heat capacity. In some embodiments, the comparing (c) compares a physical property of the three-dimensional atomic crystal structure to a calculated thermodynamic property of a three-dimensional Fc structure in the plurality of three-dimensional Fc structures, where the physical property is selected from the group consisting of (i) one or more electrostatic interactions, (ii) one or more polar interactions, (iii) one or more hydrogen-bond interactions, (iv) a comparison of buried versus accessible surface area, (v) accessible surface area, (vi) one or more hydrophobic interactions, and (vii) presence or absence of one or more buried water molecules.

### BRIEF DESCRIPTION OF THE FIGURES

Figure 1 provides an illustration of the two observed orientations of the Fc heterodimers AZ1 and AZ2, with respect to Chain A and Chain B in the crystal, in accordance with an embodiment of the present disclosure.
Figure 2 illustrates electron density at sites of mutation in the disclosed crystallographic structures, in accordance with some embodiments of the present disclosure.
Figure 3 illustrates electron density at sites of mutation in the disclosed crystallographic structures, in accordance with some embodiments of the present disclosure.
Figure 4 illustrates electron density at sites of mutation in the disclosed crystallographic structures, in accordance with some embodiments of the present disclosure.
Figure 5 compares the disclosed crystal structure of AZ1 to the predicted *in silico* model for AZ1, in accordance with some embodiments of the present disclosure.
Figure 6 also compares the disclosed crystal structure of AZ1 to the predicted *in silico* model for AZ1, in accordance with some embodiments of the present disclosure.
Figure 7 provides the superposition of CH3-CH3 domain of the disclosed AZ1 heterodimer with high resolution wild-type homodimer Fc crystal structures, in accordance with some embodiments of the present disclosure.
Figure 8 summarizes the backbone RMSD (root mean square deviation) calculations of alignments of respective crystal structures over the dimeric CH3-CH3 domains in accordance with some embodiments of the present disclosure.
Figure 9 provides a glycosylation analysis of AZ1, indicating that it has a wild-type glyco-pattern, in accordance with some embodiments of the present disclosure.
Figure 10 shows the FcgR binding affinities of the disclosed constructs, AZ1 and AZ2, relative to Trastuzumab wild type, in accordance with some embodiments of the present disclosure.
Figure 11 illustrates the ADCC activity of an anti-Her2 heterodimeric antibody (anti-her2(Herceptin)-AZ1) and parent Trastuzumab against the melanoma cell line SKOV3, measured using human peripheral blood mononuclear cells (PBMC) as effector cells, in accordance with some embodiments of the present disclosure.
Figures 12 and 13 illustrate the CDC activity of an anti-CD20 heterodimeric antibody (anti-CD20(Rituximab)-AZ1) and parent Rituximab as control was determined using human serum as a complement source, against the human CD20 B lymphocyte cell line Raji, in accordance with some embodiments of the present disclosure.
Figure 14 summarizes FcRn binding affinities for Trastuzumab WT, and trastuzumab-based heterodimeric antibodies anti-her2-AZ1, and anti-her2(Herceptin)-AZ2, in accordance with some embodiments of the present disclosure.
Figure 15 illustrates a pharmacokinetic (PK) study in which the Trastuzumab based anti-her2 AZ1 heterodimeric antibody (anti-her2(Herceptin)-AZ1) was injected intravenously into nude mice at 4 different dose levels of 1, 8, 24 and 80mg/kg, and the plasma clearances were monitored by an anti-Trastuzumab specific ELISA, in accordance with some embodiments of the present disclosure.
Figure 16 provides the primary amino acid sequences of AZ1 and AZ2, and the amino acid sequence of the portion of immunoglobulin G 1 (IgG1) isotype that served as the starting point to the derivation of AZ1 and AZ2, in accordance with some embodiments of the present disclosure.
Figure 17 provides steps in an iterative rational protein engineering strategy in accordance with some embodiments of the present disclosure.
Figure 18 provides a schematic representation of design space addressed in the current and prior work in order to achieve heterodimer pairing in a mutated Fc in accordance with some embodiments of the present disclosure.
Figure 19 provides a computational structure function analysis and screening strategy used in accordance with some embodiments of the present disclosure.
Figure 20 illustrates the wild type Fc versus an initial heterodimer design and the structural rationale for the key additional swap T366L. T366 is a hotspot in wild type Fc and, while it does not contribute to heterodimer formation, it is still present as a hotspot in the undesired wild type like homodimers. The rationale is supported by the introduced single additional swap T366L which improved the heterodimer purity from ∼90 to >95%.
Figure 21 illustrates the utility and importance of the conformational dynamics analysis of the initial negative design as described in detail herein. The predicted model after in silico mutagenesis (backbone conformation close to WT) is superimposed with a representative structure of a 50ns Molecular Dynamics simulation analysis. The figure highlights the large conformational difference in the loop region D399-S400 versus wild type, which in turn exposes the hydrophobic core to solvent and causes decreased stability of the initial heterodimer.
Figure 22 illustrates how information from the comprehensive *in silico* analysis disclosed herein and the molecular dynamics simulation was used to identify the key K392M mutation, which stabilized the loop conformation and increased the stability of the initial negative design variant by ∼4 °C (CH3-CH3 Tm).
Figure 23 illustrates how the *in silico* analysis disclosed herein indicated that one of the reasons for the lower than wild type stability of the initial heterodimer is the loss of the core interaction/packing of Y407 and T366. The initial heterodimer shows non-optimal packing at this hydrophobic core. The figure illustrates how the distal mutation L351Y was able to stabilize the heterodimer by coupling effects and improved hydrophobic packing, without impacting the initial mutations T366L/Y407V, which are essential for heterodimer specificity.
Figure 24 shows a comparison of wild type IgG1 Fc and ZW1 and illustrates the second shell, distal position of the key stabilizing mutation T350V.
Figure 25 provides the amino acid numbering used herein according to the EU index as set forth in Kabat for the CH2 and CH3 domains from human IgG1. *See,* Kabat *et al*., 1991, NIH Publication 91-3242, National Technical Information Service, Springfield, Virginia.
Figure 26 provides the structure coordinates for AZ2 (including SEQ ID NO: 4 and SEQ ID NO.: 3) in accordance with some embodiments of the present disclosure.
Figure 27 provides the structure coordinates for AZ1 (including SEQ ID NO: 2 and SEQ ID NO.: 3) in accordance with some embodiments of the present disclosure.
Figure 28 is a block diagram illustrating a system for performing aspects of the present disclosure.

### DETAILED DESCRIPTION

A rational structure and computational modeling guided IgG1 Fc engineering effort to preferentially achieve heterodimeric Fc proteins with wild type Fc like stability is disclosed. The engineering approach utilizes distinct mutations at the CH3 interface to preferentially drive heterodimer formation and prevent the formation of homodimers. The designed heterodimer achieves over 99% purity while retaining the wild type Fc like stability, as demonstrated by thermal melting (CH3 Tm of ∼82 °C) and accelerated aggregation assessment under forced degradation conditions. Further, validation of the Fc heterodimer protein by stable cell line development and early manufacturability assessment shows no impact of the CH3 mutations on the preferred wild type Fc stability.

Independent of the high specificity and stability of the designed Fc heterodimeric proteins, an additional requirement for the successful design of bispecific heterodimeric antibodies includes one or more of favorable pharmacokinetic properties, Fc effector function and decreased immunogenicity. To ensure these drug-like properties, it is desirable to preserve the wild type Fc surface characteristics and retain the natural symmetry of the wild type Fc. Introduction of asymmetric steric or electrostatic mutations at the CH3 interface as in the case of prior studies, can potentially also induce an asymmetry or shift in the naturally symmetric orientation of the two CH3 domains. This leads to an altered Fc surface area and likely presents a significantly higher risk of immunogenicity and optimal pharmacokinetic properties. In addition, distal CH3 mutations have been shown to alter FcgammaR binding and thus, breaking the natural CH3 symmetry by mutations in the CH3 interface can similarly impact the wild type Fc functionality. *See,* Shields et al., 2001, Journal of Biological Chemistry 276: 6591-6604. Thus, retaining the natural CH3 symmetry is likely an important consideration to ensure wild type like Fc functionality, optimal pharmacokinetic properties and low immunogenicity.

To investigate the impact of the CH3 interface mutations of the disclosed Fc heterodimeric proteins on the preferred wild type IgG structure and properties and to further validate the disclosed scaffold, the crystal structures of two such Fc heterodimeric proteins along with experimental data to assess Fc effector functionality and pharmacokinetic properties was elucidated and is disclosed herein.

In order that the invention described herein may be more fully understood, the following detailed description is set forth.

Throughout the specification, the word "comprise" or variations such as "comprises" or "comprising" will be understood to imply the inclusion of a stated integer or groups of integers but not the exclusion of any other integer or groups of integers.

The following abbreviations are used throughout the application:

The following abbreviations are used herein for amino acids: A = Ala = alanine; T = Thr = threonine; V = Val = valine; C = Cys = cysteine; L = Leu = leucine; Y = Tyr = tyrosine; I = Ile = isoleucine, N = Asn = asparagine; P = Pro = proline; Q = Gln = glutamine; F = Phe = phenylalanine; D = Asp = aspartic acid; W = Trp = tryptophan; E = Glu = glutamic acid; M = Met = methionine; K = Lys = lysine; G = Gly = glycine; R = Arg = arginine; S = Ser = serine; and H = His = histidine;

As used herein, the following definitions shall apply unless otherwise indicated. Also, combinations of substituents or variables are permissible only if such combinations result in stable compounds.

The term "about" when used in the context of RMSD (root mean square deviation) values takes into consideration the standard error of the RMSD value, which is ± 0.1 Å.

The term "aliphatic" refers to straight chain or branched hydrocarbons that are completely saturated or that contain one or more units of unsaturation. For example, aliphatic groups include substituted or unsubstituted linear or branched alkyl, alkenyl and alkynyl groups. Unless indicated otherwise, the term "aliphatic" encompasses both substituted and unsubstituted hydrocarbons. The term "alkyl", used alone or as part of a larger moiety, refers to both straight and branched saturated chains containing one to twelve carbon atoms. The terms "alkenyl" and "alkynyl", used alone or as part of a larger moiety, encompass both straight and branched chains containing two to twelve carbon atoms and at least one unit of unsaturation. An alkenyl group contains at least one carbon-carbon double bond and an alkynyl group contains at least one carbon-carbon triple bond.

The term "structure coordinates" refers to Cartesian coordinates derived from mathematical equations related to the patterns obtained on diffraction of a monochromatic beam of X-rays by the atoms (scattering centers) of a protein or protein complex in crystal form. The diffraction data are used to calculate an electron density map of the repeating unit of the crystal. The electron density maps are then used to establish the positions of the individual atoms of a complex or native macromolecular structure.

The term "CH3-CH3 domain" refers to the pair of CH3 domains that are part of the IgG antibody Fc structure. The CH3 domain in each of the two chains of the Fc interface contact the complementary chain at the CH3 domain interface (also referred to herein as "CH3 interface").

The term "CH2-CH3 domain" refers to the CH2 and CH3 domains present in tandem in each of the two chains of IgG antibody. An Fc structure comprises two CH2-CH3 domains.

The term "Fc heterodimer protein" refers to an Fc structure in which the two chains constituting the Fc structure do not have the same primary protein sequence. The primary protein sequence of both the CH3 domains in a native (wild type) IgG1 antibody is the same leading to the formation of a homodimeric Fc structure. On the other hand, engineering at the CH3-CH3 domain interface is performed to achieve a Fc heterodimer protein wherein the two CH3 domains no longer have the same primary protein sequence.

The term "heterodimeric Fc chain pair" refers to the two polypeptide chains constituting the Fc structure where the two polypeptide chains do not have the same primary protein sequence.

Disclosed is a transferable immunglobulin G-based Fc heterodimeric protein which achieves over 99% heterodimer purity while retaining wild type Fc-like stability. The Fc heterodimeric protein is a scaffold that can be used to prepare bispecific heterodimeric antibodies. The Fc heterodimeric protein has been successfully validated for stable cell line development and early manufacturability assessment using industry standard processes for cell line and downstream process development. Apart from the manufacturability of the bispecific heterodimeric antibodies other additional requirements for successful design of a therapeutic scaffold include favorable pharmacokinetic properties, Fc effector function and low immunogenicity. This is particularly important in the development of bispecific heterodimeric IgG1 like scaffolds, where the introduction of mutations in the IgG constant region and the resulting reduced stability of the CH3 domain, as is observed in a number of other bispecific scaffolds, could have an impact on immunogenicity and the preferred pharmacokinetic properties. To ensure these drug-like properties in the development of a modular scaffold, it is important to also validate that the engineered Fc mutations in the bispecific scaffold preserve the wild type Fc surface characteristics and retain the natural symmetry of the wild type Fc, as these are determinants of immunogenicity and Fc effector function.

*Rational Engineering Strategy.* The design of variant Fc heterodimeric proteins from wild type homodimers is illustrated by the concept of positive and negative design in the context of protein engineering by balancing stability vs. specificity, in which mutations are introduced with the goal of driving heterodimer formation over homodimer formation. Negative design strategies focus on maximizing unfavorable interactions for the formation of homodimers, by e.g. introducing mutations that lead to steric clashes or electrostatic repulsion in homodimer formation. In contrast, in positive design approaches, amino acid modifications are introduced into polypeptides to maximize favorable interactions within or between proteins. This strategy assumes that when introducing multiple mutations that specifically stabilize the desired heterodimer while neglecting the effect on the homodimers, the net effect will be a preference for the desired heterodimer interactions over the homodimers and hence a greater heterodimer specificity. It is understood in the context of protein engineering that positive design strategies optimize the stability of the desired protein interactions, but rarely achieve >90% specificity, whereas negative design approaches have successfully been employed to achieve close to 100% specificity, but with significant loss in stability of the desired product.

A challenge in protein-protein engineering for altered specificity and in designing heterodimers from natural homodimers is to achieve close to 100% specificity while maintaining the wild-type complex/homodimer affinity and stability. This is likely more challenging if the natural complex has a high affinity and complex stability, like *e.g.* the Fc CH3-CH3 domain, which has been reported to have a natural affinity in the pM range.

This challenge is reflected in the Fc heterodimeric protein designs by point mutations in the CH3-CH3 domain, which have achieved high selectivity of >95% heterodimer purity, but with significantly lower stability as indicated by the CH3-CH3 Tm. *See* Table 1, below. For example, the knobs-into-holes strategy developed by Genentech, or the electrostatic steering strategy developed by Amgen have employed mainly negative design asymmetric point mutations to drive heterodimer formation, which lead to high heterodimer specificity but low stability. In a subsequent development by Genentech the initial knobs-into-holes design was optimized for higher stability by experimental library screening and by disulfide stabilization. While the library approach only gained stabilization by 1-2 deg to ∼70 °C, the disulfide stabilization was more successful with an increase of CH3 Tm to >77 °C. Since the engineered disulfide is partially solvent exposed, it remains questionable whether this stabilization is a viable option to ensure long term stability and in vivo stability. In the disclosed approach, disulfide engineering for heterodimer stabilization is avoided in order to prevent potential complications in manufacturability and formulation.

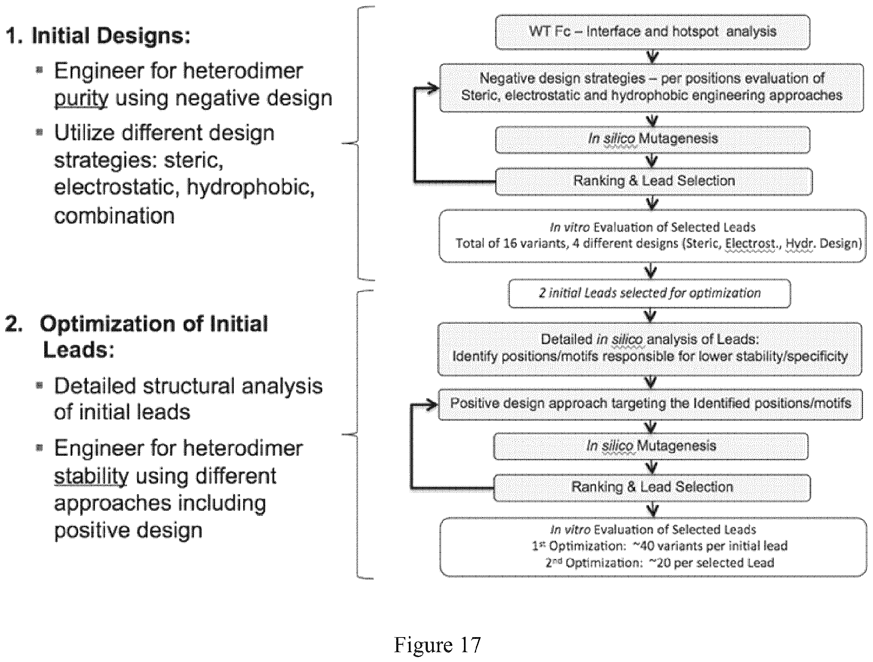

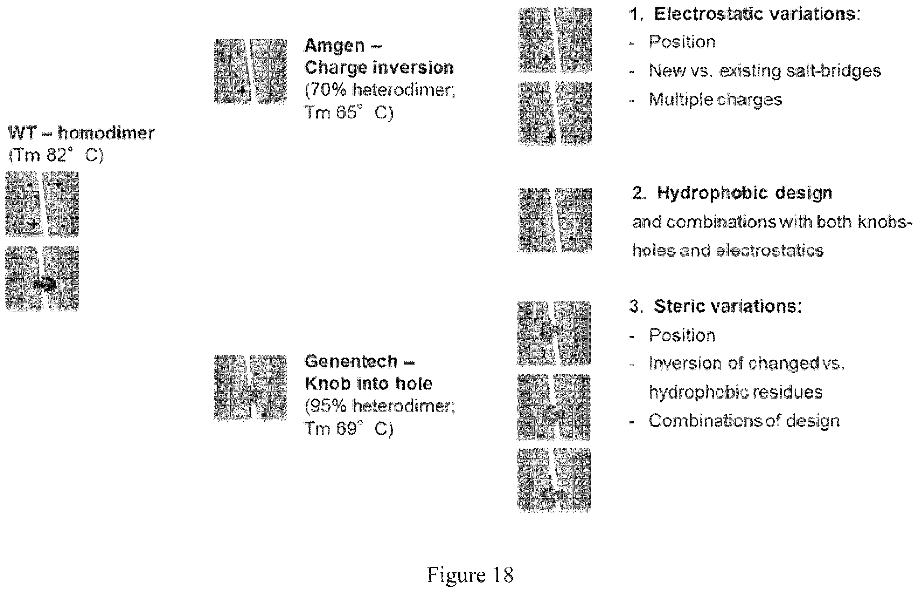

To address the challenges in Fc heterodimeric protein engineering, disclosed is the implementation of a two stage approach that specifically combines negative and positive design strategies to achieve 100% specificity and wild-type like CH3-CH3 stability as summarized in Figure 17. Specifically, in the initial design phase the core interface positions were computationally screened using different negative design strategies, including steric-, electrostatic- and hydrophobic-design approaches as shown in Figure 18, and the variants with predicted high heterodimer specificity were tested experimentally for expression and stability as described below. A total of sixteen variants based on four core designs were experimentally characterized in the initial design phase. From this initial set of negative design Fc variant heterodimers, which were expected to have lower stability, the Fc variant heterodimers with greater than 90% purity and a melting temperature of about 68 °C or greater were selected for further development. In the second design phase the selected Fc variant heterodimers were each analyzed with computational methods and comprehensive structure function analysis to identify the structural reasons these Fc variants had a lower stability than the wild-type Fc homodimer, which is 83 °C for IgG1. Following a detailed computational and structural analysis those selected Fc variant heterodimers were further modified to drive both stability and purity using positive design strategies.

**Table 1: Published Fc Heterodimeric Antibodies.**

| | **Chains** | **Engineering Approach** | **Source** | **Purity** | **Tm °C** |
|---|---|---|---|---|---|
| **Wild-Type** | - | | | | 83 |
| | - | | | | |
| Control 1 | K409D_K392D | | Gunaskekaran et al, 2010, J. Biol. Chem. 285(25);19637 -19646 | >95% | 67 |
| | D399K_E356K | Electrostatic steering | | | |
| Control 2 | K409D_K392D | | Gunaskekaran et al, 2010, J. Biol. Chem. 285(25);19637 -19646 | <80% | - |
| | D399K | Electrostatic steering | | | |
| Control 3 | T366S_L368A_Y407V | Knobs-into-holes (KH) | Atwell et al., 1997, J. Mol. Biol. 270: 26-35. | >95% | 69 |
| | T366W | | | | |
| Control 4 | Y349C_T366S_L368A Y407V | Knobs-into-holes (KH) plus disulfide | Merchant et al., 1998, Nature Biotechnology 16:677-681. | >95% | ** |
| | S354C_T366W | | | | |
| Control 5 | IgG-IgA chimera | Strand Exchange | Muda et al., 2011, Protein Engineering, Design and Selection 24:447-454. | 90% | 68 |

| | | | | | |
|---|---|---|---|---|---|
| ** A Tm greater than 77 °C was observed for control 4 in the assay system used; the Tm for this variant has not been published in the literature. | | | | | |

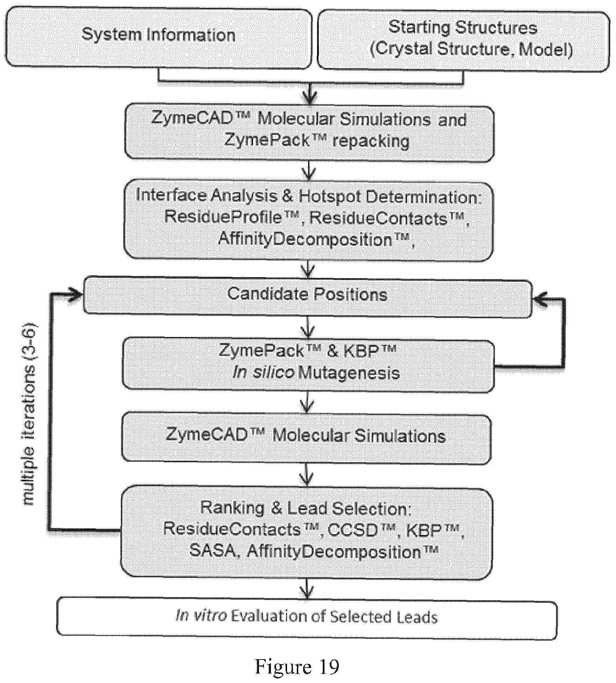

*Computational Engineering Strategy.* The computational tools and structure-function analysis included molecular dynamic (MD) analysis, protein amino acid sidechain/backbone re-packing, bioinformatics sequence and structural database derived statistical potentials (KBP), cavity and (hydrophobic) packing analysis Lennar-Jones interactions, contact density estimates (CCSD), changes in solvent accessibility of different functional groups in the protein (SASA), electrostatic-GB calculations, and coupling analysis as indicated in Figure 19.

An aspect of the disclosed protein engineering approach relied on combining structural information of the Fc IgG protein derived from X-ray crystallography with computational modeling and simulation of the wild type and variant forms of the CH3 domain. This allowed for gaining novel structural and physico-chemical insights about the potential role of individual amino acids and their cooperative action. These structural and physico-chemical insights, obtained from multiple variant CH3 domains, along with the resulting empirical data pertaining to their stability and purity helped us develop an understanding for the relationship between purity and stability of the Fc heterodimer as compared to the Fc homodimers and the simulated structural models. In order to execute these simulations, complete and realistic models were built and the quality of the wild type Fc structure of an IgG1 antibody was refined. Protein structures derived from X-ray crystallography are lacking in detail regarding certain features of the protein in aqueous medium under physiological condition and the refinement procedures addressed these limitations.

Molecular dynamics (MD) was employed to simulate the protein structure, to evaluate the intrinsic dynamic nature of the Fc homodimer and the variant CH3 domains in an aqueous environment. Molecular dynamics simulations track the dynamic trajectory of a molecule resulting from motions arising out of interactions and transient forces acting between all the atomic entities in the protein and its local environment, in this case the atoms constituting the Fc and its surrounding water molecules.

The impact of mutations on the local environment of the site of mutation was studied in detail. The formation of a well packed core at the CH3 interface between chain A and B is critical for the pairing of the two chains in a stable Fc structure. Good packing is the result of strong structural complementarity between interacting molecular partners coupled with favorable interactions between the contacting groups. The favorable interactions result from either buried hydrophobic contacts well removed from solvent exposure or from the formation of complementary electrostatic contacts between hydrophilic polar groups. These hydrophobic and hydrophilic contacts have entropic and enthalpic contributions to the free energy of dimer formation at the CH3 interface. A variety of algorithms were employed to accurately model the packing at the CH3 interface between chain A and chain B and subsequently evaluate the thermodynamic properties of the interface by scoring a number of relevant physicochemical properties.

Protein-packing methods were employed including mean field and dead-end elimination methods along with flexible backbones to optimize and prepare model structures for the large number of variants being screened computationally. Following packing, a number of features were scored including contact density, clash score, hydrophobicity and electrostatics. Use of the Generalized Born method allowed for the accurate modeling of the effect of solvent environment and to contrast the free energy differences following mutation of specific positions in the protein to alternate residue types. Contact density and clash score provided a measure of complementarity, one aspect of effective protein packing. These screening procedures are based on the application of knowledge-based potentials as well as coupling analysis schemes relying on pair-wise residue interaction energy and entropy computations.

This comprehensive in-silico analysis provided a detailed understanding of the differences of each Fc variant compared to wild-type with respect to interface hotspots, sites of asymmetry, cavities and poorly packed regions, structural dynamics of individual sites and sites of local unfolding. The computational analysis helped identify specific residues, sequence/structural motifs and cavities that were not optimized and in combination were responsible for the lower stability (e.g., Tm of 68 °C) and/or lower specificity of <90% purity. In the second design phase, targeted positive design was used to specifically address these sites with additional point-mutations and tested these by in-silico modeling using the above described methodology and analysis.

*Optimization of Initial Variants and Structural Rational.* To improve the initial negative design Fc variants for stability and purity, the structural and computational strategies described above were employed. The in depth structure-function analysis of the initial negative design variant provided a detailed understanding for each of the introduced mutations.

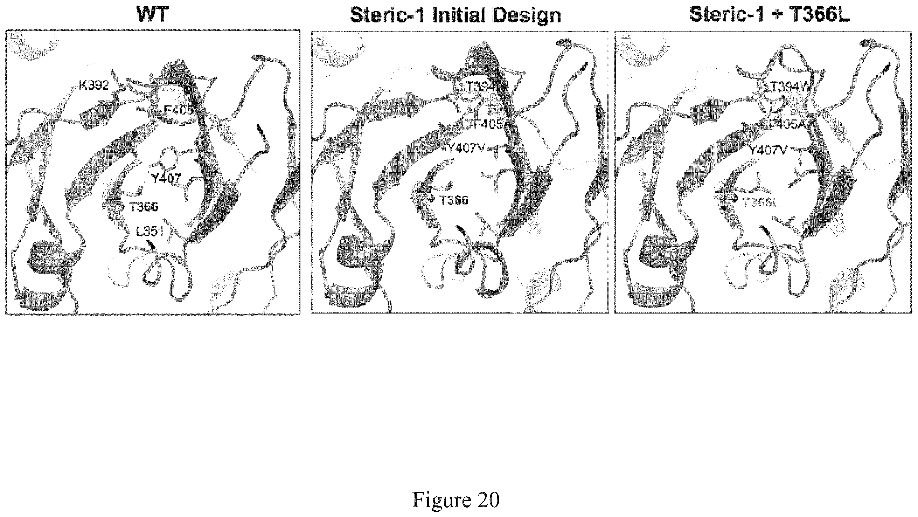

For example, the analysis showed that the important interface hotspots that are lost with respect to wild-type homodimer formation are the interactions of wild-type (chain A)F405-(chain B)K409, (chain A)Y407-(chain B)T366 and the packing of (chain AB)Y407-Y407 and -(chain A)F405 Figure 20. The analysis revealed in addition that one strong wild-type hotspot (chain A)T366 was affected but not contributing in the heterodimer formation, while likely still being present in the undesired homodimer. As illustrated in Figure 20, the single amino acid change of (chain B)T366L increased the heterodimer purity of the initial design variants from ∼80% to >95%.

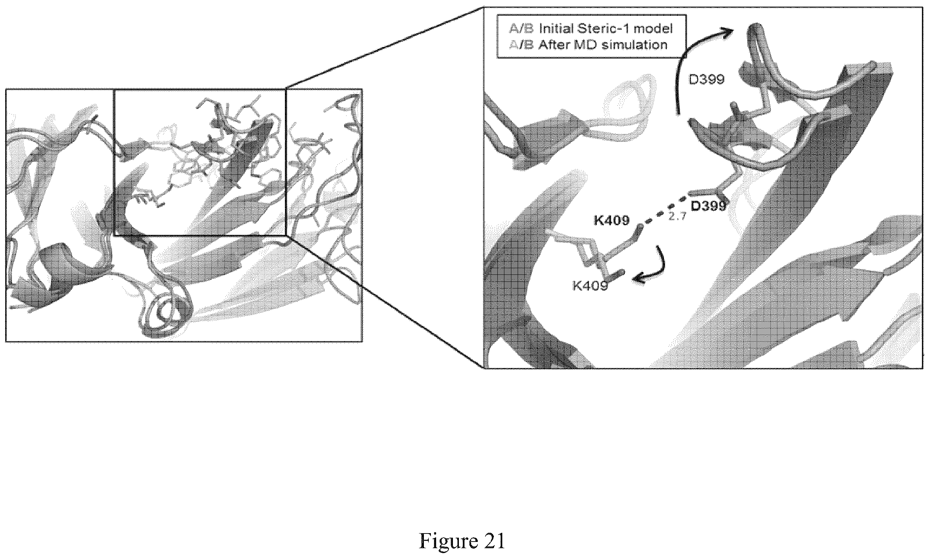

The molecular dynamics simulation of the initial heterodimer variant with low stability showed a large conformational difference in the loop region D399-S400-D401 (Figure 21) and the associated β-sheets at K370. This resulted in the loss of the interchain interactions K409-D399. In the wild type IgG1 CH3 domain these regions tether the interface at the rim and protect the hydrophobic core interactions. This analysis indicated an important factor for the lower stability of the initial heterodimer variant compared to wild type stability.

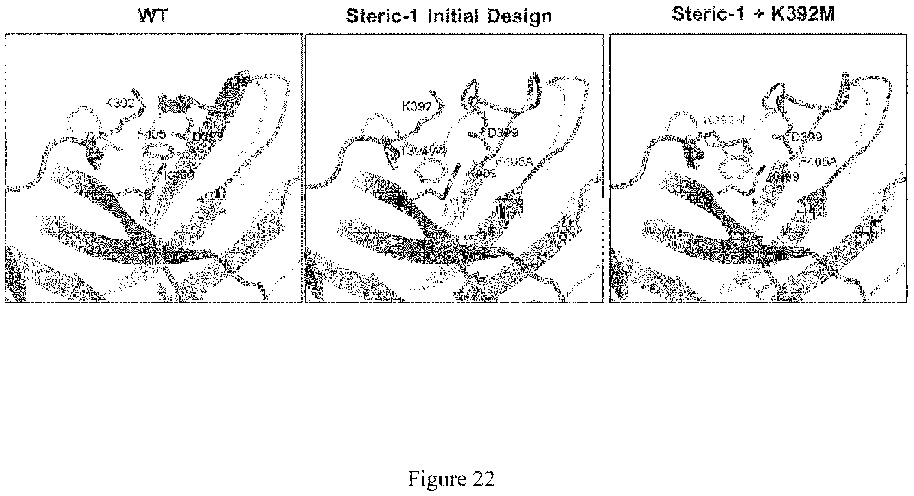

Consequently, residues and sequence motifs responsible for the low stability were identified and the subsequent positive design engineering efforts were therefore specifically focused on stabilizing the loop conformation of positions 399-401 in a more 'closed' - wild-type like conformation. In order to achieve this stabilization of the loop conformation of positions 399-401 the above described computational approach was used to evaluate different targeted design ideas. This strategy identified the single mutation difference K392M/L which leads to an increase in CH3 stability of ∼4 °C as illustrated in Figure 22.

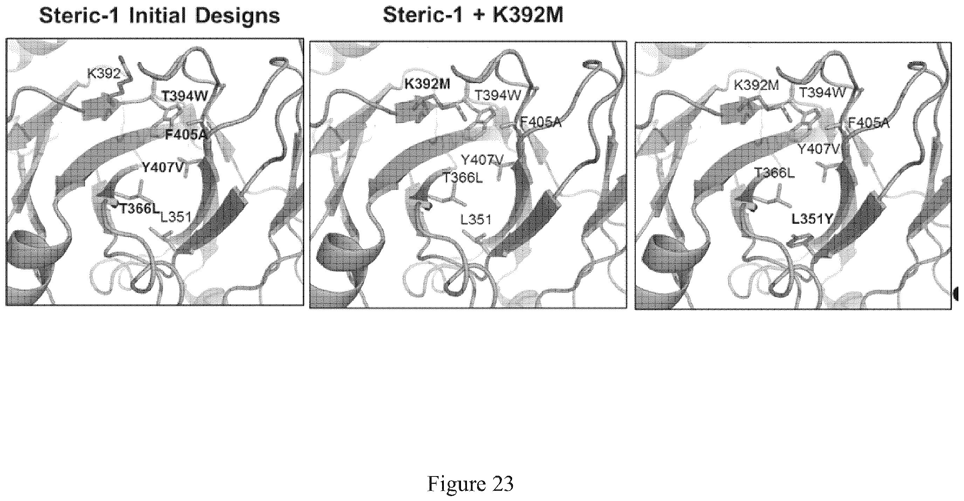

Thirdly, a cavity at the core packing positions T366, T394W and L368 was identified as a reason for the lower than wild-type stability Figure 23. To improve the core packing, the positions at T366/L368 were computationally screened and, in addition, distal positions were evaluated for stabilization of the core packing. This procedure identified the distal swap L351Y, which as a single mutations does not show any impact, but in combination with T366L and L368 gives an improved CH3 Tm of > 5 °C, indicating a strong coupling effect of the distal change L351Y.

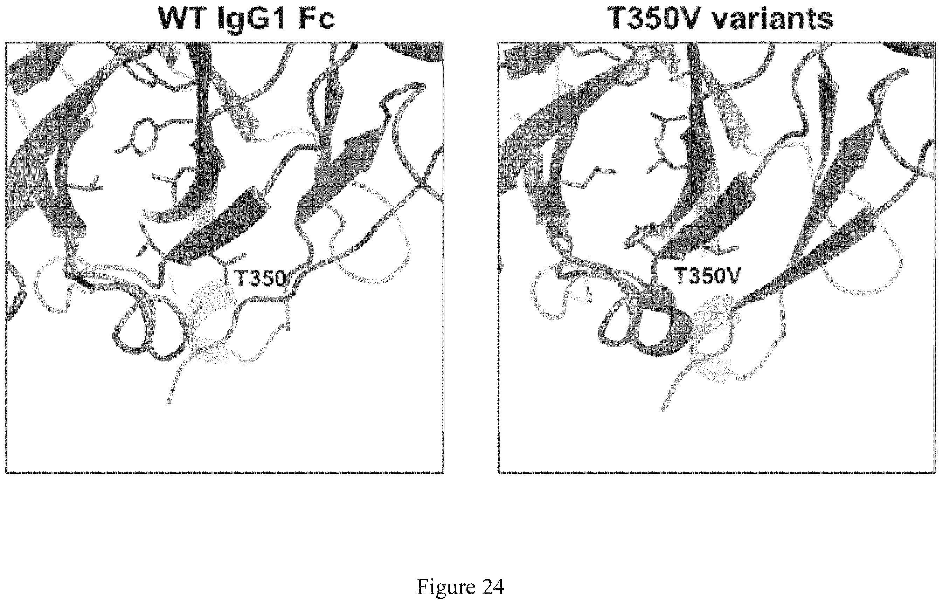

The employed engineering approach to improve the heterodimer stability is not limited to introducing mutations that increase complementarity across the two chains. Mutations of amino acids that are not directly contacting the complementary chain were evaluated as a means to improve the stability of the Fc heterodimeric protein. As an example, the second shell position T350 in the CH3 domain of IgG1 is buried and the threonine residue facing the interior of the CH3 domain. The distal second shell mutation T350V has been identified by the described computational screening and it improves the stability of the Fc domain by >2 °C Tm. See Figure 24.

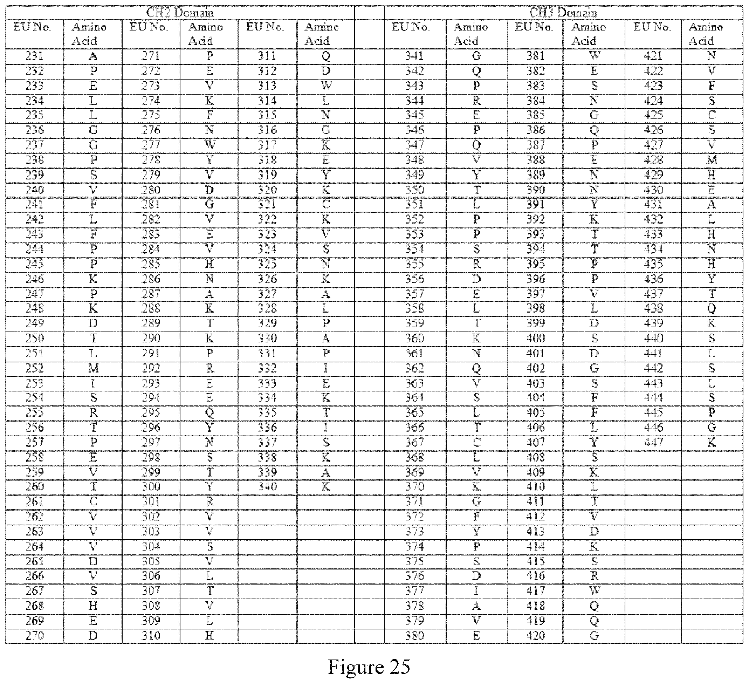

*Crystal structure of Fc heterodimeric proteins.* In a direct advancement of this validation, disclosed are the crystal structures of the Fc heterodimeric proteins AZ1 and AZ2, which are based upon the CH2 and CH3 domains of human igGl Kabat antibody. The primary amino acid sequence of AZ1 and AZ2 and the amino acid sequence of the CH2 and CH3 domains of human igG1 Kabat antibody immunoglobulin are provided in Figure 16. *See,* Kabat *et al*., 1991, NIH Publication 91-3242, National Technical Information Service, Springfield, Virginia. For convenience, Figure 25 provides the amino acids numbering used herein according to the EU index as set forth in Kabat for the CH2 and CH3 domains from human IgG1.

Crystals of the Fc heterodimeric proteins in accordance with the present disclosure were obtained in a number of different screening conditions. Using the hanging drop vapor diffusion method, the reservoir conditions refined for data collection and structure solution were ethyleneglycol, polyethylene glycol with an average molecular weight of 3350 Daltons, and ammonium iodide.

Fc heterodimeric protein constructs of AZ1 and AZ2 were transiently expressed in CHO (Chinese hamster ovary) cells and purified to homogeneity by protein A column chromatography and SEC (size exclusion chromatography). The purified Fc heterodimeric proteins were crystallized at 18 °C after -24 hours of incubation via hanging drop vapor diffusion method at a ratio of 2: 1 above a mother liquor solution composed of 5% (v/v) ethylene glycol, 18% (w/v) polyethylene glycol 3350, and 0.15 M ammonium iodide with aid of microseeding. Crystals were cryoprotected by increasing the concentration of ethylene glycol to 30% (v/v) and subsequently flash cooled in liquid nitrogen. Diffraction data from both crystals were collected at 100 K, using 0.5 degree oscillations for 200 degrees total, and processed with XDS. *See* Kabsch, 2010, Acta crystallography D Biological Crystallography 66: 125-132, for teaching on such processing of diffraction data. The structure of AZ1 was solved via molecular replacement with Phaser using PDBID: 2J6E as a query protein. *See*, McCoy, 2007, Acta Crystallography D Biological Crystallography 63: 32-41, for teachings on molecular replacement. The structure of AZ1 was then used to solve AZ2 in similar fashion. In order to accommodate the perfect twin reciprocal relationship of the heterodimer present in the crystallographic asymmetric unit (e.g., the occupancy of molecule A can be equally be described by molecule B and vice versa), two possible heterodimer pairs, each with 0.5 atomic occupancies, were modeled with Coot, and refined with Refmac. *See,* Emsley and Coot, 2004, Acta Crystallography D Biological Crystallography 60, 2126-2132; and Murshudov et al., 1997, Acta Crystallography D Biological Crystallography 53, 240-255, for teaching on Coot and Refmac. Diffraction data processing and structure refinement statistics for AZ1 and AZ2 are presented in Table 2. Figure 26 provides the structure coordinates for AZ2 (including SEQ ID NO: 4 and SEQ ID NO.: 3). Figure 27 provides the structure coordinates for AZ1 (including SEQ ID NO: 2 and SEQ ID NO.: 3).

**Table 2: Data collection and structural refinement statistics.**

| | AZ1 | AZ2 |
|---|---|---|
| **Data collection** | | |
| Synchrotron | CSLS | CSLS |
| Beam line | CMCF-BM | CMCF-BM |
| Wavelength (Å) | 0.98005 | 0.98005 |
| Space group | P2₁2₁2₁ | P2₁2₁2₁ |
| Cell dimensions | | |
| *a, b, c* (Å) | 49.54, 74.92, 148.92 | 49.67, 74.72, 148.93 |
| α, β, γ (°) | 90, 90, 90 | 90, 90, 90 |
| Resolution (Å) | 47-1.75 (1.84-1.75)* | 47-2.10 (2.21-2.10) |
| R_{sym} or *R*_{merge} | 0.043 (0.413) | 0.074 (0.502) |
| *I* / σ*I* | 26 (3.9) | 15.9 (4.0) |
| Completeness (%) | 100 (100) | 99.9 (99.9) |
| Redundancy | 7.3 (7.4) | 6.8 (7.0) |

| **Refinement** | | |
|---|---|---|
| Resolution (Å) | 1.75 | 2.15 |
| No. reflections, free | 53,467 (2,849) | 29,307 (1,557) |
| *R*_{work} / R_{free} | 17.8 / 20.8 | 20.0 / 25.9 |
| No. atoms | | |
| Protein Chains | 6704 | 6704 |
| Carbohydrate/ions | 440 / 4 | 440 / 4 |
| Solvent | 802 | 415 |
| *B*-factors | | |
| Protein Chains | 23.8 | 44.4 |
| Carbohydrate/ions | 54.3 / 20.7 | 67.0 / 41.5 |
| Solvent | 27.0 | 40.8 |
| RMS deviations | | |
| Bond lengths (Å) | 0.008 | 0.010 |
| Bond angles (°) | 1.32 | 1.53 |
| Ramachandran Data | | |
| Most favored (%, no.) | 96.8 (805) | 94.5 (786) |
| Additionally allowed (%, no.) | 2.4 (20) | 4.3 (36) |
| Disallowed (%, no.) | 0.8 (7) | 1.2 (10) |

| | | |
|---|---|---|
| *Values in parentheses are for highest-resolution shell. | | |

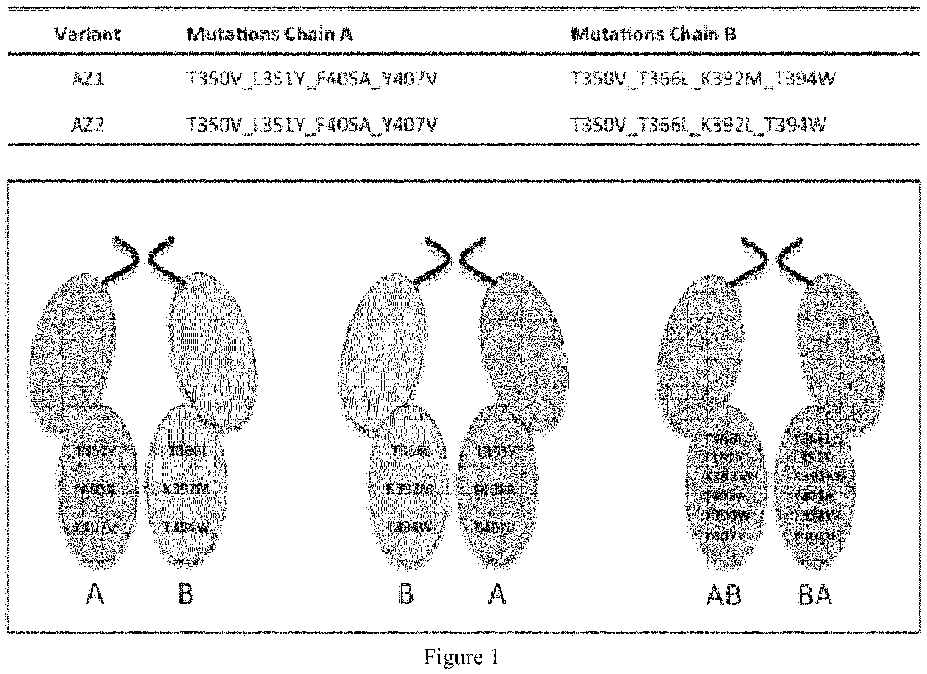

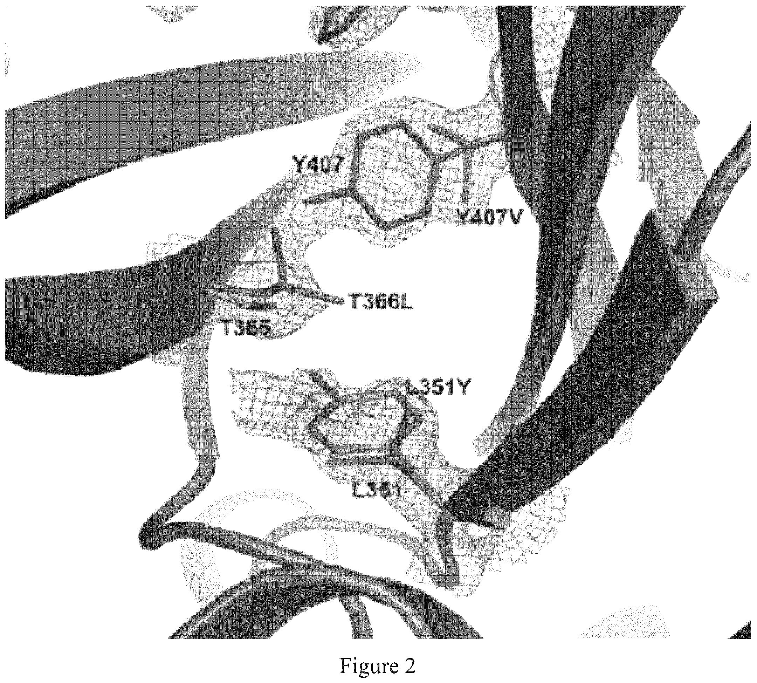

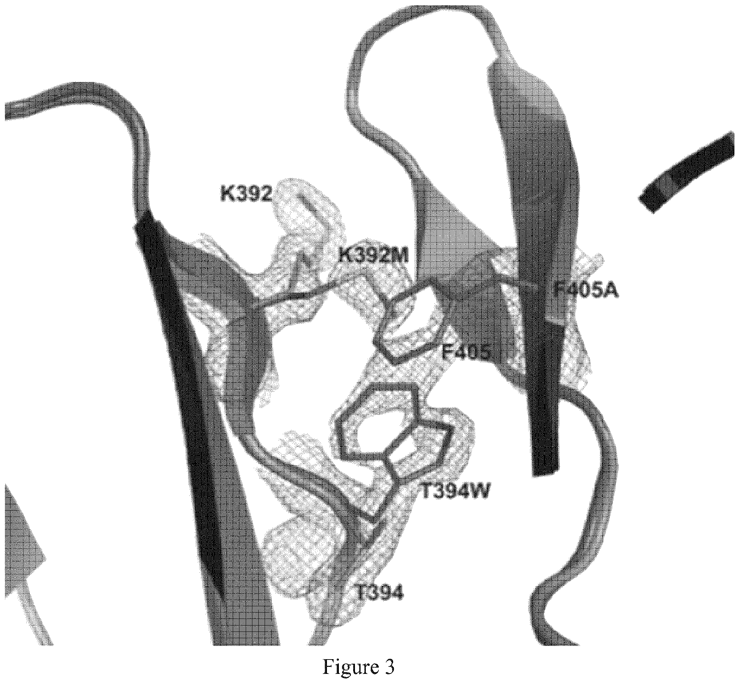

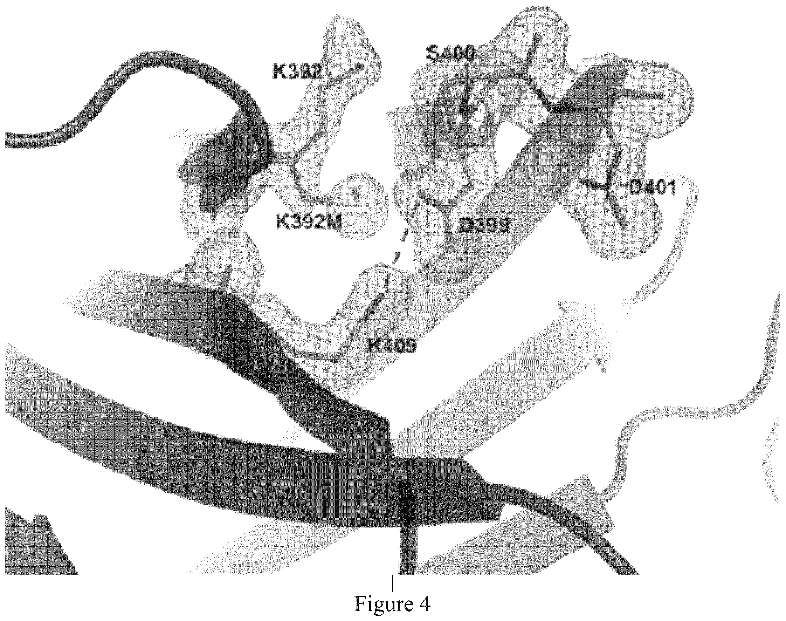

Despite the engineered asymmetry at the heterodimeric interface of the disclosed variant, the overall surface symmetry has been preserved for both AZ1 and AZ2. Crystallographically, this feature manifests in that the heterodimeric asymmetric unit can be oriented in both possible configurations when averaged throughout the crystal lattice. For example, in each asymmetric unit the occupancy of molecule A can be described with equal frequency by molecule B and *vice versa* when averaged throughout the crystal. See Figure 1 for a graphical depiction of molecule A and B in AZ1 and AZ2. Use of alternate sidechain conformations is possible when multiple conformations are evident from calculated electron density, but the sequence conflict of two different residues was not tolerated by the available software refinement packages. Similarly, creating alternate overlapping chains at the same position with 0.5 occupancies introduced steric clashes during structural refinement, and therefore did not provide a suitable solution to the duplicity of the designed interface. Therefore, while non-canonical, in order to accommodate the interface residue heterogeneity, two adjacent asymmetric units were modeled: one in which the heterodimer A:B is oriented and the other where the mirrored heterodimer B:A was modeled, and each was assigned 0.5 occupancy. The electron density using such modeling, as illustrated in Figures 2 through 4, shows the 50%:50% occupancy of the possible orientations A:B and B:A at the mutated interface residues. Inspection of the difference density at the CH3-CH3 interface showed no significant peaks, thus confirming the two possible orientations. For further validation the B-factors of the mutated interface residues of the two orientations A:B and B:A were compared to high resolution WT Fc structures. The refined B-factors of the mutated residues are very similar to the overall B-factor of the core CH3-CH3 interface residues and are comparable to the B-factors of published WT Fc structures. Further, calculation of OMIT maps at the CH3-CH3 interface did not reveal any errors of the modeled 50%:50% occupancy. This analysis confirms the modeled 50%:50% occupancy of the A:B and B:A orientation.

*Comparison of AZ1 and AZ2 crystal structures and in silico models.* Both the AZ1 and AZ2 crystal structures show overall agreement with wild type Fc structures. Importantly, detailed inspection of the engineered CH3-CH3 domain confirms that all introduced mutations are fully buried and that the wild type surface characteristics of the Fc and the CH3-CH3 domain are maintained. This is further underlined by the fact that both AZ1 and AZ2 constructs crystallized as a 50%:50% mixture of the two possible CH3-CH3 orientations A:B and B:A, and this is only possible when the natural homodimeric surface of the CH3-CH3 domain is not broken by the asymmetric CH3 interface mutations.

Maintaining the wild type Fc surface characteristics is an important aspect in reducing the risk of immunogenicity, since both surface exposed mutations and a shift in the wild type symmetric CH3-CH3 orientation due to the engineered interface mutations potentially creates new B-cell epitopes at the Fc domain which significantly increases the risk of an immunogenic response. Together with maintaining the wild type IgG1 stability, this addresses two fundamental concerns in immunogenicity and further de-risks the development of new bispecific therapeutic molecules based on the disclosed scaffold.

One desired goal in the computational design of the Fc heterodimeric proteins had been to prevent the formation of exposed mutations and exposed altered side chain conformations, which would create new potential surface epitopes. After the final round of design, the *in silico* model of AZ1 and AZ2 predicted all mutational changes to be buried in the CH3 interface residue, thus not altering the wild type CH3 surface area.

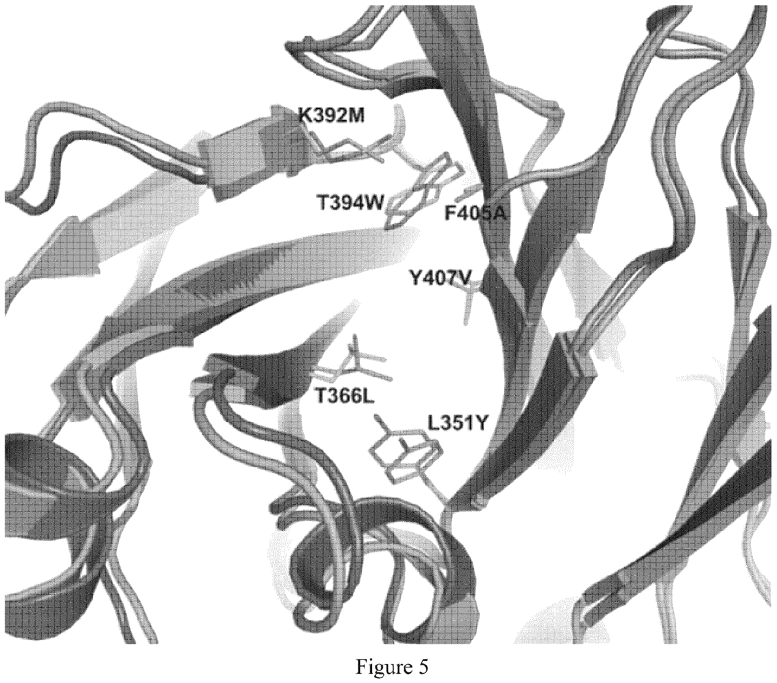

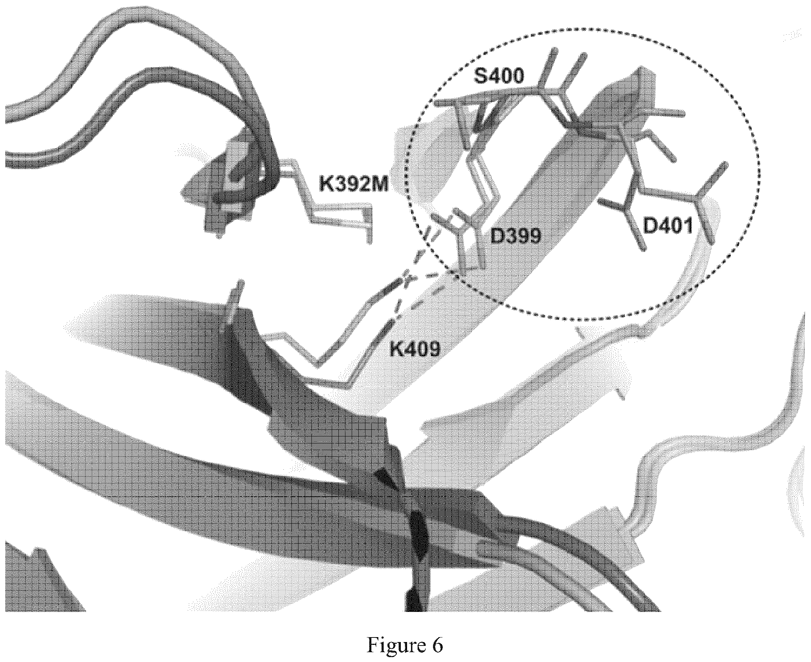

To validate the *in silico* model and the hypothesis that the introduced CH3 mutations do not create newly exposed surface area, the crystal structures of the AZ1 and AZ2 were compared to the predicted *in silico* models. See Figures 5 and 6. The superposition of the heterodimeric CH3 domains shows a agreement of the crystal structures and the *in silico* models for both variants AZ1 and AZ2 with an all atom RMSD of 0.8Å and 0.7Å. Inspection of the mutated and wild type interface residues further shows comparable side chain conformations for the crystal structures and the computational models. The crystal structures of AZ1 and AZ2 thus confirm that all mutated residues are fully buried in the CH3 interface and that the introduced mutations do not lead to altered CH3 surface area.

A particular focus in the design of a stable Fc heterodimeric protein was retaining the strong wild type salt bridge interaction at residues K409-D399. This interaction is affected by the neighboring essential heterodimer mutations, but retaining this salt bridge in the heterodimer ensures wild type stability of the CH3 interface. In addition, loss of the K409-D399 salt bridge interaction will likely lead to an altered loop conformation of D399-D401 and in turn newly exposed interface residues. As illustrated in Figure 6, the wild type Fc salt bridge interaction of K409-D399 and the loop conformation of D399-D401 is maintained in the crystal structures of both AZ1 and AZ2 Fc heterodimeric proteins.

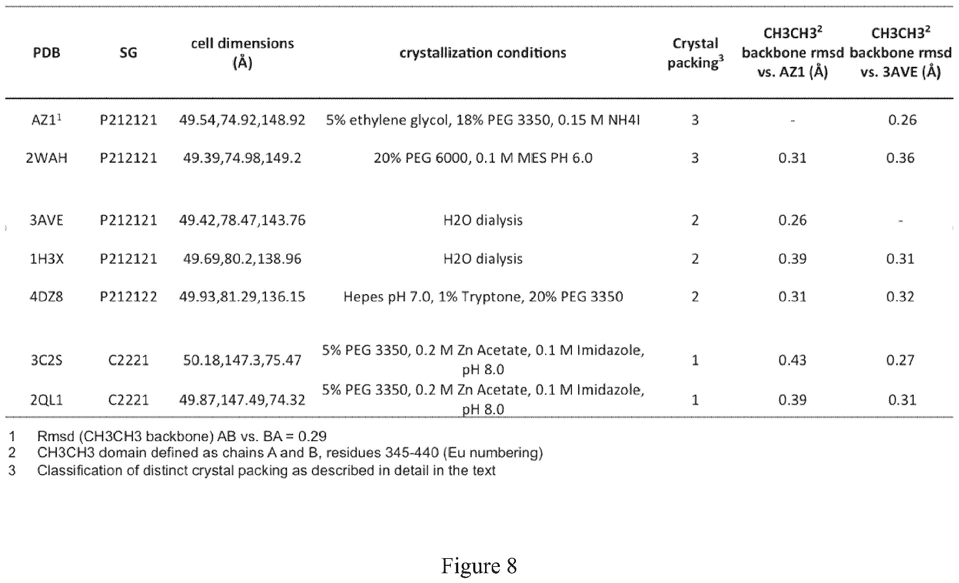

*Comparison of the CH3 domain of the AZ crystal structures and high resolution wild type Fc crystal structures.* To further evaluate potential negative effects of the heterodimer mutations on the highly conserved homodimeric CH3-CH3 structure, the AZ1 and AZ2 crystal structures were compared to a number of representative wild type Fc crystal structures, crystallized under different conditions and crystal space groups. The respective crystal structures were superimposed over the dimeric CH3-CH3 domains and the backbone RMSD was calculated. The results of this comparison are summarized in Figure 8. The comparison confirmed good overall agreement of the heterodimer CH3-CH3 domain and wild type CH3-CH3 structures.

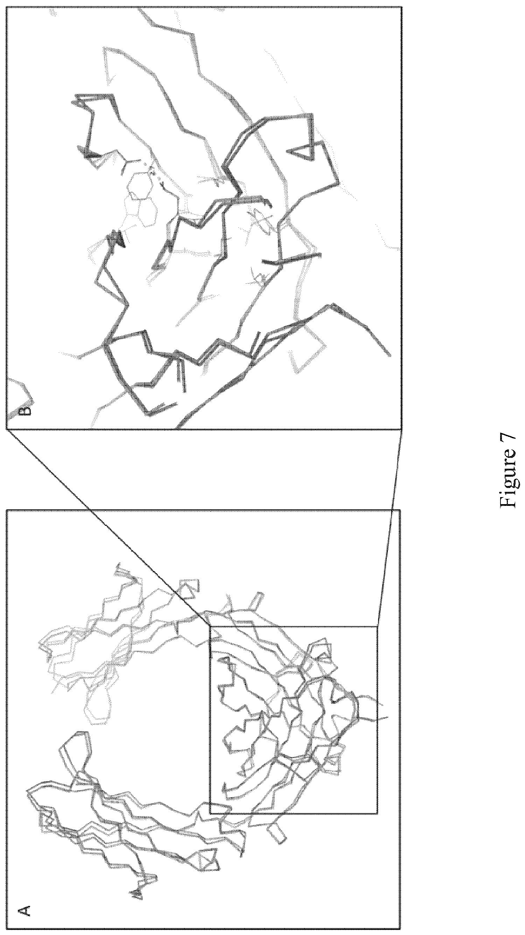

For more detailed analysis of potential effects of the heterodimer mutations on the wild type surface characteristics of the CH3 domain and the homodimeric Fc symmetry, the AZ1 and AZ2 crystal structures were compared to the two published wild type Fc crystal structures with the highest resolution, 1L6X and 3AVE (1.6Å and 1.9Å respectively). As illustrated in Figure 7, the homodimeric CH3-CH3 domains of the two representative high resolution wild type Fc crystal structures were overlaid. Per residue all atom RMSDs calculated across the entire CH3-CH3 domain of the structures showed good agreement between the structures. The per residue RMSDs of the 1L6X to 3AVE comparison was used as a reference for the naturally occurring variation of backbone and sidechain conformations between wild-type Fc structures. To uncover any differences of the heterodimeric CH3-CH3 domain to the wild type homodimeric CH3-CH3 domain apart from the engineered interface residues, the AZ1/AZ2 crystal structures were compared to 1L6X and 3AVE by per residue RMSD calculation in a similarly manner. The comparison of the per residue RMSDs of AZ1:1L6X to the RMSDs of the wild type Fc structures 1L6X:3AVE shows a similar pattern for the engineered heterodimeric CH3-CH3 and the wild type homodimeric CH3-CH3 domain. Further, the same RMSD analysis was done for the two observed heterodimer orientations of A:B and B:A (see Figure 1 for a description of this nomenclature) and the results are very similar. This analysis highlights that the asymmetric mutations at the CH3 interface do not induce an asymmetry or shift in the naturally symmetric orientation of the two CH3 domains towards each other and that the highly conserved dimeric CH3-CH3 structure is preserved in the engineered heterodimer.

*Comparison of the CH2-CH3 domain angle and the crystal packing of the AZ crystal structures and high resolution wild type Fc crystal structures.* The AZ1 and AZ2 crystal structures were compared to different Fc crystal structures with focus on the CH3-CH2 interdomain angle and the Fc structural conformations.

Overall, the comparison of wild type Fc structures shows a high identity in the CH3-CH3 domain and a significantly larger variation in the CH3-CH2 interdomain angle and the conformation of the CH2 domains. In deference to the tight dimeric nature of the CH3-CH3 domain, the interaction of the two CH2 domains is mainly mediated by the complex-type glycan attached to the conserved N297. The glycoform of the Fc has been shown to be important for Fc mediated effector function and FcgammaR and Clq binding. For example, mutation of the N297 glycosylation site to prevent N-glycosylation leads to near depletion of all Fc mediated effector functions, like antigen-dependent cellular cytotoxicity (ADCC) and complement-dependent cytotoxicity (CDC), while truncation of the complex-type Fc glycan structure by only the core-fucose moiety displays increased binding to activating Fcgamma receptors and enhanced ADCC. Further, alteration of the complex-type glycan structure to high mannose-type glycans, which also do not contain the core-fucose moiety, has been reported to yield enhanced ADCC activity while Clq mediated complement activation is reduced. The structural reasons for the observed differences in effector function due to altered Fc glycans are potentially of different nature. The recently published crystal structure of a fucosylated Fc in complex with FcgammaRIIIa revealed a unique Fc-FcgammaR carbohydrate-carbohydrate interaction which is only present in the case of a fucosylated Fc and this additional interaction has been proposed to be the reason for the higher affinity to FcgammaRIII. In contrast, complete de-glycosylation at N297 has been shown to lead to a more 'closed' conformation of the CH2 domains and this in turn is thought to prevent efficient binding of FcgammaR, which requires an 'open' CH2 conformation as determined in the Fc-FcgammaR co-complex structures. *See,* Krapp et al., 2003, Journal of Molecular Biology 325:979-989. Based on further comparisons of different glycoform Fc crystal structures and the observed variation in the CH2 conformations it has been proposed that the degree of 'openness' of the CH2 domains is influenced by the Fc glycoform and plays a role in Fc effector function.

The comparison of the AZ1 and AZ2 crystal structures to wild type Fc crystal structures reveals that AZ1 and AZ2 crystallized in an 'open' conformation that resembles the conformation observed in the PDB ID 2WAH. *See*, Crispin et al., 2009, Journal of Molecular Biology 387:1061:1066. The crystal structure 2WAH presents an immature high mannose-type glycan structure and a distinct 'open' conformation of the CH2 domains. In an independent analysis, high mannose-type glycan IgGs have been reported to display enhanced ADCC and based on this result, it has been proposed that the high mannose-type glycan induces the 'open' conformation observed in the 2WAH crystal structure, which in turn is the reason for the enhanced ADCC.

In contrast to the immature high mannose-type glycan of the 2WAH variant, the AZ1 and AZ2 crystal structures clearly show an IgG1 like core complex-type glycan structure that is similar to what has been observed in wild type Fc crystal structures. The carbohydrate-carbohydrate interaction of AZ1, AZ2 was analyzed. The glycan structures were compared by superposition to wild type Fc structures as described and detailed by Nagae and Yamaguchi. *See,* Nagae and Yamaguchi 2012, Function and 3D Structure of the N-Glycans on Glycoproteins. Int J Mol Sci. 13: 8398-8429. Nagae and Yamaguchi categorized the available wild type Fc crystal structures with respect to their carbohydrate-carbohydrate interactions and the distance of the Man-4 moieties. According to this categorization, the AZ glycan structure falls within the complex-type glycan conformations and carbohydrate-carbohydrate interactions naturally observed in wild type Fc crystal structures. This suggests that the 'open' conformation of the CH2 domains in the AZ crystal structures is not a consequence of non-wild type glycan structure, but likely has a different reason.

To further investigate the relevance of the observed 'open' conformation in AZ crystal structures, all available wild type Fc crystal structures were compared and differences in the CH2-CH3 interdomain angle, crystal space group, crystal packing and crystallization conditions were evaluated. Figure 8 lists a representative subset of Fc crystal structures grouped by distinct crystal contacts and crystal packing. Close inspection of the crystal packing of the wild type Fc structures revealed two distinct possible crystal packing within structures crystallized in the same orthorhombic space group P2₁2₁2₁.

Analysis of the differences in crystal packing, as exemplified by comparison of the AZ1 and 3AVE structures, reveals that the most significant difference in crystal contacts and packing between the 3AVE and AZ1 structure is at the CH2 domain and the hinge, which is close to the interaction region with FcgammaRs. The most prominent crystal contact in 3AVE is affects only one of the CH2 domains, while for AZ1 the adjacent Fc is bound in between two CH2 domains. This difference in crystal contacts and crystal packing is in agreement with the observed differences in 'openness' of the Fc structures.

Furthermore, in the crystal structure of AZ1 and AZ2, two tightly bound iodide ions were found at the CH2-CH3 domain interface. Analysis of the interactions of the CH2-CH3 domain and the iodide ions shows that the tight interactions are only formed in the 'open' conformation with the particular CH2-CH3 interdomain angle, as observed in the AZ1 and AZ2 crystal structures, which suggests that the presence of the iodide in the crystallization conditions might favor the 'open' conformation.

This analysis suggests that the crystallization conditions and the crystal packing might be the main determining factor for the degree of 'openness' of the CH2 conformation and the CH2-CH3 interdomain angle as observed in glycosylated Fc crystal structures.

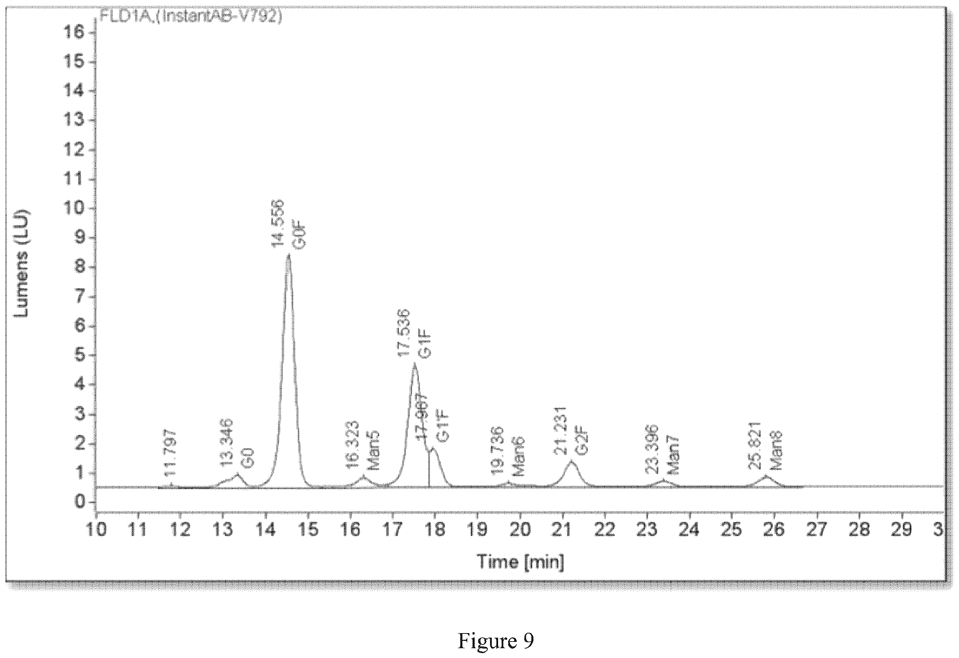

*Analysis of glycosylation pattern of Fc heterodimeric proteins.* As discussed above, the glycosylation observed in the crystal structures of Fc heterodimeric proteins AZ1 and AZ2 resembles the typical wild type like complex-type core-glycan structure. For further validation of the glycoform present in AZ1 and AZ2, the detailed glycosylation profile of full size IgG1 heterodimeric antibody was analyzed. For this analysis two proof of concept molecules were designed that on the commercial anti-HER2 antibody Trastuzumab with two identical Trastuzumab Fabs attached to AZ1 or to AZ2. The bivalent anti-her2-AZ1 heterodimeric antibody was expressed in CHO cells by transient co-expression and the heterodimer purity was confirmed using mass spectrometry. The analysis illustrated in Figure 9 shows a typical IgG1 like complex-type glycosylation pattern with GOF and G1F being the most prominent glycoforms. This reflects the core complex-type glycosylation observed in the AZ1 and AZ2 crystal structures and confirms the wild type IgG1 glycosylation of the engineered Fc heterodimeric protein. To produce the data for Figure 9, the Trastuzumab based heterodimeric antibody anti-her2-AZ1 was expressed and purified as described herein. Glycans were analyzed with GLYKOPREP™ Rapid N-Glycan Preparation with InstantAB (Prozyme) using the standard manufacturer protocol.

*FcgammaR binding and Fc effector function of Fc heterodimeric proteins.* To confirm that the engineered Fc heterodimeric protein retains all Fc mediated effector functions, the FcgammaR binding affinities were determined by surface plasmon resonance (SPR) and the ADCC and CDC activity were also determined. The SPR and ADCC experiments were performed on the anti-her2-AZ1 heterodimeric antibody, described above, and compared to similarly produced parent Trastuzumab, while the CDC activity was measured using bivalent anti-CD20(Rituximab)-AZ1 heterodimeric antibody and similarly produced parent Rituximab as control.

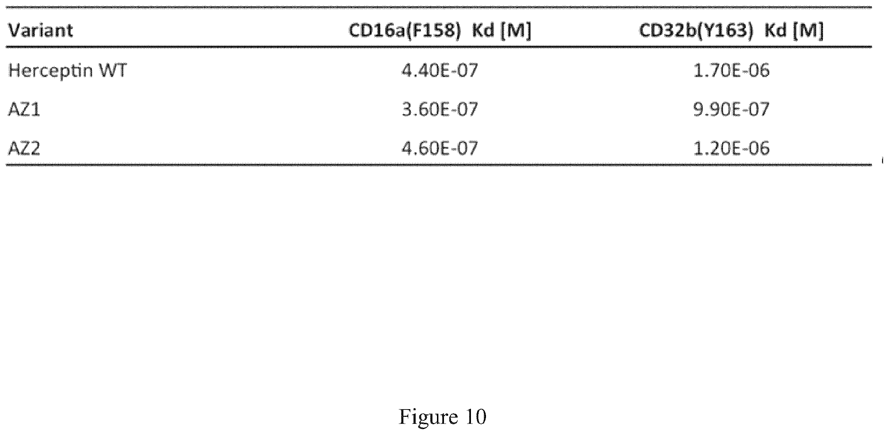

In the first set of experiments the affinities to the activating FcgammaRIIIa (CD16a(F158)) and the inhibiting FcgammaRIIb (CD32b(Y163)) were determined by SPR. The sensorgrams of the heterodimeric antibodies anti-her2-AZ1, anti-her2-AZ2 and parent Trastuzumab were highly similar, and no significant differences were detected. As detailed in Figure 10, the calculated FcgammaR affinities for the AZ1 and AZ2-based heterodimeric antibodies are very similar to wild type IgG1 control.

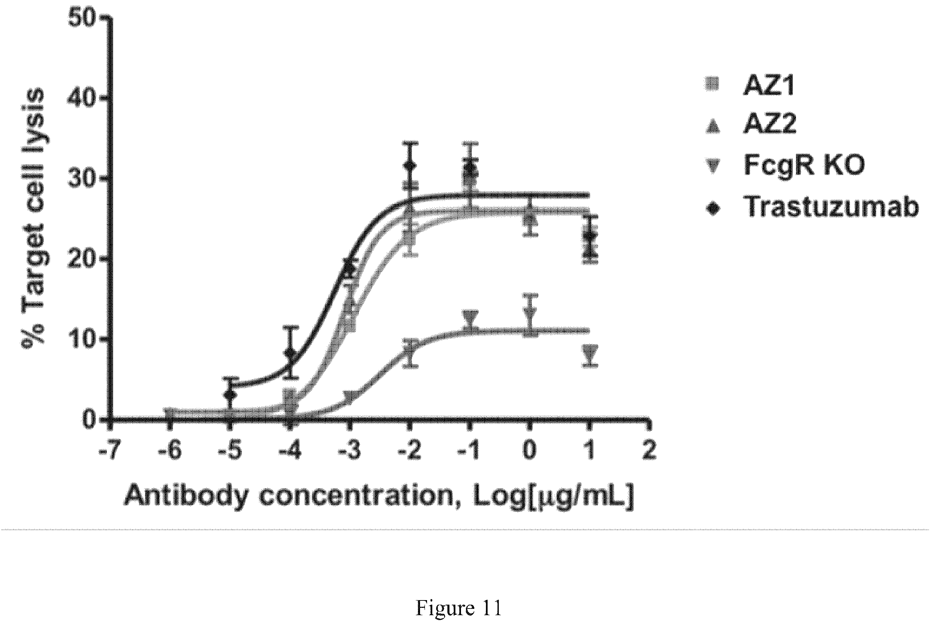

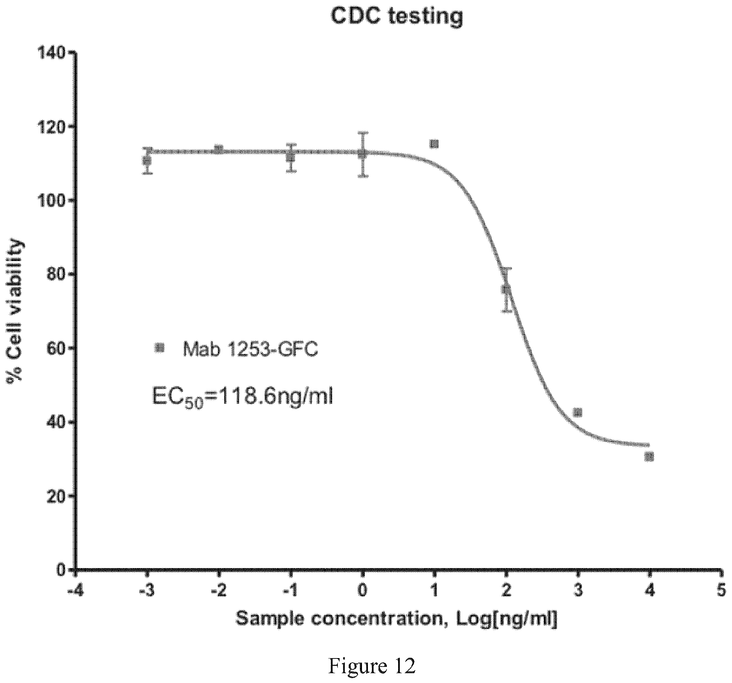

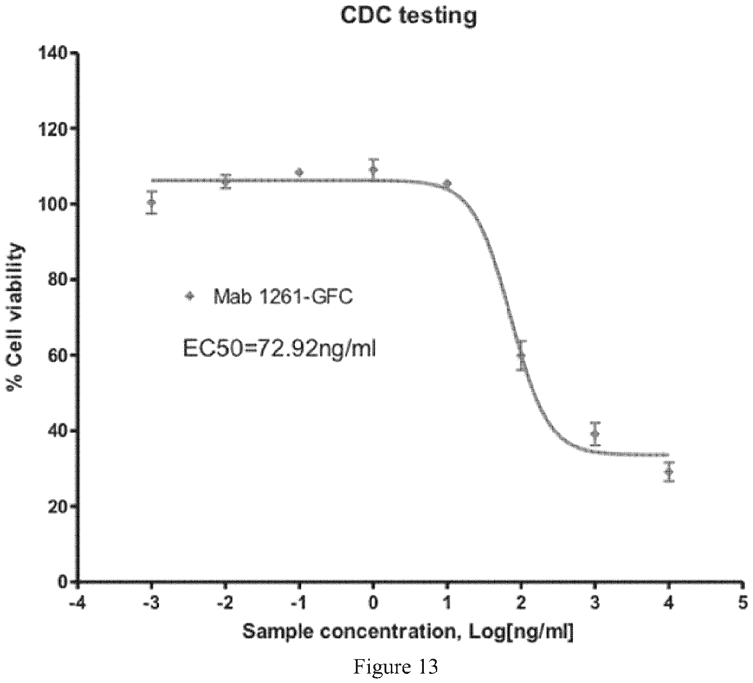

In a second set of experiments, as illustrated in Figure 11, the ADCC activity of the anti-her2-AZ1 heterodimeric antibody and parent Trastuzumab against the melanoma cell lines SKOV3 was measured using human peripheral blood mononuclear cells (PBMC) as effector cells. No significant difference between the AZ1 heterodimeric antibody and parent Trastuzumab was observed. The CDC activity of anti-CD20(Rituximab)-AZ1 heterodimeric antibody and parent Rituximab as control was determined using human serum as a complement source, against the human CD20 B lymphocyte cell line Raji. As depicted in Figures 12 and 13, no significant difference between the Rituximab based AZ1 heterodimeric antibody and parent Rituximab was observed.

Taken together, the results of the ADCC and CDC activity of AZ1-based heterodimeric antibodies compared to the wild type IgG1 controls confirms wild type IgG1 mediated effector function of the engineered heterodimeric antibodies.

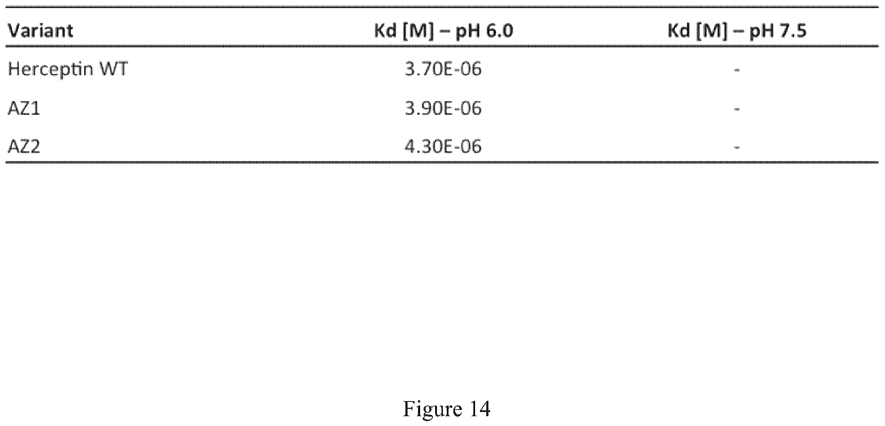

*FcRn (Neonatal Fc receptor) binding and pharmacokinetic profile of* Trastuzumab *-based AZ1 and AZ2 heterodimeric antibodies in mice.* The human FcRn binding kinetics of the Trastuzumab based AZ1 and AZ2 heterodimer antibodies (anti-her2-AZ1 or anti-her2-AZ2, as described above, and the parent trastuzumab control was estimated by SPR for binding at pH 6.0 and release at pH 7.5. The sensorgrams of the heterodimeric antibodies and parent Trastuzumab were highly similar, and no significant differences were detected. The resulting FcRn affinities for AZ1 and AZ2 anti-her2 heterodimeric antibodies in comparison to parent Trastuzumab are summarized in Figure 14.

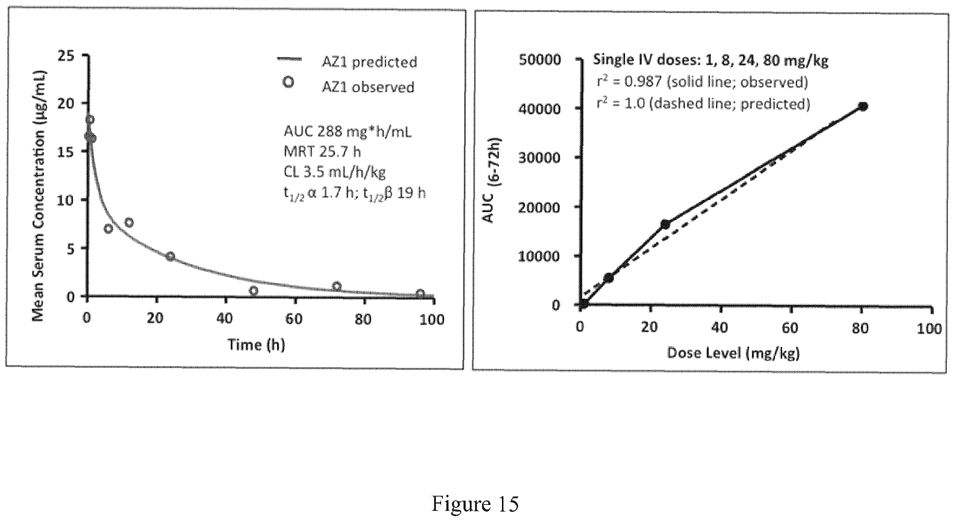

To assess the in vivo properties of the heterodimeric antibodies, a pharmacokinetic (PK) study was performed using the Trastuzumab based anti-her2 AZ1 heterodimeric antibody (anti-her2-AZ1), described above. Nude mice were injected intravenously at 4 different dose levels of 1, 8, 24 and 80mg/kg, and the plasma clearances were monitored by an anti-Trastuzumab specific ELISA, as illustrated in Figure 15. The kinetics of elimination at the different dose levels is linear over the studied dose range and the calculated pharmacokinetic properties are very similar to those published for parent Trastuzumab. The mouse PK analysis verified that the engineered heterodimeric antibody retains the preferred wild type IgG1 like pharmacokinetic properties.

In an additional set of studies, the glycosylation profile of the heterodimeric antibody was analyzed because altered glycosylation can significantly affect the Fc functionality and potentially also immunogenicity. The glycosylation analysis showed a typical IgG1 glycosylation profile for the heterodimeric antibody. Further, more detailed functional validation of heterodimeric antibodies by FcgammaR binding, ADCC and CDC analysis demonstrated that the heterodimeric antibodies retain all wild type Fc mediated effector functions. To assess the in vivo properties of the heterodimeric antibodies, a pharmacokinetic study was performed at different doses, showing no significant differences to wild type IgG1 clearance behavior. All together, the disclosed structural and functional analysis demonstrates preferred IgG1 drug like properties including Fc effector functionality, pharmacokinetics and early immunogenicity analysis, which significantly de-risk the development of bispecific therapeutics based on the Azymetric scaffold.

Despite the wild type IgG1 glycosylation and Fc effector function of the disclosed Fc heterodimeric proteins, as demonstrated here, the crystal structures of the AZ1 and AZ2 Fc heterodimeric proteins surprisingly showed an 'open' conformation of the CH2 domains and the CH2-CH3 interdomain angle. This 'open' conformation of the CH2 domains had previously been suggested to be due to altered glycosylation and has further been implicated to play a role in increased FcgammaR binding and ADCC. Here it is demonstrated that although crystallized in an 'open' conformation, the disclosed Fc heterodimeric protein neither displays altered glycosylation, nor enhanced FcgammaR binding and ADCC, questioning the hypothesized correlation of 'openness' and ADCC activity. Further detailed structural analysis of Fc crystal structures presented here suggests that the 'openness' of the CH2 domains and the CH2-CH3 interdomain angle as observed in crystal structures might in contrast be induced to the crystallization conditions and crystal packing. This observation further questions the relevance of the 'openness' of the CH2 domains in crystal structures and the correlation to function. Nevertheless, IgG1 structures with altered, namely high mannose-type, glycosylation have been reported to display enhanced ADCC activity and reduced Clq dependent CDC. Since specific to ADCC, this might on the other hand also be a consequence of the lack of core fucose in the high mannose-type glycoforms, as recently discussed for non-fucosylated complex-type glycan IgG, rather than the 'openness' of the CH2 domain. This hypothesis is supported by the data on the heterodimeric antibodies showing wild type like Fc effector function and glycosylation.

*Expression and Purification of Fc heterodimeric proteins for Crystallization.* Using separate plasmids for the two heavy chains and one light chain, CHO cells were transfected in exponential growth phase (1.5 to 2 million cells/mL) with aqueous 1mg/mL 25kDa polyethylenimine (PEI, Polysciences) at a PEI:DNA ratio of 2.5:1 (Raymond et al. 2011). For example, the transfection DNA comprised 5% GFP (green fluorescent protein), 45% salmon sperm DNA, 25% light chain and 12.5% of each of the complementary heterodimer heavy chains. At four to 48 hours after transfection in F17 serum-free media (Gibco), TN1 peptone is added to a final concentration of 0.5%. The clarified culture medium was loaded onto a MabSelect SuRe (GE Healthcare) protein-A column and washed with 10 column volumes of PBS buffer at pH 7.2. The antibody was eluted with 10 column volumes of citrate buffer at pH 3.6 with the pooled fractions containing the antibody neutralized with TRIS at pH 11. The antibody was finally desalted using an Econo-Pac 10DG column (Bio-Rad) and subsequently further purified by gel filtration. For gel filtration, 3.5mg of the purified antibody was concentrated to 1.5mL and loaded onto a Superdex 200 HiLoad 16/600 200 pg column (GE Healthcare) via an AKTA Express FPLC at a flow-rate of 1mL/min. PBS buffer at pH 7.4 was used at a flow-rate of 1mL/min.

*Binding Analysis to FcgammaR and FcRn by SPR.* All binding experiments disclosed herein (e.g., Figures 10 through 13) were carried out using a BioRad ProteOn XPR36 instrument. Briefly, recombinant HER-2/neu (p185, ErbB-2 (eBiosciences, Inc.)) was captured on the activated GLM sensorchip by injecting 4.0µg/mL in 10mM NaOAc (pH 4.5) at 25µL/min until approximately 3000 resonance units (RUs) were immobilized with the remaining active groups quenched. An aliquot of 40µg/mL of purified anti-HER-2/neu antibodies comprising the modified CH3 domains were indirectly captured on the sensorchip by binding the Her-2/neu protein when injected at 25µL/min for 240s (resulting in approximately 500RUs) following a buffer injection to establish a stable baseline. FcgammaR (CD16a(f allotype) and CD32b) concentrations (6000, 2000, 667, 222, and 74.0nM) were injected at 60µL/min for 120s with a 180s dissociation phase to obtain a set of binding sensograms. Resultant K_{D} values were determined from binding isotherms using the Equilibrium Fit model with reported values as the mean of three independent runs.

Binding to FcRn was determined by SPR in two different orientations. First, in the direct capture method, recombinant FcRn was captured on a high density surfaces at approximately 5000 RUs, using standard NHS/EDC coupling and 100nM of heterodimeric IgG and was injected in triplicate at 50 µL/min for 120 seconds with 600 second dissociation in MES pH 6 running buffer. Second, in the indirect capture experiment, a goat anti-human IgG surface was used to indirectly capture the antibodies (approximately 400RUs each), followed by an injection of a 3-fold FcRn dilution series (6000nM high conc). Running buffer was 10mM MES / 150mM NaCl / 3.4 mM EDTA /0.05 Tween20 at pH6. There was no significant binding of FcRn to the goat polyclonal surface. Both the AZ1 and AZ2-based heterodimeric antibodies showed similar to wild type sensograms. Figure 14 shows the Kd determined by the indirect immobilization method with flowing FcRn.

*Analysis of ADCC and CDC Mediated Effector Function.* The ADCC protocol was performed by harvesting SKBR3 target cells (ATCC, Cat# HTB-30) by centrifugation at 800 rpm for three minutes. The cells were washed once with assay medium and centrifuged and the medium above the pellet was completely removed. The cells were gently suspended with assay medium to make single cell solution. The number of SKBR3 cells was adjusted to 4x cell stock (10,000 cells in 50 µl assay medium). The test antibodies were then diluted to the desired concentrations as noted above.

The SKBR3 target cells were seeded in the assay plates as follows. An aliquot of 50µl of 4x target cell stock and 50µl of 4x sample diluents was added to wells of a 96-well assay plate and the plate was incubated at room temperature for thirty minutes in cell culture incubator. Effector cells (NK92/ FcgammaRIIIa(158V/V), 100µl, E/T = 5:1, i.e, 50,000 effector cells per well) were added to initiate the reaction and mixed gently by cross shaking.

Triton X-100 was added to cell controls without effector cells and antibody in a final concentration of 1% to lyse the target cells and these controls served as the maximum lysis controls. ADCC assay buffer (98% Phenol red free MEM medium, 1% Pen/Strep and 1% FBS) was added in to cell controls without effector cells and antibody and it served as the minimum LDH release control. Target cells incubated with effector cells without the presence of antibodies were set as background control of non-specific LDH release when both cells were incubated together. The plate was incubated at 37 °C/5% CO₂ incubator for 6 hours. Cell viability was assayed with an LDH kit (Roche, cat#11644793001). The absorbance data was read at OD492nm and OD650nm. Data analysis and the reported percentages of cell lysis were calculated according the formula below: Cell lysis %=100* (Experimental data-(E+T)) / (Maximum release - Minimum release).

The CDC protocol used for the CDC data disclosed herein was performed as follows. Rituximab based proof of concept of the disclosed heterodimers (anti-CD20(Rituximab)-AZ1) were tested for complement-dependent cytotoxicity using Raji cells. Cells were initially incubated for thirty minutes at 37°C. Subsequently, Raji and effector cells were combined and incubated for another two hours using 10% NHS as complement source and 5000 target cells/well. Cell titers were determined by glo cell viability assay using luminescens.

*Additional embodiments.* Those of skill in the art understand that a set of structure coordinates for a protein, a complex of proteins, or a portion thereof, such as AZ1 and AZ2, is a relative set of points that define a shape in three dimensions. Thus, it is possible that an entirely different set of coordinates could define a similar or identical shape. Moreover, slight variations in the individual coordinates will have little effect on overall shape.

The variations in coordinates discussed above may be generated because of mathematical manipulations of the AZ1 and/or AZ2 structure coordinates. For example, the structure coordinates set forth in Figures 26 or 27 could be manipulated by crystallographic permutations of the structure coordinates, fractionalization of the structure coordinates, integer additions or subtractions to sets of the structure coordinates, inversion of the structure coordinates or any combination of the above.

Alternatively, modifications in the crystal structure due to mutations, additions, substitutions, and/or deletions of amino acids, or other changes in any of the components that make up the crystal could also account for variations in structure coordinates. If such variations are within a certain root mean square deviation as compared to the original coordinates, the resulting three-dimensional shape is considered encompassed by the present disclosure.

Various computational analyses may be necessary to determine whether a macromolecule or portion thereof is sufficiently similar to AZ1 or AZ2. Such analyses may be carried out using well known software applications, such as the Molecular Similarity application of QUANTA (Molecular Simulations Inc., San Diego, Calif. 1998), CCP4 (Acta Crystallogr., D50, 760-763 (1994)) or ProFit (A. C. R. Martin, ProFit version 1.8, bioinfo.org.uk/software). In particular, the Molecular Similarity software application permits comparisons between different structures, different conformations of the same structure, and different parts of the same structure. The procedure used in Molecular Similarity to compare structures is divided into four steps: 1) load the structures to be compared; 2) define the atom equivalences in these structures; 3) perform a fitting operation; and 4) analyze the results.

Each structure in the comparison is identified by a name. One structure is identified as the target (e.g., the fixed structure); all remaining structures are working structures (*e.g*., moving structures). Since atom equivalency within QUANTA is defined by user input, for the purpose of the present disclosure, equivalent atoms are considered to be protein backbone atoms N, C, O and C_{α} for all corresponding amino acids between the two structures being compared. Moreover, the corresponding amino acids may be identified by sequence alignment programs such as the "bestfit" program available from the Genetics Computer Group which uses the local homology algorithm described by Smith and Waterman in Advances in Applied Mathematics 2, 482 (1981). The identification of equivalent residues can also be assisted by secondary structure alignment, for example, aligning secondary structure such as α-helices, β-sheets or hinge regions in the structure when present. For programs that calculate RMSD values of the backbone atoms, an RMSD cutoff value can be used to exclude pairs of equivalent atoms with extreme individual RMSD values, or in situations where the equivalent atom cannot be found in the corresponding structure.

When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atom is an absolute minimum. This number, given in angstroms, is reported by QUANTA.

For the purpose of the present disclosure, any molecule or molecular complex that is within a predetermined root mean square deviation for backbone atoms (C, O, N and C_{α}) when superimposed on the relevant backbone atoms described by structure coordinates listed in any one of Figures 26 and 27 are encompassed by the present disclosure. In some embodiments, this RMSD is not greater than about 3.0 Å. In some embodiments, this RMSD is not greater than about 1.0 Å. In some embodiments, this RMSD is not greater than about 0.5 Å. In one embodiment, this RMSD is not greater than about 0.2 Å.

In another embodiment, the root mean square deviation of the backbone atoms between the amino acid residues of a candidate molecular structure and the AZ1 or AZ2 amino acid residues according to Figures 26 or 27 is not greater than about 0.3 Å, and at least one of the amino acid residues of the candidate molecular structure is not identical to the AZ2 or AZ2 amino acid residue to which it corresponds.

In another embodiment, the root mean square deviation of the backbone atoms between the amino acid residues of a candidate molecular structure and the AZ1 or AZ2 amino acid residues according to Figures 26 or 27 is not greater than about 0.3 Å, and at least two, at least three, at least four, or at least five of the amino acid residues of the candidate molecular structure is not identical to the AZ2 or AZ2 amino acid residue to which it corresponds. Additionally, in some embodiments, the candidate molecular structure may have additional residues not found in AZ1 or AZ2, or may be missing some terminal residues found in AZ1 or AZ2.

In another embodiment, the root mean square deviation of the backbone atoms between the amino acid residues of a candidate molecular structure and the AZ1 or AZ2 amino acid residues according to Figures 26 or 27 is not greater than about 0.3 Å, at least one, at least two, at least three, at least four, or at least five of the amino acid residues of the candidate molecular structure is/are not identical to the AZ2 or AZ2 amino acid residue to which it corresponds.

*Structure Determination of Other Molecules.* The structure coordinates set forth in Figures 26 and 27 can also be used to aid in obtaining structural information about another crystallized molecule or molecular complex. This may be achieved by any of a number of well-known techniques, including molecular replacement.

In one embodiment, a computer is disclosed for determining at least a portion of the structure coordinates corresponding to X-ray diffraction data obtained from a molecule or molecular complex, where the computer comprises: a) a machine-readable data storage medium comprising a data storage material encoded with machine-readable data, where the data comprises at least a portion of the structure coordinates of AZ1 or AZ2 according to Figures 26 or 27, b) a machine-readable data storage medium comprising a data storage material encoded with machine-readable data, where the data comprises X-ray diffraction data obtained from the molecule or molecular complex; and, c) instructions for performing a Fourier transform of the machine readable data of (a) and for processing the machine readable data of (b) into structure coordinates. For example, the Fourier transform of at least a portion of the structure coordinates set forth in Figures 26 or 27 may be used to determine at least a portion of the structure coordinates of IgG1 homologs. Therefore, in another embodiment the present disclosure provides a method of utilizing molecular replacement to obtain structural information about a molecule or molecular complex whose structure is unknown comprising the steps of: a) crystallizing the molecule or molecular complex of unknown structure; b) generating an X-ray diffraction pattern from the crystallized molecule or molecular complex; and c) applying at least a portion of the AZ1 or AZ2 structure coordinates set forth in Figures 26 or 27 to the X-ray diffraction pattern to generate a three-dimensional electron density map of the molecule or molecular complex whose structure is unknown. By using molecular replacement, all or part of the structure coordinates of the AZ1 or AZ2 as provided by the present disclosure (and set forth in Figures 26 and 27) can be used to determine the structure of a crystallized molecule or molecular complex whose structure is unknown more quickly and efficiently than attempting to determine such information through more complex techniques such as multiple isomorphous replacement.

Molecular replacement provides an accurate estimation of the phases for an unknown structure. Phases are a factor in equations used to solve crystal structures that cannot be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of AZ1 or AZ2 according to Figures 26 or 27 within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex (Lattman, 1985, "Use of the Rotation and Translation Functions", in Meth. Enzymol. 115: 55-77; Rossmann, ed., 1972, "The Molecular Replacement Method", Int. Sci. Rev. Ser. 13, Gordon & Breach, New York. The structure of any portion of any crystallized molecule or molecular complex that is sufficiently homologous to any portion of the AZ1 or AZ2 can be resolved by this method.

In some embodiments, the method of molecular replacement is utilized to obtain structural information about a immunoglobulin G homologue. The structure coordinates of AZ1 and AZ2 as provided by the present disclosure are particularly useful in solving the structure of other variants of immunoglobulin G or portions thereof. For instance, the structure coordinates of AZ1 and AZ2 as provided by this invention are useful in solving the structure of immunoglobulin G proteins that have amino acid substitutions, additions and/or deletions (referred to collectively as "immunoglobulin G mutants", as compared to naturally occurring immunoglobulins.

All of the macromolecules referred to above may be studied using well-known X-ray diffraction techniques and may be refined against 1.5-3.4 Å resolution X-ray data to an R value of about 0.30 or less using computer software, such as X-PLOR (Yale University, distributed by Molecular Simulations, Inc.; see, *e.g*., Blundell & Johnson, 1985, Meth. Enzymol., 114 & 115, H. W. Wyckoff et al., eds., Academic Press.

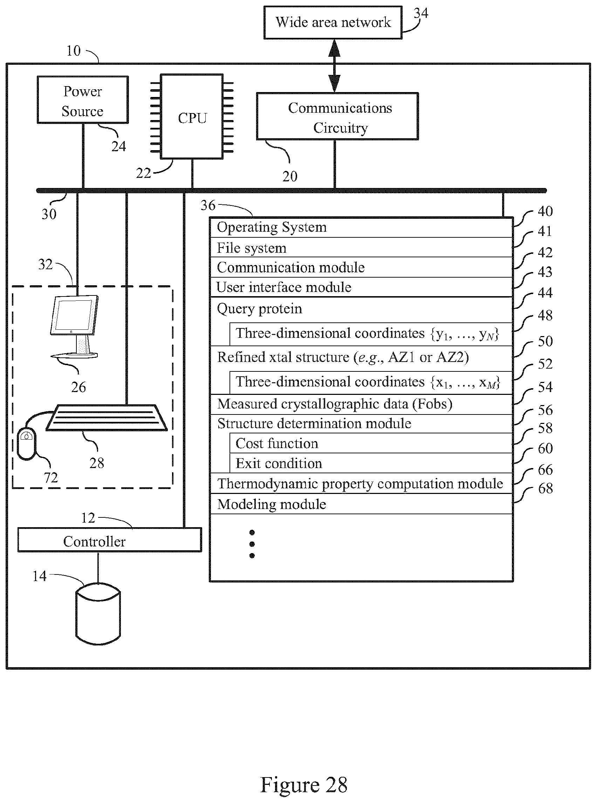

*Computer system.* Figure 28 is a block diagram illustrating a computer according to some embodiments. The computer 10 typically includes one or more processing units (CPU's, sometimes called processors) 22 for executing programs (e.g., programs stored in memory 36), one or more network or other communications interfaces 20, memory 36, a user interface 32, which includes one or more input devices (such as a keyboard 28, mouse 72, touch screen, keypads, etc.) and one or more output devices such as a display device 26, and one or more communication buses 30 for interconnecting these components. The communication buses 30 may include circuitry (sometimes called a chipset) that interconnects and controls communications between system components.

Memory 36 includes high-speed random access memory, such as DRAM, SRAM, DDR RAM or other random access solid state memory devices; and typically includes non-volatile memory, such as one or more magnetic disk storage devices, optical disk storage devices, flash memory devices, or other non-volatile solid state storage devices. Memory 36 optionally includes one or more storage devices remotely located from the CPU(s) 22. Memory 36, or alternately the non-volatile memory device(s) within memory 36, comprises a non-transitory computer readable storage medium. In some embodiments, the non-volatile components in memory 36 include one or more hard drives 14 controlled by one or more hard drive controllers 12. In some embodiments, memory 36 or the computer readable storage medium of memory 36 stores the following programs, modules and data structures, or a subset thereof:
- an operating system 40 that includes procedures for handling various basic system services and for performing hardware dependent tasks;
- a file system 41 for handling basic file I/O tasks;
- an optional communication module 42 that is used for connecting the computer 10 to other computers via the one or more communication interfaces 20 (wired or wireless) and one or more communication networks 34, such as the Internet, other wide area networks, local area networks, metropolitan area networks, and so on;
- an optional user interface module 43 that receives commands from the user via the input devices 28, 72, etc. and generates user interface objects in the display device 26;
- a query protein 44, including a set of three-dimensional coordinates {y₁, ..., y*_{N}*} 48 for the query protein (e.g., PDBID: 2J6E) to use as a starting bases for obtaining phases for a composition comprising an Fc heterodimer protein 50 in crystal form in accordance with the present disclosure;
- a refined atomic crystal structure of a composition comprising an Fc heterodimer protein 50 in crystal form in accordance with the present disclosure including three-dimensional coordinates {x₁, ..., x*_{M}*} 52 for the Fc heterodimer protein 50 (*e.g*., those disclosed in Figures 26 or 27);
- measured crystallographic data 54 for the composition comprising an Fc heterodimer protein 50 in crystal form;
- a structure determination module 56 for using the three-dimensional coordinates 48 of the query protein 44 and the measured crystallographic data 54 to determine the refined crystal structure comprising an Fc heterodimer protein 50 in accordance with the present disclosure; and
- a thermodynamic property computation module 66 for computing a thermodynamic property of all or a portion of the refined crystal structure 50.

An aspect of the present disclosure provides a method of identifying a mutation which promotes heterodimeric Fc chain pair formation. In this method, structure based modeling is performed, using a suitably programmed computer, such as computer 10 of Figure 28. The modeling is performed to identify a candidate mutation to an Fc chain using a three-dimensional atomic crystal structure of an Fc heterodimer protein. In some embodiments this three-dimensional atomic crystal structure is refined crystal structure 50. In some embodiments this three-dimensional atomic crystal structure is all or a portion of the coordinates for AZ1 or AZ2 as set forth in Figures 26 and 27. In some embodiments, this three-dimensional atomic crystal structure is defined by the atomic coordinates of any combination of chains a, b, A, and B of Figures 26 or 27 determined from an X-ray diffraction quality crystal of the Fc heterodimer protein, where the Fc heterodimer protein comprises the amino acid sequences as set forth in (i) SEQ ID NOS: 2 and 3 or (ii) SEQ ID NOS: 4 and 3 of Figure 16, and the X-ray diffraction quality crystal is in an orthorhombic space group. In some embodiments the orthorhombic space group is P2₁2₁2₁ and has unit cell dimensions a = 49 ± 2 Å, b = 75 ± 2 Å, c = 149 ± 2 Å, α = β = γ = 90°. In some embodiments, the structure based modeling is performed by modeling module 68. In some embodiments, the modeling comprises identifying a plurality of residues on the three-dimensional structure that influence heterodimeric Fc chain pair formation, (b) modeling a plurality of three-dimensional Fc structures using the three-dimensional atomic crystal structure as a template, where each three-dimensional Fc structure in the plurality of three-dimensional Fc structures includes mutations to one or more of the residues in the plurality of residues, (c) comparing each three-dimensional Fc structure in the plurality of three-dimensional Fc structures to the three-dimensional atomic crystal structure, and (d) selecting one of the three-dimensional Fc structure in the plurality of three-dimensional Fc structures based on the comparing (c).

In some embodiments the comparing (c) compares a calculated thermodynamic property of the three-dimensional atomic crystal structure to a calculated thermodynamic property of a three-dimensional Fc structure in the plurality of three-dimensional Fc structures. In some embodiments the thermodynamic property is entropy, average energy, average enthalpy, free energy or heat capacity. In some embodiments the modeling, including the calculation of the thermodynamic property, is performed using the techniques disclosed in United States Provisional Patent Application No. 61/793,203, entitled "Systems and Methods for Identifying Thermodynamic Effects of Atomic Changes to Polymers", filed March 15, 2013.

In some embodiments, the comparing (c) compares a physical property of the three-dimensional atomic crystal structure to a calculated thermodynamic property of a three-dimensional Fc structure in the plurality of three-dimensional Fc structures, where the physical property is selected from the group consisting of (i) one or more electrostatic interactions, (ii) one or more polar interactions, (iii) one or more hydrogen-bond interactions, (iv) a comparison of buried versus accessible surface area, (v) accessible surface area, (vi) one or more hydrophobic interactions, and (vii) presence or absence of one or more buried water molecules.

In some embodiments, the modeling is performed using the techniques disclosed in United States Provisional Patent Application No. 61/662,549, entitled "Systems and Methods for Identifying Thermodynamically Relevant Polymer Conformations", filed June 21, 2012. In some embodiments, such modeling is facilitated using the techniques disclosed in United States Provisional Patent Application No. 61/613,711, entitled "Systems and Methods for Making Two Dimensional Graphs of Complex Molecules", filed March 31, 2013. In some embodiments, such modeling is facilitated using the techniques disclosed in United States Patent Application No. 13/822,258, entitled "System for Molecular Packing Calculations", filed March 11, 2013, claiming priority to International Application PCT/CA11/01061. In some embodiments, such modeling is facilitated using the techniques disclosed in United States Patent Application No. 13/822,231, entitled "Simplifying Residue Relationships in Protein Design", filed March 11, 2013, claiming priority to International Application PCT/CA11/01103. In some embodiments, such modeling is facilitated using the techniques disclosed in International Application No. PCT/CA2010/001923, entitled "Combined On-Lattice / Off-Lattice Optimization Method for Rigid Body Docking", filed December 2, 2010. In some embodiments, such modeling is facilitated using the techniques disclosed in United States Provisional Patent Application No. 61/684,236, entitled "Methods for Sampling and Analysis of Protein Conformational Dynamics", filed August 17, 2012. In some embodiments, such modeling is facilitated using the techniques disclosed in United States Patent Application No. 11/441,526, entitled "System and Method for Modeling Interactions", filed May 26, 2006. In some embodiments, such modeling is facilitated using the techniques disclosed in United States Patent Application No. 11/581,075, entitled "System and Method for Simulating the Time-Dependent Behaviour of Atomic and/or Molecular Systems Subject to Static or Dynamic Fields", filed October 16, 2006. In some embodiments, such modeling is facilitated using the techniques disclosed in United States Patent Application No. 12/866,437, entitled "Methods for Determining Correlated Residues in a Protein or other Biopolymer Using Molecular Dynamics", filed October 11, 2010.

In some embodiments modeling module 68, in fact, represents one or more programs. In some embodiments, modeling module 68 comprises any or a portion of the techniques disclosed or incorporated in QUANTA (Molecular Simulations Inc., San Diego, Calif. 1998), CCP4 (Acta Crystallogr., D50, 760-763 (1994)), ProFit (A. C. R. Martin, ProFit version 1.8, bioinfo.org.uk/software); Cohen et al., 1990, "Molecular Modeling Software and Methods for Medicinal Chemistry", Journal of Medicinal Chemistry 33: 883-894; Navia and Murcko, 1992, "The Use of Structural Information in Drug Design", Current Opinions in Structural Biology 2: 202-210 (1992); Balbes et al., 1994, "A Perspective of Modern Methods in Computer-Aided Drug Design", in Reviews in Computational Chemistry 5, Lipkowitz and Boyd, Eds., VCH, New York, pp. 337-380; Guida, 1994, "Software For Structure-Based Drug Design", Current Opinion in Structural Biology 4: 777-781, Bohacek et al., 1996, "The art and practice of structure-based drug design: A molecular modeling perspective", Medicinal Research Reviews 16: 3-50; Leach, 2001, "Molecular Modelling, Principles and Applications", Second Edition, Prentice Hall, Upper Saddle River, New Jersey; and Cramer, 2004, "Essentials of Computational Chemistry: Theories and Models", Wiley, Hoboken, New Jersey.

The present disclosure can be implemented as a computer program product that comprises a computer program mechanism embedded in a non-transitory computer readable storage medium such as CD-ROM, DVD, magnetic disk storage product, and the like.

## Claims

1. A composition comprising an Fc heterodimer protein in crystalline form, wherein:
said Fc heterodimer protein comprises the amino acid sequences set forth in (i) SEQ ID NOS:2 and 3 or (ii) SEQ ID NOS:4 and 3;
said crystal is in space group P2₁2₁2₁; and
said crystal has unit cell dimensions a = 49 ± 2 Å, b = 75 ± 2 Å, c = 149 ± 2 Å, α = β = γ = 90°.

2. The composition of claim 1, wherein said Fc heterodimer protein comprises:
(a) the amino acid sequences set forth in SEQ ID NOS:2 and 3 and has a three dimensional structure **characterized by** the atomic coordinates of (i) chains A and B of Figure 27 or (ii) chains a and b of Figure 27; or
(b) the amino acid sequences set forth in SEQ ID NOS:4 and 3 and has a three dimensional structure **characterized by** the atomic coordinates of (i) chains A and B of Figure 26 or (ii) chains a and b of Figure 26.

3. The composition of claim 1, wherein said Fc heterodimer protein comprises:
(a) the amino acid sequences set forth in SEQ ID NOS: 2 and 3 forming a CH3 domain interface, and wherein the Fc heterodimer protein provides complementary hydrophobic and electrostatic surfaces, created by residues 366, 392, 394 of SEQ ID NO: 2 and residues 351, 405, 407 of SEQ ID NO: 3, at the CH3 domain interface with opposite surface complementarity to corresponding wild type Fc interface surfaces; or
(b) the amino acid sequences set forth in SEQ ID NOS: 4 and 3 forming a CH3 domain interface, and wherein the Fc heterodimer protein provides complementary hydrophobic and electrostatic surfaces, created by residues 366, 392, 394 of SEQ ID NO: 4 and residues 351, 405, 407 of SEQ ID NO: 3, at the CH3 domain interface with distinct surface complementarity relative to the corresponding wild type Fc interface surfaces.

4. The composition of claim 3, wherein said CH3 domain interface is formed in two orientations and said crystalline form of said Fc heterodimer protein comprises a 50:50 mixture of the two orientations.

5. The composition of any one of claims 1 to 4, wherein said Fc heterodimer protein comprises a D399-K409 salt bridge.

6. A method of obtaining the composition of any one of claims 1 to 5, comprising the steps of:
(a) producing and purifying said Fc heterodimer protein; and
(b) subjecting the purified Fc heterodimer protein of step a) to conditions which promote crystallization, thereby obtaining the Fc heterodimer protein in crystalline form.

7. The method of claim 6, wherein the conditions which promote crystallization comprise mixing the purified Fc heterodimer protein with a mother liquor solution, wherein the mother liquor solution comprises
(a) between 2% and 10% (v/v) ethylene glycol, between 10% and 25% (w/v) polyethylene glycol having an average molecular weight of between 2000 Daltons and 10000 Daltons, and between 0.05 M and 0.40 M ammonium iodide, or
(b) 5% (v/v) ethylene glycol, 18% (w/v) polyethylene glycol having an average molecular weight of 3350 Daltons, and 0.15 M ammonium iodide.

8. The method of claim 7, wherein the purified Fc heterodimer protein is mixed with a first aliquot of the mother liquor solution and suspended over a second aliquot of the mother liquor in a hanging drop method, optionally wherein the purified Fc heterodimer protein is mixed with the first aliquot of the mother liquor solution in a 2:1 ratio.

9. The method of claim 8, wherein the purified Fc heterodimer protein is incubated at a temperature of between 15 °C and 25 °C after the mixing.

10. A crystallizable composition comprising a mixture of
(i) a solubilized Fc heterodimer protein comprising the amino acid sequence set forth in (a) SEQ ID NOS: 2 and 3 or (b) SEQ ID NOS: 3 and 4 and
(ii) a mother liquor solution, wherein the mother liquor solution comprises between 2% and 10% (v/v) ethylene glycol, between 10% and 25% (w/v) polyethylene glycol having an average molecular weight of between 2000 Daltons and 10000 Daltons, and between 0.05 M and 0.40 M ammonium iodide.

11. The crystallizable composition of claim 10, wherein the mother liquor solution comprises 5% (v/v) ethylene glycol, 18% (w/v) polyethylene glycol having an average molecular weight of 3350 Daltons, and 0.15 M ammonium iodide.

12. A method of identifying a mutation which promotes heterodimeric Fc chain pair formation, the method comprising:
performing structure based modeling, using a suitably programmed computer, to identify a candidate mutation to an Fc chain using a three-dimensional atomic crystal structure of an Fc heterodimer protein which is defined by the atomic coordinates of any combination of chains a, b, A, and B of Figures 26 or 27 determined from an X-ray diffraction quality crystal of the Fc heterodimer protein, wherein the Fc heterodimer protein comprises the amino acid sequences as set forth in (i) SEQ ID NOS: 2 and 3 or (ii) SEQ ID NOS: 4 and 3, and said X-ray diffraction quality crystal is in an orthorhombic space group.

13. The method of claim 12, wherein said orthorhombic space group is P2₁2₁2₁ and has unit cell dimensions a = 49 ± 2 Å, b = 75 ± 2 Å, c = 149 ± 2 Å, α = β = γ = 90°.

14. The method of claim 12 or 13, wherein the structure based modeling comprises:
(a) identifying a plurality of residues on the three-dimensional structure that influence heterodimeric Fc chain pair formation;
(b) modeling a plurality of three-dimensional Fc structures using the three-dimensional atomic crystal structure as a template, wherein each three-dimensional Fc structure in the plurality of three-dimensional Fc structures includes mutations to one or more of the residues in the plurality of residues;
(c) comparing each three-dimensional Fc structure in the plurality of three-dimensional Fc structures to the three-dimensional atomic crystal structure; and
(d) selecting one of the three-dimensional Fc structures in the plurality of three-dimensional Fc structures based on the comparing (c).

15. The method of claim 14, wherein the comparing (c) compares a calculated thermodynamic property of the three-dimensional atomic crystal structure to a calculated thermodynamic property of a three-dimensional Fc structure in the plurality of three-dimensional Fc structures, optionally wherein the thermodynamic property is one or more of entropy, average energy, average enthalpy, free energy and/or heat capacity.

16. The method of claim 14, wherein the comparing (c) compares a physical property of the three-dimensional atomic crystal structure to a calculated thermodynamic property of a three-dimensional Fc structure in the plurality of three-dimensional Fc structures, wherein the physical property is selected from the group consisting of (i) one or more electrostatic interactions, (ii) one or more polar interactions, (iii) one or more hydrogen-bond interactions, (iv) a comparison of buried versus accessible surface area, (v) accessible surface area, (vi) one or more hydrophobic interactions, and (vii) presence or absence of one or more buried water molecules.

## Patentansprüche

1. Eine Zusammensetzung, umfassend ein heterodimeres Fc-Protein in kristalliner Form, wobei:
jenes heterodimere Fc-Protein die in (i) SEQ ID NOS: 2 und 3 oder (ii) SEQ ID NOS: 4 und 3 dargelegten Aminosäuresequenzen umfasst;
jener Kristall in der Raumgruppe P2₁2₁2₁ vorliegt; und
jener Kristall die Einheitszellengrößen a = 49 ± 2 Å, b = 75 ± 2 Å, c = 149 ± 2 Å, α = β = γ = 90° aufweist.

2. Die Zusammensetzung nach Anspruch 1, wobei jenes heterodimere Fc-Protein umfasst:
(a) die in SEQ ID NOS: 2 und 3 dargelegten Aminosäuresequenzen und eine dreidimensionale Struktur aufweist, die durch die Atomkoordinaten von (i) Ketten A und B von Figur 27 oder (ii) Ketten a und b von Figur 27 gekennzeichnet ist; oder
(b) die in SEQ ID NOS: 4 und 3 dargelegten Aminosäuresequenzen und eine dreidimensionale Struktur aufweist, die durch die Atomkoordinaten von (i) Ketten A und B von Figur 26 oder (ii) Ketten a und b von Figur 26 gekennzeichnet ist.

3. Die Zusammensetzung nach Anspruch 1, wobei jenes heterodimere Fc-Protein umfasst:
(a) die in SEQ ID NOS: 2 und 3 dargelegten Aminosäuresequenzen, die eine CH3-Domänen-Grenzfläche bilden, und wobei das heterodimere Fc-Protein komplementäre hydrophobe und elektrostatische Oberflächen an der CH3-Domänen-Grenzfläche mit gegensätzlicher Oberflächenkomplementarität zur entsprechenden Grenzflächenoberfläche des Wildtyp-Fc bereitstellt, die durch die Reste 366, 392, 394 von SEQ ID NO: 2 und die Reste 351, 405, 407 von SEQ ID NO:3 erzeugt werden; oder
(b) die in SEQ ID NOS: 4 und 3 dargelegten Aminosäuresequenzen, die eine CH3-Domänen-Grenzfläche bilden, und wobei das heterodimere Fc-Protein komplementäre hydrophobe und elektrostatische Oberflächen an der CH3-Domänen-Grenzfläche mit unterschiedlicher Oberflächenkomplementarität relativ zur entsprechenden Grenzflächenoberfläche des Wildtyp-Fc bereitstellt, die durch die Reste 366, 392, 394 von SEQ ID NO: 4 und die Reste 351, 405, 407 von SEQ ID NO:3 erzeugt werden.

4. Die Zusammensetzung nach Anspruch 3, wobei die CH3-Domänen-Grenzfläche in zwei Orientierungen gebildet wird und die kristalline Form des heterodimeren Fc-Proteins eine 50:50-Mischung der beiden Orientierungen umfasst.

5. Die Zusammensetzung nach einem der Ansprüche 1 bis 4, wobei das heterodimere Fc-Protein eine D399-K409-Salzbrücke umfasst.

6. Ein Verfahren zur Herstellung der Zusammensetzung nach einem der Ansprüche 1 bis 5, umfassend die Schritte:
(a) Herstellen und Reinigen des heterodimeren Fc-Proteins; und
(b) das gereinigte heterodimere Fc-Protein aus Schritt a) Bedingungen aussetzen, die die Kristallisation fördern, wodurch das heterodimere Fc-Protein in kristalliner Form erhalten wird.

7. Das Verfahren nach Anspruch 6, wobei die Bedingungen, die die Kristallisation fördern, das Vermischen des gereinigten heterodimeren Fc-Proteins mit einer Mutterlaugenlösung umfassen, wobei die Mutterlaugenlösung umfasst
(a) zwischen 2% und 10% (v/v) Ethylenglykol, zwischen 10% und 25% (w/v) Polyethylenglykol mit einem mittleren Molekulargewicht zwischen 2000 Dalton und 10000 Dalton und zwischen 0,05 M und 0,40 M Ammoniumjodid, oder
(b) 5% (v/v) Ethylenglykol, 18% (w/v) Polyethylenglykol mit einem mittleren Molekulargewicht von 3350 Dalton und 0,15 M Ammoniumjodid.

8. Das Verfahren nach Anspruch 7, wobei das gereinigte heterodimere Fc-Protein mit einem ersten Aliquot der Mutterlaugenlösung vermischt und über einem zweiten Aliquot der Mutterlauge in einem "Hanging-Drop"-Verfahren suspendiert wird, wobei wahlweise das gereinigte heterodimere Fc-Protein mit dem ersten Aliquot der Mutterlaugenlösung im 2:1-Verhältnis vermischt wird.

9. Das Verfahren nach Anspruch 8, wobei das gereinigte heterodimere Fc-Protein bei einer Temperatur zwischen 15 °C und 25 °C nach dem Vermischen inkubiert wird.

10. Eine kristallisierbare Zusammensetzung, umfassend eine Mischung aus
(i) einem solubilisierten heterodimeren Fc-Protein, umfassend die in (a) SEQ ID NO: 2 und 3 oder (b) SEQ ID NO: 3 und 4 dargelegte Aminosäuresequenz und
(ii) eine Mutterlaugenlösung, wobei die Mutterlaugenlösung zwischen 2% und 10% (v/v) Ethylenglykol, zwischen 10% und 25% (w/v) Polyethylenglykol mit einem mittleren Molekulargewicht zwischen 2000 Dalton und 10000 Dalton und zwischen 0,05 M und 0,40 M Ammoniumjodid umfasst.

11. Die kristallisierbare Zusammensetzung nach Anspruch 10, wobei die Mutterlaugenlösung 5% (v/v) Ethylenglykol, 18% (w/v) Polyethylenglykol mit einem mittleren Molekulargewicht von 3350 Dalton und 0,15 M Ammoniumjodid umfasst.

12. Ein Verfahren zur Identifizierung einer Mutation, die eine heterodimere Fc-Kettenpaarbildung fördert, wobei das Verfahren umfasst:
Durchführen einer strukturbasierten Modellierung unter Verwendung eines geeignet programmierten Computers, um eine Kandidatenmutation für eine Fc-Kette unter Verwendung einer dreidimensionalen atomaren Kristallstruktur eines heterodimeren Fc-Proteins zu identifizieren, das durch die Atomkoordinaten einer beliebigen Kombination von Ketten a, b, A und B der Fig. 26 oder 27 definiert ist, die von einem Röntgenbeugungsqualitätskristall des heterodimeren Fc-Proteins bestimmt sind, wobei das heterodimere Fc-Protein die Aminosäuresequenzen umfasst, wie in (i) SEQ ID NO: 2 und 3 oder (ii) SEQ ID NO: 4 und 3 dargelegt, und der Röntgenbeugungsqualitätskristall in einer orthorhombischen Raumgruppe vorliegt.

13. Das Verfahren von Anspruch 12, wobei jene orthorhombische Raumgruppe P2₁2₁2₁ ist und die Einheitszellengrößen a = 49 ± 2 Å, b = 75 ± 2 Å, c = 149 ± 2 Å, α = β = γ = 90° aufweist.

14. Das Verfahren von Anspruch 12 oder 13, wobei die strukturbasierte Modellierung umfasst:
(a) Identifizieren einer Vielzahl von Resten auf der dreidimensionalen Struktur, die die heterodimere Fc-Kettenpaarbildung beeinflussen;
(b) Modellieren einer Vielzahl von dreidimensionalen Fc-Strukturen unter Verwendung der dreidimensionalen atomaren Kristallstruktur als eine Matrize, wobei jede dreidimensionale Fc-Struktur in der Vielzahl von dreidimensionalen Fc-Strukturen Mutationen zu einem oder mehreren der Reste in der Vielzahl von Resten umfasst;
(c) Vergleichen jeder dreidimensionalen Fc-Struktur in der Vielzahl von dreidimensionalen Fc-Strukturen mit der dreidimensionalen atomaren Kristallstruktur; und
(d) Auswählen einer der dreidimensionalen Fc-Strukturen in der Vielzahl von dreidimensionalen Fc-Strukturen basierend auf dem Vergleich (c).

15. Das Verfahren nach Anspruch 14, wobei der Vergleich (c) eine berechnete thermodynamische Eigenschaft der dreidimensionalen atomaren Kristallstruktur mit einer berechneten thermodynamischen Eigenschaft einer dreidimensionalen Fc-Struktur in der Vielzahl von dreidimensionalen Fc-Strukturen vergleicht, wobei wahlweise die thermodynamische Eigenschaft eines oder mehrere von Entropie, mittlere Energie, durchschnittliche Enthalpie, freie Energie und/oder Wärmekapazität ist.

16. Das Verfahren nach Anspruch 14, wobei der Vergleich (c) eine physikalische Eigenschaft der dreidimensionalen atomaren Kristallstruktur mit einer berechneten thermodynamischen Eigenschaft einer dreidimensionalen Fc-Struktur in der Vielzahl von dreidimensionalen Fc-Strukturen vergleicht, wobei die physikalische Eigenschaft ausgewählt aus der Gruppe bestehend aus (i) einer oder mehreren elektrostatischen Wechselwirkungen, (ii) einer oder mehreren polaren Wechselwirkungen, (iii) einer oder mehreren Wasserstoffbrücken-Wechselwirkungen, (iv) einem Vergleich der verborgenen versus zugänglichen Oberfläche, (v) zugänglicher Oberfläche, (vi) einer oder mehreren hydrophoben Wechselwirkungen und (vii) Anwesenheit oder Abwesenheit eines oder mehrerer verborgener Wassermoleküle.

## Revendications

1. Composition comprenant une protéine Fc hétérodimère sous forme cristalline, dans laquelle :
ladite protéine Fc hétérodimère comprend les séquences d'acides aminés représentées par (i) SEQ ID NO : 2 et 3 ou (ii) SEQ ID NO : 4 et 3 ;
ledit cristal est dans le groupe d'espace P2₁2₁2₁ ; et
ledit cristal a des dimensions de cellule unitaire a = 49 ± 2 Å, b = 75 ± 2 Å, c = 149 ± 2 Å, α = β = γ = 90°.

2. Composition selon la revendication 1, dans laquelle ladite protéine Fc hétérodimère comprend :
(a) les séquences d'acides aminés représentées par SEQ ID NO : 2 et 3 et a une structure tridimensionnelle **caractérisée par** les coordonnées atomiques de (i) les chaînes A et B de la figure 27 ou (ii) les chaînes a et b de la figure 27 ; ou
(b) les séquences d'acides aminés représentées par SEQ ID NO : 4 et 3 et a une structure tridimensionnelle **caractérisée par** les coordonnées atomiques de (i) les chaînes A et B de la figure 26 ou (ii) les chaînes a et b de la figure 26.

3. Composition selon la revendication 1, dans laquelle ladite protéine Fc hétérodimère comprend :
(a) les séquences d'acides aminés représentées par SEQ ID NO : 2 et 3 formant une interface de domaine CH3, et dans laquelle la protéine Fc hétérodimère fournit des surfaces hydrophobes et électrostatiques complémentaires, créées par les résidus 366, 392, 394 de SEQ ID NO : 2 et les résidus 351, 405, 407 de SEQ ID NO : 3, au niveau de l'interface de domaine CH3 avec une complémentarité de surfaces opposée par rapport aux surfaces correspondantes de l'interface de Fc de type sauvage ; ou
(b) les séquences d'acides aminés représentées par SEQ ID NO : 4 et 3 formant une interface du domaine CH3, et dans laquelle la protéine Fc hétérodimère fournit des surfaces hydrophobes et électrostatiques complémentaires, créées par les résidus 366, 392, 394 de SEQ ID NO : 4 et les résidus 351, 405, 407 de SEQ ID NO : 3, au niveau de l'interface de domaine CH3 avec une complémentarité de surfaces distincte par rapport aux surfaces correspondantes de l'interface de Fc de type sauvage.

4. Composition selon la revendication 3, dans laquelle ladite interface du domaine CH3 est formée dans deux orientations et ladite forme cristalline de ladite protéine Fc hétérodimère comprend un mélange 50/50 des deux orientations.

5. Composition selon l'une quelconque des revendications 1 à 4, dans laquelle ladite protéine Fc hétérodimère comprend un pont salin D399-K409.

6. Procédé d'obtention de la composition selon l'une quelconque des revendications 1 à 5, comprenant les étapes de :
(a) production et purification de ladite protéine Fc hétérodimère ; et
(b) soumission de la protéine Fc hétérodimère purifiée de l'étape a) à des conditions qui favorisent la cristallisation, obtenant de cette façon la protéine Fc hétérodimère sous forme cristalline.

7. Procédé selon la revendication 6, dans lequel les conditions qui favorisent la cristallisation comprennent le mélange de la protéine Fc hétérodimère purifiée avec une solution de liqueur mère, dans lequel la solution de liqueur mère comprend
(a) entre 2 % et 10 % (v/v) d'éthylène glycol, entre 10 % et 25 % (p/v) de polyéthylène glycol ayant une masse moléculaire moyenne située entre 2000 Daltons et 10 000 Daltons, et entre 0,05 M et 0,40 M d'iodure d'ammonium, ou
(b) 5 % (v/v) d'éthylène glycol, 18 % (p/v) de polyéthylène glycol ayant une masse moléculaire moyenne de 3350 Daltons, et 0,15 M d'iodure d'ammonium.

8. Procédé selon la revendication 7, dans lequel la protéine Fc hétérodimère purifiée est mélangée avec une première aliquote de la solution de liqueur mère et mise en suspension sur une deuxième aliquote de la liqueur mère dans un procédé de la goutte suspendue, éventuellement dans lequel la protéine Fc hétérodimère est mélangée avec la première aliquote de la solution de liqueur mère dans un rapport de 2/1.

9. Procédé selon la revendication 8, dans lequel la protéine Fc hétérodimère purifiée est incubée à une température située entre 15 °C et 25 °C après le mélange.

10. Composition cristallisable comprenant un mélange de
(i) une protéine Fc hétérodimère solubilisée comprenant la séquence d'acides aminés représentée par (a) SEQ ID NO : 2 et 3 ou (b) SEQ ID NO : 3 et 4 et
(ii) une solution de liqueur mère, dans laquelle la solution de liqueur mère comprend entre 2 % et 10 % (v/v) d'éthylène glycol, entre 10 % et 25 % (p/v) de polyéthylène glycol ayant une masse moléculaire moyenne située entre 2000 Daltons et 10 000 Dalton, et entre 0,05 M et 0,40 M d'iodure d'ammonium.

11. Composition cristallisable selon la revendication 10, dans laquelle la solution de liqueur mère comprend 5 % (v/v) d'éthylène glycol, 18 % (p/v) de polyéthylène glycol ayant une masse moléculaire moyenne de 3350 Daltons, et 0,15 M d'iodure d'ammonium.

12. Procédé d'identification d'une mutation qui favorise la formation d'une paire de chaînes Fc hétérodimère, le procédé comprenant :
la réalisation d'une modélisation basée sur la structure, en utilisant un ordinateur programmé de façon appropriée, pour identifier une mutation candidate dans une chaîne Fc en utilisant une structure cristalline atomique tridimensionnelle d'une protéine Fc hétérodimère qui est définie par les coordonnées atomiques d'une combinaison quelconque des chaînes a, b, A, et B des figures 26 ou 27 déterminées à partir d'un cristal de qualité diffraction des rayons X de la protéine Fc hétérodimère, dans lequel la protéine Fc hétérodimère comprend les séquences d'acides aminés représentées par (i) SEQ ID NO : 2 et 3 ou (ii) SEQ ID NO : 4 et 3, et ledit cristal de qualité diffraction des rayons X est dans un groupe d'espace orthorhombique.

13. Procédé selon la revendication 12, dans lequel ledit groupe d'espace orthorhombique est P2₁2₁2₁, et a des dimensions de cellule unitaire a = 49 ± 2 Å, b = 75 ± 2 Å, c = 149 ± 2 Å, α = β = γ = 90°.

14. Procédé selon la revendication 12 ou 13, dans lequel la modélisation basée sur la structure comprend :
(a) l'identification d'une pluralité de résidus sur la structure tridimensionnelle qui influencent la formation d'une paire de chaînes Fc hétérodimère ;
(b) la modélisation d'une pluralité de structures tridimensionnelles de Fc en utilisant la structure cristalline atomique tridimensionnelle comme matrice, dans lequel chaque structure tridimensionnelle de Fc dans la pluralité des structures tridimensionnelles de Fc comprend des mutations sur un ou plusieurs des résidus dans la pluralité de résidus ;
(c) la comparaison de chaque structure tridimensionnelle de Fc dans la pluralité de structures tridimensionnelles de Fc à la structure cristalline atomique tridimensionnelle ; et
(d) la sélection de l'une des structures tridimensionnelles de Fc dans la pluralité de structures tridimensionnelles de Fc basée sur la comparaison (c).

15. Procédé selon la revendication 14, dans lequel la comparaison (c) compare une propriété thermodynamique calculée de la structure cristalline atomique tridimensionnelle à une propriété thermodynamique calculée d'une structure tridimensionnelle de Fc dans la pluralité des structures tridimensionnelles de Fc, éventuellement dans lequel la propriété thermodynamique est une ou plusieurs de l'entropie, l'énergie moyenne, l'enthalpie moyenne, l'énergie libre et/ou la capacité thermique.

16. Procédé selon la revendication 14, dans lequel la comparaison (c) compare une propriété physique de la structure cristalline atomique tridimensionnelle à une propriété thermodynamique calculée d'une structure tridimensionnelle de Fc dans la pluralité des structures tridimensionnelles de Fc, dans lequel la propriété physique est choisie dans le groupe constitué de (i) une ou plusieurs interactions électrostatiques, (ii) une ou plusieurs interactions polaires, (iii) une ou plusieurs interactions de liaisons hydrogène, (iv) une comparaison de la surface spécifique enfouie contre accessible, (v) la surface spécifique accessible, (vi) une ou plusieurs interactions hydrophobes, et (vii) la présence ou l'absence d'une ou de plusieurs molécules d'eau enfouies.
